(19) Europäisches Patentamt
European Patent Office
Office européen des brevets

(11) EP 3 004 084 B1

(12) # EUROPÄISCHE PATENTSCHRIFT

(45) Veröffentlichungstag und Bekanntmachung des Hinweises auf die Patenterteilung:
**01.03.2017 Patentblatt 2017/09**

(21) Anmeldenummer: **14727487.2**

(22) Anmeldetag: **30.05.2014**

(51) Int Cl.:
***C07D 413/06*** $^{(2006.01)}$ ***C07D 417/06*** $^{(2006.01)}$
***C07D 487/04*** $^{(2006.01)}$ ***C07D 263/58*** $^{(2006.01)}$
***A61K 31/423*** $^{(2006.01)}$ ***A61P 7/02*** $^{(2006.01)}$

(86) Internationale Anmeldenummer:
**PCT/EP2014/061229**

(87) Internationale Veröffentlichungsnummer:
**WO 2014/195231 (11.12.2014 Gazette 2014/50)**

(54) **SUBSTITUIERTE BENZOXAZOLE**
SUBSTITUTED BENZOXAZOLES
BENZOXAZOLES SUBSTITUÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **03.06.2013 EP 13170208**

(43) Veröffentlichungstag der Anmeldung:
**13.04.2016 Patentblatt 2016/15**

(73) Patentinhaber: **Bayer Pharma Aktiengesellschaft
13353 Berlin (DE)**

(72) Erfinder:
- **ALLERHEILIGEN, Swen
45259 Essen (DE)**
- **BUCHMÜLLER, Anja
45259 Essen (DE)**
- **ENGEL, Karen
64380 Roßdorf (DE)**
- **GERDES, Christoph
51063 Köln (DE)**
- **GERICKE, Kersten Matthias
42115 Wuppertal (DE)**
- **GERISCH, Michael
Wuppertal 42329 (DE)**
- **HEITMEIER, Stefan
42489 Wülfrath (DE)**
- **HILLISCH, Alexander
42651 Solingen (DE)**
- **KINZEL, Tom
40489 Düsseldorf (DE)**
- **LIENAU, Philip
10967 Berlin (DE)**
- **RIEDL, Bernd
42329 Wuppertal (DE)**
- **RÖHRIG, Susanne
40724 Hilden (DE)**
- **SCHMIDT, Martina Victoria
51063 Köln (DE)**
- **STRAßBURGER, Julia
42115 Wuppertal (DE)**
- **TERSTEEGEN, Adrian
42111 Wuppertal (DE)**

(74) Vertreter: **BIP Patents
c/o Bayer Intellectual Property GmbH
Alfred-Nobel-Straße 10
40789 Monheim am Rhein (DE)**

(56) Entgegenhaltungen:
**WO-A1-2007/140982 US-A1- 2003 225 131**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 004 084 B1

## Beschreibung

Die Erfindung betrifft substituierte Benzoxazole und Verfahren zu ihrer Herstellung sowie ihre Verwendung zur Herstellung von Arzneimitteln zur Behandlung und/oder Prophylaxe von Krankheiten, insbesondere von Herz-Kreislauf-Erkrankungen, vorzugsweise von thrombotischen beziehungsweise thromboembolischen Erkrankungen.

Die Blutgerinnung ist ein Schutzmechanismus des Organismus, mit dessen Hilfe Defekte in der Gefäßwand rasch und zuverlässig "abgedichtet" werden können. So kann ein Blutverlust vermieden bzw. minimiert werden. Die Blutstillung nach Gefäßverletzung erfolgt im Wesentlichen durch das Gerinnungssystem, bei dem eine enzymatische Kaskade komplexer Reaktionen von Plasmaproteinen ausgelöst wird. Hierbei sind zahlreiche Blutgerinnungsfaktoren beteiligt, von denen jeder, sobald aktiviert, die jeweils nächste inaktive Vorstufe in ihre aktive Form überführt. Am Ende der Kaskade steht die Umwandlung des löslichen Fibrinogens in das unlösliche Fibrin, so dass es zu einem Blutgerinnsel kommt. Traditionell unterscheidet man bei der Blutgerinnung zwischen dem intrinsischen und dem extrinsischen System, die in einem abschließenden gemeinsamen Reaktionsweg münden. Hierbei kommen den Faktoren Xa und IIa (Thrombin) Schlüsselrollen zu: Faktor Xa bündelt die Signale der beiden Gerinnungswege, da er sowohl über Faktor VIIa/Tissue Factor (extrinsischer Weg) wie auch den Tenase Komplex (intrisischer Weg) durch Umsetzung von Faktor X entsteht. Die aktivierte Serinprotease Xa spaltet Prothrombin zu Thrombin, das über eine Reihe von Umsetzungen die Impulse aus der Kaskade auf den Gerinnungsstatus des Blutes überträgt: Thrombin spaltet direkt Fibrinogen zu Fibrin. Es aktiviert den zur Stabilisierung des Fibringerinnsels notwendigen Faktor XIII zu Faktor XIIIa. Darüber hinaus ist Thrombin ein potenter Auslöser der Thrombozytenaggregation (über PAR-1-Aktivierung), die ebenfalls einen erheblichen Beitrag bei der Hämostase leistet. Mittels Aktivierung von TAFI (Thrombin-aktivierbarer Fibrinolyse Inhibitor) zu TAFIa hemmt Thrombin im Komplex mit Thrombomodulin die Auflösung des Gerinnsels. Aktivierung der Faktoren V und VIII führt zur Potenzierung der Thrombinproduktion und damit wiederum zur Verstärkung der Gerinnungsreaktion.

Neben frei im Blut befindlichem Thrombin sind auch gebundene Formen bekannt: Während der Entstehung eines Fibringerinnsels werden Thrombin und Prothrombinase (Faktor Xa im Komplex) an das Fibringerüst gebunden. Diese Enzymmoleküle besitzen weiterhin Aktivität und können durch das körpereigene Anti-Thrombin III nicht inhibiert werden. Gerinnsel besitzen also auf diese Weise ein generelles prokoagulantes Potenzial.

Zusätzlich ist Thrombin insbesondere über die Aktivierung von PAR-1-Rezeptoren auf Endothelzellen auch an inflammatorischen Vorgängen beteiligt, die in Interaktion mit dem Koagulationssystem beide Prozesse beschleunigt.

Eine unkontrollierte Aktivierung des Gerinnungssystems oder eine defekte Hemmung der Aktivierungsprozesse kann die Bildung von lokalen Thrombosen oder Embolien in Gefäßen (Arterien, Venen, Lymphgefäßen) oder Herzhöhlen bewirken. Darüber hinaus kann eine systemische Hyperkoagulabilität zur systemweiten Bildung von Thromben und schließlich zu einer Verbrauchskoagulopathie im Rahmen einer disseminierten intravasalen Gerinnung führen. Thromboembolische Komplikationen treten ferner auf bei mikroangiopathischen hämolytischen Anämien, extrakorporalen Blutkreisläufen, wie Hämodialyse, sowie Herzklappenprothesen und Stents.

Im Verlauf vieler Herzkreislauf- und Stoffwechselerkrankungen kommt es infolge systemischer Faktoren, wie z.B. Hyperlipidämie, Diabetes oder Rauchen, infolge von Blutflussveränderungen mit Stase, wie z.B. beim Vorhofflimmern, oder infolge pathologischer Gefäßwandveränderungen, z.B. endothelialer Dysfunktionen oder Atherosklerose, zu einer erhöhten Neigung von Gerinnungs- und Thrombozytenaktivierung, die über die Bildung fibrin- und plättchenreicher Thromben zu thromboembolischen Erkrankungen und thrombotischen Komplikationen mit lebensbedrohlichen Zuständen führen kann. Thromboembolische Erkrankungen gehören daher nach wie vor zu den häufigsten Ursachen von Morbidität und Mortalität in den meisten industrialisierten Ländern [Heart Disease: A Textbook of Cardiovascular Medicine, Eugene Braunwald, 5. Auflage, 1997, W.B. Saunders Company, Philadelphia].

Die aus dem Stand der Technik bekannten Antikoagulantien, d.h. Stoffe zur Hemmung oder Verhinderung der Blutgerinnung, weisen verschiedene Nachteile auf. In der Therapie und Prophylaxe von thromboembolischen Erkrankungen findet zum einen Heparin Verwendung, das parenteral oder subkutan appliziert wird. Aufgrund günstigerer pharmakokinetischer Eigenschaften wird zwar heutzutage zunehmend niedermolekulares Heparin bevorzugt; allerdings können auch hierdurch die im Folgenden geschilderten bekannten Nachteile nicht vermieden werden, die bei der Therapierung mit Heparin bestehen. So ist Heparin oral unwirksam und besitzt nur eine vergleichsweise geringe Halbwertszeit. Darüber hinaus besteht ein hohes Blutungsrisiko, insbesondere können Hirnblutungen und Blutungen im Gastrointestinaltrakt auftreten, und es kann zu Thrombopenie, Alopecia medicomentosa oder Osteoporose kommen [Pschyrembel, Klinisches Wörterbuch, 257. Auflage, 1994, Walter de Gruyter Verlag, Seite 610, Stichwort "Heparin"; Römpp Lexikon Chemie, Version 1.5, 1998, Georg Thieme Verlag Stuttgart, Stichwort "Heparin"]. Niedermolekulare Heparine besitzen zwar eine geringere Wahrscheinlichkeit zur Ausbildung einer Heparin-induzierten Thrombocytopenie, sind aber auch nur subkutan applizierbar. Dies gilt auch für Fondaparinux, einem synthetisch hergestellten, selektiven Faktor Xa Inhibitor mit einer langen Halbwertszeit.

Eine zweite Klasse von Antikoagulantien stellen die Vitamin K-Antagonisten dar. Hierzu gehören beispielsweise 1,3-Indandione, vor allem aber Verbindungen wie Warfarin, Phenprocoumon, Dicumarol und andere Cumarin-Derivate, die unselektiv die Synthese verschiedener Produkte bestimmter Vitamin K-abhängiger Gerinnungsfaktoren in der Leber

hemmen. Durch den Wirkmechanismus bedingt, setzt die Wirkung nur sehr langsam ein (Latenzzeit bis zum Wirkeintritt 36 bis 48 Stunden). Die Verbindungen können zwar oral appliziert werden, aufgrund des hohen Blutungsrisikos und des engen therapeutischen Indexes ist aber eine aufwendige individuelle Einstellung und Beobachtung des Patienten notwendig [J. Hirsh, J. Dalen, D.R. Anderson et al., "Oral anticoagulants: Mechanism of action, clinical effectiveness, and optimal therapeutic range" Chest 2001, 119, 8S-21S; J. Ansell, J. Hirsh, J. Dalen et al., "Managing oral anticoagulant therapy" Chest 2001, 119, 22S-38S; P.S. Wells, A.M. Holbrook, N.R. Crowther et al., "Interactions of warfarin with drugs and food" Ann. Intern. Med. 1994, 121, 676-683]. Darüber hinaus sind weitere Nebenwirkungen wie gastrointestinale Störungen, Haarausfall und Hautnekrosen beschrieben.

Neuere Ansätze für orale Antikoagulantien befinden sich in verschiedenen Phasen der klinischen Erprobung bzw. im klinischen Einsatz, haben jedoch auch Nachteile gezeigt, wie z.B. hochvariable Bioverfügbarkeit, Leberschädigung und Blutungskomplikationen, insbesondere bei Patienten mit Nierenschädigung.

Bei antithrombotischen Arzneimitteln ist die therapeutische Breite wichtig: Der Abstand zwischen der therapeutisch wirksamen Dosis zur Gerinnungshemmung und der Dosis, bei der Blutungen auftreten können, sollte möglichst groß sein, so dass eine maximale therapeutische Wirksamkeit bei minimalem Risikoprofil erreicht wird.

Insbesondere unter therapeutischen Bedingungen mit schon vorhandenem Thromben kann es vorteilhaft sein, auch den im Thrombus vorhandenen Faktor IIa zu hemmen und damit einen schnellen Thrombusabbau zu begünstigen. In verschiedenen in-vitro und in-vivo Modellen mit beispielsweise Argatroban bzw. Hirudin als FIIa- Inhibitoren wurde der vorteilhafte Effekt der FIIa-Hemmung bei schon vorliegendem Thrombus allein bzw. in Anwesenheit von Gewebe-Plaminogenaktivator (tPA) herausgearbeitet.

Eine Aufgabe der vorliegenden Erfindung ist daher die Bereitstellung neuer Verbindungen als Thrombininhibitoren zur Behandlung von Herz-Kreislauf-Erkrankungen, insbesondere von thrombotischen beziehungsweise thromboembolischen Erkrankungen, bei Menschen und Tieren, die eine große therapeutische Bandbreite aufweisen und ein gutes pharmakokinetisches Verhalten zeigen.

WO 98/37075 beschreibt unter anderem Benzoxazol-Derivate mit einem Amidino-Benzylaminosubstituenten als Thrombin Inhibitoren. Amidino-substituierte Thrombin Inhibitoren haben eine kurze Halbwertszeit und eine geringe orale Bioverfügbarkeit. Die Verbindungen eignen sich als solches nur für die parenterale Anwendung und müssen bei oraler Gabe als Prodrugs eingesetzt werden (A. Casimiro-Garcia, D. A. Dudley, R. J. Heemstra, K. J. Filipski, C. F. Bigge, J. J. Edmunds, Expert Opin. Ther. Patents 2006, 16(2), 119-145).

WO 2007/140982 beschreibt die Verwendung von Benzoxazolen als Thrombin Inhibitoren.

EP-A 0 535 521 beschreibt die Verwendung von Benzoxazolen als Leukotrien Biosynthese Inhibitoren zur Behandlung von inflammatorischen Erkrankungen.

Gegenstand der Erfindung sind Verbindungen der Formel

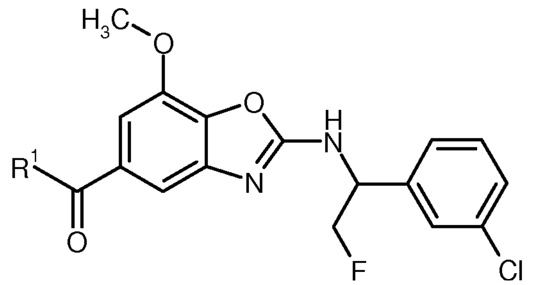 (I),

in welcher

$R^1$ für eine Gruppe der Formel

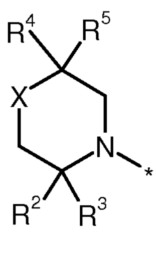 oder 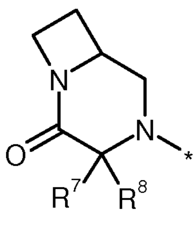 oder 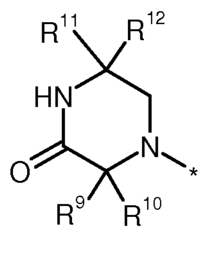 oder 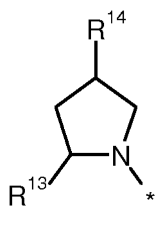

steht, wobei * die Anknüpfstelle an die Carbonylgruppe ist,

X für ein Sauerstoffatom, ein Schwefelatom oder CH-$R^6$ steht,

wobei

$R^6$ für Hydroxy steht,

$R^2$ für Wasserstoff, $C_1$-$C_6$-Alkyl, $C_3$-$C_6$-Cycloalkyl oder Phenyl steht, worin Alkyl und Cycloalkyl substituiert sein können mit einem Substituenten ausgewählt aus der Gruppe bestehend aus Hydroxy, Methoxy, Cyano, Hydroxycarbonyl, Aminocarbonyl, Methylsulfonyl, Difluormethoxy und Trifluormethoxy,
oder
worin Alkyl und Cycloalkyl substituiert sein können mit 1 bis 3 Substituenten Fluor,

$R^3$ für Wasserstoff oder $C_1$-$C_4$-Alkyl steht,

oder $R^2$ und $R^3$ zusammen mit dem Kohlenstoffatom an das sie gebunden sind einen Cyclopropyl-Ring, Cyclobutyl-Ring oder Cyclopentyl-Ring bilden,
worin der Cyclobutyl-Ring und Cyclopentyl-Ring substituiert sein können mit einem Substituenten Hydroxy,

$R^4$ für Wasserstoff oder $C_1$-$C_6$-Alkyl steht,
worin Alkyl substituiert sein kann mit einem Substituenten Hydroxy,

$R^5$ für $C_1$-$C_4$-Alkyl steht,

oder $R^4$ und $R^5$ zusammen mit dem Kohlenstoffatom an das sie gebunden sind einen Cyclopropyl-Ring, Cyclobutyl-Ring oder Cyclopentyl-Ring bilden,
worin der Cyclobutyl-Ring und Cyclopentyl-Ring substituiert sein können mit einem Substituenten Hydroxy,

$R^7$ für Wasserstoff oder $C_1$-$C_6$-Alkyl steht,
worin Alkyl substituiert sein kann mit einem Substituenten Hydroxy oder Cyano,
oder
worin Alkyl substituiert sein kann mit 1 bis 3 Substituenten Fluor,

$R^8$ für Wasserstoff steht,

$R^9$ für Wasserstoff oder $C_1$-$C_6$-Alkyl steht,
worin Alkyl substituiert sein kann mit einem Substituenten Hydroxy oder Cyano,
oder
worin Alkyl substituiert sein kann mit 1 bis 3 Substituenten Fluor,

$R^{10}$ für Wasserstoff steht,

$R^{11}$ für $C_1$-$C_4$-Alkyl steht,
worin Alkyl substituiert sein kann mit einem Substituenten Hydroxy,

$R^{12}$ für Wasserstoff oder $C_1$-$C_4$-Alkyl steht,

oder $R^{11}$ und $R^{12}$ zusammen mit dem Kohlenstoffatom an das sie gebunden sind einen Cyclopropyl-Ring, Cyclobutyl-Ring oder Cyclopentyl-Ring bilden,
worin der Cyclobutyl-Ring und Cyclopentyl-Ring substituiert sein können mit einem Substituenten Hydroxy,

$R^{13}$ für Hydroxymethyl oder Hydroxyethyl steht,

$R^{14}$ für Methoxy oder Ethoxy steht,

und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler beziehungsweise chiraler Phase.

Sofern die erfindungsgemäßen Verbindungen in tautomeren Formen vorkommen können, umfasst die vorliegenden Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie $^{2}H$ (Deuterium), $^{3}H$ (Tritium), $^{13}C$, $^{14}C$, $^{15}N$, $^{17}O$, $^{18}O$, $^{32}P$, $^{33}P$, $^{33}S$, $^{34}S$, $^{35}S$, $^{36}S$, $^{18}F$, $^{36}Cl$, $^{82}Br$, $^{123}I$, $^{124}I$, $^{129}I$ und $^{131}I$. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit $^{3}H$- oder $^{14}C$-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Offenbarung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Im Rahmen der vorliegenden Erfindung haben die Substituenten, soweit nicht anders spezifiziert, die folgende Bedeutung:

Alkyl steht für einen linearen oder verzweigten Alkylrest mit 1 bis 6 Kohlenstoffatomen, bevorzugt 1 bis 4 Kohlenstoffatomen, beispielhaft und vorzugsweise für Methyl, Ethyl, n-Propyl, iso-Propyl, n-Butyl, iso-Butyl, 1-Methylpropyl, tert-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.

Cycloalkyl steht für eine monocyclische Cycloalkylgruppe mit 3 bis 6 Kohlenstoffatomen, beispielhaft und vorzugsweise für Cycloalkyl seien genannt Cyclopropyl, Cyclobutyl, Cyclopentyl und Cyclohexyl.

In den Formeln der Gruppe, die für $R^1$ stehen kann, steht der Endpunkt der Linie, neben der jeweils ein * steht, nicht für ein Kohlenstoffatom beziehungsweise eine $CH_2$-Gruppe sondern ist Bestandteil der Bindung zu dem Atom, an das $R^1$ gebunden ist.

Bevorzugt sind Verbindungen der Formel (I), in welcher

$R^1$ für eine Gruppe der Formel

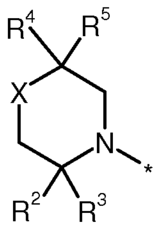 oder 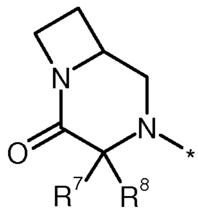 oder 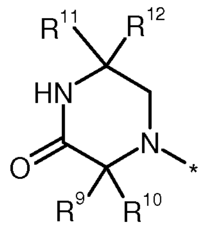 oder 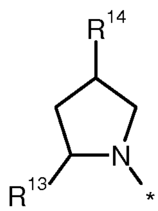

steht, wobei * die Anknüpfstelle an die Carbonylgruppe ist,

X für ein Sauerstoffatom oder CH-$R^6$ steht, wobei

$R^6$ für Hydroxy steht,

$R^2$ für Wasserstoff, $C_1$-$C_4$-Alkyl oder $C_3$-$C_6$-Cycloalkyl steht, worin Alkyl substituiert sein kann mit einem Substituenten Hydroxy oder Hydroxycarbonyl, und worin Cycloalkyl substituiert sein kann mit einem Substituenten Hydroxy,

$R^3$ für Wasserstoff oder $C_1$-$C_4$-Alkyl steht,

oder $R^2$ und $R^3$ zusammen mit dem Kohlenstoffatom an das sie gebunden sind einen Cyclobutyl-Ring bilden, worin der Cyclobutyl-Ring substituiert sein kann mit einem Substituenten Hydroxy,

$R^4$ für Wasserstoff oder $C_1$-$C_4$-Alkyl steht, worin Alkyl substituiert sein kann mit einem Substituenten Hydroxy,

$R^5$ für $C_1$-$C_4$-Alkyl steht,

$R^7$ für Wasserstoff oder $C_1$-$C_4$-Alkyl steht,

$R^8$ für Wasserstoff steht,

$R^9$ für Wasserstoff oder $C_1$-$C_4$-Alkyl steht, worin Alkyl substituiert sein kann mit einem Substituenten Hydroxy,

$R^{10}$ für Wasserstoff steht,

$R^{11}$ und $R^{12}$ zusammen mit dem Kohlenstoffatom an das sie gebunden sind einen Cyclopropyl-Ring bilden,

$R^{13}$ für Hydroxymethyl oder Hydroxyethyl steht,

$R^{14}$ für Methoxy oder Ethoxy steht,

und ihre Salze, ihre Solvate und die Solvate ihrer Salze.

Bevorzugt sind auch Verbindungen der Formel (I), in welcher

$R^1$ für eine Gruppe der Formel

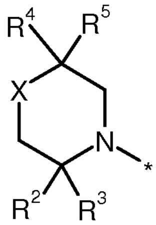 oder 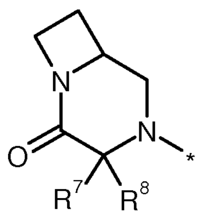 oder 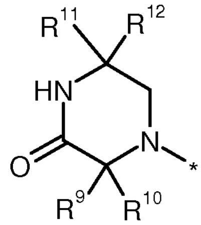 oder 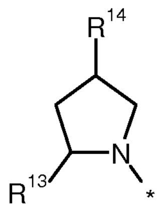

steht, wobei * die Anknüpfstelle an die Carbonylgruppe ist,

X für ein Sauerstoffatom steht,

$R^2$ für Methyl oder Ethyl steht, worin Methyl und Ethyl substituiert sind mit einem Substituenten Hydroxy,

$R^3$ für Wasserstoff steht,

oder $R^2$ und $R^3$ zusammen mit dem Kohlenstoffatom an das sie gebunden sind einen Cyclobutyl-Ring bilden, worin der Cyclobutyl-Ring substituiert ist mit einem Substituenten Hydroxy,

$R^4$ für Wasserstoff oder Methyl steht,

$R^5$ für Methyl steht,

$R^7$ für Wasserstoff oder Methyl steht,

$R^8$ für Wasserstoff steht,

$R^9$ für Methyl steht,

$R^{10}$ für Wasserstoff steht,

$R^{11}$ und $R^{12}$ zusammen mit dem Kohlenstoffatom an das sie gebunden sind einen Cyclopropyl-Ring bilden,

$R^{13}$ für Hydroxymethyl oder Hydroxyethyl steht,

$R^{14}$ für Ethoxy steht,

und ihre Salze, ihre Solvate und die Solvate ihrer Salze.

Bevorzugt sind auch Verbindungen der Formel (I), in welcher

$R^1$ für eine Gruppe der Formel

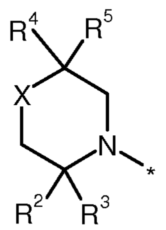

oder

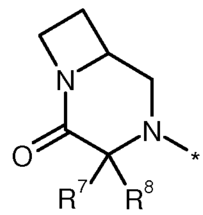

steht, wobei * die Anknüpfstelle an die Carbonylgruppe ist,

X für ein Sauerstoffatom, ein Schwefelatom oder CH-$R^6$ steht, wobei

$R^6$ für Hydroxy steht,

$R^2$ für Wasserstoff, $C_1$-$C_6$-Alkyl, $C_3$-$C_6$-Cycloalkyl oder Phenyl steht, worin Alkyl und Cycloalkyl substituiert sein können mit einem Substituenten ausgewählt aus der Gruppe bestehend aus Hydroxy, Methoxy, Cyano, Hydroxycarbonyl, Aminocarbonyl, Methylsulfonyl, Difluormethoxy und Trifluormethoxy, oder worin Alkyl und Cycloalkyl substituiert sein können mit 1 bis 3 Substituenten Fluor,

$R^3$ für Wasserstoff oder $C_1$-$C_4$-Alkyl steht,

oder $R^2$ und $R^3$ zusammen mit dem Kohlenstoffatom an das sie gebunden sind einen Cyclopropyl-Ring, Cyclobutyl-Ring oder Cyclopentyl-Ring bilden, worin der Cyclobutyl-Ring und Cyclopentyl-Ring substituiert sein können mit einem Substituenten Hydroxy,

$R^4$ für Wasserstoff oder $C_1$-$C_6$-Alkyl steht, worin Alkyl substituiert sein kann mit einem Substituenten Hydroxy,

$R^5$ für $C_1$-$C_4$-Alkyl steht,

oder $R^4$ und $R^5$ zusammen mit dem Kohlenstoffatom an das sie gebunden sind einen Cyclopropyl-Ring, Cyclobutyl-Ring oder Cyclopentyl-Ring bilden, worin der Cyclobutyl-Ring und Cyclopentyl-Ring substituiert sein können mit einem Substituenten Hydroxy,

$R^7$ für Wasserstoff oder $C_1$-$C_6$-Alkyl steht, worin Alkyl substituiert sein kann mit einem Substituenten Hydroxy oder Cyano,

oder
worin Alkyl substituiert sein kann mit 1 bis 3 Substituenten Fluor,

$R^8$ für Wasserstoff steht,

und ihre Salze, ihre Solvate und die Solvate ihrer Salze.

Bevorzugt sind auch Verbindungen der Formel (I), in welcher

$R^1$ für eine Gruppe der Formel

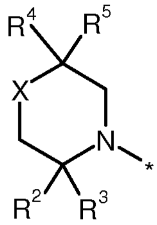

oder

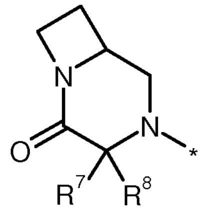

steht, wobei * die Anknüpfstelle an die Carbonylgruppe ist,

X für ein Sauerstoffatom oder $CH-R^6$ steht,
wobei

$R^6$ für Hydroxy steht,

$R^2$ für Wasserstoff, $C_1$-$C_4$-Alkyl oder $C_3$-$C_6$-Cycloalkyl steht,
worin Alkyl substituiert sein kann mit einem Substituenten Hydroxy oder Hydroxycarbonyl, und
worin Cycloalkyl substituiert sein kann mit einem Substituenten Hydroxy,

$R^3$ für Wasserstoff oder $C_1$-$C_4$-Alkyl steht,

oder $R^2$ und $R^3$ zusammen mit dem Kohlenstoffatom an das sie gebunden sind einen Cyclobutyl-Ring bilden,
worin der Cyclobutyl-Ring substituiert sein kann mit einem Substituenten Hydroxy,

$R^4$ für Wasserstoff oder $C_1$-$C_4$-Alkyl steht,
worin Alkyl substituiert sein kann mit einem Substituenten Hydroxy,

$R^5$ für $C_1$-$C_4$-Alkyl steht,

$R^7$ für Wasserstoff oder $C_1$-$C_4$-Alkyl steht,

$R^8$ für Wasserstoff steht,

und ihre Salze, ihre Solvate und die Solvate ihrer Salze.

Bevorzugt sind auch Verbindungen der Formel (I), in welcher

$R^1$ für eine Gruppe der Formel

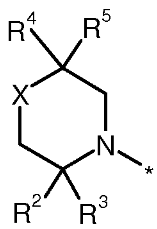

oder

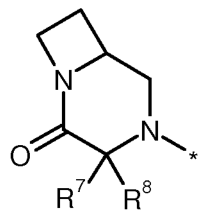

steht, wobei * die Anknüpfstelle an die Carbonylgruppe ist,

X für ein Sauerstoffatom oder $CH-R^6$ steht,
wobei

$R^6$ für Hydroxy steht,

$R^2$ für Wasserstoff, Methyl, Ethyl oder Cyclobutyl steht,
worin Methyl und Ethyl substituiert sein können mit einem Substituenten Hydroxy oder Hydroxycarbonyl,
und
worin Cyclobutyl substituiert sein kann mit einem Substituenten Hydroxy,
$R^3$ für Wasserstoff oder Methyl steht,
oder $R^2$ und $R^3$ zusammen mit dem Kohlenstoffatom an das sie gebunden sind einen Cyclobutyl-Ring bilden,
worin der Cyclobutyl-Ring substituiert sein kann mit einem Substituenten Hydroxy,
$R^4$ für Wasserstoff, Methyl, Ethyl oder Propyl steht, worin Methyl, Ethyl und Propyl substituiert sein können mit einem Substituenten Hydroxy,
$R^5$ für Methyl steht,
$R^7$ für Wasserstoff, Methyl oder Ethyl steht,
$R^8$ für Wasserstoff steht,

und ihre Salze, ihre Solvate und die Solvate ihrer Salze.

Bevorzugt sind auch Verbindungen der Formel (I), in welcher

$R^1$ für eine Gruppe der Formel

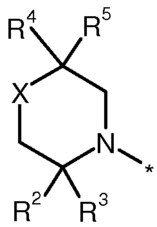
oder
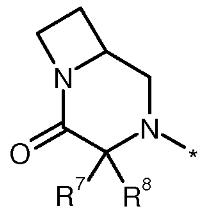

steht, wobei * die Anknüpfstelle an die Carbonylgruppe ist,
X für ein Sauerstoffatom steht,
$R^2$ für Methyl oder Ethyl steht,
worin Methyl und Ethyl substituiert sind mit einem Substituenten Hydroxy,
$R^3$ für Wasserstoff steht,
oder $R^2$ und $R^3$ zusammen mit dem Kohlenstoffatom an das sie gebunden sind einen Cyclobutyl-Ring bilden,
worin der Cyclobutyl-Ring substituiert ist mit einem Substituenten Hydroxy,
$R^4$ für Wasserstoff oder Methyl steht,
$R^5$ für Methyl steht,
$R^7$ für Wasserstoff oder Methyl steht,
$R^8$ für Wasserstoff steht,

und ihre Salze, ihre Solvate und die Solvate ihrer Salze.

Bevorzugt sind auch Verbindungen der Formel (I), in welcher

$R^1$ für eine Gruppe der Formel

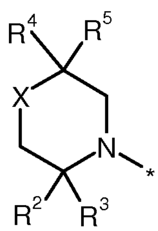

steht, wobei * die Anknüpfstelle an die Carbonylgruppe ist,
X für ein Sauerstoffatom, ein Schwefelatom oder CH-$R^6$ steht, wobei

$R^6$ für Hydroxy steht,

$R^2$ für Wasserstoff, $C_1$-$C_6$-Alkyl, $C_3$-$C_6$-Cycloalkyl oder Phenyl steht, worin Alkyl und Cycloalkyl substituiert sein können mit einem Substituenten ausgewählt aus der Gruppe bestehend aus Hydroxy, Methoxy, Cyano, Hydroxycarbonyl, Aminocarbonyl, Methylsulfonyl, Difluormethoxy und Trifluormethoxy,
oder
worin Alkyl und Cycloalkyl substituiert sein können mit 1 bis 3 Substituenten Fluor,

$R^3$ für Wasserstoff oder $C_1$-$C_4$-Alkyl steht,

oder $R^2$ und $R^3$ zusammen mit dem Kohlenstoffatom an das sie gebunden sind einen Cyclopropyl-Ring, Cyclobutyl-Ring oder Cyclopentyl-Ring bilden,
worin der Cyclobutyl-Ring und Cyclopentyl-Ring substituiert sein können mit einem Substituenten Hydroxy,

$R^4$ für Wasserstoff oder $C_1$-$C_6$-Alkyl steht,
worin Alkyl substituiert sein kann mit einem Substituenten Hydroxy,

$R^5$ für $C_1$-$C_4$-Alkyl steht,

oder $R^4$ und $R^5$ zusammen mit dem Kohlenstoffatom an das sie gebunden sind einen Cyclopropyl-Ring, Cyclobutyl-Ring oder Cyclopentyl-Ring bilden,
worin der Cyclobutyl-Ring und Cyclopentyl-Ring substituiert sein können mit einem Substituenten Hydroxy,

und ihre Salze, ihre Solvate und die Solvate ihrer Salze.

Bevorzugt sind auch Verbindungen der Formel (I), in welcher

$R^1$ für eine Gruppe der Formel

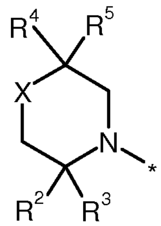

steht, wobei * die Anknüpfstelle an die Carbonylgruppe ist,

X für ein Sauerstoffatom oder CH-$R^6$ steht,
wobei

$R^6$ für Hydroxy steht,

$R^2$ für Wasserstoff, Methyl, Ethyl oder Cyclobutyl steht,
worin Methyl und Ethyl substituiert sein können mit einem Substituenten Hydroxy oder Hydroxycarbonyl,
und
worin Cyclobutyl substituiert sein kann mit einem Substituenten Hydroxy,

$R^3$ für Wasserstoff oder Methyl steht,

oder $R^2$ und $R^3$ zusammen mit dem Kohlenstoffatom an das sie gebunden sind einen Cyclobutyl-Ring bilden,
worin der Cyclobutyl-Ring substituiert sein kann mit einem Substituenten Hydroxy,

$R^4$ für Wasserstoff, Methyl, Ethyl oder Propyl steht,
worin Methyl, Ethyl und Propyl substituiert sein können mit einem Substituenten Hydroxy,

$R^5$ für Methyl steht,

und ihre Salze, ihre Solvate und die Solvate ihrer Salze.

Bevorzugt sind auch Verbindungen der Formel (I), in welcher

$R^1$ für eine Gruppe der Formel

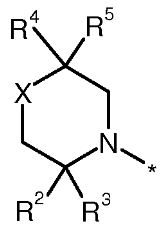

steht, wobei * die Anknüpfstelle an die Carbonylgruppe ist,

X für ein Sauerstoffatom steht,

$R^2$ für Methyl, Ethyl oder Cyclobutyl steht,
worin Methyl und Ethyl substituiert sind mit einem Substituenten Hydroxy,
und
worin Cyclobutyl substituiert ist mit einem Substituenten Hydroxy,

$R^3$ für Wasserstoff steht,

$R^4$ für Wasserstoff oder Methyl steht,

und $R^5$ für Methyl steht,

oder $R^2$ für Methyl steht,

$R^3$ für Wasserstoff oder Methyl steht,

$R^4$ für Methyl, Ethyl oder Propyl steht,
worin Methyl, Ethyl und Propyl substituiert sind mit einem Substituenten Hydroxy,

und $R^5$ für Methyl steht,

oder $R^2$ und $R^3$ zusammen mit dem Kohlenstoffatom an das sie gebunden sind einen Cyclobutyl-Ring bilden,
worin der Cyclobutyl-Ring substituiert ist mit einem Substituenten Hydroxy,

$R^4$ für Wasserstoff oder Methyl steht,

und $R^5$ für Methyl steht,

und ihre Salze, ihre Solvate und die Solvate ihrer Salze.

Bevorzugt sind auch Verbindungen der Formel (I), in welcher

$R^1$ für eine Gruppe der Formel

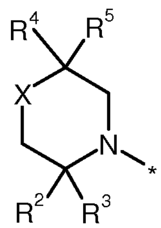

steht, wobei * die Anknüpfstelle an die Carbonylgruppe ist,

X für ein Sauerstoffatom steht,

$R^2$ für Methyl oder Ethyl steht,
worin Methyl und Ethyl substituiert sind mit einem Substituenten Hydroxy,

$R^3$ für Wasserstoff steht,

oder $R^2$ und $R^3$ zusammen mit dem Kohlenstoffatom an das sie gebunden sind einen Cyclobutyl-Ring bilden,
worin der Cyclobutyl-Ring substituiert ist mit einem Substituenten Hydroxy,

$R^4$ für Wasserstoff oder Methyl steht,

$R^5$ für Methyl steht,

und ihre Salze, ihre Solvate und die Solvate ihrer Salze.

Bevorzugt sind auch Verbindungen der Formel (I), in welcher

$R^1$ für eine Gruppe der Formel

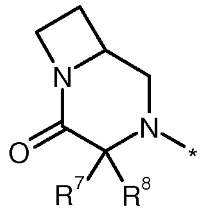

steht, wobei * die Anknüpfstelle an die Carbonylgruppe ist,
$R^7$ für Wasserstoff oder $C_1$-$C_6$-Alkyl steht,
worin Alkyl substituiert sein kann mit einem Substituenten Hydroxy oder Cyano,
oder
worin Alkyl substituiert sein kann mit 1 bis 3 Substituenten Fluor,
$R^8$ für Wasserstoff steht,

und ihre Salze, ihre Solvate und die Solvate ihrer Salze.

Bevorzugt sind auch Verbindungen der Formel (I), in welcher

$R^1$ für eine Gruppe der Formel

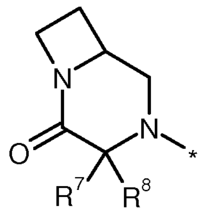

steht, wobei * die Anknüpfstelle an die Carbonylgruppe ist,
$R^7$ für Wasserstoff, Methyl oder Ethyl steht,
$R^8$ für Wasserstoff steht,

und ihre Salze, ihre Solvate und die Solvate ihrer Salze.

Bevorzugt sind auch Verbindungen der Formel (I), in welcher

$R^1$ für eine Gruppe der Formel

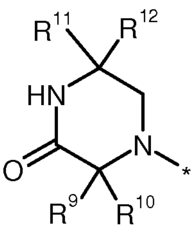

steht, wobei * die Anknüpfstelle an die Carbonylgruppe ist,
$R^9$ für Wasserstoff oder $C_1$-$C_6$-Alkyl steht,
worin Alkyl substituiert sein kann mit einem Substituenten Hydroxy oder Cyano,
oder
worin Alkyl substituiert sein kann mit 1 bis 3 Substituenten Fluor,
$R^{10}$ für Wasserstoff steht,
$R^{10}$ für $C_1$-$C_4$-Alkyl steht,
worin Alkyl substituiert sein kann mit einem Substituenten Hydroxy,
$R^{12}$ für Wasserstoff oder $C_1$-$C_4$-Alkyl steht,
oder $R^{11}$ und $R^{12}$ zusammen mit dem Kohlenstoffatom an das sie gebunden sind einen Cyclopropyl-Ring, Cyclobutyl-Ring oder Cyclopentyl-Ring bilden,
worin der Cyclobutyl-Ring und Cyclopentyl-Ring substituiert sein können mit einem Substituenten

Hydroxy,

und ihre Salze, ihre Solvate und die Solvate ihrer Salze.

Bevorzugt sind auch Verbindungen der Formel (I), in welcher

$R^1$ für eine Gruppe der Formel

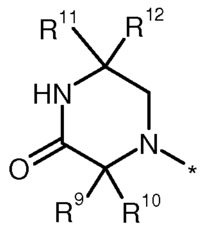

steht, wobei * die Anknüpfstelle an die Carbonylgruppe ist,
$R^9$ für Methyl steht,
$R^{10}$ für Wasserstoff steht,
$R^{11}$ und $R^{12}$ zusammen mit dem Kohlenstoffatom an das sie gebunden sind einen Cyclopropyl-Ring bilden,

und ihre Salze, ihre Solvate und die Solvate ihrer Salze.

Bevorzugt sind auch Verbindungen der Formel (I), in welcher

$R^1$ für eine Gruppe der Formel

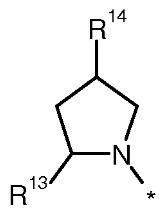

steht, wobei * die Anknüpfstelle an die Carbonylgruppe ist,
$R^{13}$ für Hydroxymethyl oder Hydroxyethyl steht,
$R^{14}$ für Methoxy oder Ethoxy steht,

und ihre Salze, ihre Solvate und die Solvate ihrer Salze.

Bevorzugt sind auch Verbindungen der Formel (I), in welcher

$R^1$ für eine Gruppe der Formel

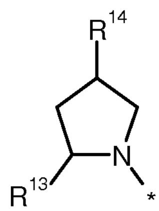

steht, wobei * die Anknüpfstelle an die Carbonylgruppe ist,
$R^{13}$ für Hydroxymethyl oder Hydroxyethyl steht,
$R^{14}$ für Ethoxy steht,

und ihre Salze, ihre Solvate und die Solvate ihrer Salze.

Bevorzugt sind auch Verbindungen, welche die Formel (Ia) aufweisen

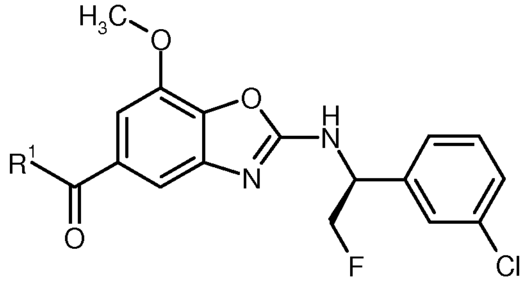

(Ia)

worin $R^1$ wie oben definiert ist.

Bevorzugt ist auch

(2-{[1-(3-Chlorphenyl)-2-fluorethyl]amino}-7-methoxy-1,3-benzoxazol-5-yl)[(5*S*)-5-(2-hydroxyethyl)-2-methylmorpholin-4-yl]methanon [enantiomerenreines Isomer 2]
oder

(2-{[1-(3-Chlorphenyl)-2-fluorethyl]amino}-7-methoxy-1,3-benzoxazol-5-yl)[5-(3-hydroxycyclobutyl)-2-methylmorpholin-4-yl]methanon [enantiomerenreines Isomer 4]
oder

(2-{[1-(3-Chlorphenyl)-2-fluorethyl]amino}-7-methoxy-1,3-benzoxazol-5-yl)(*cis*-2-hydroxy-7-methyl-8-oxa-5-azaspiro[3.5]non-5-yl)methanon [enantiomerenreines Isomer 2]
oder

4-[(2-{[1-(3-Chlorphenyl)-2-fluorethyl]amino}-7-methoxy-1,3-benzoxazol-5-yl)carbonyl]-3-methyl-1,4-diazabicyclo[4.2.0]octan-2-on [enantiomerenreines Isomer]
oder

{(3S)-4-[(2-{[1-(3-Chlorphenyl)-2-fluorethyl]amino}-7-methoxy-1,3-benzoxazol-5-yl)carbonyl]-6-methylmorpholin-3-yl}essigsäure [enantiomerenreines Isomer]
oder

(2-{[1-(3-Chlorphenyl)-2-fluorethyl]amino}-7-methoxy-1,3-benzoxazol-5-yl)[(5*R*)-5-(2-hydroxyethyl)-2,2-dimethylmorpholin-4-yl]methanon [Diastereomerengemisch, 2 Isomere]

oder eines des Salze, der Solvate oder der Solvate der Salze dieser Verbindungen.

Besonders bevorzugt ist (2-{[1-(3-Chlorphenyl)-2-fluorethyl]amino}-7-methoxy-1,3-benzoxazol-5-yl)[(5*S*)-5-(2-hydroxyethyl)-2-methylmorpholin-4-yl]methanon [enantiomerenreines Isomer 2] mit der folgenden Formel

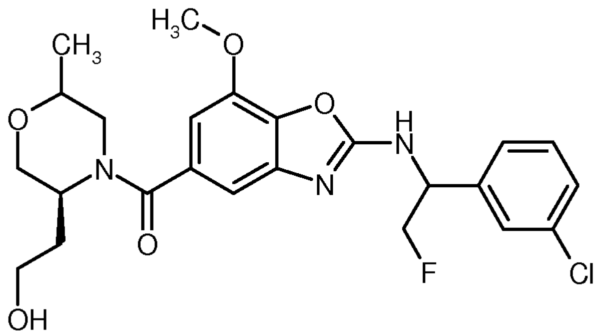

oder eines ihrer Salze, ihrer Solvate oder der Solvate ihrer Salze.

Besonders bevorzugt ist auch (2-{[(1*S*)-1-(3-Chlorphenyl)-2-fluorethyl]amino}-7-methoxy-1,3-benzoxazol-5-yl)[(2*S*,5*S*)-5-(2-hydroxyethyl)-2-methylmorpholin-4-yl]methanon mit der folgenden Formel

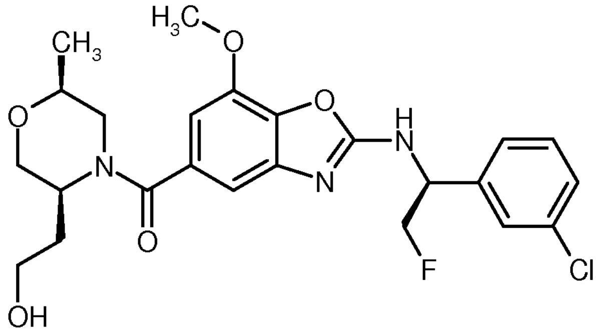

oder eines ihrer Salze, ihrer Solvate oder der Solvate ihrer Salze.

Gegenstand der Erfindung ist auch die Verbindung 2-(6-Methylmorpholin-3-yl)ethanol [Racemat] mit der folgenden Formel

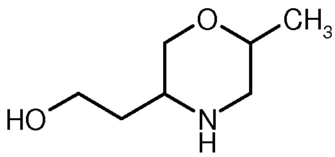

oder eines ihrer Salze, ihrer Solvate oder der Solvate ihrer Salze.

Die Verbindung 2-(6-Methylmorpholin-3-yl)ethanol [Racemat] kann nach dem Fachmann bekannten Methoden, zum Beispiel durch Chromatograpie an chiraler Phase, in ihre Enantiomere getrennt werden.

Bevorzugt ist auch die Verbindung 2-[(3S,6S)-6-Methylmorpholin-3-yl]ethanol mit der folgenden Formel

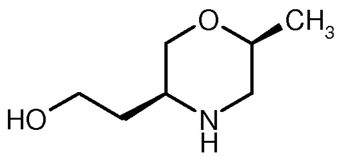

oder eines ihrer Salze, ihrer Solvate oder der Solvate ihrer Salze.

Gegenstand der Erfindung ist weiterhin ein Verfahren zur Herstellung der Verbindungen der Formel (I), oder ihrer Salze, ihrer Solvate oder der Solvate ihrer Salze, wobei die Verbindung der Formel

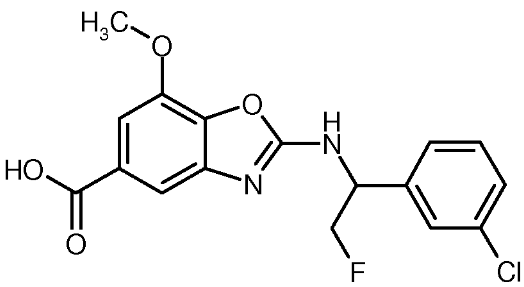 (II),

mit Verbindungen der Formel

$R^1$-H (III),

in welcher

$R^1$ die oben angegebene Bedeutung hat,

mit Dehydratisierungsreagenzien umgesetzt werden.

Die Umsetzung erfolgt im Allgemeinen in inerten Lösungsmitteln, gegebenenfalls in Gegenwart einer Base,

bevorzugt in einem Temperaturbereich von 0°C bis Raumtemperatur bei Normaldruck.

Als Dehydratisierungsreagenzien eignen sich hierbei beispielsweise Carbodiimide wie z.B. *N,N'*-Diethyl-, *N,N'*-Dipropyl-, *N,N'*-Diisopropyl-, *N,N'*-Dicyclohexylcarbodiimid, *N*-(3-Dimethylamino-isopropyl)-*N'*-ethylcarbodiimid-Hydrochlorid (EDC) (gegebenenfalls in Gegenwart von Pentafluorphenol (PFP)), *N*-Cyclohexylcarbodiimid-*N'*-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder *O*-(Benzotriazol-1-yl)-*N,N,N',N'*-tetra-methyluronium-hexafluorophosphat (HBTU), 2-(2-Oxo-1-(2H)-pyridyl)-1,1,3,3-tetramethyluroniumtetrafluoroborat (TPTU), (Benzotriazol-1-yloxy)bisdimethylaminomethyliumfluoroborat (TBTU) oder *O*-(7-Azabenzotriazol-1-yl)-*N,N,N',N'*-tetramethyl-uroniumhexafluorophosphat (HATU), oder 1-Hydroxybenztriazol (HOBt), oder Benzotriazol-1-yloxytris(dimethylamino)-phosphoniumhexafluorophosphat (BOP), oder Mischungen aus diesen, mit Basen. Vorzugsweise wird die Kondensation mit HATU durchgeführt.

Basen sind beispielsweise Alkalicarbonate, wie z.B. Natrium- oder Kaliumcarbonat, oder -hydrogencarbonat, oder organische Basen wie Trialkylamine, z.B. Triethylamin, N-Methylmorpholin, N-Methylpiperidin, 4-Dimethylaminopyridin oder Diisopropylethylamin, bevorzugt ist Diisopropylethylamin.

Inerte Lösungsmittel sind beispielsweise Halogenkohlenwasserstoffe wie Dichlormethan oder Trichlormethan, Kohlenwasserstoffe wie Benzol, oder andere Lösungsmittel wie Nitromethan, Dioxan, Dimethylformamid, Dimethylsulfoxid oder Acetonitril, oder Gemische der Lösungsmittel, bevorzugt ist Dimethylformamid.

Die Verbindungen der Formel (III) sind bekannt, lassen sich nach bekannten Verfahren aus den entsprechenden Ausgangsverbindungen synthetisieren oder können analog den im Beispielteil beschriebenen Verfahren hergestellt werden.

Die Verbindung der Formel (II) ist bekannt oder kann hergestellt werden, indem die Verbindungen der Formel

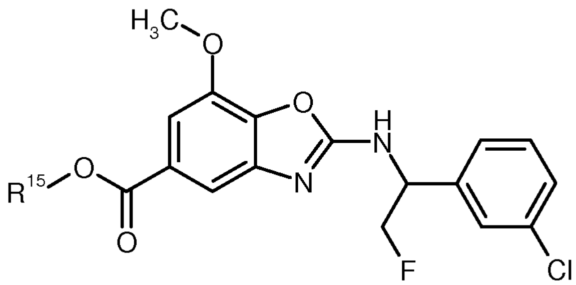

(IV),

in welcher

$R^{15}$ für Methyl oder Ethyl steht,

mit einer Base umgesetzt werden.

Die Umsetzung erfolgt im Allgemeinen in inerten Lösungsmitteln, bevorzugt in einem Temperaturbereich von 0°C bis Raumtemperatur bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt ist Natriumhydroxid.

Inerte Lösungsmittel sind beispielsweise Halogenkohlenwasserstoffe wie Dichlormethan, Trichlormethan, Tetrachlormethan, Trichlorethan, Tetrachlorethan, 1,2-Dichlorethan oder Trichlorethylen, Ether wie Diethylether, Methyl-tert.-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Dimethylsulfoxid, Acetonitril oder Pyridin, oder Gemische von Lösungsmitteln, bevorzugt ist Dioxan.

Die Verbindungen der Formel (IV) sind bekannt oder können hergestellt werden, indem Verbindungen der Formel

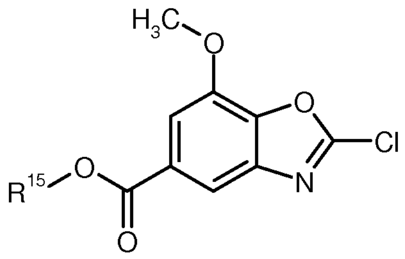

(V),

in welcher

$R^{15}$ für Methyl oder Ethyl steht,

mit der Verbindung der Formel

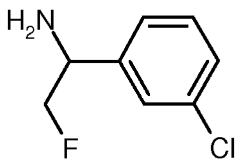

(VI),

in Gegenwart einer Base umgesetzt werden.

Die Umsetzung erfolgt im Allgemeinen in inerten Lösungsmitteln, bevorzugt in einem Temperaturbereich von Raumtemperatur bis zum Rückfluss der Lösungsmittel bei Normaldruck.

Die Verbindungen der Formeln (V) und (VI) sind bekannt, lassen sich nach bekannten Verfahren aus den entsprechenden Ausgangsverbindungen synthetisieren oder können analog den im Beispielteil beschriebenen Verfahren hergestellt werden.

Die Herstellung der Ausgangsverbindungen und der Verbindungen der Formel (I) kann durch das folgende Syntheseschema verdeutlicht werden.

**Schema 1:**

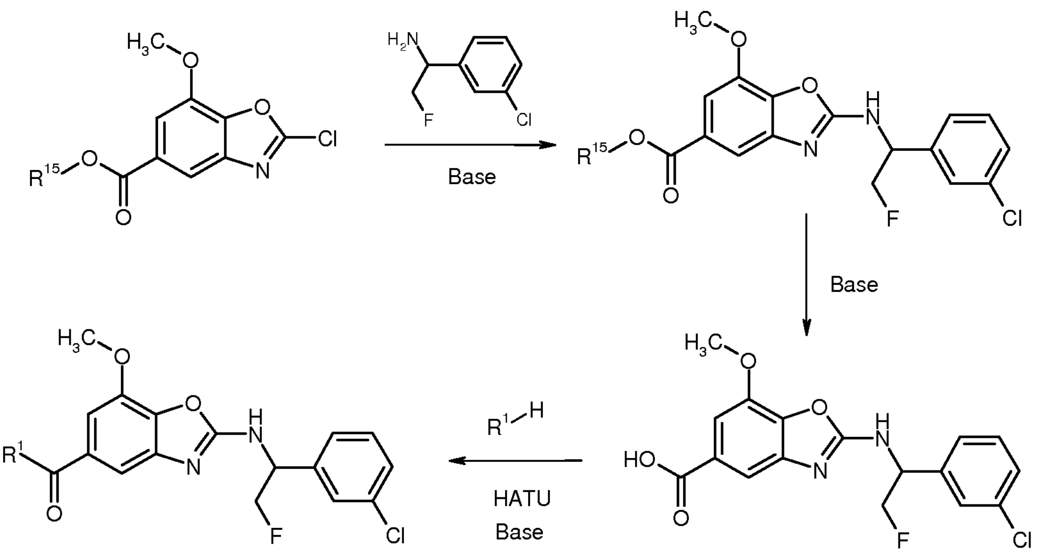


Die erfindungsgemäßen Verbindungen zeigen ein nicht vorhersehbares, wertvolles pharmakologisches Wirkspektrum und ein gutes pharmakokinetisches Verhalten. Es handelt sich dabei um Verbindungen, die die proteolytische Aktivität der Serinprotease Thrombin beeinflussen. Die erfindungsgemäßen Verbindungen hemmen die von Thrombin katalysierte enzymatische Spaltung von Substraten, die eine wesentliche Rolle in der Aktivierung der Blutgerinnung, der Aggregation von Blutplättchen (via PAR-1-Aktivierung der Plättchen) und bei von Thrombin-induzierten Inflammations-, Fibrose- und Angiogenese-Prozessen einnehmen.

Sie eigenen sich daher zur Verwendung als Arzneimittel zur Behandlung und/oder Prophylaxe von Krankheiten bei Menschen und Tieren.

Weiterer Gegenstand der vorliegenden Erfindung ist der Einsatz der erfindungsgemäßen Verbindungen zur Behandlung und/oder Prophylaxe von Erkrankungen, insbesondere von Herz-Kreislauf-Erkrankungen, vorzugsweise von thrombotischen beziehungsweise thromboembolischen Erkrankungen und/oder thrombotischen beziehungsweise thromboembolischen Komplikationen.

Als Schlüsselenzym am Ende der Koagulationskaskade übersetzt Thrombin über eine Reihe von Umsetzungen die Impulse aus der Kaskade in den Gerinnungsstatus des Blutes. Durch die Umwandlung von Fibrinogen zu unlöslichem Fibrin kommt es zu Fibringerinnselbildung, die durch das ebenfalls durch Thrombin aktivierte Faktor XIIIa stabilisiert werden. Mittels Aktivierung von TAFI (Thrombin-aktivierbarer Fibrinolyse Inhibitor) zu TAFIa hemmt Thrombin im Komplex mit Thrombomodulin die Auflösung des Gerinnsels. Aktivierung der Faktoren V und VIII führt zur Potenzierung der Thrombinproduktion und damit wiederum zur Verstärkung der Gerinnungsreaktion. Darüber hinaus ist Thrombin ein potenter Auslöser der Thrombozytenaggregation (über PAR-1-Aktivierung), die ebenfalls einen erheblichen Beitrag bei der Hämostase leistet.

Daher eignen sich die erfindungsgemäßen Verbindungen zur Behandlung und/oder Prophylaxe von Erkrankungen oder Komplikationen, die durch Gerinnselbildung entstehen beziehungsweise entstehen können.

Zu den "thrombotischen beziehungsweise thromboembolischen Erkrankungen" im Sinne der vorliegenden Erfindung zählen Erkrankungen, die sowohl im arteriellen als auch im venösen Gefäßbett auftreten und mit den erfindungsgemäßen Verbindungen behandelt werden können, insbesondere Erkrankungen in den Koronararterien des Herzens, wie das akute Koronarsyndrom (ACS), Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, aber auch thrombotische beziehungsweise thromboembolische Erkrankungen in weiteren Gefäßen, die zu peripheren arteriellen Verschlusskrankheiten, Lungenembolien, venösen Thromboembolien, venösen Thrombosen, insbesondere in tiefen Beinvenen und Nierenvenen, transitorische ischämische Attacken sowie thrombotischer Hirnschlag und thromboembolischer Hirnschlag führen.

Die Stimulation des Gerinnungssystems kann durch verschiedene Ursachen beziehungsweise Begleiterkrankungen erfolgen. Unter anderem kann im Rahmen von chirurgischen Eingriffen, Immobilität, Bettlägerigkeit, Infektionen oder einer Krebserkrankung beziehungsweise Krebstherapie das Gerinnungssystem stark angeregt sein und es zu thrombotischen Komplikationen, insbesondere venösen Thrombosen, kommen. Die erfindungsgemäßen Verbindungen eignen sich daher zur Thromboseprophylaxe im Rahmen von chirurgischen Eingriffen bei Patienten, die eine Krebserkrankung haben. Die erfindungsgemäßen Verbindungen eignen sich daher auch zur Thromboseprophylaxe in Patienten mit aktiviertem Gerinnungssystem, beispielsweise unter den beschriebenen Stimulationssituationen.

Die erfindungsgemäßen Verbindungen eignen sich daher auch zur Prävention und Behandlung von kardiogenen Thromboembolien, wie beispielsweise Hirn-Ischämien, Schlaganfall und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit künstlichen Herzklappen.

Thromboembolische Komplikationen treten ferner auf bei mikroangiopathischen hämolytischen Anämien, extrakorporalen Blutkreisläufen, wie Hämodialyse, sowie Herzklappenprothesen.

Außerdem kommen die erfindungsgemäßen Verbindungen insbesondere zur Behandlung von Erkrankungen in Betracht, bei denen das Gerinnsel schon vorliegt, da insbesondere in das Gerinnsel korporierte Thrombin zur Gerinnselstabilität beiträgt. Da die Hemmung dieser Thrombinmoleküle den Gerinnselabbau beschleunigt, können die erfindungsgemäßen Verbindungen zur Behandlung vorhandener Gerinnsel eingesetzt werden. Diese Gerinnsel können im gesamten Gefäßsystem entstehen und in verschiedenen Organen zu schwerwiegenden Komplikationen insbesondere durch Ischämie, Entzündungsreaktionen oder Embolusbildung führen, wie beispielsweise Myokardinfarkt oder Hirninfarkt, aber auch Lungenembolie oder postthrombotisches Syndrom insbesondere nach tiefen Beinvenenthrombosen. Die erfindungsgemäßen Verbindungen kommen daher auch zur Behandlung von venösen und arteriellen Verschlüssen der Augengefäße in Betracht, die durch Gerinnsel bedingt sind, beispielsweise altersbedingter Makula-Degeneration.

Aufgrund der beobachteten synergistischen Effekte mit lytischen Therapieprinzipien wie dem Gewebeplasminogenaktivator (tPA) eignen sich die Verbindungen für den adjuvanten Einsatz im Rahmen einer Thrombolyse-Therapie.

Außerdem kommen die erfindungsgemäßen Verbindungen zur Behandlung und/oder Prophylaxe von Erkrankungen in Betracht, bei denen Mikrogerinnselbildungen beziehungweise Fibrinablagerungen in Gehirngefäßen auftreten, die zu Demenzerkrankungen, wie beispielsweise vaskulärer Demenz oder Morbus Alzheimer führen können. Hierbei kann das Gerinnsel sowohl über Okklusionen als auch über die Bindung weiterer krankheitsrelevanter Faktoren zur Erkrankung beitragen.

Außerdem kommen die erfindungsgemäßen Verbindungen insbesondere zur Behandlung und/oder Prophylaxe von Erkrankungen in Betracht, bei denen neben der prokoagulanten auch die proinflammatorische Komponente der Thrombin-Wirkung eine wesentliche Rolle spielt. Insbesondere die gegenseitige Verstärkung von Koagulation und Inflammation kann durch die erfindungsgemäßen Verbindungen unterbunden und daher die Wahrscheinlichkeit für eine thrombotische Komplikation entscheidend gesenkt werden. Hier kommen unter anderem die Behandlung und/oder Prophylaxe im Rahmen von atherosklerotischen Gefäßerkrankungen, Entzündungen im Rahmen von rheumatischen Erkrankungen des Bewegungsapparates, entzündlichen Erkrankungen der Lunge, wie beispielsweise Lungenfibrosen, entzündliche Erkrankungen der Niere, wie beispielsweise Glomerulonephritiden, entzündliche Erkrankungen des Darms, wie beispielsweise Morbus Crohn oder Colitis ulcerosa, oder Erkrankungen, die im Rahmen einer diabetischen Grunderkrankung vorliegen können, wie beispielsweise diabetische Retinopathie beziehungsweise Nephropathie in Betracht.

Außerdem können die erfindungsgemäßen Verbindungen zur Inhibition des Tumorwachstums und der Metastasenbildung sowie zur Prophylaxe und/oder Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Außerdem kommen die erfindungsgemäßen Verbindungen auch für die Prophylaxe und/oder Behandlung von pulmonaler Hypertonie in Betracht.

Der Begriff "pulmonale Hypertonie" umfasst im Rahmen der vorliegenden Erfindung die pulmonale arterielle Hypertonie, die pulmonale Hypertonie bei Erkrankungen des linken Herzens, die pulmonale Hypertonie bei Lungenerkrankung und/oder Hypoxie und die Pulmonale Hypertonie aufgrund chronischer Thrombembolien (CTEPH).

Die "pulmonale arterielle Hypertonie" beinhaltet die Idiopathische Pulmonale Arterielle Hypertonie (IPAH, früher auch als primäre pulmonale Hypertonie bezeichnet), die Familiär bedingte Pulmonale Arterielle Hypertonie (FPAH) und die Assoziierte Pulmonal-Arterielle Hypertonie (APAH), die assoziiert ist mit Kollagenosen, kongenitalen systemisch-pulmonalen Shuntvitien, portaler Hypertension, HIV-Infektionen, der Einnahme bestimmter Drogen und Medikamente, mit anderen Erkrankungen (Schilddrüsenerkrankungen, Glykogenspeicherkrankheiten, Morbus Gaucher, hereditäre Teleangiektasie, Hämoglobinopathien, myeloproliferative Erkrankungen, Splenektomie), mit Erkrankungen mit einer signifikanten venösen/kapillären Beteiligung wie der pulmonal-venookklusiven Erkrankung und der pulmonal-kapillären Hämangiomatose, sowie die persistierende pulmonale Hypertonie der Neugeborenen.

Die pulmonale Hypertonie bei Erkrankungen des linken Herzens beinhaltet die Erkrankung des linken Vorhofes oder Ventrikels und Mitral- oder Aortenklappenfehler.

Die pulmonale Hypertonie bei Lungenerkrankung und/oder Hypoxie beinhaltet chronisch obstruktive Lungenerkrankungen, interstitielle Lungenerkrankung, Schlafapnoe-Syndrom, alveolärer Hypoventilation, chronische Höhenkrankheit und anlagebedingte Fehlbildungen.

Die Pulmonale Hypertonie aufgrund chronischer Thrombembolien (CTEPH) beinhaltet den thrombembolischen Verschluss proximaler Lungenarterien, den thrombembolischen Verschluss distaler Lungenarterien und nicht-thrombotische Lungenembolien (Tumor, Parasiten, Fremdkörper).

Weiterer Gegenstand der vorliegenden Erfindung ist die Verwendung der erfindungsgemäßen Verbindungen zur Herstellung von Arzneimitteln zur Behandlung und/oder Prophylaxe von pulmonaler Hypertonie bei Sarkoidose, Histiozytose X und Lymphangiomatosis.

Außerdem kommen die erfindungsgemäßen Substanzen auch zur Behandlung von pulmonalen und hepatischen Fibrosen in Betracht.

Außerdem kommen die erfindungsgemäßen Verbindungen auch für die Behandlung und/oder Prophylaxe einer Disseminierten intravasalen Koagulation im Rahmen einer Infektionserkrankung, und/oder von Systemic Inflammatory Syndrome (SIRS), septischer Organdysfunktion, septischem Organversagen und Multiorganversagen, Acute Respiratory Distress Syndrome (ARDS), Acute Lung Injury (ALI), Septischer Schock und/oder des septischen Organversagens in Betracht.

Im Verlauf einer Infektion kann es zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es es zur endothelialen Schädigung mit Erhöhung der Gefäßpermeabilität und Austritt von Flüssigkeit und Proteinen in den Extravasalraum kommen. Im weiteren Verlauf kann ein Versagen eines Organs (z.B. Nierenversagen, Leberversagen, Atemversagen, zentralnervöse Defizite und Herz-/Kreislaufversagen) oder zum Multiorganversagen kommen.

Bei der DIC kommt es an der Oberfäche von geschädigten Endothelzellen, Fremdkörperoberflächen oder veretztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin und Fibrinogen) und Plättchen, wodurch die Gerinnungsfähigkeit des Blutes herabgesetzt wird und schwere Blutungen auftreten können.

Ganz besonders eigenen sich die erfindungsgemäßen Verbindungen für die Behandlung und/oder Prophylaxe von akutem Koronarsyndrom (ACS), venösen Thromboembolien, venösen Thrombosen, insbesondere in tiefen Beinvenen und Nierenvenen, Lungenembolien, Schlaganfall und/oder Thromboseprophylaxe im Rahmen von chirurgischen Eingriffen, insbesondere im Rahmen von chirurgischen Eingriffen bei Patienten, die eine Krebserkrankung haben.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind die erfindungsgemäßen Verbindungen zur Verwendung in einem Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe.

Die erfindungsgemäßen Verbindungen können darüber hinaus auch zur Verhinderung von Koagulation *ex vivo* eingesetzt werden, z.B. zum Schutz von zu transplantierenden Organen vor durch Gerinnselbildung verursachten Organschäden und zum Schutz des Organ-Empfängers vor Thromboemboli aus dem transplantierten Organ, zur Konservierung von Blut- und Plasmaprodukten, zur Reinigung/Vorbehandlung von Kathetern und anderen medizinischen Hilfsmitteln und Geräten, zur Beschichtung künstlicher Oberflächen von *in vivo* oder *ex vivo* eingesetzten medizinischen Hilfsmitteln und Geräten oder bei biologischen Proben, die Faktor IIa enthalten könnten.

Weiterer Gegenstand der vorliegenden Erfindung ist ein Verfahren zur Verhinderung der Blutkoagulation *in vitro*, insbesondere bei Blutkonserven oder biologischen Proben, die Faktor IIa enthalten könnten, das dadurch gekennzeichnet ist, dass eine antikoagulatorisch wirksame Menge der erfindungsgemäßen Verbindung zugegeben wird.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:

- Lipidsenker, insbesondere HMG-CoA-(3-Hydroxy-3-methylglutaryl-Coenzym A)-Reduktase-Inhibitoren wie beispielsweise Lovastatin (Mevacor), Simvastatin (Zocor), Pravastatin (Pravachol), Fluvastatin (Lescol) und Atorvastatin (Lipitor);

- Koronartherapeutika/Vasodilatatoren, insbesondere ACE-(Angiotensin-Converting-Enzyme)-Inhibitoren wie beispielsweise Captopril, Lisinopril, Enalapril, Ramipril, Cilazapril, Benazepril, Fosinopril, Quinapril und Perindopril, oder AII-(Angiotensin II)-Rezeptor-Antagonisten wie beispielsweise Embusartan, Losartan, Valsartan, Irbesartan, Candesartan, Eprosartan und Temisarta, oder β-Adrenozeptor-Antagonisten wie beispielsweise Carvedilol, Alprenolol, Bisoprolol, Acebutolol, Atenolol, Betaxolol, Carteolol, Metoprolol, Nadolol, Penbutolol, Pindolol, Propanolol und Timolol, oder alpha-1-Adrenozeptor-Antagonisten wie beispielsweise Prazosin, Bunazosin, Doxazosin und Terazosin, oder Diuretika wie beispielsweise Hydrochlorothiazid, Furosemid, Bumetanid, Piretanid, Torasemid, Amilorid und Dihydralazin, oder Calciumkanal-Blocker wie beispielsweise Verapamil und Diltiazem, oder Dihydropyridin-Derivate wie beispielsweise Nifedipin (Adalat) und Nitrendipin (Bayotensin), oder Nitropräparate wie beispielsweise Isosorbid-5-mononitrat, Isosorbiddinitrat und Glyceroltrinitrat, oder Substanzen, die eine Erhöhung von cyclischem Guanosinmonophosphat (cGMP) bewirken, wie beispielsweise Stimulatoren der löslichen Guanylatcyclase, wie beispielsweise Riociguat;

- Plasminogen-Aktivatoren (Thrombolytika/Fibrinolytika) und die Thrombolyse/Fibrinolyse steigernde Verbindungen wie Inhibitoren des Plasminogen-Aktivator-Inhibitors (PAI-Inhibitoren) oder Inhibitoren des Thrombin-aktivierten Fibrinolyse-Inhibitors (TAFI-Inhibitoren) wie beispielsweise Gewebsplasminogen-Aktivator (t-PA, beispielsweise Actilyse®), Streptokinase, Reteplase und Urokinase;

- antikoagulatorisch wirksame Substanzen (Antikoagulantien) wie beispielsweise Heparin (UFH), niedermolekulare Heparine (NMH) wie beispielsweise Tinzaparin, Certoparin, Parnaparin, Nadroparin, Ardeparin, Enoxaparin, Reviparin, Dalteparin, Danaparoid, Semuloparin (AVE 5026), Adomiparin (M118) und EP-42675/ORG42675;

- direkte Thrombin Inhibitoren (DTI) wie beispielsweise Pradaxa (Dabigatran), Atecegatran (AZD-0837), DP-4088, SSR-182289A, Argatroban, Bivalirudin und Tanogitran (BIBT-986 und prodrug BIBT-1011), Hirudin;

- direkte Faktor Xa-Inhibitoren wie beispielsweise Rivaroxaban, Apixaban, Edoxaban (DU-176b), Betrixaban (PRT-54021), R-1663, Darexaban (YM-150), Otamixaban (FXV-673/RPR-130673), Letaxaban (TAK-442), Razaxaban (DPC-906), DX-9065a, LY-517717, Idraparinux und Fondaparinux;

- plättchenaggregationshemmende Substanzen (Plättchenaggregationshemmer, Thrombozytenaggregationshemmer) wie beispielsweise Acetylsalicylsäure (wie beispielsweise Aspirin), Ticlopidin (Ticlid), Clopidogrel (Plavix), Prasugrel, Ticagrelor, Cangrelor, Elinogrel, Vorapaxar;

- Fibrinogen-Rezeptor-Antagonisten (Glycoprotein-IIb/IIIa-Antagonisten) wie beispielsweise Abciximab, Eptifibatide, Tirofiban, Lamifiban, Lefradafiban und Fradafiban;

- rekombinantes humanes aktiviertes Protein C wie beispielsweise Xigris;

- sowie Antiarrhythmika.

Weiterer Gegenstand der vorliegenden Erfindung ist eine Kombination enthaltend (A) eine Verbindung der Formel (I) und (B) 5-Chlor-*N*-({(5*S*)-2-oxo-3-[4-(3-oxo-4-morpholinyl)-phenyl]-1,3-oxazolidin-5-yl}-methyl)-2-thiophen-carboxamid (Rivaroxaban) [WO 01/47919] mit der Strukturformel

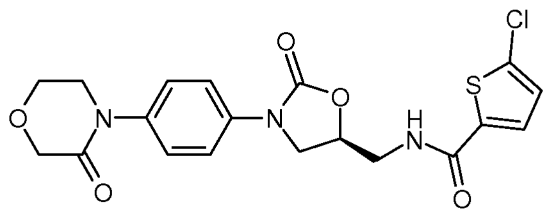

Weiterer Gegenstand der vorliegenden Erfindung ist auch eine Kombination enthaltend (A) (2-{[1-(3-Chlorphenyl)-2-fluorethyl]amino}-7-methoxy-1,3-benzoxazol-5-yl)[(5*S*)-5-(2-hydroxy-ethyl)-2-methylmorpholin-4-yl]methanon [enantiomerenreines Isomer 2] mit der folgenden Formel

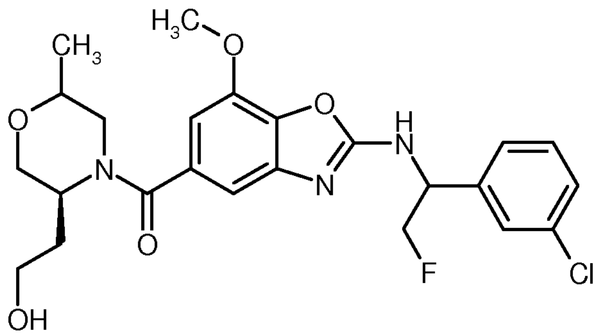

oder eines ihrer Salze, ihrer Solvate oder der Solvate ihrer Salze
und (B) 5-Chlor-*N*-({(5*S*)-2-oxo-3-[4-(3-oxo-4-morpholinyl)-phenyl]-1,3-oxazolidin-5-yl}-methyl)-2-thiophen-carboxamid (Rivaroxaban) mit der Strukturformel

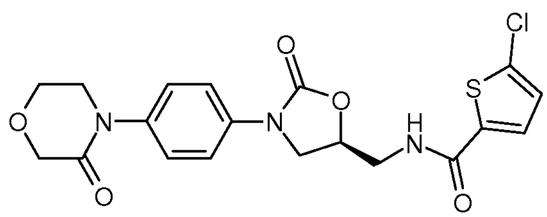

Weiterer Gegenstand der vorliegenden Erfindung ist auch eine Kombination enthaltend (A) (2-{[(1*S*)-1-(3-Chlorphenyl)-2-fluorethyl]amino}-7-methoxy-1,3-benzoxazol-5-yl)[2*S*,5*S*)-5-(2-hydroxyethyl)-2-methylmorpholin-4-yl]methanon mit der folgenden Formel

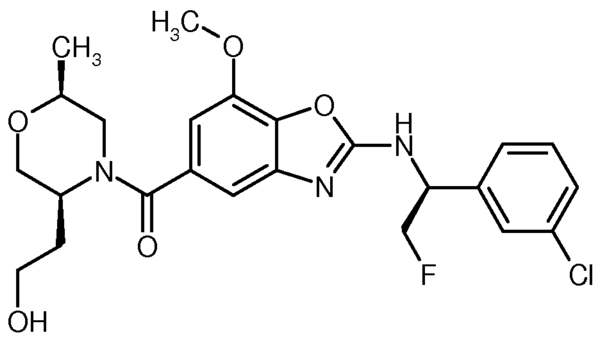

oder eines ihrer Salze, ihrer Solvate oder der Solvate ihrer Salze und (B) 5-Chlor-*N*-({(5*S*)-2-oxo-3-[4-(3-oxo-4-morpholinyl)-phenyl]-1,3-oxazolidin-5-yl}-methyl)-2-thiophen-carboxamid (Rivaroxaban) mit der Strukturformel

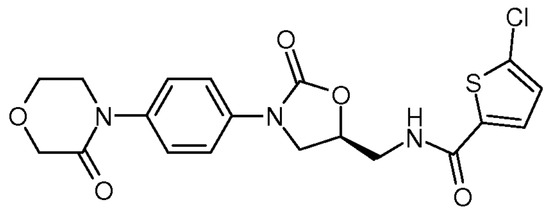

Weiterer Gegenstand der vorliegenden Erfindung ist auch eine Kombination enthaltend (A) (2-{[(1*S*)-1-(3-Chlorphenyl)-2-fluorethyl]amino}-7-methoxy-1,3-benzoxazol-5-yl)[(2*S*,5*S*)-5-(2-hydroxyethyl)-2-methylmorpholin-4-yl]methanon mit der folgenden Formel

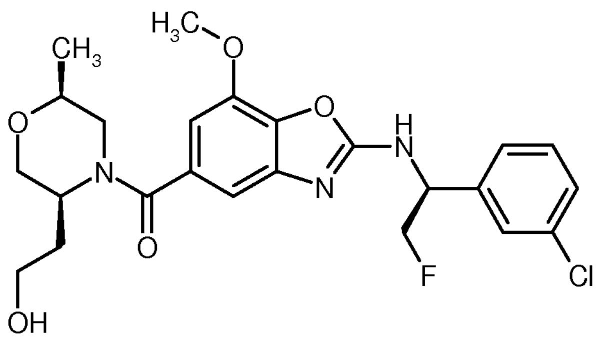

und (B) 5-Chlor-*N*-({(5*S*)-2-oxo-3-[4-(3-oxo-4-morpholinyl)-phenyl]-1,3-oxazolidin-5-yl}-methyl)-2-thiophen-carboxamid (Rivaroxaban) mit der Strukturformel

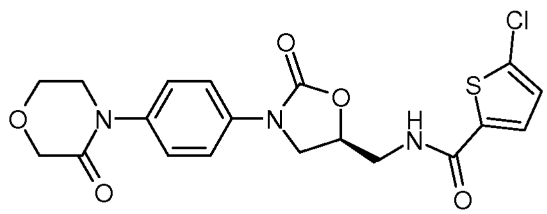

Unter "Kombinationen" im Sinne der Erfindung werden nicht nur Darreichungsformen, die alle Komponenten enthalten (sog. Fixkombinationen) und Kombinationspackungen, die die Komponenten voneinander getrennt enthalten, verstanden, sondern auch gleichzeitig oder zeitlich versetzt applizierte Komponenten, sofern sie zur Prophylaxe und/oder Behandlung derselben Krankheit eingesetzt werden. Ebenso ist es möglich, zwei oder mehr Wirkstoffe miteinander zu kombinieren, es handelt sich dabei also jeweils um zwei- oder mehrfach-Kombinationen.

Die Wirkstoffe (2-{[(1*S*)-1-(3-Chlorphenyl)-2-fluorethyl]amino}-7-methoxy-1,3-benzoxazol-5-yl)[(2*S*,5*S*)-5-(2-hydroxyethyl)-2-methylmorpholin-4-yl]methanon und 5-Chlor-*N*-(1(5*S*)-2-oxo-3-[4-(3-oxo-4-morpholinyl)-phenyl]-1,3-oxazolidin-5-yl}-methyl)-2-thiophen-carboxamid (Rivaroxaban) werden in der Kombination bevorzugt in jeweils subtherapeutischer Menge eingesetzt.

Die Wirkstoffe (2-{[(1*S*)-1-(3-Chlorphenyl)-2-fluorethyl]amino}-7-methoxy-1,3-benzoxazol-5-yl)[(2*S*,5*S*)-5-(2-hydroxyethyl)-2-methylmorpholin-4-yl]methanon und 5-Chlor-*N*-({(5*S*)-2-oxo-3-[4-(3-oxo-4-morpholinyl)-phenyl]-1,3-oxazolidin-5-yl}-methyl)-2-thiophen-carboxamid (Rivaroxaban) werden in der Kombination bevorzugt in jeweils subtherapeutischer Menge eingesetzt, wobei die subtherapeutische Menge bezogen ist auf die Erkrankungen, in der die Kombination Verwendung findet.

Bevorzugt werden 20 mg bis 120 mg je 24 Stunden, besonders bevorzugt 20 mg bis 80 mg je 24 Stunden, von (2-{[(1*S*)-1-(3-Chlorphenyl)-2-fluorethyl]amino}-7-methoxy-1,3-benzoxazol-5-yl)[(2*S*,5*S*)-5-(2-hydroxyethyl)-2-methylmorpholin-4-yl]methanon in der Kombination eingesetzt.

Bevorzugt werden 2.5 mg bis 10 mg je 24 Stunden, besonders bevorzugt 2.5 mg, 5 mg oder 10 mg je 24 Stunden, von 5-Chlor-*N*-({(5*S*)-2-oxo-3-[4-(3-oxo-4-morpholinyl)-phenyl]-1,3-oxazolidin-5-yl}-methyl)-2-thiophen-carboxamid (Rivaroxaban) in der Kombination eingesetzt.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Bevorzugt ist die orale Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 500 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

## A) Beispiele

### Abkürzungen:

| | |
|---|---|
| br. d. | breites Dublett (bei NMR) |
| br. m. | breites Multiplett (bei NMR) |
| br. s. | breites Singulett (bei NMR) |
| ca. | circa |
| d | Tag(e), Dublett (bei NMR) |
| DC | Dünnschicht-Chromatographie |
| dd | doppeltes Dublett (bei NMR) |
| DMSO | Dimethylsulfoxid |
| dt | doppeltes Triplett (bei NMR) |
| d. Th. | der Theorie (bei Ausbeute) |
| ESI | Elektrospray-Ionisation (bei MS) |
| GC-MS | Gaschromatographie-gekoppelte Massenspektroskopie |
| h | Stunde(n) |
| HATU | *O*-(7-Azabenzotriazol-1-yl)-*N,N,N',N'*-tetramethyluronium-Hexafluorphosphat |
| HPLC | Hochdruck-, Hochleistungsflüssigchromatographie |
| konz. | konzentriert(e)(er) |
| LC-MS | Flüssigchromatographie-gekoppelte Massenspektroskopie |
| m | Multiplett (bei NMR) |
| M | molar |
| $m_c$ | zentriertes Multiplett (bei NMR) |
| min | Minute(n) |
| MS | Massenspektroskopie |
| N | normal |
| NMR | Kernresonanzspektroskopie |
| quant. | quantitativ |
| Q | Quartett (bei NMR) |
| quin | Quintett (bei NMR) |
| RP | Reverse Phase |
| RT | Raumtemperatur (20-25°C) |
| $R_t$ | Retentionszeit (bei HPLC) |
| s | Singulett (bei NMR) |
| t | Triplett (bei NMR) |
| UV | Ultraviolett |
| UPLC | Ultrahochdruck-, Ultraleistungsflüssigchromatographie |

### LC-MS Methoden:

Methode 1A: Instrument: Waters ACQUITY SQD UPLC System; Säule: Waters Acquity UPLC HSS T3 1.8 μ 50 x 1 mm; Eluent A: 1 L Wasser + 0.25 mL 99%ige Ameisensäure, Eluent B: 1 L Acetonitril + 0.25 mL 99%ige Ameisensäure; Gradient: 0.0 min 90% A → 1.2 min 5% A → 2.0 min 5% A; Ofen: 50°C; Fluss: 0.40 mL/min; UV-Detektion: 208 - 400 nm.

Methode 2A: Instrument: Waters ACQUITY SQD UPLC System; Säule: Waters Acquity UPLC HSS T3 1.8 μ 30 x 2 mm; Eluent A: 1 L Wasser + 0.25 mL 99%ige Ameisensäure, Eluent B: 1 L Acetonitril + 0.25 mL 99%ige Ameisensäure; Gradient: 0.0 min 90% A → 1.2 min 5% A → 2.0 min 5% A; Ofen: 50°C; Fluss: 0.60 mL/min; UV-Detektion: 208 - 400 nm.

Methode 3A: Instrument: Micromass Quattro Premier mit Waters UPLC Acquity; Säule: Thermo Hypersil GOLD 1.9 μ 50 x 1 mm; Eluent A: 1 L Wasser + 0.5 mL 50%ige Ameisensäure, Eluent B: 1 L Acetonitril + 0.5 mL 50%ige Ameisensäure; Gradient: 0.0 min 97% A → 0.5 min 97% A → 3.2 min 5% A → 4.0 min 5% A; Ofen: 50°C; Fluss: 0.3 mL/min; UV-Detektion: 210 nm.

Methode 4A: Instrument MS: Waters (Micromass) Quattro Micro; Instrument HPLC: Agilent 1100 Serie; Säule: YMC-Triart C18 3 μ 50 x 3 mm; Eluent A: 1 L Wasser + 0.01 mol Ammoniumcarbonat, Eluent B: 1 L Acetonitril; Gradient: 0.0 min 100% A → 2.75 min 5% A → 4.5 min 5% A; Ofen: 40°C; Fluss: 1.25 mL/min; UV-Detektion: 210 nm.

Methode 5A: Instrument MS: Waters (Micromass) QM; Instrument HPLC: Agilent 1100 Serie; Säule: Agient ZORBAX Extend-C18 3.0 x 50mm 3.5-Micron; Eluent A: 1 L Wasser + 0.01 mol Ammoniumcarbonat, Eluent B: 1 L Acetonitril; Gradient: 0.0 min 98% A → 0.2 min 98% A → 3.0 min 5% A → 4.5 min 5% A; Ofen: 40°C; Fluss: 1.75 mL/min; UV-Detektion: 210 nm.

Methode 6A: Instrument MS: Waters (Micromass) ZQ; Instrument HPLC: Agilent 1100 Serie; Säule: Agient ZORBAX Extend-C18 3.0 x 50mm 3.5-Micron; Eluent A: 1 L Wasser + 0.01 mol Ammoniumcarbonat, Eluent B: 1 L Acetonitril; Gradient: 0.0 min 98% A → 0.2 min 98% A → 3.0 min 5% A → 4.5 min 5% A; Ofen: 40°C; Fluss: 1.75 mL/min; UV-Detektion: 210 nm.

Methode 7A: Instrument: Waters ACQUITY SQD UPLC System; Säule: Waters Acquity UPLC HSS T3 1.8 μ 50 x 1 mm; Eluent A: 1 L Wasser + 0.25 mL 99%ige Ameisensäure, Eluent B: 1 L Acetonitril + 0.25 mL 99%ige Ameisensäure; Gradient: 0.0 min 95% A → 6.0 min 5% A → 7.5 min 5% A; Ofen: 50°C; Fluss: 0.35 mL/min; UV-Detektion: 210 - 400 nm.

GC-MS-Methoden:

Methode 1B: Instrument: Thermo DFS, Trace GC Ultra; Säule: Restek RTX-35, 15 m x 200 μm x 0.33 μm; konstanter Fluss mit Helium: 1.20 mL/min; Ofen: 60°C; Inlet: 220°C; Gradient: 60°C, 30°C/min → 300°C (3.33 min halten).

Methode 2B: Instrument: Micromass GCT, GC6890; Säule: Restek RTX-35, 15 m x 200 μm x 0.33 μm; konstanter Fluss mit Helium: 0.88 mL/min; Ofen: 70°C; Inlet: 250°C; Gradient: 70°C, 30°C/min → 310°C (3 min halten).

MS-Methoden:

Methode 1C: Instrument: Thermo Fisher-Scientific DSQ; chemische Ionisierung; Reaktantgas $NH_3$; Quellentemperatur: 200°C; Ionisierungsenergie 70eV.

Methode 2C: Instrument: Waters ZQ 2000; Elektrospray-Ionisierung; Eluent A: 1 L Wasser + 0.25 mL 99%ige Ameisensäure, Eluent B: 1 L Acetonitril + 0.25 ml 99%ige Ameisensäure; 25% A, 75% B; Fluss: 0.25 mL/min.

**Präparative Enantiomeren-/Diastereomerentrennung an chiraler Phase:**

Methode 1D: Phase: Daicel Chiralpak AZ-H, 5 μm 250 mm x 30 mm, Eluent: iso-Hexan/Ethanol 50:50; Fluss: 40 mL/min; Temperatur: 20°C; UV-Detektion: 220 nm.

Methode 2D: Phase: Daicel Chiralpak AZ-H, 5 μm 250 mm x 30 mm, Eluent: iso-Hexan/Ethanol 50:50; Fluss: 40 mL/min, Temperatur: 25°C; UV-Detektion: 220 nm.

Methode 3D: Phase: Daicel Chiralpak AD-H SFC, 10 μm 250 mm x 20 mm, Eluent: Kohlendioxid /Ethanol 70:30; Fluss: 100 mL/min, Makeupflowrate: 30 mL/min, Backpressure: 80 bar; Temperatur: 40°C; UV-Detektion: 220 nm.

Methode 4D: Phase: Daicel Chiralpak AD-H, 5 μm 250 mm x 20 mm, Eluent: iso-Hexan/iso-Propanol 70:30; Fluss: 20 mL/min; Temperatur: 25°C; UV-Detektion: 230 nm.

Methode 5D: Phase: Daicel Chiralpak AZ-H, 5 μm 250 mm x 30 mm, Eluent: iso-Hexan/Ethanol 90:10; Fluss: 40 mL/min; Temperatur: 25°C; UV-Detektion: 220 nm.

Methode 6D: Phase: Daicel Chiralpak AY-H, 5 μm 250 mm x 20 mm, Eluent: iso-Hexan/Ethanol 90:10; Fluss: 40 mL/min; Temperatur: 40°C; UV-Detektion: 220 nm.

Methode 7D: Phase: Daicel Chiralpak AS-H, 5 μm 250 mm x 20 mm, Eluent: iso-Hexan/Ethanol 70:30; Fluss: 20 mL/min; Temperatur: 25°C; UV-Detektion: 230 nm.

Methode 8D: Phase: Daicel Chiralpak AZ-H, 5 μm 250 mm x 30 mm, Eluent: iso-Hexan/Ethanol 50:50; Fluss: 20 mL/min; Temperatur: 25°C; UV-Detektion: 220 nm.

Methode 9D: Phase: Daicel Chiralpak OZ-H, 5 μm 250 mm x 20 mm, Eluent: iso-Hexan/Ethanol 50:50; Fluss: 15 mL/min; Temperatur: 30°C; UV-Detektion: 220 nm.

Methode 10D: Phase: Daicel Chiralpak OD-H, 5 μm 250 mm x 20 mm, Eluent: iso-Hexan/Ethanol 60:40; Fluss: 20 mL/min; Temperatur: 22°C; UV-Detektion: 230 nm.

**Analytische Enantiomeren-/Diastereomerentrennung an chiraler Phase:**

Methode 1E: Phase: Daicel Chiralcel OZ-H, 5 μm 250 mm x 4.6 mm; Eluent: iso-Hexan/Ethanol 50:50; Fluss: 1 mL/min; Temperatur: 30°C; UV-Detektion: 220 nm.

Methode 2E: Phase: Daicel Chiralcel AZ-H, 5 μm 250 mm x 4.6 mm; Eluent: iso-Hexan/Ethanol 50:50; Fluss: 1 mL/min; Temperatur: 30°C; UV-Detektion: 220 nm.

Methode 3E: Phase: Daicel Chiralpak AD-H SFC, 5 μm 250 mm x 4.6 mm; Eluent: Kohlendioxid/Ethanol 70:30; Fluss: 3 mL/min; Temperatur: 30°C; UV-Detektion: 220 nm.

Methode 4E: Phase: Daicel Chiralpak AD-H, 5 μm 250 mm x 4.6 mm, Eluent: iso-Hexan/iso-Propanol 50:50; Fluss: 1 mL/min; Temperatur: 30°C; UV-Detektion: 220 nm.

Methode 5E: Phase: LUX Amylose-2, 5 μm 250 mm x 4.6 mm; Eluent: iso-Hexan/Ethanol 90:10; Fluss: 1 mL/min; Temperatur: 30°C; UV-Detektion: 220 nm.

Methode 6E: Phase: Daicel Chiralpak AS-H, 5 μm 250 mm x 4.6 mm, Eluent: iso-Hexan/iso-Propanol 50:50; Fluss: 1 mL/min; Temperatur: 30°C; UV-Detektion: 220 nm.

Methode 7E: Phase: Daicel Chiralcel OD-H, 5 μm 250 mm x 4.6 mm; Eluent: iso-Hexan/Ethanol 80:20 + 0.2% Diethylamin; Fluss: 1 mL/min; Temperatur: 40°C; UV-Detektion: 220 nm.

Methode 8E: Phase: Daicel Chiralpak AD-H, 5 μm 250 mm x 4.6 mm, Eluent: iso-Hexan/Ethanol 50:50; Fluss: 1 mL/min; Temperatur: 30°C; UV-Detektion: 220 nm.

Methode 9E: Phase: Daicel Chiralcel OZ-H, 5 μm 250 mm x 4.6 mm; Eluent: iso-Hexan/Ethanol 50:50; Fluss: 1 mL/min; Temperatur: 40°C; UV-Detektion: 220 nm.

Methode 10E: Phase: Daicel Chiralcel OD-H, 5 μm 250 mm x 4.6 mm; Eluent: iso-Hexan/Ethanol 50:50; Fluss: 1 mL/min; Temperatur: 30°C; UV-Detektion: 220 nm.

Methode 11E: Phase: Daicel Chiralcel AZ-H, 5 μm 250 mm x 4.6 mm; Eluent: iso-Hexan/Ethanol 90:10; Fluss: 1 mL/min; Temperatur: 30°C; UV-Detektion: 220 nm.

**Präparative Reinigung:**

Methode 1F: Phase: Sunfire C-18, 5 μm 250 mm x 20 mm, Eluent: Wasser/Acetonitril Gradient 80:20 → 5:95, Fluss: 23.75 mL/min + konstante Zugabe von 2%iger Ameisensäure, Fluss: 1.25 mL/min, UV-Detektion: 210 nm.

**Präparative Diastereomerentrennung an achiraler Phase:**

Methode 1G: Phase: Sunfire C-18, 5 μm 250 mm x 20 mm, Eluent: Wasser/Methanol 60:40, Fluss: 60 mL/min, Temperatur: 23°C, UV-Detektion: 210 nm.

**Mikrowelle**

Als Mikrowellenreaktor wurde ein "single mode" Gerät vom Typ Biotage Initiator Microwave Synthesizer verwendet.

Bei Aufreinigungen von erfindungsgemäßen Verbindungen per präparativer HPLC nach den oben beschriebenen Methoden, in denen die Elutionsmittel Zusatzstoffe wie beispielsweise Trifluoressigsäure, Ameisensäure oder Ammoniak enthalten, können die erfindungsgemäßen Verbindungen in Salz-Form, beispielsweise als Trifluoracetat, Formiat oder Ammonium-Salz anfallen, sofern die erfindungsgemäßen Verbindungen eine ausreichend basische bzw. saure Funktionalität enthalten. Ein solches Salz kann durch verschiedene dem Fachmann bekannte Methoden in die entsprechende freie Base beziehungsweise Säure überführt werden.

Wenn bei den im Folgenden beschriebenen Synthese-Intermediaten und Ausführungsbeispielen der Erfindung eine Verbindung in der Form eines Salzes der korrespondierenden Base beziehungseise Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Trifluoracetat", "Natrium-Salz" bzw. "x HCl", "x $CF_3COOH$", "x $Na^+$" bei solchen Salzen nicht stöchiometrisch zu verstehen, sondern haben allein deskriptiven Charakter bezüglich der enthaltenen salzbildenden Komponenten.

Sinngemäß gleiches gilt für den Fall, dass Synthese-Intermediate oder Ausführungsbeispiele oder Salze hiervon nach den beschriebenen Herstell- und/oder Reinigungsverfahren in Form von Solvaten, wie beispielsweise Hydraten, erhalten wurden, deren stöchiometrische Zusammensetzung (sofern definierter Art) nicht bekannt ist.

**Ausgangsverbindungen**

**Beispiel 1A**

7-Methoxy-2-thioxo-2,3-dihydro-1,3-benzoxazol-5-carbonsäuremethylester

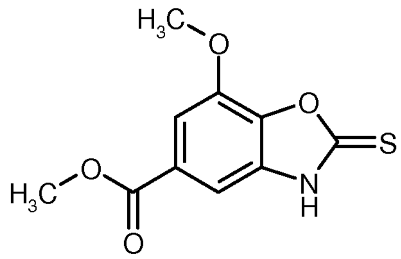

Es wurden 20.0 g (101 mmol) 3-Amino-4-hydroxy-5-methoxybenzoesäuremethylester und 17.9 g (112 mmol) Kalium-O-ethyldithiocarbonat in Pyridin (400 mL) gelöst und für 3 h unter Rückfluss gerührt (analog Lit.: R. Lok et al., J. Org. Chem. 1996, 61, 3289-3297). Anschließend wurde die Reaktionsmischung abgekühlt und auf eine Mischung von Eis (600 g) und konzentrierter wässriger Hydrogenchlorid-Lösung (60 mL) gegeben. Der entstandene Feststoff wurde im Vakuum abfiltriert und mit Wasser (5 x 200 mL) gewaschen. Der Feststoff wurde zunächst bei 50°C/40 mbar und anschließend am Hochvakuum getrocknet. Ausbeute: 23.3 g (96% d. Th.).
LC-MS (Methode 1A): $R_t$ = 0.79 min; MS (ESIpos): m/z = 240 $[M+H]^+$;
$^1$H-NMR (400 MHz, DMSO-$d_6$): δ [ppm] = 14.1 (br. s., 1H), 7.45 (d, 1H), 7.32 (d, 1H), 4.00 (s, 3H), 3.88 (s, 3H).

**Beispiel 2A**

2-Chlor-7-methoxy-1,3-benzoxazol-5-carbonsäuremethylester

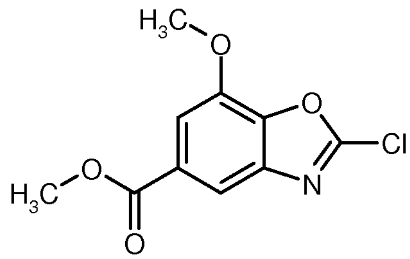

150 g (627 mmol) 7-Methoxy-2-thioxo-2,3-dihydro-1,3-benzoxazol-5-carbonsäure-methylester wurden in Thionylchlorid (450 mL) suspendiert, mit katalytischen Mengen an *N,N*-Dimethylformamid (1.0 mL) versetzt und anschließend für 3 h gerührt (analog Lit.: R. Lok et al., J. Org. Chem. 1996, 61, 3289-3297). Es wurde erneut *N,N*-Dimethylformamid (1.0 mL) zugegeben und bis zum Ende der Gasentwicklung (ca. 4 h) bei 70°C gerührt. Die Reaktionslösung wurde im Vakuum eingeengt und der Rückstand zur vollständigen Entfernung des Thionylchlorids mit Dichlormethan (3 x 200 mL) koevaporiert. Der Feststoff wurde am Hochvakuum getrocknet und anschließend durch Säulenchromatographie an Kieselgel (Dichlormethan) gereinigt. Alternativ kann auch das Rohprodukt direkt weiter verwendet werden. Ausbeute: 125.6 g (82% d. Th.).
LC-MS (Methode 1A): $R_t$ = 1.00 min; MS (ESIpos): m/z = 242 $[M+H]^+$;
$^1$H-NMR (400 MHz, $CDCl_3$): δ [ppm] = 7.99 (d, 1H), 7.62 (d, 1H), 4.07 (s, 3H), 3.96 (s, 3H).

**Beispiel 3A**

1-(3-Chlorphenyl)-2-fluorethanon

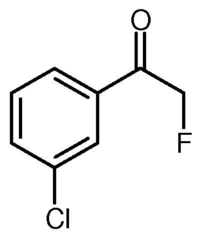

Methode 1:

Es wurden 50.7g (161 mmol) Tetra-*n*-butylammoniumfluorid Trihydrat, 22.1 g (214 mmol) Zinkfluorid und 6.22 g (107 mmol) Kaliumfluorid in Acetonitril (850 mL) vorgelegt und 1 h bei 80°C gerührt (analog Lit.: X. Zou et al., J. Fluorane Chem. 2010, 131, 340-344.). Anschließend wurden bei dieser Temperatur 50.0 g (214 mmol) 2-Brom-1-(3-chlorphenyl)ethanon in Acetonitril (210 mL) innerhalb von 3 h zugetropft und nachfolgend für weitere 3 h bei 80°C gerührt. Die Reaktionslösung wurde auf RT abgekühlt und die ausgefallenen Salze durch eine Glasfritte abfiltriert. Das Filtrat wurde im Vakuum eingeengt, der Rückstand mit Wasser versetzt und mehrfach mit *tert*-Butylmethylether extrahiert. Weitere ausgefallene Salze wurden durch Filtration entfernt. Die organischen Phasen wurden über Magnesiumsulfat getrocknet, filtriert und im Vakuum eingeengt. Das Rohprodukt wurde anschließend durch Flash-Chromatographie an Kieselgel (Petrolether, Petrolether/Essigsäureethylester 10:1) gereinigt. Ausbeute: 27.0 g (58% d. Th., Reinheit: 80%).
GC-MS (Methode 1B): $R_t$ = 3.73 min; MS (EIpos): m/z = 172 $[M]^+$;
$^1$H-NMR (400 MHz, DMSO-$d_6$): $\delta$ [ppm] = 7.91 ($m_c$, 1H), 7.85 ($d_{br}$, 1H), 7.77 (dd, 1H), 7.60 ($m_c$, 1H), 5.85 (d, 2H).

Methode 2:

10.0 g (64.7 mmol) 1-(3-Chlorphenyl)ethanon in Methanol (80.0 mL) wurden mit 45.8 g (129 mmol) 1-(Chlormethyl)-4-fluor-1,4-diazoniabicyclo[2.2.2]octan-bis-tetrafluoroborat (Selectfluor) versetzt und anschließend in zehn Portionen für 2.5 h bei 110°C in der Mikrowelle (Biotage Synthesizer) gerührt (analog Lit.: B. H. Hoff et al., Tetrahedron 2009, 65, 9550-9556.). Dann wurde jeweils mit 5 mL Wasser versetzt und für 1 h bei 110°C in der Mikrowelle gerührt. Nachfolgend wurden die Reaktionsgemische vereinigt, das Methanol im Vakuum entfernt, der Rückstand mit Wasser verdünnt und anschließend mit Essigsäureethylester extrahiert. Die organische Phase wurde über Natriumsulfat getrocknet, filtriert und im Vakuum eingeengt. Das Rohprodukt wurde ohne weitere Reinigung in der nächsten Stufe eingesetzt. Ausbeute: 12.5 g (86% d. Th., Reinheit: 77%).
GC-MS (Methode 2B): $R_t$ = 3.56 min; MS (EIpos): m/z = 172 $[M]^+$.

**Beispiel 4A**

1-(3-Chlorphenyl)-2-fluorethanamin [Racemat]

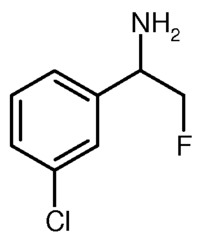

Methode 1:

24.9 g (144.0 mmol) 1-(3-Chlorphenyl)-2-fluorethanon in 2 M ethanolischer Ammoniaklösung (361 mL, 722 mmol) wurden tropfenweise mit 82.0 g (86.3 mL, 289 mmol) Titantetraisopropoxid versetzt, wobei die Temperatur durch Eiskühlung bei 20°C gehalten wurde, und dann über Nacht bei RT gerührt. Anschließend wurde bei 10°C portionsweise 8.19 g (216 mmol) Natriumborhydrid zugegeben und für 6 h bei RT gerührt. Anschließend wurden weitere 1.64 g (43.2 mmol) Natriumborhydrid zugegeben und über Nacht gerührt. Die Reaktionslösung wurde mit halbkonzentrierter wässriger Hydrogenchlorid-Lösung (300 mL) versetzt und anschließend mit Wasser (1.0 L) verdünnt (pH = 2). Es wurde mit

*tert*-Butylmethylether (3 x 500 mL) extrahiert, anschließend die organischen Phasen über Natriumsulfat getrocknet, filtriert und im Vakuum eingeengt. Ausbeute: 10.5 g (Reinheit: 58%).

Die wässrige Phase wurde mit 45%iger wässriger Natriumhydroxid-Lösung auf pH = 10 eingestellt, mit Natriumchlorid gesättigt und mit *tert*-Butylmethylether (3 x 500 mL) extrahiert. Die vereinigten organischen Phasen wurden über Natriumsulfat getrocknet, filtriert und im Vakuum eingeengt. Es wurden weitere 12.9 g (51% d. Th.) des gewünschten Produktes erhalten.
LC-MS (Methode 5A): $R_t$ = 1.93 min; MS (ESIpos): m/z = 174 $[M+H]^+$;
$^1$H-NMR (400 MHz, DMSO-$d_6$): $\delta$ [ppm] = 7.50 (s, 1H), 7.40-7.27 (m, 3H), 4.45 ($m_c$, 1H), 4.32 ($m_c$, 1H), 4.12 (dt, 1H), 2.10 (br. s., 2H).

Die Produktfraktionen wurden vereinigt und in der nächsten Stufe ohne weitere Reinigung eingesetzt.

Methode 2:

Unter Argonatmosphäre wurden 8.20 g (38.0 mmol, Reinheit: 80%) 1-(3-Chlorphenyl)-2-fluorethanon in 2 M ethanolischer Ammoniaklösung (95 mL, 190 mmol) gelöst, mit 21.6 g (22.8 mL, 76.0 mmol) Titantetraisopropoxid versetzt und für 16 h bei RT gerührt. Anschließend wurden 1.51 g (57.3 mmol) Natriumborhydrid zugegeben und 5 h bei RT gerührt. Es wurden weitere 700 mg (18.5 mmol) Natriumborhydrid zugegeben und über Nacht bei RT gerührt. Die Reaktionslösung wurde mit 6 M wässriger Hydrogenchlorid-Lösung auf pH = 2 gebracht und anschließend dreimal mit *tert*-Butylmethylether extrahiert. Die wässrige Phase wurde mit Natriumhydroxid auf pH = 10 gebracht, mit Natriumchlorid gesättigt und viermal mit *tert*-Butylmethylether extrahiert. Die vereinigten organischen Phasen wurden über Natriumsulfat getrocknet, filtriert und im Vakuum eingeengt. Der Rückstand wurde in Dichlormethan aufgenommen und mit gesättigter wässriger Natriumhydrogencarbonat-Lösung gewaschen. Die organische Phase wurde über Natriumsulfat getrocknet, filtriert und im Vakuum eingeengt. Die Zielverbindung wurde ohne weitere Reinigung in der nächsten Stufe eingesetzt. Ausbeute: 7.32 g (90% d. Th., Reinheit: 81%).
LC-MS (Methode 5A): $R_t$ = 1.92 min; MS (ESIpos): m/z = 174 $[M+H]^+$.

**Beispiel 5A**

1-(3-Chlorphenyl)-2-fluorethanamin Hydrochlorid [Racemat]

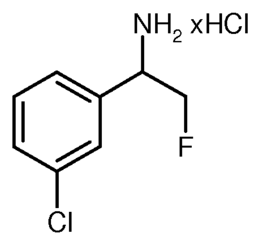

Methode 1:

Unter Argonatmosphäre wurden 2.00 g (11.5 mmol) 1-(3-Chlorphenyl)-2-fluorethanon in 2 M ethanolischer Ammoniaklösung (28.7 mL, 57.4 mmol) mit 6.52 g (6.87 mL, 23.0 mmol) Titantetraisopropoxid versetzt und 16 h bei RT gerührt. Anschließend wurden 654 mg (17.3 mmol) Natriumborhydrid zugegeben und 5 h bei RT gerührt. Es wurden weitere 350 mg (9.25 mmol) Natriumborhydrid zugegeben und über Nacht bei RT gerührt. Die Reaktionslösung wurde in 25%iger wässriger Ammoniaklösung (100 mL) gegossen und anschließend über Kieselgur filtriert. Das Filtrat wurde mit *tert*-Butylmethylether (200 mL) versetzt und extrahiert. Nach Phasentrennung wurde die wässrige Phase mit *tert*-Butylmethylether (100 mL) extrahiert. Die vereinigten organischen Phasen wurden über Natriumsulfat getrocknet, filtriert und im Vakuum eingeengt. Das Rohprodukt wurde in Diethylether/Tetrahydrofuran (5:1; 60 mL) gelöst und anschließend mit 4 N Hydrogenchlorid-Lösung in 1,4-Dioxan (10.0 mL) versetzt. Der entstandene Feststoff wurde im Vakuum abfiltriert, mit wenig Diethylether gewaschen und am Hochvakuum getrocknet. Ausbeute: 1.54 g (63% d. Th.).
LC-MS (Methode 5A): $R_t$ = 1.94 min; MS (ESIpos): m/z = 174 $[M+H-HCl]^+$;
$^1$H-NMR (400 MHz, DMSO-$d_6$): $\delta$ [ppm] = 8.98 (br. s., 3H), 7.70 (s, 1H), 7.60-7.47 (m, 3H), 4.88-4.67 (m, 3H).

Methode 2:

335 g (1.94 mol) 1-(3-Chlorphenyl)-2-fluorethanon in 2 M ethanolischer Ammoniaklösung (4.85 L, 9.71 mol) wurden tropfenweise mit 1.10 kg (1.16 L, 3.88 mol) Titantetraisopropoxid versetzt (Temperatur durch Eiskühlung bei 20°C gehalten) und über Nacht bei RT gerührt. Dann wurde bei 10°C in vier Portionen 110 g (2.91 mol) Natriumborhydrid zugegeben und 36 h bei RT gerührt. Es wurden weitere 29.4 g (776 mmol) Natriumborhydrid zugegeben und 1 h gerührt bei RT gerührt. Die Reaktionslösung wurde auf 2 M wässrige Ammoniak-Lösung (4.85 L) gegossen und anschließend die ausgefallenen Salze über eine Fritte im Vakuum abfiltriert. Das Filtrat wurde mit *tert*-Butylmethylether (14 L) und Wasser (50 L) versetzt, extrahiert und die Mischung anschließend zur Phasentrennung mit 5%iger wässriger Natriumchlorid-Lösung versetzt. Nach Phasentrennung wurde die wässrige Phase erneut mit *tert*-Butylmethylether (5 L) extrahiert, die vereinigten organischen Phasen über Natriumsulfat getrocknet, filtriert und im Vakuum eingeengt. Das Rohprodukt wurde in Diethylether/Tetrahydrofuran (10:1; 1.1 L) gelöst und anschließend unter Rühren und unter Eiskühlung mit 4 N Hydrogenchlorid-Lösung in 1,4-Dioxan (385 mL) versetzt. Der ausgefallene weiße Feststoff wurde im Vakuum abfiltriert, mit wenig Diethylether gewaschen und am Hochvakuum getrocknet. Ausbeute: 261 g (77% d. Th.).
LC-MS (Methode 5A): $R_t$ = 1.93 min; MS (ESIpos): m/z = 174 $[M+H-HCl]^+$;

**Beispiel 6A**

*tert*-Butyl-[1-(3-chlorphenyl)-2-fluorethyl]carbamat [Racemat]

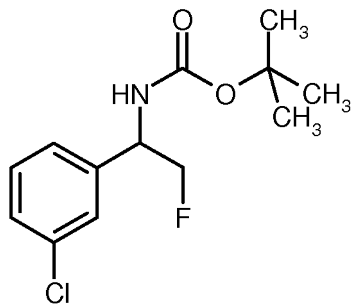

Methode 1:

7.47 g (43.3 mmol) 1-(3-Chlorphenyl)-2-fluorethanamin [Racemat] wurden in Dichlormethan (150 mL) suspendiert, zunächst mit 9.14 g (12.6 mL, 90.4 mmol) Triethylamin und anschließend mit 10.3 g (47.3 mmol) Di-*tert*-butyldicarbonat versetzt und über Nacht bei RT gerührt. Dann wurde die Reaktionslösung mit 0.5 N wässriger Hydrogenchlorid-Lösung (100 mL), gesättigter wässriger Natriumhydrogencarbonat-Lösung (100 mL) und Wasser (100 mL) gewaschen. Die organische Phase wurde über Natriumsulfat getrocknet, filtriert und im Vakuum eingeengt. Das Rohprodukt wurde durch präparative RP-HPLC (Wasser/Acetonitril) gereinigt. Ausbeute: 7.23 g (61% d. Th.).
LC-MS (Methode 1A): $R_t$ = 1.10 min; MS (ESIpos): m/z = 218 $[M+H-C_4H_9]^+$;
$^1$H-NMR (400 MHz, DMSO-$d_6$): $\delta$ [ppm] = 7.72 (d, 1H), 7.45 (s, 1H), 7.41-7.29 (m, 3H), 4.89 ($m_c$, 1H), 4.59-4.45 (m, 1H), 4.45-4.32 (m, 1H), 1.38 (s, 9H).

Methode 2:

Unter Argonatmosphäre wurden 41.5 g (198 mmol) 1-(3-Chlorphenyl)-2-fluorethanamin Hydrochlorid [Racemat] in Dichlormethan (200 mL) suspendiert, dann zunächst mit 80.0 g (110 mL, 790 mmol) Triethylamin und anschließend mit Dichlormethan (200 mL) versetzt. Es wurden 31.0 g (142 mmol) Di-*tert*-butyldicarbonat in Dichlormethan (100 mL) zugegeben und über Nacht bei RT gerührt. Es wurden weitere 9.91 g (45.4 mmol) Di-*tert*-butyldicarbonat zugegeben und bis zum nahezu vollständigen Umsatz (DC-Kontrolle) gerührt. Die Reaktionslösung wurde mit 1 N wässriger Hydrogenchlorid-Lösung (2 x 100 mL) und gesättigter wässriger Natriumhydrogen-carbonat-Lösung (100 mL) gewaschen. Die organische Phase wurde über Natriumsulfat getrocknet, filtriert und im Vakuum eingeengt. Ausbeute: 51.0 g (94% d. Th.).
LC-MS (Methode 7A): $R_t$ = 3.25 min; MS (ESIpos): m/z = 218 $[M+H-C_4H_9]^+$.

**Beispiel 7A**

*tert*-Butyl-[1-(3-chlorphenyl)-2-fluorethyl]carbamat [enantiomerenreines Isomer 1]

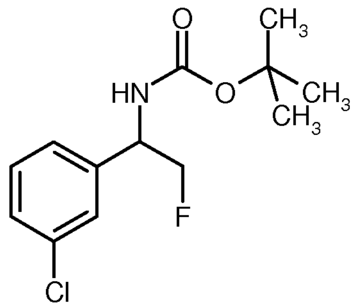

Enantiomerentrennung an chiraler Phase von 7.23 g der Verbindung aus Beispiel 6A nach Methode 6D ergab 3.05 g des Beispiels 7A (enantiomerenreines Isomer 1) und 3.05 g des Beispiels 8A (enantiomerenreines Isomer 2).
HPLC (Methode 6E): $R_t$ = 5.01 min, 99.0% ee;
LC-MS (Methode 7A): $R_t$ = 3.26 min; MS (ESIpos): m/z = 218 $[M+H-C_4H_9]^+$;
$^1$H-NMR (400 MHz, DMSO-$d_6$): $\delta$ [ppm] = 7.72 (d, 1H), 7.45 (s, 1H), 7.42-7.27 (m, 3H), 4.89 ($m_c$, 1H), 4.59-4.45 (m, 1H), 4.44-4.31 (m, 1H), 1.38 (s, 9H).

**Beispiel 8A**

*tert*-Butyl-[1-(3-chlorphenyl)-2-fluorethyl]carbamat [enantiomerenreines Isomer 2]

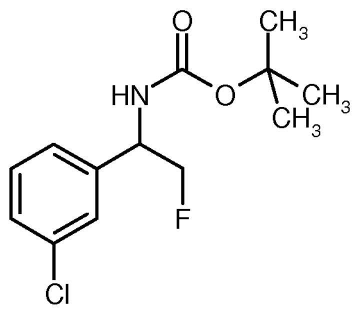

Enantiomerentrennung an chiraler Phase von 7.23 g der Verbindung aus Beispiel 6A nach Methode 6D ergab 3.05 g des Beispiels 7A (enantiomerenreines Isomer 1) und 3.05 g des Beispiels 8A (enantiomerenreines Isomer 2).
HPLC (Methode 6E): $R_t$ = 7.46 min, 99.0% ee;
LC-MS (Methode 7A): $R_t$ = 3.26 min; MS (ESIpos): m/z = 218 $[M+H-C_4H_9]^+$;
$^1$H-NMR (400 MHz, DMSO-$d_6$): $\delta$ [ppm] = 7.72 (d, 1H), 7.45 (s, 1H), 7.42-7.28 (m, 3H), 4.89 ($m_c$, 1H), 4.58-4.45 (m, 1H), 4.44-4.30 (m, 1H), 1.38 (s, 9H).

**Beispiel 9A**

1-(3-Chlorphenyl)-2-fluorethanamin Hydrochlorid [enantiomerenreines Isomer]

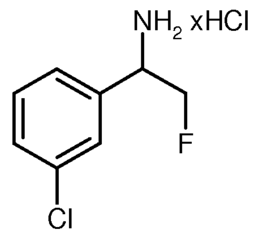

Es wurden 17.3 g (63.2 mmol) *tert*-Butyl-[1-(3-chlorphenyl)-2-fluorethyl]carbamat [enantiomerenreines Isomer 2] in 1,4-Dioxan (50 mL) vorgelegt und anschließend bei RT mit 79 mL (316 mmol) 4 N Hydrogenchlorid-Lösung in 1,4-Dioxan versetzt. Es bildete sich nach kurzer Zeit ein Feststoff. 1,4-Dioxan (250 mL) und anschließend 31.6 mL (126 mmol) 4 N Hydrogenchlorid-Lösung in 1,4-Dioxan wurden hinzugefügt und es wurde bei RT über Nacht gerührt. Die entstandene Suspension wurde vollständig im Vakuum eingeengt, der Rückstand mit *tert*-Butylmethylether (200 mL) verrührt, filtriert und der Filterrückstand mit *tert*-Butylmethylether (2 x 50 mL) gewaschen. Der entstandene Feststoff wurde am Hochvakuum getrocknet. Ausbeute: 13.2 g (99% d. Th.).

Drehwert: $[\alpha]_D^{19.9} = 27.06°$ (c = 0.51, Methanol),

LC-MS (Methode 5A): $R_t$ = 1.94 min; MS (ESIpos): m/z = 174 $[M+H]^+$;

$^1$H-NMR (400 MHz, DMSO-$d_6$): $\delta$ [ppm] = 8.91 (br. s., 3H), 7.68 (s, 1H), 7.55-7.47 (m, 3H), 4.89-4.66 (m, 3H).

**Beispiel 10A**

2-{[1-(3-Chlorphenyl)-2-fluorethyl]amino}-7-methoxy-1,3-benzoxazol-5-carbonsäuremethylester [Racemat]

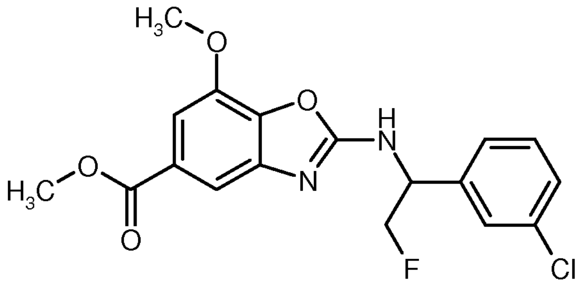

Es wurden 1.82 g (5.73 mmol, Reinheit: 78%) 2-Chlor-7-methoxy-1,3-benzoxazol-5-carbonsäuremethylester und 2.03 g (5.73 mmol, Reinheit: 49%) 1-(3-Chlorphenyl)ethanamin [Racemat] in 1,4-Dioxan (78 mL) bei RT mit 2.22 g (2.99 mL, 17.2 mmol) *N,N*-Diisopropylethylamin versetzt und anschließend für 1 h gerührt. Die Reaktionslösung wurde 5 h unter Rückfluss gerührt und anschließend über Nacht bei RT nachgerührt. Dann wurde im Vakuum eingeengt, der Rückstand in Diethylether aufgenommen und mit wenig Dichlormethan versetzt, gerührt und der ausgefallene Feststoff im Vakuum filtriert und mit Diethylether gewaschen. Ausbeute: 1.42 g (37% d. Th., Reinheit: 56%). Das Filtrat wurde durch präparative RP-HPLC (Acetonitril/Wasser) gereinigt und man erhielt weitere 665 mg (22% d. Th., Reinheit: 71%) des gewünschten Produktes.
LC-MS (Methode 1A): $R_t$ = 1.10 min; MS (ESIpos): m/z = 379 $[M+H]^+$.

**Beispiel 11A**

2-{[1-(3-Chlorphenyl)-2-fluorethyl]amino}-7-methoxy-1,3-benzoxazol-5-carbonsäure [Racemat]

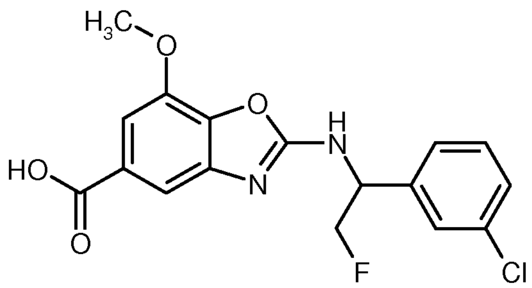

Es wurden 628 mg (1.31 mmol, Reinheit: 79%) 2-{[1-(3-Chlorphenyl)-2-fluorethyl]amino}-7-methoxy-1,3-benzoxazol-5-carbonsäuremethylester [Racemat] in 1,4-Dioxan (80 mL) gelöst und anschließend mit 1 N wässriger Natriumhydroxid-Lösung (20 mL) versetzt. Es wurde für 18 h bei RT gerührt. Dann wurde 1,4-Dioxan im Vakuum weitgehend entfernt, der Rückstand in 1 N wässriger Hydrogenchlorid-Lösung aufgenommen und mehrfach mit Essigsäureethylester extrahiert. Die organischen Phasen wurden über Natriumsulfat getrocknet, filtriert und im Vakuum eingeengt. Der Rückstand wurde mit Acetonitril verrührt, der ausgefallene Feststoff im Vakuum abfiltriert und am Hockvakuum getrocknet. Ausbeute: 134 mg (17% d. Th., Reinheit: 60%). Das Filtrat wurde durch präprative RP-HPLC (Acetonitril/Wasser) gereinigt und eine zweite Menge Produkt erhalten. Ausbeute: 137 mg (23% d. Th., Reinheit: 80%).
LC-MS (Methode 1A): $R_t$ = 0.93 min; MS (ESIpos): m/z = 365 $[M+H]^+$.

__Beispiel 12A__

2-{[1-(3-Chlorphenyl)-2-fluorethyl]amino}-7-methoxy-1,3-benzoxazol-5-carbonsäuremethylester [enantiomerenreines Isomer]

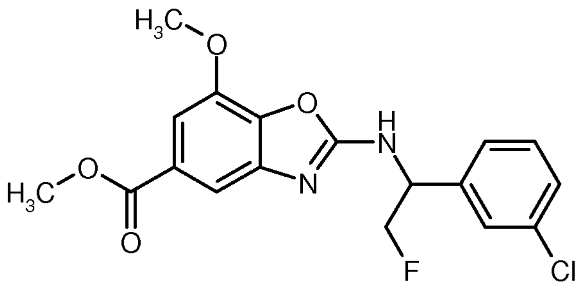

__Methode 1:__

Unter Argon wurden 15.2 g (62.8 mmol) 2-Chlor-7-methoxy-1,3-benzoxazol-5-carbonsäuremethylester in *N,N*-Dimethylformamid (100 mL) vorgelegt und anschließend bei RT mit 13.2 g (62.8 mmol) 1-(3-Chlorphenyl)-2-fluorethanamin Hydrochlorid [Beispiel 9A, enantiomerenreines Isomer] und 32.5 g (43.8 mL, 251 mol) *N,N*-Diisopropylethylamin versetzt. Die Reaktionslösung wurde 2 h bei 70°C (Ölbadtemperatur) gerührt und mit weiteren 264 mg (1.26 mmol) 1-(3-Chlorphenyl)-2-fluorethanamin Hydrochlorid [Beispiel 9A] versetzt. Es wurde 1 h bei 70°C (Ölbadtemperatur) gerührt, anschließend abgekühlt und auf Eiswasser gegossen. Nach Phasentrennung wurde die wässrige Phase mit *tert*-Butylmethylether (500 mL) versetzt und 10 min gerührt. Die entstandene Suspension wurde über eine Glasfritte im Vakuum filtriert, der Feststoff mehrfach mit *tert*-Butylmethylether verrührt und im Vakuum filtriert. Die wässrige Phase wurde mit *tert*-Butylmethylether (2 x 300 mL) extrahiert und die vereinigten organischen Phasen wurden im Vakuum eingeengt. Der Rückstand wurde erneut mit *tert*-Butylmethylether verrührt, filtriert und im Vakuum eingeengt. Beide Feststofffraktionen wurden anschließend vereinigt und am Hochvakuum getrocknet. Ausbeute: 18.4 g (77% d. Th.).
LC-MS (Methode 5A): $R_t$ = 2.56 min; MS (ESIpos): m/z = 379 $[M+H]^+$;
$^1$H-NMR (400 MHz, DMSO-$d_6$): δ [ppm] = 9.10 (d, 1H), 7.59 (s, 1H), 7.50-7.35 (m, 4H), 7.31 (d, 1H), 5.26 ($m_c$, 1H), 4.82-4.53 (m, 2H), 3.96 (s, 3H), 3.84 (s, 3H).

Methode 2:

Unter Argon wurden 125 g (517 mmol) 2-Chlor-7-methoxy-1,3-benzoxazol-5-carbonsäure-methylester in *N,N*-Dimethylformamid (850 mL) vorgelegt und bei RT mit 114 g (543 mmol) 1-(3-Chlorphenyl)-2-fluorethanamin Hydrochlorid und 267 g (360 mL, 2.07 mol) *N,N*-Diisopropylethylamin versetzt. Die Reaktionslösung wurde 4 h bei 70°C (Ölbadtemperatur) gerührt, dann mit 8.69 g (41.4 mmol) 1-(3-Chlorphenyl)-2-fluorethanamin Hydrochlorid versetzt und über Nacht (ca. 14 h) bei RT gerührt. Es wurden weitere 1.09 g (5.17 mmol) 1-(3-Chlorphenyl)-2-fluorethanamin Hydrochlorid zugesetzt, 2 h bei 70°C (Ölbadtemperatur) gerührt und anschließend im Vakuum eingeengt. Der Rückstand wurde in *tert*-Butylmethylether (2.0 L) aufgenommen und die organische Phase mit Wasser (3 x 1.0 L) gewaschen. Die organische Phase wurde im Vakuum bis auf ca. ein Drittel an Volumen eingeengt und nachfolgend der ausgefallene Feststoff im Vakuum abfiltriert. Der in der wässrigen Phase ausgefallene Feststoff wurde ebenfalls im Vakuum abfiltriert und anschließend mit *tert*-Butylmethylether (2 x 100 mL) gewaschen. Die vereinigten Feststoffe wurden am Hochvakuum getrocknet. Ausbeute: 181 g (92% d. Th.).

Drehwert: $[\alpha]_D^{20.2} = 77.20°$ (c = 0.465, Methanol);

LC-MS (Methode 5A): $R_t$ = 2.57 min; MS (ESIpos): m/z = 379 $[M+H]^+$;

$^1$H-NMR (400 MHz, DMSO-$d_6$): $\delta$ [ppm] = 9.11 (d, 1H), 7.59 (s, 1H), 7.51-7.35 (m, 4H), 7.31 (s, 1H), 5.27 ($m_c$, 1H), 4.83-4.50 (m, 2H), 3.96 (s, 3H), 3.84 (s, 3H).

**Beispiel 13A**

2-{[1-(3-Chlorphenyl)-2-fluorethyl]amino}-7-methoxy-1,3-benzoxazol-5-carbonsäure [enantiomerenreines Isomer]

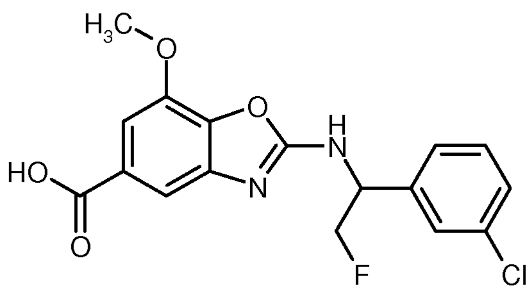

Es wurden 181 g (478 mmol) 2-{[1-(3-Chlorphenyl)-2-fluorethyl]amino}-7-methoxy-1,3-benzoxazol-5-carbonsäuremethylester [enantiomerenreines Isomer] in 1,4-Dioxan (2.4 L) vorgelegt und anschließend mit einer kalten (ca. 8°C) Lösung von 425 g (287 mL, 4.78 mol) 45%iger wässriger Natriumhydroxid-Lösung in Wasser (2.35 L) versetzt. Es wurde für 4 h bei RT gerührt und anschließend mit Wasser (2.0 L) verdünnt. Die Reaktionslösung wurde mit *tert*-Butylmethylether (2 x 1.0 L) extrahiert, die wässrige Phase mit Eis versetzt und mit konzentrierter Hydrogenchlorid-Lösung (ca. 450 mL) sauer gestellt. Es wurde 15 min bei 10°C gerührt, der ausgefallene Feststoff im Vakuum abfiltriert, der Rückstand mit Wasser (2 x 1.0 L) gewaschen, über Nacht bei RT belassen, dann 4 h bei 60°C und abschließend bei 50°C im Vakuum bis zur Massenkonstanz getrocknet. Ausbeute: 172 g (98% d. Th., Reinheit: 89%).
LC-MS (Methode 1A): $R_t$ = 0.92 min; MS (ESIpos): m/z = 365 $[M+H]^+$;
$^1$H-NMR (400 MHz, DMSO-$d_6$): $\delta$ [ppm] = 12.85 (br. s., 1H), 9.06 (d, 1H), 7.59 (s, 1H), 7.51-7.46 (m, 1H), 7.45-7.35 (m, 3H), 7.31 (d, 1H), 5.32-5.20 (m, 1H), 4.81-4.54 (m, 2H), 3.95 (s, 3H).

**Beispiel 14A**

3-Methylpiperidin-4-ol [racemisches *trans*-Diastereomer, 2 Isomere]

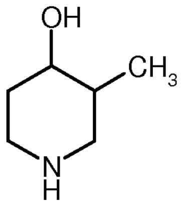

Es wurden 2.82 g (13.7 mmol) 1-Benzyl-3-methylpiperidin-4-ol [racemisches *trans*-Diastereomer, 2 Isomere; Lit.: M.-J. Blanco-Pilado et al., WO 2004/094380 A1] in Ethanol (250 mL) vorgelegt, mit 1.46 g Palladium auf Kohle (10%ig) versetzt und über Nacht bei RT und 3.5 bar WasserstoffAtmosphäre in einer Parr-Apparatur geschüttelt. Die Reaktionslösung wurde zur Entfernung des Katalysators filtriert und das Filtrat im Vakuum eingeengt. Ausbeute: 1.26 g (79% d. Th.).
GC-MS (Methode 2B): $R_t$ = 2.23 min; MS (EIpos): m/z = 115 $[M]^+$;
$^1$H-NMR (400 MHz, DMSO-$d_6$): $\delta$ [ppm] = 4.48 (br. s., 1H), 2.95-2.75 (m, 3H), 2.37 (td, 1H), 2.05-1.96 (m, 1H), 1.68 ($m_c$, 1H), 1.27-1.13 (m, 2H), 0.83 (d, 3H).

**Beispiel 15A**

*N*-Benzyl-2-chlor-*N*-(1,3-dihydroxypropan-2-yl)propanamid [Racemat]

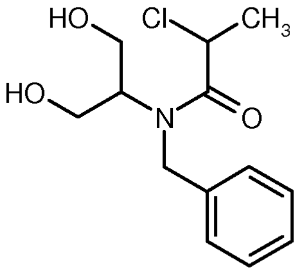

Es wurden 60.5 g (334 mmol) 2-(Benzylamino)propan-1,3-diol [Lit.: W. Lacôte et al., Org. Lett. 2011, 13, 5990-5993] in *iso*-Propanol (0.93 L) vorgelegt, auf 0°C abgekühlt und mit 50.7 g (69.8 mL, 501 mmol) Triethylamin versetzt. Anschließend wurden 50.9 g (38.9 mL, 401 mmol) 2-Chlorpropionylchlorid [Racemat] zugetropft. Man ließ die Reaktionslösung auf RT erwärmen und engte anschließend im Vakuum ein. Der Rückstand wurde mit 0.5 N wässriger Hydrogenchlorid-Lösung versetzt und mit Dichlormethan extrahiert. Die organischen Phasen wurden über Natriumsulfat getrocknet, filtriert und im Vakuum eingeengt. Das Rohprodukt wurde ohne weitere Reinigung in der nächsten Stufe eingesetzt. Ausbeute: 91.7 g (94% d. Th.).
LC-MS (Methode 1A): $R_t$ = 0.71 min; MS (ESIpos): m/z = 272 $[M+H]^+$.

**Beispiel 16A**

4-Benzyl-5-(hydroxymethyl)-2-methylmorpholin-3-on [Diastereomerengemisch, 4 Isomere]

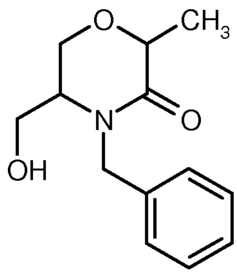

Es wurden 81.3 g (272 mmol, Reinheit: 91%) *N*-Benzyl-2-chlor-*N*-(1,3-dihydroxypropan-2-yl)propanamid [Racemat] in *iso*-Propanol (600 mL) vorgelegt, auf 0°C gekühlt und mit 91.6 g (817 mmol) Kalium-*tert*-butylat versetzt. Man ließ die Reaktionslösung auf RT erwärmen und rührte über Nacht bei RT. *Iso*-Propanol wurde im Vakuum weitgehend entfernt und der Rückstand in Dichlormethan aufgenommen. Es wurde mit Wasser gewaschen, die organische Phase über Magnesiumsulfat getrocknet, filtriert und im Vakuum eingeengt. Das Rohprodukt wurde ohne weitere Reinigung in der nächsten Stufe eingesetzt. Ausbeute: 61.7 g (96% d. Th., Diastereomerenverhältnis ca. 7:3).
LC-MS (Methode 2A): $R_t$ = 0.61 min (Diastereomer 1, 2 Isomere), $R_t$ = 0.62 min (Diastereomer 2, 2 Isomere);
MS (ESIpos): m/z = 236 $[M+H]^+$.

**Beispiel 17A**

4-Benzyl-5-({[*tert*-butyl(dimethyl)silyl]oxy}methyl)-2-methylmorpholin-3-on [Diastereomerengemisch, 4 Isomere]

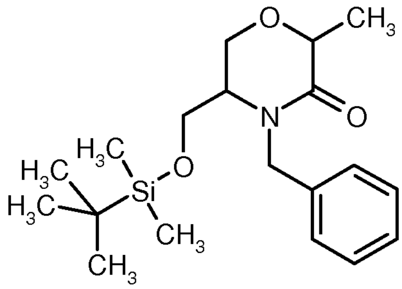

Es wurden 21.5 g (91.4 mmol) 4-Benzyl-5-(hydroxymethyl)-2-methylmorpholin-3-on [Diastereomerengemisch, 4 Isomere] in *N,N*-Dimethylformamid (126 mL) vorgelegt und bei RT mit 12.4 g (183 mmol) Imidazol und anschließend 14.5 g (96.0 mmol) *tert*-Butyldimethylsilylchlorid versetzt. Es wurde 2 h gerührt und nachfolgend das Lösungsmittel weitgehend im Vakuum entfernt. Der Rückstand wurde in Essigsäureethylester/Wasser aufgenommen und die organische Phase mit Wasser, 0.4 N wässriger Hydrogenchlorid-Lösung, gesättigter wässriger Natrium-hydrogencarbonat-Lösung und Wasser gewaschen. Die organische Phase wurde über Natriumsulfat getrocknet, filtriert und im Vakuum eingeengt. Das Rohprodukt wurde ohne weitere Reinigung in der nächsten Stufe eingesetzt. Ausbeute: 31.2 g (97% d. Th., Diastereomerenverhältnis ca. 7:3).
LC-MS (Methode 1A): $R_t$ = 1.41 min; MS (ESIpos): m/z = 350 $[M+H]^+$;
$^1$H-NMR (400 MHz, DMSO-$d_6$): $\delta$ [ppm] = 7.38-7.18 (m, 5H), 5.00 (d, 0.3H), 4.95 (d, 0.7H), 4.32-4.19 (m, 2H), 3.92-3.85 (m, 1H), 3.75-3.62 (m, 3H), 3.32-3.26 (m, 0.3H), 3.19-3.13 (m., 0.7H), 1.35 (d, 0.9H), 1.32 (d, 2.1H), 0.84-0.80 (m, 9H), 0.04--0.03 (m, 6H).

**Beispiel 18A**

4-Benzyl-5-({[*tert*-butyl(dimethyl)silyl]oxy}methyl)-2,2-dimethylmorpholin-3-on [Racemat]

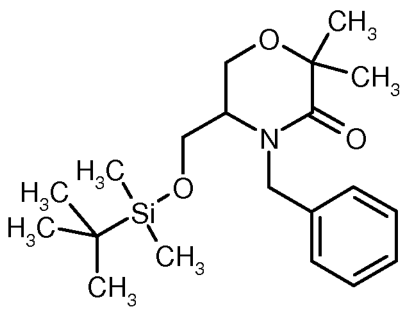

Es wurden 17.0 g (70.7 mmol) 4-Benzyl-5-({[*tert*-butyl(dimethyl)silyl]oxy}methyl)-2-methylmorpholin-3-on [Diastereomerengemisch, 4 Isomere] in Tetrahydrofuran (340 mL) vorgelegt und bei -78°C mit 32.4 mL (58.4 mmol) Lithiumdiisopropylamid-Lösung (1.8 M in Tetrahydrofuran/*n*-Heptan/Ethylbenzol) versetzt. Es wurde langsam auf 0°C erwärmt und anschließend mit 8.97 g (3.94 mL, 63.2 mmol) Iodmethan versetzt. Nach 1.5 h wurde erneut auf-78°C abgekühlt und es wurden 5.40 mL (9.73 mmol) Lithiumdiisopropylamid-Lösung (1.8 M in Tetrahydrofuran/n-Heptan/Ethylbenzol) zugeben. Dann wurde auf 0°C erwärmt und mit 2.07 g (0.91 mL, 14.6 mmol) Iodmethan versetzt. Nach 1 h wurde die Reaktionslösung unter Kühlung mit Wasser versetzt, Tetrahydrofuran im Vakuum entfernt, der Rückstand in Essigsäureethylester aufgenommen und dann mit Wasser sowie gesättigter wässriger Natriumchlorid-Lösung gewaschen. Die organische Phase wurde über Natriumsulfat getrocknet, filtriert und im Vakuum eingeengt. Das Rohprodukt wurde ohne weitere Reinigung in der nächsten Stufe eingesetzt. Ausbeute: 19.8 g (98% d. Th., Reinheit: 88%).
LC-MS (Methode 1A): $R_t$ = 1.45 min; MS (ESIpos): m/z = 364 $[M+H]^+$.

**Beispiel 19A**

4-Benzyl-5-(hydroxymethyl)-2,2-dimethylmorpholin-3-on [Racemat]

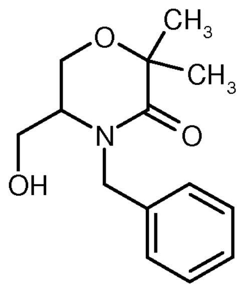

Es wurden 18.1 g (43.8 mmol, Reinheit: 88%) 4-Benzyl-5-({[*tert*-butyl(dimethyl)silyl]oxy} methyl)-2,2-dimethylmorpholin-3-on [Racemat] in Tetrahydrofuran (329 mL) vorgelegt, bei RT mit 110 mL (110 mmol) Tetra-*n*-butylammoniumfluorid-Lösung (1.0 M in Tetrahydrofuran) versetzt und über Nacht gerührt. Dann wurde die Reaktionslösung im Vakuum eingeengt, der Rückstand in Essigsäureethylester aufgenommen und mit Wasser gewaschen. Die organische Phase wurde über Natriumsulfat getrocknet, filtriert und im Vakuum eingeengt. Das Rohprodukt wurde durch Säulenchromatographie an Kieselgel (Dichlormethan, Dichlormethan/Methanol 100:3) gereinigt. Ausbeute: 9.99 g (89% d. Th.).
LC-MS (Methode 1A): $R_t$ = 0.73 min; MS (ESIpos): m/z = 250 $[M+H]^+$;
$^1$H-NMR (400 MHz, DMSO-$d_6$): $\delta$ [ppm] = 7.43-7.35 (m, 2H), 7.34-7.21 (m, 3H), 5.09-4.98 (m, 2H), 4.23 (d, 1H), 3.90-3.75 (m, 2H), 3.65-3.55 (m, 2H), 3.15 (br. t., 1H), 1.42 (s, 3H), 1.39 (s, 3H).

**Beispiel 20A**

(4-Benzyl-6,6-dimethylmorpholin-3-yl)methanol [Racemat]

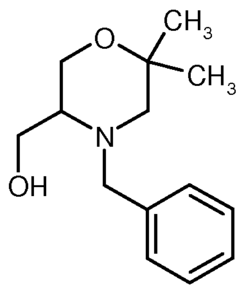

Es wurden 1.00 g (4.01 mmol) 4-Benzyl-5-(hydroxymethyl)-2,2-dimethylmorpholin-3-on [Racemat] in Tetrahydrofuran (39 mL) vorgelegt, unter Argon mit 8.02 mL (16.0 mmol) 2 M Boran-Dimethylsulfid-Komplex-Lösung in Tetrahydrofuran versetzt und 2 h unter Rückfluss gerührt. Nachfolgend wurde auf 0°C gekühlt, vorsichtig mit Methanol (10 mL) versetzt und 30 min unter Rückfluss gerührt. Anschließend wurde im Vakuum vollständig eingeengt, der Rückstand

in Acetonitril aufgenommen und mittels präparativer RP-HPLC (Acetonitril/Wasser, isokratisch) gereinigt. Ausbeute: 587 mg (58% d. Th.).
LC-MS (Methode 5A): $R_t$ = 2.19 min; MS (ESIpos): m/z = 236 $[M+H]^+$.

**Beispiel 21A**

(6,6-Dimethylmorpholin-3-yl)methanol [Racemat]

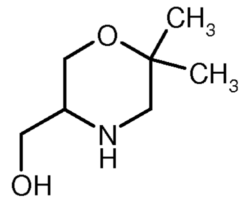

Es wurden 587 mg (2.49 mmol) (4-Benzyl-6,6-dimethylmorpholin-3-yl)methanol [Racemat] in Ethanol (20 mL) vorgelegt und unter Argon mit 58.7 mg Palladium auf Kohle (10%ig) und 29.4 mg Palladiumhydroxid auf Kohle (20%ig) versetzt und anschließend über Nacht unter Wasserstoffatmosphäre unter Normaldruck gerührt. Die Reaktionslösung wurde über Kieselgur filtriert und der Rückstand mit Ethanol gewaschen. Es wurde im Vakuum eingeengt und das Produkt am Hochvakuum getrocknet. Ausbeute: 385 mg (quant.).
LC-MS (Methode 6A): $R_t$ = 0.68 min; MS (ESIpos): m/z = 146 $[M+H]^+$;
$^1$H-NMR (400 MHz, DMSO-$d_6$): $\delta$ [ppm] = 4.54 (t, 1H), 3.46 (dd, 1H), 3.30-3.22 (m, 3H), 2.62-2.55 (m, 2H), 2.45 (d, 1H), 2.17 (br. s., 1H), 1.17 (s, 3H), 1.04 (s, 3H).

**Beispiel 22A**

*N*-Benzyl-2-chlor-*N*-[(*2R*)-1-hydroxypropan-2-yl]propanamid [Diastereomerengemisch, 2 Isomere]

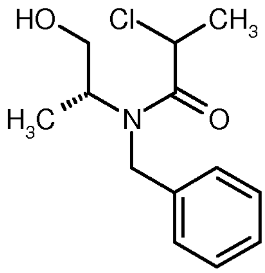

Es wurden 16.4 g (99.3 mmol) (*2R*)-2-(Benzylamino)propan-1-ol [Lit.: T. J. Tewson et al., Synthesis 2002, 6, 766-770] in *iso*-Propanol (500 mL) vorgelegt, auf 0°C abgekühlt und mit 20.1 g (27.7 mL, 199 mmol) Triethylamin versetzt. Anschließend wurden 13.9 g (10.8 mL, 109 mmol) 2-Chlorpropionylchlorid [Racemat] zugetropft, man ließ die Reaktionslösung auf RT erwärmen, rührte über Nacht und engte anschließend im Vakuum ein. Der Rückstand wurde mit 0.5 N wässriger Hydrogenchlorid-Lösung versetzt und mit Essigsäureethylester extrahiert. Die organischen Phasen wurden über Natriumsulfat getrocknet, filtriert und im Vakuum eingeengt. Das Rohprodukt wurde ohne weitere Reinigung in der nächsten Stufe eingesetzt. Ausbeute: 24.3 g (88% d. Th., Reinheit: 92%, Diastereomerenverhältnis ca. 1:1).
LC-MS (Methode 1A): $R_t$ = 0.80 min (Diastereomer 1), $R_t$ = 0.84 min (Diastereomer 2);
MS (ESIpos): m/z = 256 $[M+H]^+$.

**Beispiel 23A**

(*5R*)-4-Benzyl-2,5-dimethylmorpholin-3-on [Diastereomerengemisch, 2 Isomere]

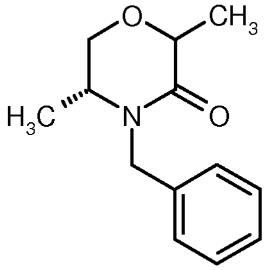

Es wurden 30.0 g (109 mmol, Reinheit: 93%) *N*-Benzyl-2-chlor-*N*-[(*2R*)-1-hydroxypropan-2-yl]propanamid [Diastereomerengemisch, 2 Isomere] in *iso*-Propanol (588 mL) vorgelegt, auf 0°C gekühlt und mit 49.0 g (436 mmol) Kalium-*tert*-butylat versetzt. Man ließ die Reaktionslösung auf RT erwärmen und rührte über Nacht. *Iso*-Propanol wurde im Vakuum weitgehend entfernt und der Rückstand in Wasser aufgenommen. Es wurde mit Essigsäureethylester extrahiert, die organischen Phasen über Magnesiumsulfat getrocknet, filtriert und im Vakuum eingeengt. Das Rohprodukt wurde ohne weitere Reinigung in der nächsten Stufe eingesetzt. Ausbeute: 22.8 g (93% d. Th.).
LC-MS (Methode 1A): $R_t$ = 0.85 min; MS (ESIpos): m/z = 220 $[M+H]^+$.

**Beispiel 24A**

(*5R*)-4-Benzyl-2,5-dimethylmorpholin [enantiomerenreine Isomere 1+2]

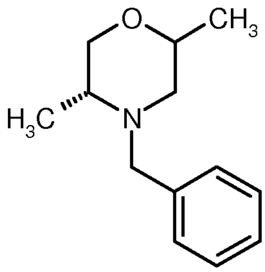

Es wurden 27.0 g (123 mmol) (*5R*)-4-Benzyl-2,5-dimethylmorpholin-3-on [Diastereomerengemisch, 2 Isomere] in Tetrahydrofuran (400 mL) vorgelegt, unter Argon mit 184 mL (369 mmol) 2 M Boran-Dimethylsulfid-Komplex-Lösung in Tetrahydrofuran versetzt und 2 h unter Rückfluss gerührt. Nachfolgend wurde der Ansatz auf 0°C gekühlt, vorsichtig mit Methanol (200 mL) versetzt und 2 h unter Rückfluss gerührt. Anschließend wurde im Vakuum vollständig eingeengt, der Rückstand in Acetonitril aufgenommen und mittels präparativer RP-HPLC (Acetonitril/Wasser, isokratisch) gereinigt und in die Diastereomeren getrennt. Man erhielt 2.60 g (10% d. Th.) des zuerst eluierenden enantiomerenreinen Diastereomers 1 sowie 9.00 g (35% d. Th.) des später eluierenden enantiomerenreinen Diastereomers 2.

Enantiomerenreines Isomer 1:

LC-MS (Methode 6A): $R_t$ = 2.30 min; MS (ESIpos): m/z = 206 $[M+H]^+$;

Enantiomerenreines Isomer 2:

LC-MS (Methode 6A): $R_t$ = 2.46 min; MS (ESIpos): m/z = 206 $[M+H]^+$.

**Beispiel 25A**

(*5R*)-2,5-Dimethylmorpholin Hydrochlorid [enantiomerenreines Isomer 1]

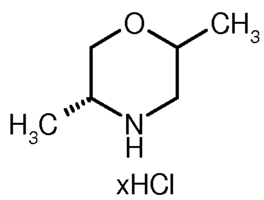

Es wurden 2.60 g (12.7 mmol) (*5R*)-4-Benzyl-2,5-dimethylmorpholin (Beispiel 24A, enantiomerenreines Isomer 1) in Ethanol (127 mL) vorgelegt und mit 2 N wässriger Hydrogenchlorid-Lösung (10.0 mL) versetzt. Unter Argon wurden 363 mg Palladium auf Kohle (10%ig) und 181 mg Palladiumhydroxid auf Kohle (20%ig) zugegeben und anschließend über Nacht unter Wasserstoffatmosphäre unter Normaldruck gerührt. Die Reaktionslösung wurde über Kieselgur filtriert und der Filterrückstand mit Ethanol gewaschen. Es wurde im Vakuum eingeengt und das gewünschte Produkt am Hochvakuum getrocknet. Ausbeute: 2.35 g (quant.).
MS (Methode 1C): m/z = 116 $[M+H-HCl]^+$;
$^1$H-NMR (400 MHz, DMSO-$d_6$): $\delta$ [ppm] = 9.80-9.34 (br. M., 2H), 3.94-3.71 (m, 2H), 3.48-3.38 (m, 1H), 3.23-3.11 (d, 2H), 2.66 (br. q., 1H), 1.15 (d, 3H), 1.11 (d, 3H).

**Beispiel 26A**

(*5R*)-2,5-Dimethylmorpholin Hydrochlorid [enantiomerenreines Isomer 2]

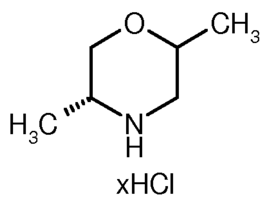

Es wurden 9.00 g (43.8 mmol) (*5R*)-4-Benzyl-2,5-dimethylmorpholin (Beispiel 24A, enantiomerenreines Isomer 2) in Ethanol (441 mL) vorgelegt und mit 2 N wässriger Hydrogenchlorid-Lösung (40.0 mL) versetzt. Unter Argon wurden 1.26 g Palladium auf Kohle (10%ig) und 628 mg Palladiumhydroxid auf Kohle (20%ig) zugegeben und anschließend über Nacht unter Wasserstoffatmosphäre unter Normaldruck gerührt. Die Reaktionslösung wurde über Kieselgur filtriert und der Filterrückstand mit Ethanol nachgewaschen. Es wurde im Vakuum eingeengt und das Produkt am Hochvakuum getrocknet. Ausbeute: 7.83 g (quant.).
MS (Methode 1C): m/z = 116 $[M+H-HCl]^+$;
$^1$H-NMR (400 MHz, DMSO-$d_6$): $\delta$ [ppm] = 9.97 (br. s., 1H), 9.43 (br. s., 1H), 3.90-3.71 (m, 2H), 3.62 (d, 1H), 3.40 (d, 1H), 3.06-2.91 (m, 1H), 2.89-2.71 (m, 1H), 1.32 (d, 3H), 1.14 (d, 3H).

**Beispiel 27A**

(*5R*)-2-Allyl-4-benzyl-2,5-dimethylmorpholin-3-on [Diastereomerengemisch, 2 Isomere]

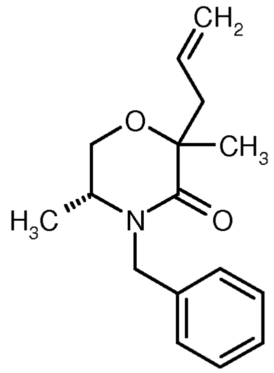

Es wurden 22.8 g (104 mmol) (*5R*)-4-Benzyl-2,5-dimethylmorpholin-3-on [Diastereomerengemisch, 2 Isomere] in Tetrahydrofuran (1.34 L) vorgelegt, unter Argon bei -78°C mit 146 mL (146 mmol) 1 M Lithiumhexamethyldisilazid-Lösung in Tetrahydrofuran versetzt und 15 min gerührt. Nachfolgend wurde bei -78°C 21.0 g (11.4 mL, 125 mmol) Allyliodid zugegeben, das Reaktionsgemisch ließ man auf RT erwärmen und rührte 3 h. Die Reaktion wurde durch Zugabe von gesättigter wässriger Ammoniumchlorid-Lösung beendet und anschließend wurde mit Essigsäureethylester extrahiert. Die organische Phase wurde mit gesättigter wässriger Natriumchlorid-Lösung gewaschen, über Natriumsulfat getrocknet, filtriert und im Vakuum eingeengt. Das Rohprodukt wurde ohne weitere Reinigung in der nächsten Stufe eingesetzt. Ausbeute: 27.5 g (77% d. Th., Reinheit: 75%).
LC-MS (Methode 1A): $R_t$ = 0.99 min; MS (ESIpos): m/z = 260 $[M+H]^+$.

**Beispiel 28A**

[(*5R*)-4-Benzyl-2,5-dimethyl-3-oxomorpholin-2-yl]acetaldehyd [Diastereomerengemisch, 2 Isomere]

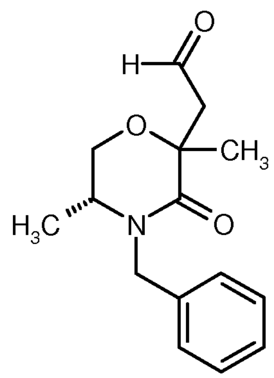

Es wurden 27.4 g (79.9 mmol, Reinheit: 75%) (*5R*)-2-Allyl-4-benzyl-2,5-dimethylmorpholin-3-on [Diastereomerengemisch, 2 Isomere] in Tetrahydrofuran (620 mL) und Wasser (370 mL) vorgelegt und bei 0°C mit 4.35 mL (1.60 mmol) 2.5%ige Osmiumtetroxid-Lösung in *tert*-Butanol und 51.2 g (240 mmol) Natriumperiodat versetzt. Man ließ die Reaktionslösung auf RT erwärmen und rührte über Nacht. Die Reaktionslösung wurde über Kieselgur filtriert und der Filterrückstand mit Tetrahydrofuran gewaschen. Es wurde im Vakuum eingeengt und der Rückstand in Essigsäureethylester und Wasser aufgenommen. Nach Trennung der Phasen wurde die organische Phase mit 1 N wässriger Natriumsulfit-Lösung (2 x 400 mL) gewaschen, über Natriumsulfat getrocknet, filtriert und im Vakuum eingeengt. Das Rohprodukt wurde ohne weitere Reinigung in der nächsten Stufe eingesetzt. Ausbeute: 23.6 g Rohprodukt.
LC-MS (Methode 1A): $R_t$ = 0.81 min (Diastereomer 1), $R_t$ = 0.84 min (Diastereomer 2);
MS (ESIpos): m/z = 262 $[M+H]^+$.

**Beispiel 29A**

(*5R*)-4-Benzyl-2-(2-hydroxyethyl)-2,5-dimethylmorpholin-3-on [Diastereomerengemisch, 2 Isomere]

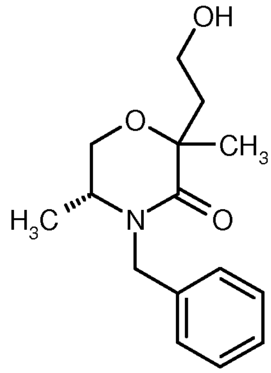

Es wurden 7.00 g (ca. 26.8 mmol, Rohprodukt) [(*5R*)-4-Benzyl-2,5-dimethyl-3-oxomorpholin-2-yl]acetaldehyd [Diastereomerengemisch, 2 Isomere] in Methanol (200 mL) vorgelegt und bei 0°C mit 3.04 g (80.4 mmol) Natriumborhydrid versetzt. Man ließ die Reaktionslösung auf RT erwärmen und rührte 30 min. Die Reaktionslösung wurde mit Wasser versetzt, das Methanol im Vakuum weitgehend entfernt und der Rückstand mit Essigsäureethylester extrahiert. Die organische Phase wurde über Natriumsulfat getrocknet, filtriert und im Vakuum eingeengt. Das Rohprodukt wurde mittels präparativer RP-HPLC (Acetonitril/Wasser) gereinigt. Ausbeute: 6.82 g (70% d. Th., Reinheit: 73%).
LC-MS (Methode 1A): $R_t$ = 0.71 min; MS (ESIpos): m/z = 264 $[M+H]^+$.

**Beispiel 30A**

2-[(*5R*)-4-Benzyl-2,5-dimethylmorpholin-2-yl]ethanol [enantiomerenreine Isomere 1+2]

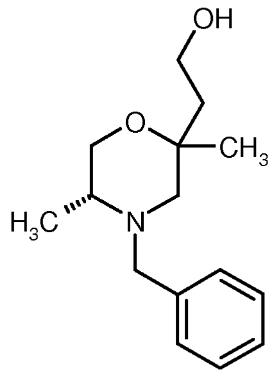

Es wurden 6.80 g (18.9 mmol, Reinheit: 73%) (*5R*)-4-Benzyl-2-(2-hydroxyethyl)-2,5-dimethylmorpholin-3-on [Diastereomerengemisch, 2 Isomere] in Tetrahydrofuran (191 mL) vorgelegt, unter Argon mit 37.7 mL (75.4 mmol) 2 M Boran-Dimethylsulfid-Komplex-Lösung in Tetrahydrofuran versetzt und 2 h unter Rückfluss gerührt. Nachfolgend wurde auf 0°C gekühlt, vorsichtig mit Methanol (37 mL) versetzt und 30 min unter Rückfluss gerührt. Es wurde im Vakuum vollständig eingeengt, der Rückstand in Acetonitril aufgenommen und direkt mittels präparativer RP-HPLC (Acetonitril/Wasser, isokratisch) einer Reinigung und Diastereomerentrennung unterzogen. Enantiomerenreines Isomer 1 wurde als erste Verbindung eluiert. Ausbeute: 1.34 g (28% d. Th., enantiomerenreines Isomer 1). Enantiomerenreines Isomer 2 wurde als zweite Verbindung eluiert. Ausbeute: 2.28 g (47% d. Th., enantiomerenreines Isomer 2).

Enantiomerenreines Isomer 1:

LC-MS (Methode 4A): $R_t$ = 2.55 min; MS (ESIpos): m/z = 250 $[M+H]^+$;

Enantiomerenreines Isomer 2:

LC-MS (Methode 4A): $R_t$ = 2.64 min; MS (ESIpos): m/z = 250 $[M+H]^+$.

**Beispiel 31A**

2-[(*5R*)-2,5-Dimethylmorpholin-2-yl]ethanol [enantiomerenreines Isomer]

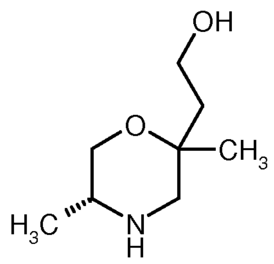

Es wurden 2.25 g (9.02 mmol) 2-[(*5R*)-4-Benzyl-2,5-dimethylmorpholin-2-yl]ethanol [enantiomerenreines Isomer 2] aus Beispiel 30A in Ethanol (90.7 mL) vorgelegt, unter Argon mit 227 mg Palladium auf Kohle (10%ig) und 113 mg Palladiumhydroxid auf Kohle (20%ig) versetzt und über Nacht unter Wasserstoffatmosphäre unter Normaldruck gerührt. Die Reaktionslösung wurde über Kieselgur filtriert und der Filterrückstand mit Ethanol gewaschen. Es wurde im Vakuum eingeengt und das Produkt am Hochvakuum getrocknet. Ausbeute: 1.46 g (quant.).
MS (Methode 1C): m/z = 160 $[M+H]^+$;
$^1$H-NMR (400 MHz, DMSO-$d_6$): δ [ppm] = 4.21 (t, 1H), 3.53-3.44 (d, 2H), 3.34 (dd, 1H), 3.14 (t, 1H), 2.65-2.52 (m, 3H), 2.07 (br. s., 1H), 1.52 (td, 2H), 1.18 (s, 3H), 0.85 (d, 3H).

**Beispiel 32A**

(*5R*)-4-Benzyl-2-(2-hydroxypropyl)-2,5-dimethylmorpholin-3-on [Diastereomerengemisch, 4 Isomere]

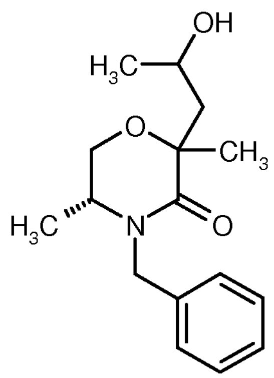

Es wurden 16.8 g (ca. 64.3 mmol, Rohprodukt) [(*5R*)-4-Benzyl-2,5-dimethyl-3-oxomorpholin-2-yl]acetaldehyd [Beispiel 28A, Diastereomerengemisch, 2 Isomere] in Tetrahydrofuran (275 mL) vorgelegt und bei -78°C mit 77.2 mL (77.2 mmol) 1 M Methylmagnesiumbromid-Lösung in Tetrahydrofuran versetzt. Es wurde 15 min bei -78°C gerührt und man ließ dann die Reaktionslösung auf RT erwärmen. Anschließend wurde die Reaktionslösung vorsichtig mit gesättigter wässriger Ammoniumchlorid-Lösung (400 mL) versetzt, das Tetrahydrofuran weitgehend im Vakuum entfernt und der Rückstand in Dichlormethan aufgenommen. Nach Trennung der Phasen wurde die organische Phase mit Wasser gewaschen, über Natriumsulfat getrocknet, filtriert und im Vakuum eingeengt. Das Rohprodukt wurde ohne weitere Reinigung in der nächsten Stufe eingesetzt. Ausbeute: 16.2 g Rohprodukt.
LC-MS (Methode 1A): $R_t$ = 0.78, 0.80 min; MS (ESIpos): m/z = 278 $[M+H]^+$.

**Beispiel 33A**

1-[(*5R*)-4-Benzyl-2,5-dimethylmorpholin-2-yl]propan-2-ol [enantiomerenreine Isomere 1+2+3+4]

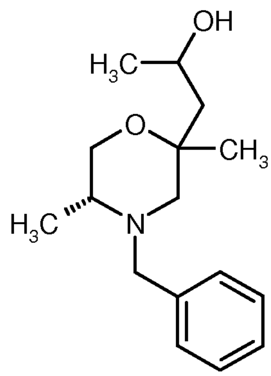

Es wurden 16.2 g (ca. 39.1 mmol, Rohprodukt) (*5R*)-4-Benzyl-2-(2-hydroxypropyl)-2,5-dimethylmorpholin-3-on [Diastereomerengemisch, 4 Isomere] in Tetrahydrofuran (397 mL) vorgelegt, unter Argon mit 78.3 mL (157 mmol) 2 M Boran-Dimethylsulfid-Komplex-Lösung in Tetrahydrofuran versetzt und für 2 h unter Rückfluss gerührt. Nachfolgend wurde der Ansatz auf 0°C gekühlt, vorsichtig mit Methanol (80 mL) versetzt und 30 min unter Rückfluss gerührt. Anschließend wurde im Vakuum vollständig eingeengt, der Rückstand in Acetonitril aufgenommen und direkt mittels präparativer RP-HPLC (Acetonitril/Wasser, isokratisch) einer Reinigung und Diastereomerentrennung unterzogen. Die Zielverbindung eluierte hierbei als dritte Komponente. Ausbeute: Zielverbindung: 3.11 g (29% d. Th.; enantiomerenreines Isomer 3); enantiomerenreines Isomer 1: 2.12 g (20% d. Th.); enantiomerenreines Isomer 2: 506 mg (5% d. Th.); enantiomerenreines Isomer 4: 1.72 g (16% d. Th.).

Enantiomerenreines Isomer 3:

LC-MS (Methode 1A): $R_t$ = 0.39 min; MS (ESIpos): m/z = 264 $[M+H]^+$;

$^1$H-NMR (400 MHz, DMSO-$d_6$): $\delta$ [ppm] = 7.34-7.18 (m, 5H), 4.10 (d, 1H), 3.96 (d, 1H), 3.79 ($m_c$, 1H), 3.48 (dd, 1H), 3.36 ($m_c$, 1H), 3.04 (d, 1H), 2.46 (d, 1H), 2.28 ($m_c$, 1H), 1.88 (d, 1H), 1.44 (dd, 1H), 1.36 (dd, 1H), 1.23 (s, 3H), 1.01 (d, 3H), 0.98 (d, 3H).

Enantiomerenreines Isomer 1:

LC-MS (Methode 1A): $R_t$ = 0.43 min; MS (ESIpos): m/z = 264 $[M+H]^+$;

$^1$H-NMR (400 MHz, DMSO-$d_6$): $\delta$ [ppm] = 7.33-7.18 (m, 5H), 4.16 (d, 1H), 3.90 (d, 1H), 3.76 ($m_c$, 1H), 3.50 (dd, 1H), 3.26 (dd, 1H), 3.10 (d, 1H), 2.43 (d, 1H), 2.32 ($m_c$, 1H), 2.10 (dd, 1H), 1.84 (d, 1H), 1.27 (dd, 1H), 1.09-1.06 (m, 6H), 0.98 (d, 3H).

Enantiomerenreines Isomer 2:

LC-MS (Methode 1A): $R_t$ = 0.45 min; MS (ESIpos): m/z = 264 $[M+H]^+$;

$^1$H-NMR (400 MHz, DMSO-$d_6$): $\delta$ [ppm] = 7.32-7.20 (m, 5H), 4.11 (d, 1H), 3.92 (d, 1H), 3.57 ($m_c$, 1H), 3.51 (dd, 1H), 3.41 (dd, 1H), 3.06 (d, 1H), 2.47 (d, 1H), 2.34 ($m_c$, 1H), 1.85-1.74 (m, 2H), 1.59 (dd, 1H), 1.06 (s, 3H), 1.03-0.97 (t, 6H).

Enantiomerenreines Isomer 4:

LC-MS (Methode 1A): $R_t$ = 0.44 min; MS (ESIpos): m/z = 264 $[M+H]^+$;

$^1$H-NMR (400 MHz, DMSO-$d_6$): $\delta$ [ppm] = 7.49 (s, 5H), 4.69 (d, 1H), 4.28-4.15 (m, 2H), 3.86-3.73 (m, 3H), 3.63

(t, 1H), 3.32 (t, 1H), 3.21 (br. s., 1H), 2.84 (d, 1H), 1.52-1.38 (m, 4H), 1.28 (s, 3H), 1.01 (d, 3H).

**Beispiel 34A**

1-[(*5R*)-2,5-Dimethylmorpholin-2-yl]propan-2-ol [enantiomerenreines Isomer]

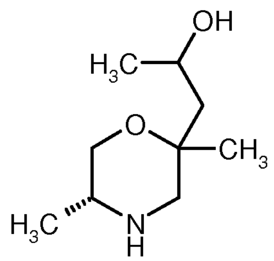

Es wurden 3.10 g (11.8 mmol) 1-[(*5R*)-4-Benzyl-2,5-dimethylmorpholin-2-yl]propan-2-ol [Beispiel 33A, enantiomerenreines Isomer 3] in Ethanol (118 mL) vorgelegt, unter Argon mit 296 mg Palladium auf Kohle (10%ig) und 148 mg Palladiumhydroxid auf Kohle (20%ig) versetzt und anschließend über Nacht unter Wasserstoffatmosphäre unter Normaldruck gerührt. Die Reaktionslösung wurde über Kieselgur filtriert und der Filterrückstand mit heißem Ethanol (100 mL) gewaschen. Es wurde im Vakuum eingeengt und das Produkt am Hochvakuum getrocknet. Ausbeute: 2.06 g (quant.).
GC-MS (Methode 1B): $R_t$ = 3.86 min; MS (EIpos): m/z = 173 $[M]^+$;
$^1$H-NMR (400 MHz, DMSO-$d_6$): $\delta$ [ppm] = 4.20 (d, 1H), 3.87 (br. s., 1H), 3.35 (dd, 1H), 3.16 (t, 1H), 2.67-2.53 (m, 3H), 2.05 (br. s., 1H), 1.44 (dd, 1H), 1.36 (dd, 1H), 1.23 (s, 3H), 1.04 (d, 3H), 0.85 (d, 3H).

**Beispiel 35A**

*N*-Benzyl-2-chlor-*N*-(1,4-dihydroxybutan-2-yl)propanamid [Diastereomerengemisch, 4 Isomere]

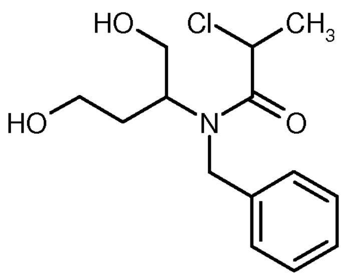

Es wurden 20.6 g (106 mmol) 2-(Benzylamino)butan-1,4-diol [Racemat] [Lit.: B. L. Feringa, B. de Lange, Heterocycles 1988, 27, 1197-1205] in *iso*-Propanol (500 mL) vorgelegt, auf 0°C abgekühlt und mit 21.4 g (29.4 mL, 211 mmol) Triethylamin versetzt. Anschließend wurden 16.1 g (12.6 mL, 127 mmol) 2-Chlorpropionylchlorid [Racemat] zugetropft. Nach 30 min Rühren wurden weitere 10.4 g (8.37 mL, 84.4 mmol) 2-Chlorpropionylchlorid [Racemat] zugetropft und man ließ die Reaktionslösung auf RT erwärmen und engte anschließend im Vakuum ein. Der Rückstand wurde in Essigsäureethylester (500 mL) aufgenommen und mit 0.5 N wässriger Hydrogenchlorid-Lösung (400 mL) gewaschen. Die wässrige Phase wurde mehrfach mit Essigsäureethylester extrahiert. Die organischen Phasen wurde über Natriumsulfat getrocknet, filtriert und im Vakuum eingeengt. Das Rohprodukt wurde ohne weitere Reinigung in der nächsten Stufe eingesetzt. Ausbeute: 37.5 g (78% d. Th., Reinheit: 63%, Diastereomerenverhältnis ca. 2:1).
LC-MS (Methode 1A): $R_t$ = 0.71 min (Diastereomer 1, 2 Isomere), $R_t$ = 0.72 min (Diastereomer 2, 2 Isomere);
MS (ESIpos): m/z = 286 $[M+H]^+$.

**Beispiel 36A**

4-Benzyl-5-(2-hydroxyethyl)-2-methylmorpholin-3-on [Diastereomerengemisch, 4 Isomere]

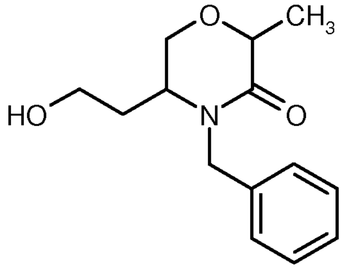

Es wurden 37.5 g (82.5 mmol, Reinheit: 63%) *N*-Benzyl-2-chlor-*N*-(1,4-dihydroxybutan-2-yl)propanamid [Diastereomerengemisch, 4 Isomere] in *iso*-Propanol (500 mL) vorgelegt und auf 0°C gekühlt. Dann wurden 73.5 g (655 mmol) Kalium-*tert*-butylat in einer Portion zugegeben und es wurde für 1 h bei 0°C gerührt. *Iso*-Propanol wurde im Vakuum weitgehend entfernt, der Rückstand in Essigsäureethylester aufgenommen und mit 1 N wässriger Hydrogenchlorid-Lösung (400 mL) gewaschen. Die organische Phase wurde über Natriumsulfat getrocknet, filtriert und im Vakuum eingeengt. Das Rohprodukt wurde ohne weitere Reinigung in der nächsten Stufe eingesetzt. Ausbeute: 28.8 g (quant., Reinheit: 82%, Diastereomerenverhältnis ca. 2.5:1).
LC-MS (Methode 7A:): $R_t$ = 1.42 min (Diastereomer 1, 2 Isomere), $R_t$ = 1.46 min (Diastereomer 2, 2 Isomere); MS (ESIpos): m/z = 250 $[M+H]^+$.

**Beispiel 37A**

2-(4-Benzyl-6-methylmorpholin-3-yl)ethanol [Racemat]

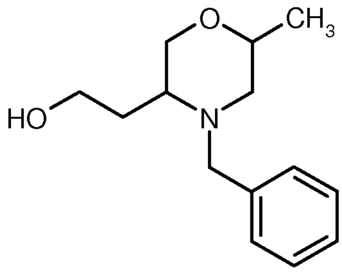

Es wurden 28.8 g (94.7 mmol, Reinheit: 82%) 4-Benzyl-5-(2-hydroxyethyl)-2-methylmorpholin-3-on [Diastereomerengemisch, 4 Isomere] in Tetrahydrofuran (800 mL) vorgelegt, unter Argon mit 231 mL (462 mmol) 2 M Boran-Dimethylsulfid-Komplex-Lösung in Tetrahydrofuran versetzt und anschließend 2 h unter Rückfluss gerührt. Nachfolgend wurde der Ansatz auf 0°C gekühlt, vorsichtig mit Methanol (220 mL) versetzt und 30 min unter Rückfluss gerührt. Anschließend wurde im Vakuum vollständig eingeengt, 6.0 g des Rückstandes in Acetonitril aufgenommen und mittels präparativer RP-HPLC (Acetonitril/Wasser, isokratisch) einer Reinigung und Diastereomerentrennung unterzogen. Die Zielverbindung eluierte hierbei als zweite Komponente (Diastereomer 2, 2 Isomere). Ausbeute: Diastereomer 2 (2 Isomere): 1.95 g; Diastereomer 1 (2 Isomere): 698 mg.

Diastereomer 2, 2 Isomere:

LC-MS (Methode 4A): $R_t$ = 2.33 min; MS (ESIpos): m/z = 236 $[M+H]^+$.

Diastereomer 1, 2 Isomere:

LC-MS (Methode 4A): $R_t$ = 2.23 min; MS (ESIpos): m/z = 236 $[M+H]^+$.

**Beispiel 38A**

2-(6-Methylmorpholin-3-yl)ethanol [Racemat]

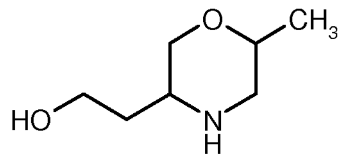

Es wurden 1.95 g (8.29 mmol) 2-(4-Benzyl-6-methylmorpholin-3-yl)ethanol [Diastereomer 2, 2 Isomere aus Beispiel 37A] in Ethanol (83 mL) vorgelegt, unter Argon mit 208 mg Palladium auf Kohle (10%ig) und 104 mg Palladiumhydroxid auf Kohle (20%ig) versetzt und anschließend über Nacht unter Wasserstoffatmosphäre unter Normaldruck gerührt. Die Reaktionslösung wurde über Kieselgur filtriert und der Filterrückstand mit Ethanol gewaschen. Es wurde im Vakuum eingeengt und das Produkt am Hochvakuum getrocknet. Ausbeute: 1.37 g (quant.).
MS (Methode 1C): m/z = 146 $[M+H]^+$;
$^1$H-NMR (400 MHz, DMSO-$d_6$): $\delta$ [ppm] = 3.53-3.41 (m, 5H), 2.69 ($m_c$, 1H), 2.60-2.43 (m, 2H), 1.82-1.69 (m, 1H), 1.58-1.44 (m, 1H), 1.03 (d, 3H), zwei Protonen nicht sichtbar.

**Beispiel 39A**

*N*-Benzyl-2-chlor-*N*-[(2*S*)-1,4-dihydroxybutan-2-yl]propanamid [Diastereomerengemisch, 2 Isomere]

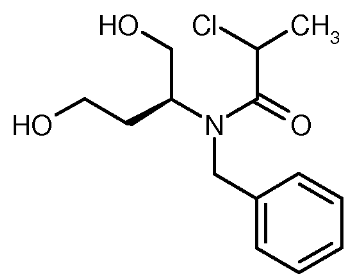

Es wurden 45.1 g (199 mmol, Reinheit: 86%) (2*S*)-2-(Benzylamino)butan-1,4-diol [F. Horiuchi, M. Matsui, Agr. Biol.Chem. 1973, 37, 1713-1716] in *iso*-Propanol (1.00 L) vorgelegt, auf 0°C abgekühlt und mit 40.2 g (55.4 mL, 397 mmol) Triethylamin versetzt. Anschließend wurden 37.8 g (29.6 mL, 298 mmol) 2-Chlorpropionylchlorid [Racemat] zugetropft. Nach 30 min Rühren wurden weitere 18.9 g (14.8 mL, 149 mmol) 2-Chlorpropionylchlorid [Racemat] zugetropft und man ließ die Reaktionslösung auf RT erwärmen und engte anschließend im Vakuum ein. Der Rückstand wurde in Essigsäureethylester (1.00 L) aufgenommen und mit Wasser gewaschen. Die organische Phase wurde über Natriumsulfat getrocknet, filtriert und im Vakuum eingeengt. Das Rohprodukt wurde ohne weitere Reinigung in der nächsten Stufe eingesetzt. Ausbeute: 71.8 g (quant., Reinheit: 82%, Diastereomerenverhältnis ca. 1:1).
LC-MS (Methode 1A): $R_t$ = 0.65 min (enantiomerenreines Isomer 1), $R_t$ = 0.67 min (enantiomerenreines Isomer 2);
MS (ESIpos): m/z = 286 $[M+H]^+$.

**Beispiel 40A**

(5*S*)-4-Benzyl-5-(2-hydroxyethyl)-2-methylmorpholin-3-on [Diastereomerengemisch, 2 Isomere]

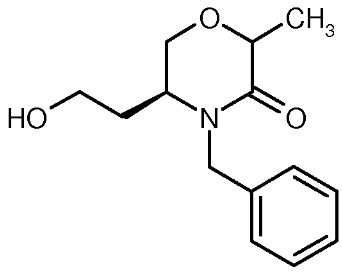

Es wurden 71.8 g (206 mmol, Reinheit: 82%) *N*-Benzyl-2-chlor-*N*-[(2*S*)-1,4-dihydroxybutan-2-yl]propanamid [Diastereomerengemisch, 2 Isomere] in *iso*-Propanol (1.30 L) vorgelegt und auf 0°C gekühlt. Dann wurden 92.4 g (824 mmol) Kalium-*tert*-butylat in einer Portion zugegeben, 30 min bei 0°C gerührt. Man ließ die Reaktionslösung auf RT erwärmen und entfernte *iso*-Propanol im Vakuum. Der Rückstand wurde in Essigsäureethylester (500 mL) aufgenommen. Es wurde mit Wasser (600 mL) versetzt, extrahiert und nach Phasentrennung die wässrige Phase mit Essigsäureethylester (2 x 300 mL) extrahiert. Die organischen Phasen wurden über Natriumsulfat getrocknet, filtriert und im Vakuum eingeengt. Das Rohprodukt wurde ohne weitere Reinigung in der nächsten Stufe eingesetzt. Ausbeute: 58.6 g (quant., Reinheit: 90%, Diastereomerenverhältnis ca. 3:2).
LC-MS (Methode 3A): $R_t$ = 1.51 min (enantiomerenreines Isomer 1), $R_t$ = 1.53 min (enantiomerenreines Isomer 2); MS (ESIpos): m/z = 250 $[M+H]^+$.

**Beispiel 41A**

2-[(3*S*)-4-Benzyl-6-methylmorpholin-3-yl]ethanol [enantiomerenreines Isomer]

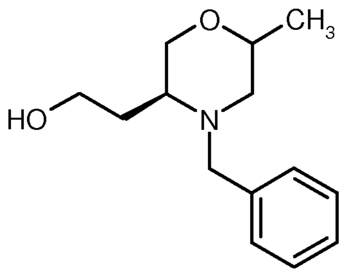

Es wurden 30.0 g (108 mmol) (5*S*)-4-Benzyl-5-(2-hydroxyethyl)-2-methylmorpholin-3-on [Diastereomerengemisch, 2 Isomere] in Tetrahydrofuran (1.10 L) vorgelegt, unter Argon mit 217 mL (433 mmol) 2 M Boran-Dimethylsulfid-Komplex-Lösung in Tetrahydrofuran versetzt und 2 h unter Rückfluss gerührt. Nachfolgend wurde der Ansatz auf 0°C gekühlt, vorsichtig mit Methanol (200 mL) versetzt und 30 min unter Rückfluss gerührt. Anschließend wurde im Vakuum vollständig eingeengt, der Rückstand in Acetonitril aufgenommen und mittels präparativer RP-HPLC (Acetonitril/Wasser, isokratisch) einer Reinigung und Diastereomerentrennung unterzogen. Die Zielverbindung eluierte hierbei als zweite Komponente (enantiomerenreines Isomer 2). Ausbeute: enantiomerenreines Isomer 2: 12.1 g (47% d. Th.); enantiomerenreines Isomer 1: 6.23 g (24% d. Th.).

Enantiomerenreines Isomer 2:

LC-MS (Methode 4A): $R_t$ = 2.33 min; MS (ESIpos): m/z = 236 $[M+H]^+$;

$^1$H-NMR (400 MHz, DMSO-$d_6$): $\delta$ [ppm] = 7.36-7.18 (m, 5H), 4.42 (t, 1H), 3.69-3.35 (m, 7H), 2.65-2.56 (m, 1H), 2.36-2.29 (m, 1H), 2.26-2.16 (m, 1H), 1.81-1.65 (m, 2H), 1.00 (d, 3H).

Enantiomerenreines Isomer 1:

LC-MS (Methode 4A): $R_t$ = 2.23 min; MS (ESIpos): m/z = 236 $[M+H]^+$;

$^1$H-NMR (400 MHz, DMSO-$d_6$): $\delta$ [ppm] = 7.37-7.19 (m, 5H), 4.49 (t, 1H), 4.10 (d, 1H), 3.76 (dd, 1H), 3.58-3.38

(m, 3H), 3.33-3.20 (m, 1H), 2.95 (d, 1H), 2.27 ($m_c$, 1H), 1.80 ($m_c$, 1H), 1.68 (dd, 1H), 1.48 ($m_c$, 1H), 0.94 (d, 3H).

**Beispiel 42A**

2-[(3*S*)-6-Methylmorpholin-3-yl]ethanol [enantiomerenreines Isomer]

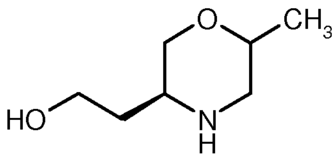

Es wurden 58.0 g (246 mmol) 2-[(3*S*)-4-Benzyl-6-methylmorpholin-3-yl]ethanol [enantiomerenreines Isomer 2, Beispiel 41A (Die eingesetzte Substanzmenge stammt aus mehreren Reaktionen in Analogie zu Beispiel 41A.)] in Ethanol (1.50 L) vorgelegt, unter Argon mit 2.90 g Palladium auf Kohle (10%ig) und 2.90 g Palladiumhydroxid auf Kohle (20%ig) versetzt und anschließend über Nacht unter Wasserstoffatmosphäre unter Normaldruck gerührt. Die Reaktionslösung wurde über Kieselgur filtriert und der Filterrückstand mit heißem Ethanol (100 mL) gewaschen. Es wurde im Vakuum eingeengt und das Produkt am Hochvakuum getrocknet. Ausbeute: 35.5 g (99% d. Th.).

Drehwert: $[\alpha]_D^{19.5} = 89.7°$ (c = 0.565, Chloroform);

LC-MS (Methode 5A): $R_t$ = 0.54 min; MS (ESIpos): m/z = 146 $[M+H]^+$;

LC-MS (Methode 1A): MS (ESIpos): m/z = 146 $[M+H]^+$;

$^1$H-NMR (400 MHz, $CDCl_3$): δ [ppm] = 3.88-3.80 (m, 2H), 3.71 (br. d., 1H), 3.63 (br. d., 1H), 3.50 ($m_c$, 1H), 3.12 (br. s., 2H), 2.94 ($m_c$, 1H), 2.81 (dd, 1H), 2.68 (dd, 1H), 2.39-2.25 (m, 1H), 1.45 ($m_c$, 1H), 1.15 (d, 3H).

**Beispiel 43A**

Methyl-[3-(benzyloxy)cyclobutyliden][(*tert*-butoxycarbonyl)amino]acetat

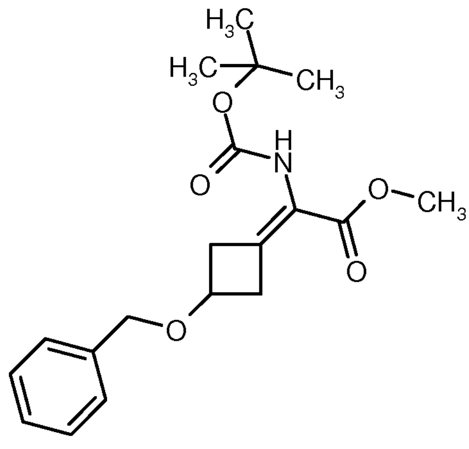

Es wurden 928 mg (3.12 mmol) Methyl-[(tert-butoxycarbonyl)amino](dimethoxyphosphoryl) acetat [Racemat] und 500 mg (2.84 mmol) 3-(Benzyloxy)cyclobutanon [K. Ogura, G. Tsuchihashi et al., Bull. Chem. Soc. Jpn. 1984, 57, 1637-1642] in Dichlormethan (50 mL) vorgelegt, bei RT mit 605 mg (0.590 mL, 3.97) 1,8-Diazabicyclo[5.4.0]undec-7-en versetzt und anschließend über Nacht gerührt. Die Reaktionslösung wurde im Vakuum eingeengt und der Rückstand in Essigsäureethylester aufgenommen. Die organische Phase wurde mit Wasser, 0.5 N wässriger Hydrogenchlorid-Lösung, gesättigter wässriger Natriumhydrogencarbonat-Lösung und gesättigter wässriger Natriumchlorid-Lösung gewaschen, über Natriumsulfat getrocknet, filtriert und im Vakuum eingeengt. Der Rückstand wurde mittels präparativer RP-HPLC (Acetonitril/Wasser) gereinigt. Ausbeute: 651 mg (60% d. Th.).
LC-MS (Methode 1A): $R_t$ = 1.15 min; MS (ESIpos): m/z = 348 $[M+H]^+$;

$^1$H-NMR (400 MHz, DMSO-$d_6$): δ [ppm] = 8.11 (br. s., 1H), 7.41-7.25 (m, 5H), 4.42 (s, 2H), 4.13 (quin, 1H), 3.63 (s, 3H), 3.25 (br. d., 1H), 2.99 (br. d., 1H), 2.85 (br. d., 1H), 2.65 (m, 1H), 1.37 (s, 9H).

**Beispiel 44A**

Methyl-[3-(benzyloxy)cyclobutyl][(*tert*-butoxycarbonyl)amino]acetat [*cis*- und *trans*-Isomerengemisch, 4 Isomere]

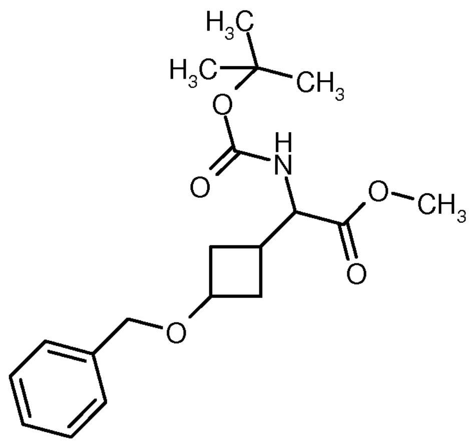

Es wurden 650 mg (1.87 mmol) Methyl-[3-(benzyloxy)cyclobutyliden][(*tert*-butoxycarbonyl)-amino]acetat und 455 mg (18.7 mmol) Magnesium-Späne in Methanol (50 mL) vorgelegt und bei RT 3 h im Ultraschallbad [Elma, Transsonic T 780] umgesetzt. Die Reaktionslösung wurde mit halbgesättigter wässriger Ammoniumchlorid-Lösung versetzt und mehrfach mit Dichlormethan extrahiert. Die organischen Phasen wurden über Natriumsulfat getrocknet, filtriert und im Vakuum eingeengt. Das Rohprodukt wurde ohne weitere Reinigung in der nächsten Stufe eingesetzt. Ausbeute: 630 mg (96% d. Th.).
LC-MS (Methode 1A): $R_t$ = 1.16 min; MS (ESIpos): m/z = 350 $[M+H]^+$, 250 $[M+H-COOC(CH_3)_3]$;
$^1$H-NMR (400 MHz, DMSO-$d_6$): δ [ppm] = 7.39-7.20 (m, 6H), 4.34 (s, 2H), 4.07 (quin, 0.3H), 3.99-3.73 (m, 1.7 H), 3.60 (s, 3H), 2.34-1.94 (m, 3.5H), 1.74-1.59 (m, 1.5H), 1.45-1.27 (m, 9H).

**Beispiel 45A**

*tert*-Butyl-{1-[3-(benzyloxy)cyclobutyl]-2-hydroxyethyl}carbamat [*cis*- und *trans*-Isomerengemisch, 4 Isomere]

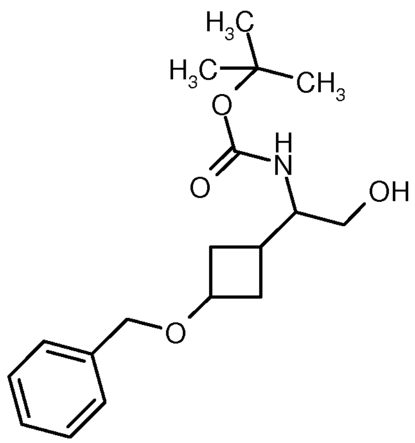

Es wurden 620 mg (1.77 mmol) Methyl-[3-(benzyloxy)cyclobutyl][(tert-butoxycarbonyl)amino]-acetat [*cis*- und *trans*-Isomerengemisch, 4 Isomere] in Tetrahydrofuran (6.0 mL) vorgelegt und bei 0°C mit 4.44 mL (8.87 mmol) 2 M Lithiumborhydrid-Lösung in Tetrahydrofuran versetzt. Anschließend wurde 4 h gerührt wobei man auf RT erwärmen

ließ. Die Reaktion wurde durch Zugabe von Essigsäureethylester (50.0 mL) beendet und die Reaktionslösung anschließend mit 0.5 N wässriger Hydrogenchlorid-Lösung gewaschen. Die organische Phase wurde über Natriumsulfat getrocknet, filtriert und im Vakuum eingeengt. Das Rohprodukt wurde ohne weitere Reinigung in der nächsten Stufe eingesetzt. Ausbeute: 560 mg (96% d. Th.).
LC-MS (Methode 1A): $R_t$ = 0.99 min; MS (ESIpos): m/z = 322 $[M+H]^+$, 222 [M+H-Boc];
$^1$H-NMR (400 MHz, DMSO-$d_6$): $\delta$ [ppm] = 7.47-7.15 (m, 5H), 6.65-6.41 (m, 1H), 4.46 (br. s., 0.5H), 4.33 (s, 2H), 3.88-3.70 (m, 0.7H), 3.67-3.09 (m, 3.8H), 2.36-1.78 (m, 3.5H), 1.74-1.48 (m, 1.5H), 1.38 (s, 9H).

**Beispiel 46A**

2-Amino-2-[3-(benzyloxy)cyclobutyl]ethanol Trifluoracetat [*cis-* und *trans*-Isomerengemisch, 4 Isomere]

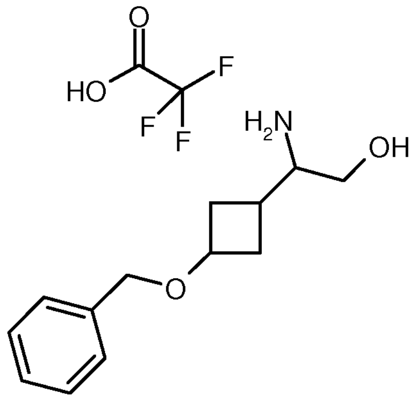

Es wurden 560 mg (1.74 mmol) *tert*-Butyl-{1-[3-(benzyloxy)cyclobutyl]-2-hydroxyethyl}carbamat [*cis-* und *trans*-Isomerengemisch, 4 Isomere] in Dichlormethan (8.0 mL) vorgelegt, bei RT mit 1.0 mL (12.9 mmol) Trifluoressigsäure versetzt und 2 h gerührt. Dann wurde die Reaktionslösung vollständig im Vakuum eingeengt und überschüssige Trifluoressigsäure durch mehrfache Koevaporation mit Dichlormethan entfernt. Das Rohprodukt wurde ohne weitere Reinigung in der nächsten Stufe eingesetzt. Ausbeute: 580 mg (95% d. Th.).
LC-MS (Methode 4A): $R_t$ = 2.10 min; MS (ESIpos): m/z = 222 $[M+H\text{-}TFA]^+$.

**Beispiel 47A**

*N*-{1-[3-(Benzyloxy)cyclobutyl]-2-hydroxyethyl}-2-chlorpropanamid [Diastereomerengemisch, 8 Isomere]

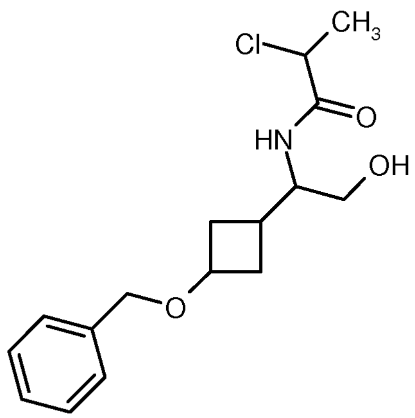

Es wurden 580 mg (1.73 mmol) 2-Amino-2-[3-(benzyloxy)cyclobutyl]ethanol Trifluoracetat [*cis-* und *trans*-Isomerengemisch, 4 Isomere] in *iso*-Propanol (15 mL) vorgelegt, auf 0°C abgekühlt und mit 700 mg (960 µL, 6.92 mmol)

Triethylamin versetzt. Anschließend wurden 242 mg (190 µL, 1.90 mmol) 2-Chlorpropionylchlorid [Racemat] zugetropft, 1 h bei 0°C gerührt und anschließend vollständig im Vakuum eingeengt. Der Rückstand wurde mit 0.5 N wässriger Hydrogenchlorid-Lösung (50 mL) versetzt und mehrfach mit Dichlormethan extrahiert. Die organischen Phasen wurden über Natriumsulfat getrocknet, filtriert und im Vakuum eingeengt. Das Rohprodukt wurde ohne weitere Reinigung in der nächsten Stufe eingesetzt. Ausbeute: 638 mg (91% d. Th., Reinheit: 77%).
LC-MS (Methode 4A): $R_t$ = 2.36 min; MS (ESIpos): m/z = 312 $[M+H]^+$.

**Beispiel 48A**

5-[3-(Benzyloxy)cyclobutyl]-2-methylmorpholin-3-on [Diastereomerengemisch, 8 Isomere]

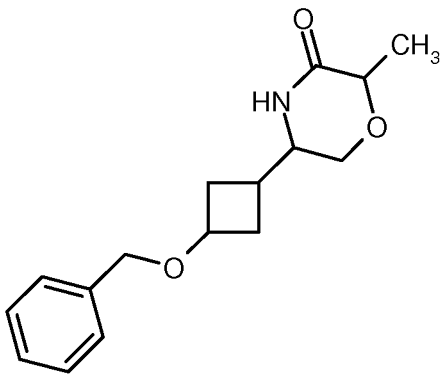

Es wurden 1.15 g (3.69 mmol) *N*-{1-[3-(Benzyloxy)cyclobutyl]-2-hydroxyethyl}-2-chlorpropanamid [Diastereomerengemisch, 8 Isomere] in *iso*-Propanol (30.0 mL) vorgelegt, auf 0°C gekühlt und anschließend 1.66 g (14.8 mmol) Kalium-*tert*-butylat in einer Portion zugegeben. Es wurde auf RT kommen gelassen und anschließend 1 h bei 50°C gerührt. Das *iso*-Propanol wurde im Vakuum weitgehend entfernt und der Rückstand in Essigsäureethylester aufgenommen. Die organische Phase wurde mit 1 N wässriger Hydrogenchlorid-Lösung gewaschen, über Natriumsulfat getrocknet, filtriert und im Vakuum eingeengt. Der Rückstand wurde mittels präparativer RP-HPLC (Acetonitril/Wasser) gereinigt. Ausbeute: 953 mg (93% d. Th.).
LC-MS (Methode 1A): $R_t$ = 0.88 min; MS (ESIpos): m/z = 276 $[M+H]^+$;
$^1$H-NMR (400 MHz, $CDCl_3$): δ [ppm] = 7.43-7.27 (m, 5H), 6.40 (br. s., 0.16H), 6.24 (br. s., 0.38H), 6.12-5.94 (m, 0.46H), 4.41 (s, 2H), 4.24-4.05 (m, 1.25H), 4.03-3.86 (m, 1.25H), 3.82-3.51 (m, 1.5H), 3.31-3.21 (m, 1H), 2.54-1.57 (m, 5H), 1.48-1.41 (m, 3H).

**Beispiel 49A**

5-[3-(Benzyloxy)cyclobutyl]-2-methylmorpholin [Diastereomerengemisch, 8 Isomere]

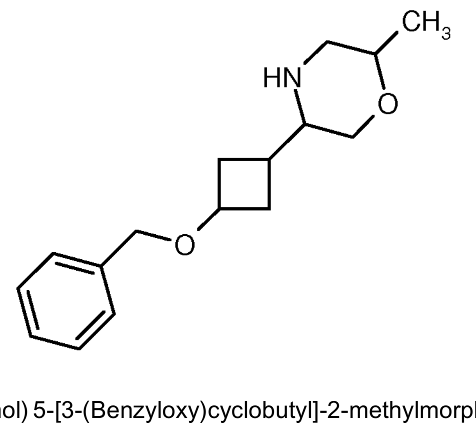

Es wurden 953 mg (3.46 mmol) 5-[3-(Benzyloxy)cyclobutyl]-2-methylmorpholin-3-on [Diastereomerengemisch,

8 Isomere] in Tetrahydrofuran (10 mL) vorgelegt, unter Argon mit 6.92 mL (13.8 mmol) 2 M Boran-Dimethylsulfid-Komplex-Lösung in Tetrahydrofuran versetzt und 3 h unter Rückfluss gerührt. Nachfolgend wurde die Reaktionslösung vorsichtig in Ethanol (50.0 mL) getropft und 8 h unter Rückfluss gerührt. Dann wurde im Vakuum eingeengt, der Rückstand in Acetonitril aufgenommen und mittels präparativer RP-HPLC (Acetonitril/Wasser) gereinigt. Ausbeute: 780 mg (84% d. Th.).
LC-MS (Methode 1A): $R_t$ = 0.57, 0.60 min; MS (ESIpos): m/z = 262 $[M+H]^+$;
$^1$H-NMR (400 MHz, DMSO-$d_6$): δ [ppm] = 7.39-7.24 (m, 5H), 4.37-4.31 (m, 2H), 4.11-3.98 (m, 0.3H), 3.92-3.78 (m, 0.7H), 3.72-3.54 (m, 0.5H), 3.50-3.40 (m, 1.5H), 2.94-2.70 (m, 1H), 2.61 (td, 0.3H), 2.48-1.82 (m, 5.7H), 1.73-1.40 (m, 2H), 1.06-0.94 (m, 3H), ein Proton verdeckt.

**Beispiel 50A**

Benzyl-5-[3-(benzyloxy)cyclobutyl]-2-methylmorpholin-4-carboxylat [Diastereomerengemisch, 4 Isomere]

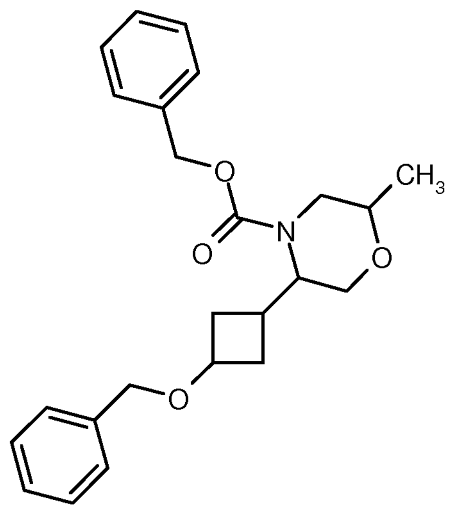

Es wurden 900 mg (3.44 mmol) 5-[3-(Benzyloxy)cyclobutyl]-2-methylmorpholin [Diastereomerengemisch, 8 Isomere] und 890 mg (1.20 mL, 6.89 mmol) *N,N,*-Diisopropylethylamin in Dichlormethan (45.0 mL) vorgelegt, bei 0°C mit 881 mg (0.74 mL, 5.17 mmol) Chlorameisensäurebenzylester tropfenweise versetzt und über Nacht gerührt wobei man auf RT erwärmen ließ. Die Reaktionslösung wurde im Vakuum eingeengt und der Rückstand in Acetonitril aufgenommen. Reinigung und Diastereomerentrennung mittels RP-HPLC an achiraler Phase (Acetonitril/Wasser) ergaben 537 mg (36% d. Th.) der Zielverbindung des Beispiels 50A (Diastereomerengemisch, 4 Isomere) und 588 mg (43% d. Th.) der Zielverbindung des Beispiels 51A (Diastereomerengemisch, 4 Isomere).
LC-MS (Methode 1A): $R_t$ = 1.26 min; MS (ESIpos): m/z = 396 $[M+H]^+$;
$^1$H-NMR (400 MHz, DMSO-$d_6$): δ [ppm] = 7.41-7.24 (m, 10H), 5.22-5.01 (m, 2H), 4.33-4.26 (m, 2H), 4.09-3.66 (m, 4H), 3.51 (d, 1H), 3.29-3.10 (m, 2H), 2.82 (br. s., 0.3H), 2.48-1.79 (m, 3.3H), 1.69-1.52 (m, 1.4H), 1.14-1.07 (m, 3H).

**Beispiel 51A**

Benzyl-5-[3-(benzyloxy)cyclobutyl]-2-methylmorpholin-4-carboxylat [Diastereomerengemisch, 4 Isomere]

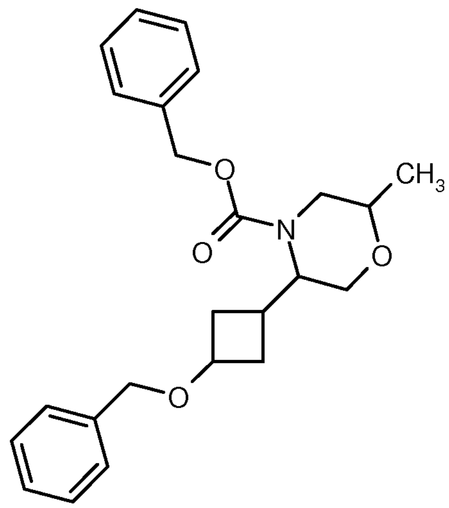

Es wurden 900 mg (3.44 mmol) 5-[3-(Benzyloxy)cyclobutyl]-2-methylmorpholin [Diastereomerengemisch, 8 Isomere] und 890 mg (1.20 mL, 6.89 mmol) *N,N,*-Diisopropylethylamin in Dichlormethan (45.0 mL) vorgelegt, bei 0°C mit 881 mg (0.74 mL, 5.17 mmol) Chlorameisensäurebenzylester tropfenweise versetzt und über Nacht gerührt wobei man auf RT erwärmen ließ. Die Reaktionslösung wurde im Vakuum eingeengt und der Rückstand in Acetonitril aufgenommen. Reinigung und Diastereomerentrennung mittels RP-HPLC an achiraler Phase (Acetonitril/Wasser) ergaben 537 mg (36% d. Th.) der Zielverbindung des Beispiels 50A (Diastereomerengemisch, 4 Isomere) und 588 mg (43% d. Th.) der Zielverbindung des Beispiels 51A (Diastereomerengemisch, 4 Isomere).
LC-MS (Methode 1A): $R_t$ = 1.29 min; MS (ESIpos): m/z = 396 $[M+H]^+$;
$^1$H-NMR (400 MHz, DMSO-$d_6$): $\delta$ [ppm] = 7.44-7.22 (m, 10H), 5.20-4.98 (m, 2H), 4.36-4.20 (m, 2H), 4.14-3.34 (m, 6H), 2.88-2.57 (m, 1.5H), 2.44-1.53 (m, 4.5H), 1.10-1.03 (m, 3H).

## Beispiel 52A

3-(6-Methylmorpholin-3-yl)cyclobutanol [Diastereomerengemisch, 4 Isomere]

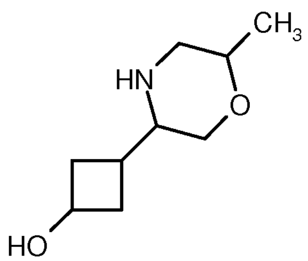

Es wurden 580 mg (1.47 mmol) Benzyl-5-[3-(benzyloxy)cyclobutyl]-2-methylmorpholin-4-carboxylat [Beispiel 51A, Diastereomerengemisch, 4 Isomere] in Ethanol (100 mL) vorgelegt, unter Argon mit 58 mg Palladium auf Kohle (10%ig) und 58 mg Palladiumhydroxid auf Kohle (20%ig) versetzt und anschließend über Nacht unter Wasserstoffatmosphäre unter Normaldruck gerührt. Die Reaktionslösung wurde über Kieselgur filtriert und der Filterrückstand mit Ethanol gewaschen. Es wurde im Vakuum eingeengt und das Produkt am Hochvakuum getrocknet. Ausbeute: 245 mg (97% d. Th.).
GC-MS (Methode 1B): $R_t$ = 4.60, 4.67 min; MS (EIpos): m/z = 171 $[M]^+$;
$^1$H-NMR (400 MHz, DMSO-$d_6$): $\delta$ [ppm] = 4.94-4.84 (m, 1H), 4.16-4.05 (d, 0.6H), 3.93-3.82 (m, 0.7H), 3.55-3.40 (m, 3.3H), 3.19-3.14 (m, 0.7H), 3.17 (d, 1H), 2.47-1.76 (m, 6H), 1.58-1.28 (m, 1.5H), 1.08-0.94 (m, 3.5H).

**Beispiel 53A**

[1-Amino-3-(benzyloxy)cyclobutyl]methanol [Diastereomerengemisch, 2 Isomere, *cis/trans* ca. 4:1]

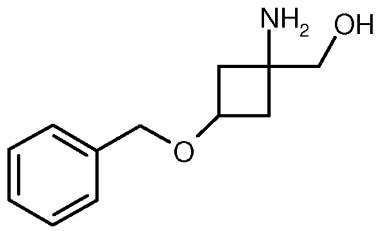

I) Es wurden 5.00 g (20.3 mmol) 2-(Benzyloxy)-5,7-diazaspiro[3.4]octan-6,8-dion [Diastereomerengemisch, 2 Isomere, *cis/trans* ca. 4:1; T. M. Shoup, M. M. Goodman, J. Labelled. Cpd. Radiopharm. 1999, 42, 215-225; US2006/292073 A1] in Wasser (100 mL) vorgelegt und mit 32.0 g (102 mmol) Bariumhydroxid Octahydrat versetzt. Die Suspension wurde in sieben Portionen in der Mikrowelle (Biotage Synthesizer) für jeweils 1.5 h bei 140°C gerührt. Die Suspensionen wurden vereinigt und mit 6 N wässriger Schwefelsäure-Lösung auf einen pH von ca. 4 eingestellt. Der ausgefallene Feststoff wurde im Vakuum abfiltriert, das Filtrat anschließend im Vakuum eingeengt und der erhaltene Feststoff am Hochvakuum getrocknet. Man erhielt 6.2 g Rohprodukt.

II) Es wurden zu 49.1 mL 2 M Lithiumborhydrid-Lösung in Tetrahydrofuran (98.2 mmol) tropfenweise 21.3 g (24.9 mL, 196 mmol) Chlortrimethylsilan gegeben. Die erhaltene Suspension wurde auf 0°C abgekühlt und dann portionsweise mit 5.43 g des Rohproduktes aus I) versetzt. Es wurde anschließend auf RT erwärmt und dann bei RT über Nacht gerührt. Die Reaktion wurde durch tropfenweise Zugabe von Methanol (15 mL) beendet und die Reaktionslösung nachfolgend im Vakuum eingeengt. Der Rückstand wurde in Essigsäureethylester aufgenommen und mit 2 N wässriger Natriumhydroxid-Lösung gewaschen. Die wässrige Phase wurde mit Essigsäureethylester extrahiert, die vereinigten organischen Phasen über Natriumsulfat getrocknet, filtriert und im Vakuum eingeengt. Das Rohprodukt wurde ohne weitere Reinigung in der nächsten Stufe eingesetzt. Ausbeute: 3.76 g (Rohprodukt).
LC-MS (Methode 4A): $R_t$ = 2.10 min; MS (ESIpos): m/z = 208 $[M+H]^+$;
$^1$H-NMR (400 MHz, DMSO-$d_6$): δ [ppm] = 7.39-7.22 (m, 5H), 4.66 (br. s., 1H), 4.32 (s, 2H), 4.15 (quin, 0.2H), 3.70 (quin, 0.8H), 3.22-3.14 (m, 2H), 2.34-2.26 (m, 2H), 1.91-1.74 (m, 2H), 1.72-1.61 (m, 2H).

**Beispiel 54A**

*tert*-Butyl-[3-(benzyloxy)-1-(hydroxymethyl)cyclobutyl]carbamat [enantiomerenreines *cis*- und trans-Isomer]

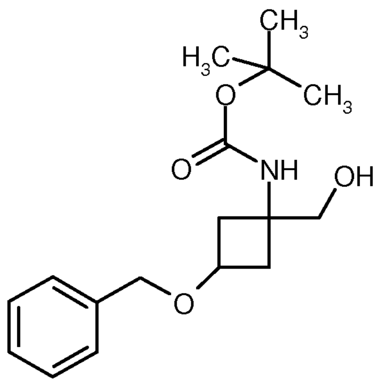

Es wurden 3.76 g (18.1 mmol) [1-Amino-3-(benzyloxy)cyclobutyl]methanol [Diastereomerengemisch, 2 Isomere *cis/trans* ca. 4:1] in Dichlormethan (150 mL) vorgelegt, bei RT mit 4.36 g (20.0 mmol) Di-*tert*-butyldicarbonat sowie 3.86 g (5.31 mL, 38.1 mmol) Triethylamin versetzt und über Nacht bei RT gerührt. Dann wurde mit 0.5 N wässriger Hydrogenchlorid-Lösung, gesättigter wässriger Natriumhydrogencarbonat-Lösung und Wasser gewaschen, die organische

Phase über Natriumsulfat getrocknet, filtriert und im Vakuum eingeengt. Das Rohprodukt (6.4 g) wurde mittels präparativer RP-HPLC (Methode 1G) gereinigt und in die Diastereomere aufgetrennt. Das schneller eluierende Haupt-Diastereomer war hierbei das *cis*-Isomer und das langsamer eluierende Minder-Diastereomer das trans-Isomer. Ausbeute: 3.45 g (61% d. Th., enantiomerenreines *cis*-Isomer); 690 mg (12% d. Th., enantiomerenreines *trans-Isomer*).
enantiomerenreines *cis*-Diastereomer:

LC-MS (Methode 1A): $R_t$ = 2.00 min; MS (ESIpos): m/z = 308 $[M+H]^+$;

enantiomerenreines trans-Diastereomer:

LC-MS (Methode 1A): $R_t$ = 2.02 min; MS (ESIpos): m/z = 308 $[M+H]^+$.

**Beispiel 55A**

[*cis*-1-Amino-3-(benzyloxy)cyclobutyl]methanol Hydrochlorid [enantiomerenreines *cis*-Isomer]

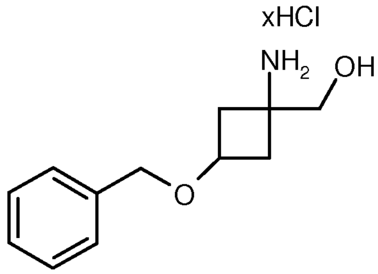

Es wurden 3.45 g (11.2 mmol) *tert*-Butyl-[*cis*-3-(benzyloxy)-1-(hydroxymethyl)cyclobutyl]-carbamat [enantiomerenreines *cis*-Isomer aus Beispiel 54A] in 1,4-Dioxan (30 mL) vorgelegt, bei RT mit 11.2 mL 4 N Hydrogenchlorid-Lösung in 1,4-Dioxan/Wasser versetzt und 20 h bei RT gerührt. Anschließend wurde im Vakuum eingeengt und der Rückstand am Hochvakuum getrocknet. Das Rohprodukt wurde ohne weitere Reinigung in der nächsten Stufe eingesetzt. Ausbeute: 2.81 g (quant.).
LC-MS (Methode 1A): $R_t$ = 0.40 min; MS (ESIpos): m/z = 208 $[M+H-HCl]^+$;
$^1$H-NMR (400 MHz, DMSO-$d_6$): $\delta$ [ppm] = 8.24 (br. s., 3H), 7.43-7.25 (m, 5H), 5.54 (br. s., 1H), 4.39 (s, 2H), 3.90 (quin, 1H), 3.46 (br. d., 2H), 2.42 ($m_c$, 2H), 2.12 ($m_c$, 2H).

**Beispiel 56A**

*N*-[*cis*-3-(Benzyloxy)-1-(hydroxymethyl)cyclobutyl]-2-chlorpropanamid [Racemat]

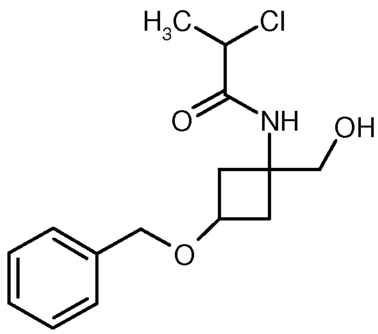

Es wurden 2.81 g (11.5 mmol) [*cis*-1-Amino-3-(benzyloxy)cyclobutyl]methanol Hydrochlorid [enantiomerenreines *cis*-Isomer] in *iso*-Propanol (70.0 mL) vorgelegt, auf 0°C abgekühlt und mit 4.67 g (6.43 mL, 46.1 mmol) Triethylamin versetzt. Anschließend wurden 1.61 (1.26mol, 12.7 mmol) 2-Chlorpropionylchlorid [Racemat] zugetropft. Man ließ die

Reaktionslösung auf RT erwärmen, rührte 1 h und engte anschließend die Reaktionslösung im Vakuum ein. Der Rückstand wurde in Dichlormethan aufgenommen und mit 1 N wässriger Hydrogenchlorid-Lösung gewaschen. Die organischen Phasen wurden über Natriumsulfat getrocknet, filtriert und im Vakuum eingeengt. Das Rohprodukt wurde ohne weitere Reinigung in der nächsten Stufe eingesetzt. Ausbeute: 3.38 g (97% d. Th.).
LC-MS (Methode 1A): $R_t$ = 0.85 min; MS (ESIpos): m/z = 298 $[M+H]^+$.

**Beispiel 57A**

*cis*-2-(Benzyloxy)-7-methyl-8-oxa-5-azaspiro[3.5]nonan-6-on [Racemat]

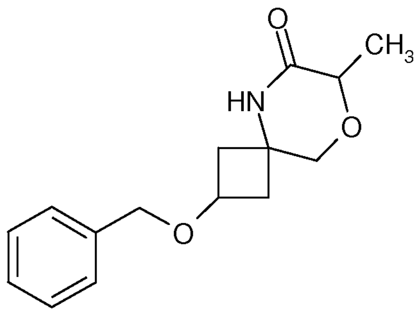

Es wurden 3.38 g (11.4 mmol) *N*-[*cis*-3-(Benzyloxy)-1-(hydroxymethyl)cyclobutyl]-2-chlorpropanamid [Racemat] in *iso*-Propanol (250 mL) vorgelegt, auf 0°C gekühlt und mit 3.82 g (34.1 mmol) Kalium-*tert*-butylat in einer Portion versetzt. Man ließ auf RT erwärmen und rührte 1 h bei 50°C. Dann wurde *iso*-Propanol im Vakuum weitgehend entfernt und der Rückstand in Dichlormethan aufgenommen. Die organische Phase wurde mit 1 N wässriger Hydrogenchlorid-Lösung gewaschen, über Natriumsulfat getrocknet, filtriert und im Vakuum eingeengt. Der Rückstand wurde mittels präparativer RP-HPLC (Acetonitril/Wasser) gereinigt. Ausbeute: 2.96 g (99% d. Th.).
LC-MS (Methode 1A): $R_t$ = 0.86 min; MS (ESIpos): m/z = 262 $[M+H]^+$.

**Beispiel 58A**

*cis*-2-(Benzyloxy)-7-methyl-8-oxa-5-azaspiro[3.5]nonan [Racemat]

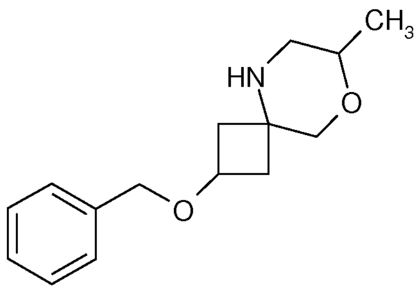

Es wurden 2.96 g (11.3 mmol) *cis*-2-(Benzyloxy)-7-methyl-8-oxa-5-azaspiro[3.5]nonan-6-on [Racemat] in Tetrahydrofuran (200 mL) vorgelegt, unter Argon mit 22.7 mL (45.3 mmol) 2 M Boran-Dimethylsulfid-Komplex-Lösung in Tetrahydrofuran versetzt und 2 h unter Rückfluss gerührt. Nachfolgend wurde die Reaktionslösung auf 0°C abgekühlt, es wurde vorsichtig Methanol (100 mL) zugetropft und 12 h unter Rückfluss gerührt. Anschließend wurde im Vakuum vollständig eingeengt, der Rückstand in Acetonitril aufgenommen und direkt mittels präparativer RP-HPLC (Acetonitril/Wasser) gereinigt. Ausbeute: 2.80 g (91% d. Th.).
LC-MS (Methode 1A): $R_t$ = 0.61 min; MS (ESIpos): m/z = 248 $[M+H]^+$.

**Beispiel 59A**

*tert*-Butyl-*cis*-2-(benzyloxy)-7-methyl-8-oxa-5-azaspiro[3.5]nonan-5-carboxylat [Racemat]

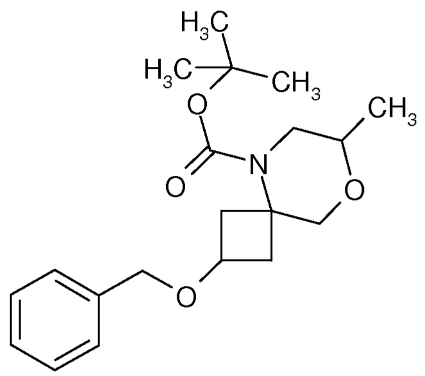

Es wurden 2.80 g (11.3 mmol) *cis*-2-(Benzyloxy)-7-methyl-8-oxa-5-azaspiro[3.5]nonan [Racemat] in Dichlormethan (150 mL) vorgelegt, bei RT mit 3.71 g (17.0 mmol) Di-*tert*-butyldicarbonat und 5.73 g (7.89 mL, 56.6 mmol) Triethylamin versetzt und über Nacht bei RT gerührt. Die Reaktionslösung wurde mit 0.5 N wässriger Hydrogenchlorid-Lösung, gesättigter wässriger Natriumhydrogencarbonat-Lösung und Wasser gewaschen. Die organische Phase wurde über Natriumsulfat getrocknet, filtriert und im Vakuum eingeengt. Ausbeute: 3.33 g (84% d. Th.).
LC-MS (Methode 1A): $R_t$ = 1.28 min; MS (ESIpos): m/z = 348 $[M+H]^+$.

**Beispiel 60A**

*tert*-Butyl-*cis*-2-(benzyloxy)-7-methyl-8-oxa-5-azaspiro[3.5]nonan-5-carboxylat [enantiomerenreines Isomer 1]

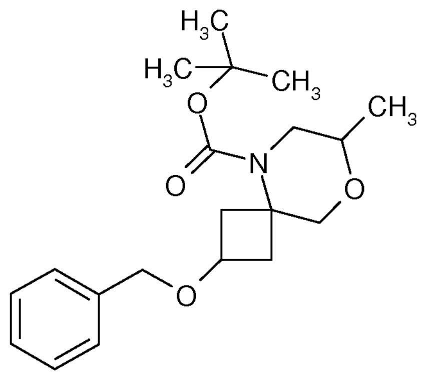

Enantiomerentrennung von 3.33 g der Verbindung aus Beispiel 59A (Methode 5D) ergaben 1.06 g der Verbindung aus Beispiel 60A [enantiomerenreines Isomer 1] und 928 mg der Verbindung aus Beispiel 61A [enantiomerenreines Isomer 2].
HPLC (Methode 11E): $R_t$ = 5.06 min, 99.9% ee;
LC-MS (Methode 1A): $R_t$ = 1.30 min; MS (ESIpos): m/z = 348 $[M+H]^+$;
$^1$H-NMR (400 MHz, DMSO-$d_6$): $\delta$ [ppm] = 7.40-7.23 (m, 5H), 4.37 ($m_c$, 2H), 3.79 (quin, 1H), 3.62 (dd, 1H), 3.49-3.33 (m, 3H), 2.69-2.56 (m, 2H), 2.43 (dd, 1H), 2.32-2.23 (m, 1H), 1.78 ($m_c$, 1H), 1.38 (s, 9H), 1.01 (d, 3H).

**Beispiel 61A**

*tert*-Butyl-*cis*-2-(benzyloxy)-7-methyl-8-oxa-5-azaspiro[3.5]nonan-5-carboxylat [enantiomerenreines Isomer 2]

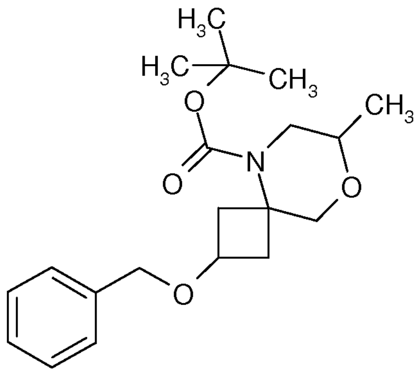

Enantiomerentrennung von 3.33 g der Verbindung aus Beispiel 59A (Methode 5D) ergaben 1.06 g der Verbindung aus Beispiel 60A [enantiomerenreines Isomer 1] und 928 mg der Verbindung aus Beispiel 61A [enantiomerenreines Isomer 2].
HPLC (Methode 11E): $R_t$ = 13.5 min, 99.9% ee;
LC-MS (Methode 1A): $R_t$ = 1.30 min; MS (ESIpos): m/z = 348 $[M+H]^+$;
$^1$H-NMR (400 MHz, DMSO-$d_6$): $\delta$ [ppm] = 7.38-7.25 (m, 5H), 4.37 ($m_c$, 1H), 3.79 (quin, 1H), 3.62 (dd, 1H), 3.47-3.34 (m, 2H), 2.68-2.56 (m, 2H), 2.43 (dd, 1H), 2.32-2.22 (m, 1H), 1.78 ($m_c$, 1H), 1.38 (s, 9H), 1.01 (d, 3H).

**Beispiel 62A**

*cis*-2-(Benzyloxy)-7-methyl-8-oxa-5-azaspiro[3.5]nonan Hydrochlorid [enantiomerenreines Isomer 1]

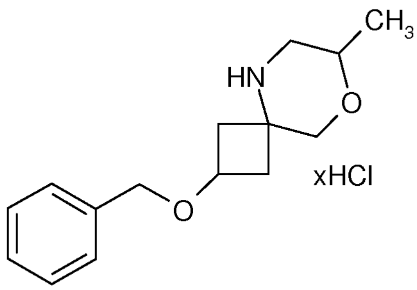

Es wurden 1.06 g (3.06 mmol) *tert-Butyl- cis*-2-(benzyloxy)-7-methyl-8-oxa-5-azaspiro[3.5]nonan-5-carboxylat [enantiomerenreines Isomer 1 aus Beispiel 60A] in 1,4-Dioxan (30 mL) vorgelegt und bei RT mit 10.0 mL 4 N Hydrogenchlorid-Lösung in 1,4-Dioxan versetzt. Es wurde über Nacht bei RT gerührt, anschließend im Vakuum eingeengt und das Produkt am Hochvakuum getrocknet. Ausbeute: 1.04 g (quant.).
LC-MS (Methode 1A): $R_t$ = 0.48 min; MS (ESIpos): m/z = 248 $[M+H-HCl]^+$.

**Beispiel 63A**

*cis*-2-(Benzyloxy)-7-methyl-8-oxa-5-azaspiro[3.5]nonan Hydrochlorid [enantiomerenreines Isomer 2]

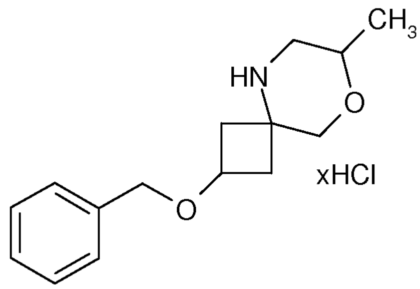

Es wurden 928 mg (2.67 mmol) *tert*-Butyl-*cis*-2-(benzyloxy)-7-methyl-8-oxa-5-azaspiro[3.5]-nonan-5-carboxylat [enantiomerenreines Isomer 2 aus Beispiel 61A] in 1,4-Dioxan (30 mL) vorgelegt und bei RT mit 10.0 mL 4 N Hydrogenchlorid-Lösung in 1,4-Dioxan versetzt. Es wurde über Nacht bei RT gerührt, anschließend im Vakuum eingeengt und das Produkt am Hochvakuum getrocknet. Ausbeute: 1.16 g (quant.).
LC-MS (Methode 1A): $R_t$ = 0.51 min; MS (ESIpos): m/z = 248 $[M+H-HCl]^+$.

**Beispiel 64A**

*cis*-7-Methyl-8-oxa-5-azaspiro[3.5]nonan-2-ol Hydrochlorid [enantiomerenreines Isomer 1]

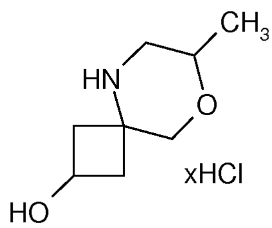

Es wurden 1.03 g (3.66 mmol) *cis*-2-(Benzyloxy)-7-methyl-8-oxa-5-azaspiro[3.5]nonan Hydrochlorid [enantiomerenreines Isomer 1 aus Beispiel 62A] in Methanol (36.7 mL) sowie 3.34 mL 2 N wässrige Hydrogenchlorid-Lösung vorgelegt, unter Argon mit 119 mg Palladium auf Kohle (10%ig) und 59.7 mg Palladiumhydroxid auf Kohle (20%ig) versetzt und anschließend über Nacht unter Wasserstoffatmosphäre unter Normaldruck gerührt. Die Reaktionslösung wurde über Kieselgur filtriert und der Filterrückstand mit Methanol nachgewaschen. Es wurde im Vakuum eingeengt und das Produkt am Hochvakuum getrocknet. Ausbeute: 785 mg (99% d. Th.).
MS (Methode 1C): m/z = 158 $[M+H-HCl]^+$;
$^1$H-NMR (400 MHz, DMSO-$d_6$): δ [ppm] = 9.84 (br. s., 1H), 9.57 (br. s., 1H), 3.78-3.60 (m, 4H), 3.11 (d, 1H), 2.27-2.18 (m, 1H), 2.13-2.00 (m, 2H), 1.09 (d, 3H), drei Protonen verdeckt.

**Beispiel 65A**

*cis*-7-Methyl-8-oxa-5-azaspiro[3.5]nonan-2-ol Hydrochlorid [enantiomerenreines Isomer 2]

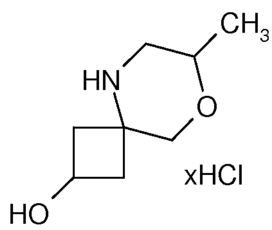

Es wurden 1.16 g (4.11 mmol) *cis*-2-(Benzyloxy)-7-methyl-8-oxa-5-azaspiro[3.5]nonan Hydrochlorid [enantiomerenreines Isomer 2 aus Beispiel 63A] in Methanol (41.3 mL) und 3.75 mL 2 N wässrige Hydrogenchlorid-Lösung vorgelegt und unter Argon mit 134 mg Palladium auf Kohle (10%ig) und 67.1 mg Palladiumhydroxid auf Kohle (20%ig) versetzt und anschließend über Nacht unter Wasserstoffatmosphäre unter Normaldruck gerührt. Die Reaktionslösung wurde über Kieselgur filtriert und der Filterrückstand mit Methanol nachgewaschen. Es wurde im Vakuum eingeengt und das Produkt am Hochvakuum getrocknet. Ausbeute: 870 mg (98% d. Th.).
MS (Methode 1C): m/z = 158 $[M+H-HCl]^+$;
$^1$H-NMR (400 MHz, DMSO-$d_6$): $\delta$ [ppm] = 9.96 (br. s., 1H), 9.67 (br. s., 1H), 3.84-3.59 (m, 4H), 3.10 (d, 1H), 2.29-2.17 (m, 1H), 2.15-1.99 (m, 2H), 1.09 (d, 3H), drei Protonen verdeckt.

**Beispiel 66A**

*tert*-Butyl-2-[(benzylamino)methyl]azetidin-1-carboxylat [Racemat]

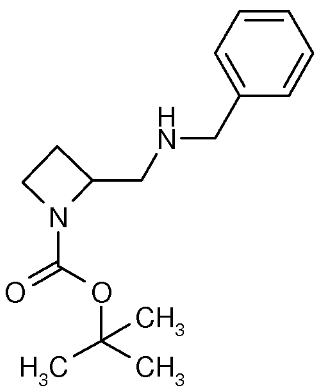

10.0 g (53.7 mmol) *tert*-Butyl-2-(aminomethyl)azetidin-1-carboxylat und 2.03 g (37.8 mmol) Benzaldehyd in 100 mL Methanol wurden 2.5 h unter Rückfluss erhitzt. Dann wurde auf 0°C abgekühlt und bei dieser Temperatur innerhalb von 15 min Natriumborhydrid langsam hinzugegeben. Es wurde über Nacht bei RT gerührt. Anschließend wurde im Vakuum eingeengt, der Rückstand mit Dichlormethan und Wasser versetzt, die Phasen getrennt und die wässrige Phase wurde zweimal mit Dichlormethan extrahiert. Die vereinigten organischen Phasen wurden mit gesättigter wässriger Natriumchlorid- Lösung gewaschen, über Natriumsulfat getrocknet, filtriert und das Filtrat wurde im Vakuum eingeengt. Der erhaltene Rückstand wurde mit Dichlormethan versetzt und mittels Kieselgel-Chromatographie (Dichlormethan, dann Dichlormethan:Methanol = 100:4) gereinigt. Ausbeute: 7.43 g (50% d. Th.).
LC-MS (Methode 6A): $R_t$ = 2.41 min; MS (ESIpos): m/z = 277 $[M+H]^+$.

**Beispiel 67A**

*tert*-Butyl-2-{[benzyl(1-methoxy-1-oxopropan-2-yl)amino]methyl}azetidin-1-carboxylat [Diastereomerengemisch, 4 Isomere]

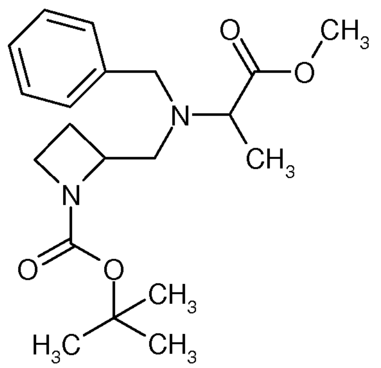

Es wurden 2.50 g (9.05 mmol) *tert*-Butyl-2-[(benzylamino)methyl]azetidin-1-carboxylat [Racemat] in Dichlormethan (150 mL) gelöst, mit 5.55 mL (4.03 g, 39.8 mmol) Triethylamin und 3.04 mL (4.53 g, 27.1 mmol) 2-Brompropansäuremethylester [Racemat] versetzt und über Nacht bei RT gerührt. Dann wurde mit 5.55 mL (4.03 g, 39.8 mmol) Triethylamin und 3.04 mL (4.53 g, 27.1 mmol) 2-Brompropansäuremethylester [Racemat] versetzt und über Nacht bei 40°C gerührt. Anschließend wurde erneut mit 5.55 mL (4.03 g, 39.8 mmol) Triethylamin und 3.04 mL (4.53 g, 27.1 mmol) 2-Brompropansäuremethylester [Racemat] versetzt und über Nacht bei 40°C gerührt. Nach Abkühlen auf Raumtemperatur wurde mit Wasser und Dichlormethan verdünnt und die Phasen wurden getrennt. Die wässrige Phase wurde zweimal mit Dichlormethan extrahiert und die vereinten organischen Phasen wurden mit gesättigter wässriger Natriumchlorid-Lösung gewaschen, über Natriumsulfat getrocknet, filtriert und dann im Vakuum vom Lösungsmittel befreit. Das erhaltene Rohprodukt wurde mittels Kieselgel-Chromatographie (Dichlormethan, dann Dichlormethan:Methanol = 100:1) gereinigt. Ausbeute: 3.22 g (94% d. Th.).
LC-MS (Methode 1A): $R_t$ = 1.00 min (Diastereomer 1), $R_t$ = 1.13 min (Diastereomer 2);
MS (ESIpos): m/z = 363 $[M+H]^+$;
$^1$H-NMR (400 MHz, DMSO-$d_6$): $\delta$ [ppm] = 7.35-7.28 (m, 4H), 7.27-7.20 (m, 1H), 4.18-3.98 (m, 1H), 3.85-3.73 (m, 1H), 3.71-3.51 (m, 6H), 3.51-3.38 (m, 1H), 3.04-2.88 (m, 1H), 2.85-2.69 (m, 1H), 2.15-1.96 (m, 1H), 1.93-1.65 (m, 1H), 1.34 (d, 9H), 1.26-1.15 (m, 3H).

**Beispiel 68A**

Methyl-*N*-(azetidin-2-ylmethyl)-*N*-benzylalaninat Hydrochlorid [Diastereomerengemisch, 4 Isomere]

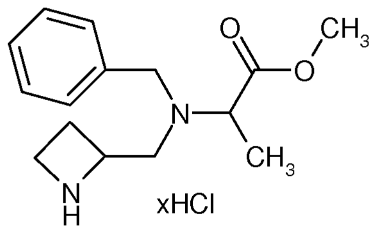

3.2 g (8.5 mmol) *tert*-Butyl-2-{[benzyl(1-methoxy-1-oxopropan-2-yl)amino]methyl}-azetidin-1-carboxylat [Diastereomerengemisch, 4 Isomere] in Dioxan (74 mL) wurden mit 14.9 mL (59.7 mmol) 4 N Hydrogenchlorid-Lösung in 1,4-Dioxan versetzt und über Nacht bei Raumtemperatur gerührt. Anschließend wurde erneut mit 14 mL (59.7 mmol) 4 N Hydrogenchlorid-Lösung in 1,4-Dioxan versetzt und über Nacht bei RT gerührt. Dann wurde im Vakuum eingeengt und das Produkt am Hochvakuum getrocknet. Ausbeute: 3.13 g (98% d. Th., Reinheit: 80%).
LC-MS (Methode 1A): $R_t$ = 0.68 min (Diastereomer 1), $R_t$ = 0.70 min (Diastereomer 2);
MS (ESIpos): m/z = 263 $[M+H-HCl]^+$.

**Beispiel 69A**

4-Benzyl-3-methyl-1,4-diazabicyclo[4.2.0]octan-2-on [enantiomerenreines Isomer 3]

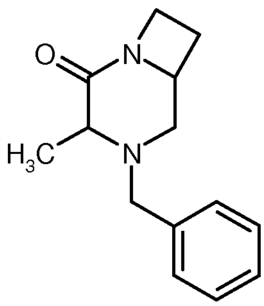

Es wurden 21.8 g (51.0 mmol, Reinheit: 70%) Methyl-N-(azetidin-2-ylmethyl)-N-benzylalaninat [Diastereomerengemisch, 4 Isomere] in Methanol (562 mL) vorgelegt, mit 28.2 g (204 mmol) Kaliumcarbonat versetzt und anschließend für 2.5 d bei RT gerührt. Die Reaktionslösung wurde filtriert und bei 20°C wurde das Lösungsmittel im Vakuum weitgehend entfernt. Der Rückstand wurde in Wasser aufgenommen und mehrfach mit Dichlormethan und Chloroform/*iso*-Propanol (7:3) extrahiert. Die gesammelten organischen Phasen wurden über Natriumsulfat getrocknet, filtriert und im Vakuum eingeengt. Das Rohprodukt (12.1 g) wurde nach Methode 7D in die entsprechenden Isomere aufgetrennt. Die Zielverbindung eluierte hierbei als dritte Komponente. Ausbeute: 2.47 g (21% d. Th.).
HPLC (Methode 6E): $R_t$ = 7.49 min, 99.0% ee;
LC-MS (Methode 1A): $R_t$ = 0.50 min; MS (ESIpos): m/z = 231 $[M+H]^+$.

**Beispiel 70A**

3-Methyl-1,4-diazabicyclo[4.2.0]octan-2-on [enantiomerenreines Isomer 3]

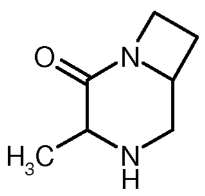

Es wurden 2.40 g (10.4 mmol) 4-Benzyl-3-methyl-1,4-diazabicyclo[4.2.0]octan-2-on [enantiomerenreines Isomer 3] in Ethanol (85 mL) vorgelegt und unter Argon mit 250 mg Palladium auf Kohle (10%ig) und 130 mg Palladiumhydroxid auf Kohle (20%ig) versetzt und anschließend über Nacht unter Wasserstoffatmosphäre unter Normaldruck gerührt. Die Reaktionslösung wurde über Kieselgur filtriert und der Filterrückstand mit heißem Ethanol (100 mL) gewaschen. Es wurde im Vakuum eingeengt und das Produkt am Hochvakuum getrocknet. Ausbeute: 1.56 g (quant.).
GC-MS (Methode 2B): $R_t$ = 4.50 min; MS (EIpos): m/z = 140 $[M]^+$;
MS (Methode 1C): m/z = 141 $[M+H]^+$;
$^1$H-NMR (400 MHz, DMSO-$d_6$): $\delta$ [ppm] = 4.59 ($m_c$, 1H), 4.09-3.89 (m, 2H), 3.27 (q, 1H), 2.95 (dd, 1H), 2.58-2.53 (m, 2H), 2.33-2.04 (m, 2H), 1.12 (d, 3H).

**Beispiel 71A**

*N*-Benzyl-2-chlor-*N*-[(2*R*)-1,4-dihydroxybutan-2-yl]propanamid [Diastereomerengemisch, 2 Isomere]

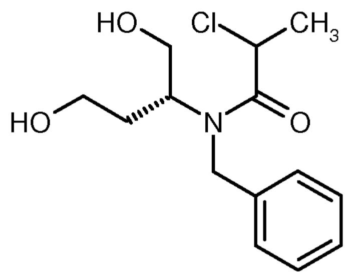

Es wurden 45.1 g (55.3 mmol, Reinheit: 72%) (2*R*)-2-(Benzylamino)butan-1,4-diol [B. L. Feringa, Tetrahedron 1989, 45, 6799-6818] in *iso*-Propanol (239 mL) vorgelegt, auf 0°C abgekühlt und mit 11.2 g (15.4 mL, 111 mmol) Triethylamin versetzt. Anschließend wurden 10.5 g (8.23 mL, 83.0 mmol) 2-Chlorpropionylchlorid [Racemat] zugetropft. Nach 10 min Rühren wurde die Reaktionslösung im Vakuum eingeengt, der Rückstand in Essigsäureethylester aufgenommen und mit Wasser gewaschen. Die organische Phase wurde über Natriumsulfat getrocknet, filtriert und im Vakuum eingeengt. Das Rohprodukt wurde ohne weitere Reinigung in der nächsten Stufe eingesetzt. Ausbeute: 21.4 g (quant., Reinheit: 82%, Diastereomerenverhältnis ca. 3:2).
LC-MS (Methode 1A): $R_t$ = 0.65 min (enantiomerenreines Isomer 1), $R_t$ = 0.67 min (enantiomerenreines Isomer 2);
MS (ESIpos): m/z = 286 $[M+H]^+$.

**Beispiel 72A**

(*5R*)-4-Benzyl-5-(2-hydroxyethyl)-2-methylmorpholin-3-on [Diastereomerengemisch, 2 Isomere]

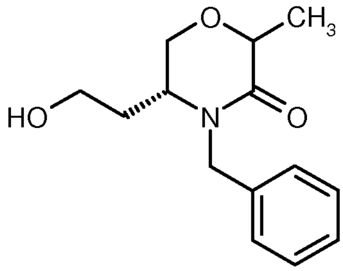

Es wurden 21.4 g (62.1 mmol, Reinheit: 82%) *N*-Benzyl-2-chlor-*N*-[(2*R*)-1,4-dihydroxybutan-2-yl]propanamid [Diastereomerengemisch, 2 Isomere] in *iso*-Propanol (335 mL) vorgelegt, auf 0°C gekühlt und anschließend mit 27.9 g (249 mmol) Kalium-*tert*-butylat in einer Portion versetzt. Es wurde über Nacht gerührt wobei man die Reaktion auf RT erwärmen ließ. *Iso*-Propanol wurde im Vakuum weitgehend entfernt, der Rückstand in Wasser (300 mL) aufgenommen und mit Essigsäureethylester extrahiert. Die organischen Phasen wurden über Natriumsulfat getrocknet, filtriert und im Vakuum eingeengt. Das Rohprodukt wurde ohne weitere Reinigung in der nächsten Stufe eingesetzt. Ausbeute: 13.3 g (69% d. Th., Reinheit: 81%, Diastereomerenverhältnis ca. 3:2).
LC-MS (Methode 7A): $R_t$ = 3.23 min (enantiomerenreines Isomer 1), $R_t$ = 3.34 min (enantiomerenreines Isomer 2); MS (ESIpos): m/z = 250 $[M+H]^+$.

**Beispiel 73A**

(5*R*)-4-Benzyl-5-(2-{[*tert*-butyl(dimethyl)silyl]oxy}ethyl)-2-methylmorpholin-3-on [Diastereomerengemisch, 2 Isomere]

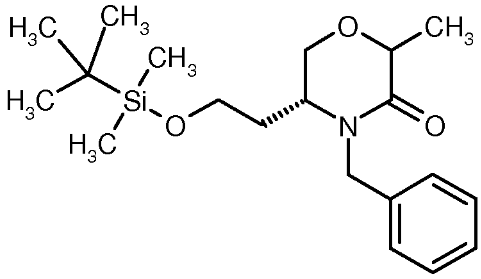

Es wurden 13.3 g (43.3 mmol) (5*R*)-4-Benzyl-5-(2-hydroxyethyl)-2-methylmorpholin-3-on [Diastereomerengemisch, 2 Isomere] in *N,N*-Dimethylformamid (60.0 mL) vorgelegt und bei RT mit 8.85 g (130 mmol) Imidazol versetzt. Anschließend wurden bei 0°C 9.80 g (65.0 mmol) *tert*-Butyldimethylsilylchlorid zugegeben, über Nacht gerührt wobei man die Reaktionslösung auf RT erwärmen ließ. Nachfolgend wurde im Vakuum eingeengt, in Essigsäureethylester aufgenommen und mehrfach mit Wasser und einmal mit gesättigter wässriger Natriumchlorid-Lösung gewaschen. Die organische Phase wurde über Magnesiumsulfat getrocknet, filtriert und im Vakuum eingeengt. Das Rohprodukt wurde anschließend durch Chromatographie an Kieselgel (Cyclohexan/ Essigsäureethylester 6:1, dann Cyclohexan/Essigsäureethylester 5:1) gereinigt. Ausbeute: 8.03 g (49% d. Th, Diastereomerenverhältnis: ca. 2.3:1).
LC-MS (Methode 1A): $R_t$ = 1.41 min; MS (ESIpos): m/z = 364 $[M+H]^+$;
$^1$H-NMR (400 MHz, DMSO-$d_6$): $\delta$ [ppm] = 7.40-7.18 (m, 5H), 5.12-5.03 (m, 1H), 4.33-4.21 (m, 1H), 4.14 (d, 0.3H), 4.05 (m, 0.7H), 3.95-3.84 (m, 1H), 3.74-3.56 (m, 3H), 3.39 (dd, 0.3H), 3.28 (d, 0.7H), 1.98-1.70 (m, 2H), 1.39 (d, 0.9H), 1.35 (d, 2.1H), 0.82 (s, 9H), 0.02 (s, 1.8H), 0.00 (s, 4.2H).

**Beispiel 74A**

(5*R*)-4-Benzyl-5-(2-{[*tert*-butyl(dimethyl)silyl]oxy}ethyl)-2,2-dimethylmorpholin-3-on [enantiomerenreines Isomer]

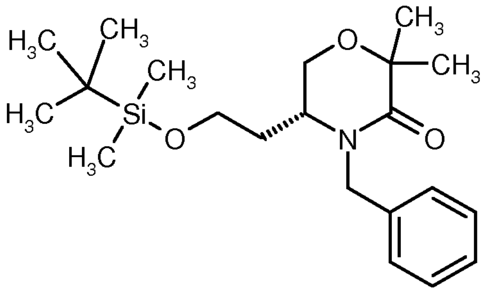

Es wurden 7.00 g (18.6 mmol) (5*R*)-4-Benzyl-5-(2-{[*tert*-butyl(dimethyl)silyl]oxy}ethyl)-2-methylmorpholin-3-on [Diastereomerengemisch, 2 Isomere] in Tetrahydrofuran (233 mL) vorgelegt und bei -78°C tropfenweise mit 13.0 mL (26.1 mmol) Lithiumdiisopropylamid-Lösung (2.0 M in Tetrahydrofuran/*n*-Heptan/Ethylbenzol) versetzt. Es wurde für 15 min gerührt und anschließend mit 3.17 g (1.39 mL, 22.4 mmol) Iodmethan versetzt. Man ließ die Reaktionslösung auf RT erwärmen und rührte 2 h. Die Reaktion wurde durch Zugabe von gesättigter wässriger Ammoniumchlorid-Lösung beendet und die Mischung mit Essigsäureethylester extrahiert. Die organische Phase wurde mit gesättigter wässriger Natriumchlorid-Lösung gewaschen, über Magnesiumsulfat getrocknet, filtriert und im Vakuum eingeengt. Das Rohprodukt wurde ohne weitere Reinigung in der nächsten Stufe eingesetzt. Ausbeute: 8.36 g (70% d. Th., Reinheit: 59%).
LC-MS (Methode 1A): $R_t$ = 1.47 min; MS (ESIpos): m/z = 378 $[M+H]^+$.

**Beispiel 75A**

(5*R*)-4-Benzyl-5-(2-{[*tert*-butyl(dimethyl)silyl]oxy}ethyl)-2,2-dimethylmorpholin [enantiomerenreines Isomer]

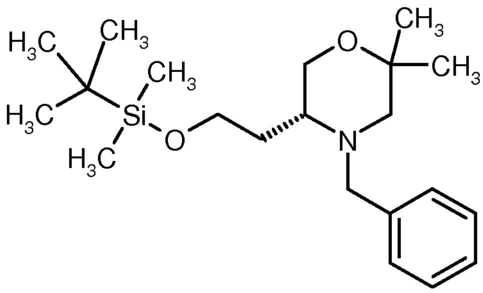

Es wurden 8.36 g (13.1 mmol, Reinheit: 59%) (5*R*)-4-Benzyl-5-(2-{[*tert*-butyl(dimethyl)silyl]-oxy}ethyl)-2,2-dimethylmorpholin-3-on [enantiomerenreines Isomer] in Tetrahydrofuran (133 mL) vorgelegt, unter Argon mit 26.2 mL (52.3 mmol) 2 M Boran-Dimethylsulfid-Komplex-Lösung in Tetrahydrofuran versetzt und 4 h unter Rückfluss gerührt. Dann wurde auf 0°C gekühlt, vorsichtig mit Methanol (30 mL) versetzt, 30 min unter Rückfluss gerührt und anschließend im Vakuum eingeengt. Das Rohprodukt wurde ohne weitere Reinigung in der nächsten Stufe eingesetzt. Ausbeute: 8.39 g (96% d. Th., Reinheit: 55%).
LC-MS (Methode 1A): $R_t$ = 1.15 min; MS (ESIpos): m/z = 364 $[M+H]^+$.

**Beispiel 76A**

2-[(3*R*)-4-Benzyl-6,6-dimethylmorpholin-3-yl]ethanol [Enantiomerengemisch, 2 Isomere]

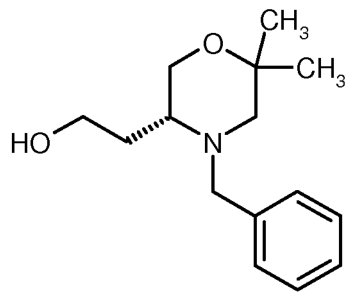

Es wurden 7.39 g (11.2 mmol, Reinheit: 55%) (5*R*)-4-Benzyl-5-(2-{[*tert*-butyl(dimethyl)silyl]-oxy}ethyl)-2,2-dimethylmorpholin [enantiomerenreines Isomer] in Tetrahydrofuran (148 mL) vorgelegt und bei RT mit 30.5 mL (30.5 mmol) Tetra-*n*-butylammoniumfluorid-Lösung (1.0 M in Tetrahydrofuran) versetzt. Es wurde 1 h bei RT gerührt und anschließend im Vakuum eingeengt. Der Rückstand wurde mittels präparativer RP-HPLC (Acetonitril/Wasser, isokratisch) gereinigt. Ausbeute: 1.97 g (38% d. Th., Enantiomerenverhältnis: ca. 85:15); es wurde auf dieser Stufe eine anteilige Isomerisierung des Stereozentrums auf einer der vorherigen Vorstufen festgestellt.
HPLC (Methode 7E): $R_t$ = 4.41 min, 85:15 *R*:*S*-Enantiomerenverhältnis;
LC-MS (Methode 1A): $R_t$ = 0.35 min; MS (ESIpos): m/z = 250 $[M+H]^+$;
$^1$H-NMR (400 MHz, DMSO-$d_6$): $\delta$ [ppm] = 7.31 (d, 4H), 7.22 ($m_c$, 1H), 4.45 (t, 1H), 3.93 (d, 1H), 3.60 (dd, 1H), 3.54-3.40 (m, 3H), 3.10 (d, 1H), 2.40-2.29 (m, 2H), 1.85 (d, 1H), 1.79-1.69 (m, 1H), 1.59 ($m_c$, 1H), 1.14 (s, 3H), 1.04 (s, 3H).

**Beispiel 77A**

2-[(3R)- 6,6-Dimethylmorpholin -3-yl]ethanol [Enantiomerengemisch, 2 Isomere]

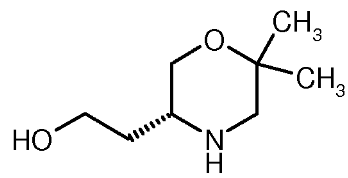

Es wurden 1.00 g (4.01 mmol) 2-[(3*R*)-4-Benzyl-6,6-dimethylmorpholin-3-yl]ethanol [Enantiomerengemisch, Enantiomerenverhältnis: ca. 85:15] in Ethanol (40.0 mL) vorgelegt, unter Argon mit 150 mg Palladium auf Kohle (10%ig) und 150 mg Palladiumhydroxid auf Kohle (20%ig) versetzt und anschließend für 4 h unter Wasserstoffatmosphäre unter Normaldruck gerührt. Die Reaktionslösung wurde über Kieselgur filtriert und im Vakuum eingeengt. Ausbeute: 680 mg (quant.).
GC-MS (Methode 2B): $R_t$ = 3.71 min; MS (EIpos): m/z = 159 $[M]^+$;
$^1$H-NMR (500 MHz, DMSO-$d_6$): $\delta$ [ppm] = 4.32 (br. s., 1H), 3.46 (t, 2H), 3.38 (dd, 1H), 3.21 (t, 1H), 2.64-2.54 (m, 2H), 2.47-2.42 (m, 1H), 1.36 ($m_c$, 2H), 1.18 (s, 3H), 1.02 (s, 3H), ein Proton verdeckt.

**Beispiel 78A**

1-Benzyl-2-ethyl-(4*R*)-4-ethoxypyrrolidin-1,2-dicarboxylat [Diastereomerengemisch, 2 Isomere]

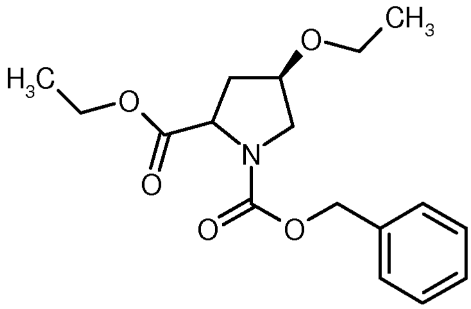

Es wurden 10.0 g (37.7 mmol) (4*R*)-1-[(Benzyloxy)carbonyl]-4-hydroxy-L-prolin in *N,N*-Dimethylformamid (110 mL) unter Argon vorgelegt und bei 0°C mit 1.96 g (49.0 mmol, 60%ige Suspension in Paraffinöl) Natriumhydrid versetzt. Die Reaktionsmischung wurde für 30 min gerührt und anschließend mit 7.54 mL (14.7 g, 94.2 mmol) Iodethan versetzt. Man ließ auf RT erwärmen, kühlte dann nochmals auf 0°C, versetzte mit 1.96 g (49.0 mmol, 60%ige Suspension in Paraffinöl) Natriumhydrid und rührte 30 min. Es wurden weitere 7.54 mL (14.7 g, 94.2 mmol) Iodethan zugegeben, wieder auf RT erwärmt und über Nacht gerührt. Die Reaktionsmischung wurde vorsichtig mit Wasser versetzt und mit Essigsäureethylester extrahiert. Die organische Phase wurde über Natriumsulfat getrocknet, filtriert und im Vakuum eingeengt. Das Rohprodukt wurde ohne weitere Reinigung in der nächsten Stufe eingesetzt. Ausbeute: 14.8 g (94% d. Th., Reinheit: 77%).
LC-MS (Methode 1A): $R_t$ = 1.06 min; MS (ESIpos): m/z = 322 $[M+H]^+$.

**Beispiel 79A**

Benzyl-(4*R*)-4-ethoxy-2-(hydroxymethyl)pyrrolidin-1-carboxylat [Diastereomerengemisch, 2 Isomere]

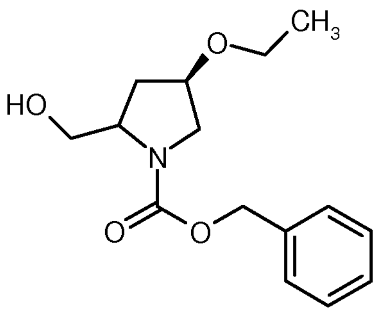

Es wurden 13.5 g (32.6 mmol, Reinheit: 77%) 1-Benzyl-2-ethyl-(4*R*)-4-ethoxypyrrolidin-1,2-dicarboxylat [Diastereomerengemisch, 2 Isomere] unter Argon in Tetrahydrofuran (150 mL) vorgelegt und bei 0°C mit 817 mg (37.5 mmol) Lithiumborhydrid versetzt. Man ließ die Reaktionsmischung auf RT erwärmen und rührte anschließend über Nacht bei RT. Es wurde vorsichtig mit Wasser (100 mL) versetzt, mit 2 N wässriger Hydrogenchlorid-Lösung auf pH = 1 eingestellt und anschließend mit Essigsäureethylester extrahiert. Die organische Phasen wurden mit gesättigter Natriumchlorid-Lösung gewaschen, über Natriumsulfat getrocknet, filtriert und im Vakuum eingeengt. Das Rohprodukt wurde mittels präparativer RP-HPLC (Acetonitril/Wasser) gereinigt. Ausbeute: 4.78 g (52% d. Th., Diastereomerenverhältnis: ca. 2:1).
LC-MS (Methode 1A): $R_t$ = 0.81 min (Diastereomer 1), $R_t$ = 0.83 min (Diastereomer 2);
MS (ESIpos): m/z = 280 $[M+H]^+$.

**Beispiel 80A**

[(4*R*)-4-Ethoxypyrrolidin-2-yl]methanol [Diastereomerengemisch, 2 Isomere]

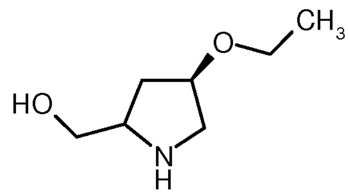

Es wurden 2.00 g (7.16 mmol) Benzyl-(4*R*)-4-ethoxy-2-(hydroxymethyl)pyrrolidin-1-carboxylat [Diastereomerengemisch, 2 Isomere] in Methanol (46.3 mL) vorgelegt, unter Argon mit 221 mg Palladium auf Kohle (10%ig) und 111 mg Platin(IV)oxid versetzt und unter Wasserstoffatmosphäre unter Normaldruck bis zum Ende der Wasserstoffaufnahme gerührt. Die Reaktionslösung wurde über Kieselgur filtriert, mit Methanol nachgewaschen und im Vakuum eingeengt. Ausbeute: 1.17 g (quant.).
MS (Methode 2C): m/z = 146 $[M+H]^+$;
$^1$H-NMR (400 MHz, DMSO-$d_6$): δ [ppm] = 4.04-2.67 (m, 10H), 2.10-1.30 (m, 2H), 1.22-0.97 (m, 3H).

**Beispiel 81A**

Benzyl-(4*R*)-4-ethoxy-2-formylpyrrolidin-1-carboxylat [Diastereomerengemisch, 2 Isomere]

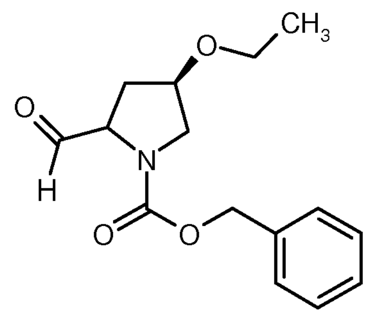

Es wurden 2.60 g (9.31 mmol) Benzyl-(4*R*)-4-ethoxy-2-(hydroxymethyl)pyrrolidin-1-carboxylat [Diastereomerengemisch, 2 Isomere] in Dichlormethan (46.6 mL) vorgelegt und bei 0°C mit 4.36 g (3.96 mL, 55.9 mmol) Dimethylsulfoxid, 9.62 g (13.0 mL, 129 mmol) *N,N*-Diisopropylethylamin und 5.93 g (37.2 mmol) Schwefeltrioxid-Pyridin-Komplex versetzt. Man ließ die Reaktionslösung auf RT erwärmen und rührte 3 h bei RT. Die Reaktionslösung wurde mit Dichlormethan verdünnt, mit Wasser und gesättigter Natriumchlorid-Lösung gewaschen, über Natriumsulfat getrocknet, filtriert und im Vakuum eingeengt. Das Rohprodukt wurde ohne weitere Reinigung in der nächsten Stufe eingesetzt. Ausbeute: 4.10 g (quant., Reinheit: 65%).
LC-MS (Methode 1A): $R_t$ = 0.96 min (enantiomerenreines Isomer 1), $R_t$ = 0.97 min (enantiomerenreines Isomer 2);
MS (ESIpos): m/z = 278 $[M+H]^+$.

**Beispiel 82A**

Benzyl-(4*R*)-4-ethoxy-2-vinylpyrrolidin-1-carboxylat [Diastereomerengemisch, 2 Isomere]

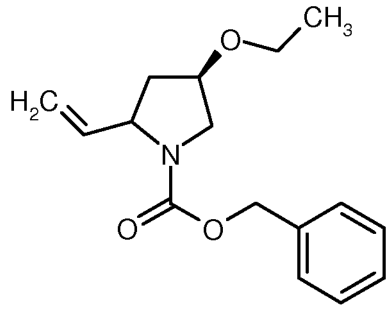

Zu 4.81 g (13.5 mmol) Methyltriphenylphosphoniumbromid in Tetrahydrofuran (30.8 mL) wurden bei 0°C unter Argon 4.23 mL (10.6 mmol, 2.5 M Lösung in n-Hexan) n-Butyllithium getropft. Die Reaktionslösung ließ man auf RT erwärmen. Es wurde 30 min bei RT gerührt, dann erneut auf 0°C abgekühlt und es wurden 4.10 g (9.61 mmol, Reinheit: 65%) Benzyl-(4R)-4-ethoxy-2-formylpyrrolidin-1-carboxylat [Diastereomerengemisch, 2 Isomere] in THF über 10 min zugetropft. Es wurde 30 min gerührt und anschließend die Reaktionlösung in Eiswasser gegossen. Es wurde mit Diethylether extrahiert, die organischen Phasen wurden über Natriumsulfat getrocknet, filtriert und im Vakuum eingeengt. Das Rohprodukt wurde mittels präparativer RP-HPLC (Acetonitril/Wasser) gereinigt. Ausbeute: 954 mg (36% d. Th., Diastereomerenverhältnis: ca. 2:1).
LC-MS (Methode 1A): $R_t$ = 1.11 min (Diastereomer 1), $R_t$ = 1.13 min (Diastereomer 2);
MS (ESIpos): m/z = 276 $[M+H]^+$.

**Beispiel 83A**

Benzyl-(4*R*)-4-ethoxy-2-(2-hydroxyethyl)pyrrolidin-1-carboxylat [Diastereomerengemisch, 2 Isomere]

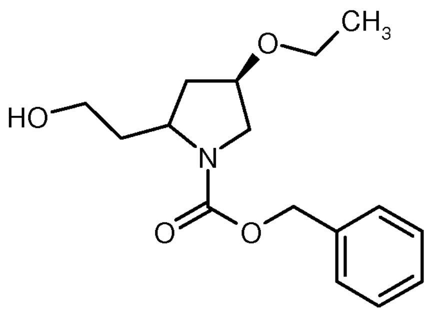

Zu 954 mg (3.47 mmol) Benzyl-(4*R*)-4-ethoxy-2-vinylpyrrolidin-1-carboxylat [Diastereomerengemisch, 2 Isomere] in Tetrahydrofuran (53 mL) wurden bei 0°C 34.7 mL (17.3 mmol, 0.5 M Lösung in Tetrahydrofuran) 9-Borabicyclo[3.3.1]nonan getropft. Man ließ die Reaktionslösung langsam auf RT erwärmen. Anschließend wurde bei 0°C langsam 1 N wässrige NatriumcarbonatLösung (40 mL) und anschließend 30%ige wässrige Wasserstoffperoxid-Lösung (40 mL) zugegeben. Die Reaktionslösung wurde auf RT erwärmt und 30 min gerührt. Die Reaktionslösung wurde anschließend mit Essigsäureethylester versetzt und die organische Phase mit Wasser und gesättigter Natriumchlorid-Lösung gewaschen. Die organischen Phasen wurden über Natriumsulfat getrocknet, filtriert und im Vakuum eingeengt. Das Rohprodukt wurde mittels präparativer RP-HPLC (Acetonitril/Wasser) gereinigt. Ausbeute: 781 mg (75% d. Th., Diastereomerenverhältnis: ca. 2.5:1).
LC-MS (Methode 1A): $R_t$ = 0.87 min (Diastereomer 1), $R_t$ = 0.90 min (Diastereomer 2);
MS (ESIpos): m/z = 294 $[M+H]^+$.

**Beispiel 84A**

2-[(4*R*)-4-Ethoxypyrrolidin-2-yl]ethanol [Diastereomerengemisch, 2 Isomere]

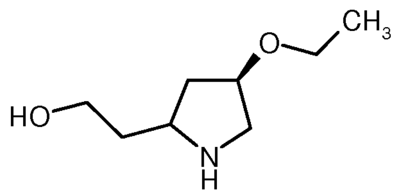

Es wurden 780 mg (2.66 mmol) Benzyl-(4*R*)-4-ethoxy-2-(2-hydroxyethyl)pyrrolidin-1-carboxylat [Diastereomerengemisch, 2 Isomere] in Methanol (17.2 mL) vorgelegt, unter Argon mit 82.2 mg Palladium auf Kohle (10%ig) und 41.1 mg Platin(IV)oxid versetzt und anschließend unter Wasserstoffatmosphäre unter Normaldruck bis zum Ende der Wasserstoffaufnahme gerührt. Die Reaktionslösung wurde über Kieselgur filtriert, mit Methanol nachgewaschen und im Vakuum eingeengt. Ausbeute: 465 mg (quant.).
MS (Methode 1C): m/z = 160 $[M+H-HC1]^+$.

**Beispiel 85A**

*tert*-Butyl-4-benzyl-5-oxo-4,7-diazaspiro[2.5]octan-7-carboxylat

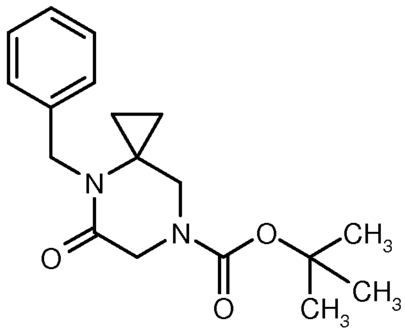

Unter Argon wurde bei 0°C zu 2.50 g (8.84 mmol) *tert*-Butyl-5-oxo-4,7-diazaspiro[2.5]octan-7-carboxylat in 80 mL THF portionsweise 2.47 g (61.9 mmol) Natriumhydrid gegeben und es wurde 30 min bei 0°C gerührt. Dann wurden 1.26 mL (1.81 g, 10.6 mmol) Benzylbromid zugetropft und es wurde über Nacht bei Raumtemperatur gerührt. Anschließend wurde auf 0°C gekühlt, mit 1.24 g (30.9 mmol) Natriumhydrid versetzt und 30 min bei 0°C gerührt. 0.63 mL (0.91 g, 5.3 mmol) Benzylbromid wurden zugetropft und es wurde über Nacht bei Raumtemperatur gerührt. Anschließend wurde bei 0°C zunächst mit Ethanol und dann mit Wasser und Essigsäureethylester versetzt. Nach Trennung der Phasen wurde die wässrige Phase zweimal mit Essigsäureethylester extrahiert und die vereinigten organischen Phasen wurden über Natriumsulfat getrocknet. Nach Filtration wurde das Filtrat im Vakuum eingeengt, der Rückstand am Hochvakuum getrocknet und mittels Kieselgelchromatographie (Cyclohexan/ Essigsäureethylester 10:1) und anschließend durch präparative HPLC (RP18 Säule, Laufmittel: Acetonitril/Wasser-Gradient) gereinigt. Man erhielt 1.98 g (71% d. Th.) des gewünschten Produktes.
LC-MS (Methode 1A): $R_t$ = 1.09 min; MS (ESIpos): m/z = 317 $[M+H]^+$
$^1$H-NMR (400 MHz, DMSO-$d_6$): $\delta$ [ppm] = 7.38-7.14 (m, 5H), 4.41 (s, 2H), 4.16 (br. s., 2H), 1.40 (br. s., 9H), 0.98-0.89 (m, 2H), 0.79-0.72 (m, 2H).

**Beispiel 86A**

*tert*-Butyl-4-benzyl-6-methyl-5-oxo-4,7-diazaspiro[2.5]octan-7-carboxylat [Racemat]

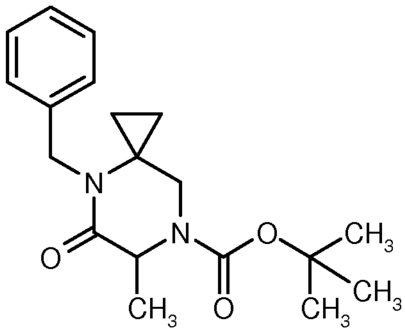

Bei -78°C wurden unter Argon zu 1.20 g (3.79 mmol) *tert*-Butyl-4-benzyl-5-oxo-4,7-diazaspiro[2.5]octan-7-carboxylat in 48 mL THF 11.38 mL (11.38 mmol) einer 1 M Lithiumhexamethyldisilazid-Lösung in THF getropft und es wurde 30 min bei -78°C gerührt. Dann wurden 0.47 mL (7.59 mmol) Methyliodid zugetropft und es wurde 1.5 h gerührt. Anschließend wurde bei 0°C zunächst mit gesättigter wässriger Ammoniumchlorid-Lösung und anschließend mit Essigsäureethylester versetzt. Nach Trennung der Phasen wurde die wässrige Phase zweimal mit Essigsäureethylester extrahiert, die vereinigten organischen Phasen wurden mit gesättigter wässriger Natriumchlorid-Lösung gewaschen und dann über Natriumsulfat getrocknet. Nach Filtration wurde das Filtrat im Vakuum eingeengt, der Rückstand am Hochvakuum getrocknet, in Acetonitril und Wasser gelöst und durch präparative HPLC (RP18 Säule, Laufmittel: Acetonitril/Wasser-Gradient) gereinigt. Man erhielt 0.54 g (41% d. Th.) des gewünschten Produktes.

**Beispiel 87A**

*tert*-Butyl-6-methyl-5-oxo-4,7-diazaspiro[2.5]octan-7-carboxylat [Racemat]

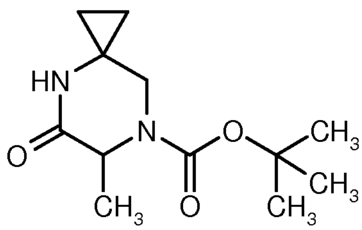

Bei -78°C wurden 10 mL (7.70 g, 452 mmol) Ammoniak mit 107 mg (15.5 mmol) Lithium versetzt und einige Minuten gerührt. Dann wurden 540 mg (1.55 mmol) *tert*-Butyl-4-benzyl-6-methyl-5-oxo-4,7-diazaspiro[2.5]octan-7-carboxylat [Racemat] in 5 mL THF zugetropft, langsam auf Raumtemperatur erwärmt und dann über Nacht bei Raumtemperatur gerührt. Anschließend wurde bei 0°C zunächst mit gesättigter wässriger Ammoniumchlorid-Lösung und dann mit Essigsäureethylester versetzt. Nach Trennung der Phasen wurde die wässrige Phase zweimal mit Essigsäureethylester extrahiert, die vereinigten organischen Phasen wurden mit gesättigter wässriger Natriumchlorid-Lösung gewaschen und über Natriumsulfat getrocknet. Nach Filtration wurde das Filtrat im Vakuum eingeengt und der Rückstand am Hochvakuum getrocknet. Man erhielt 353 mg des Rohproduktes, das ohne weitere Aufreinigung eingesetzt wurde.
MS (Methode 1C): m/z = 241 $[M+H]^+$.

**Beispiel 88A**

6-Methyl-4,7-diazaspiro[2.5]octan-5-on Trifluoracetat [Racemat]

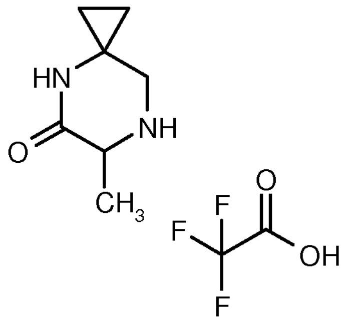

Zu 331 mg (1.38 mmol) *tert*-Butyl-6-methyl-5-oxo-4,7-diazaspiro[2.5]octan-7-carboxylat [Racemat] in 10 mL Dichlormethan wurden 1.06 mL (1.57 g, 13.8 mmol) Triflouressigsäure gegeben und es wurde 2 h bei Raumtemperatur gerührt. Dann wurde im Vakuum eingeengt und der Rückstand in Dichlormethan gelöst. Es wurde im Vakuum eingeengt und der erhaltene Rückstand erneut in Dichlormethan gelöst, im Vakuum vom Lösungsmittel befreit und im Hochvakuum getrocknet. Das erhaltene Rohprodukt (605 mg) wurde ohne Aufreinigung weiter eingesetzt.
MS (Methode 1C): m/z = 141 $[M+H]^+$.

## Ausführungsbeispiele

### Beispiel 1

(2-{[1-(3-Chlorphenyl)-2-fluorethyl]amino}-7-methoxy-1,3-benzoxazol-5-yl)(4-hydroxy-3-methylpiperidin-1-yl)methanon [1:1 *trans*-Diastereomerengemisch, 2 Isomere]

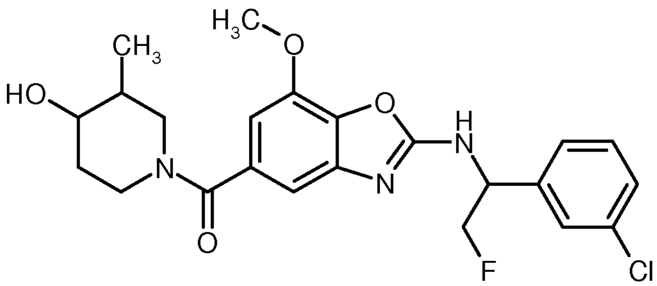

Es wurden 200 mg (0.250 mmol, Reinheit: 46%) 2-{[1-(3-Chlorphenyl)-2-fluorethyl]amino}-7-methoxy-1,3-benzoxazol-5-carbonsäure [enantiomerenreines Isomer] und 34.9 mg (0.300 mmol) 3-Methylpiperidin-4-ol [racemisches *trans*-Isomer, 2 Isomere] in *N,N*-Dimethylformamid (2.00 mL) vorgelegt und mit 131 mg (176 μL, 1.01 mmol) *N,N*-Diisopropylethylamin versetzt. Anschließend wurden bei RT 115 mg (0.300 mmol) HATU zugegeben und 1 h gerührt. Es wurden 17.5 mg (0.150 mmol) 3-Methylpiperidin-4-ol [racemisches *trans*-Isomer], 66.5 mg (88 μL, 0.505 mmol) *N,N*-Diisopropylethylamin und 57.5 mg (0.150 mmol) HATU zugegeben und nachfolgend über Nacht gerührt. Die Reaktionslösung wurde ohne weitere Aufarbeitung mittels präparativer RP-HPLC (Acetonitril/Wasser) gereinigt. Ausbeute: 76.6 mg (65% d. Th.).
LC-MS (Methode 1A): $R_t$ = 0.88 min; MS (ESIpos): m/z = 462 $[M+H]^+$;
$^1$H-NMR (400 MHz, DMSO-$d_6$): δ [ppm] = 8.99 (d, 1H), 7.58 (s, 1H), 7.50-7.28 (m, 3H), 6.82 (s, 1H), 6.69 (s, 1H), 5.24 ($m_c$, 1H), 4.84-4.49 (m, 3H), 4.25 (br. s., 1H), 3.91 (s, 3H), 3.50 (br. s., 1H), 3.22-3.08 (m, 1H), 3.07-2.78 (br. m., 1H), 1.91-1.64 (br. m., 1H), 1.49-1.20 (m, 2H), 0.85 (br. d., 3H).

### Beispiel 2

(2-{[1-(3-Chlorphenyl)-2-fluorethyl]amino}-7-methoxy-1,3-benzoxazol-5-yl)(4-hydroxy-3-methylpiperidin-1-yl)methanon [enantiomerenreines *trans*-Isomer 1]

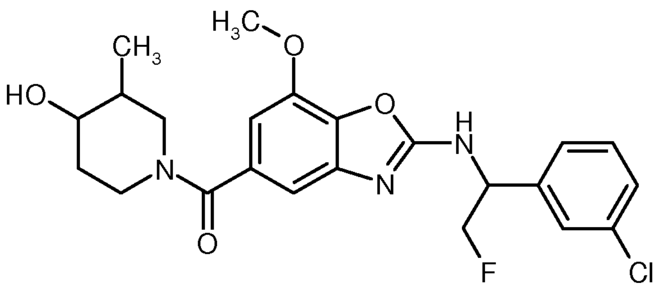

Diastereomerentrennung an chiraler Phase von 70.0 mg der Verbindung aus Beispiel 1 nach Methode 3D ergab 32.0 mg des Beispiels 2 (enantiomerenreines *trans*-Isomer 1) und 32.0 mg des Beispiels 3 (enantiomerenreines *trans-Isomer* 2).
HPLC (Methode 3E): $R_t$ = 9.37 min, 99.0% *de;*
LC-MS (Methode 1A): $R_t$ = 0.91 min; MS (ESIpos): m/z = 462 $[M+H]^+$;
$^1$H-NMR (400 MHz, DMSO-$d_6$): δ [ppm] = 8.99 (d, 1H), 7.58 (s, 1H), 7.50-7.28 (m, 3H), 6.82 (s, 1H), 6.69 (s, 1H), 5.24 ($m_c$, 1H), 4.84-4.49 (m, 3H), 4.25 (br. s., 1H), 3.91 (s, 3H), 3.50 (br. s., 1H), 3.22-3.08 (m, 1H), 3.07-2.78 (br. m., 1H), 1.91-1.64 (br. m., 1H), 1.49-1.20 (m, 2H), 0.85 (br. d., 3H).

**Beispiel 3**

(2-{[1-(3-Chlorphenyl)-2-fluorethyl]amino}-7-methoxy-1,3-benzoxazol-5-yl)(4-hydroxy-3-methylpiperidin-1-yl)methanon [enantiomerenreines trans-Diastereomer 2]

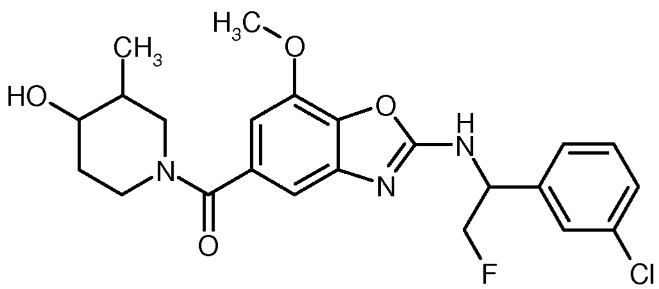

Diastereomerentrennung an chiraler Phase von 70.0 mg der Verbindung aus Beispiel 1 nach Methode 3D ergab 32.0 mg des Beispiels 2 (enantiomerenreines Isomer 1) und 32.0 mg des Beispiels 3 (enantiomerenreines Isomer 2).
HPLC (Methode 3E): $R_t$ = 15.1 min, 99.0% *de;*
LC-MS (Methode 1A): $R_t$ = 0.91 min; MS (ESIpos): m/z = 462 $[M+H]^+$;
$^1$H-NMR (400 MHz, DMSO-$d_6$): δ [ppm] = 8.99 (d, 1H), 7.58 (s, 1H), 7.50-7.28 (m, 3H), 6.82 (s, 1H), 6.69 (s, 1H), 5.24 ($m_c$, 1H), 4.84-4.49 (m, 3H), 4.25 (br. s., 1H), 3.91 (s, 3H), 3.50 (br. s., 1H), 3.22-3.08 (m, 1H), 3.07-2.78 (br. m., 1H), 1.91-1.64 (br. m., 1H), 1.49-1.20 (m, 2H), 0.85 (br. d., 3H).

**Beispiel 4**

(2-{[1-(3-Chlorphenyl)-2-fluorethyl]amino}-7-methoxy-1,3-benzoxazol-5-yl)[5-(hydroxymethyl)-2,2-dimethylmorpholin-4-yl]methanon [1:1 Diastereomerengemisch, 2 Isomere]

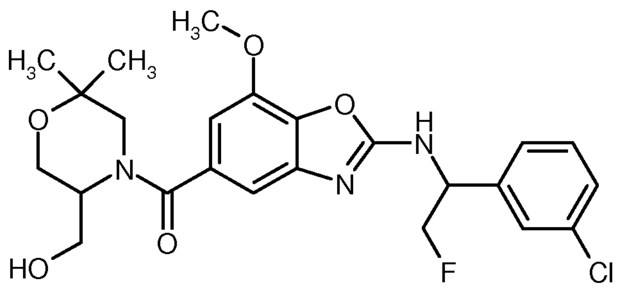

Es wurden 80.0 mg (0.101 mmol, Reinheit: 46%) 2-{[1-(3-Chlorphenyl)-2-fluorethyl]amino}-7-methoxy-1,3-benzoxazol-5-carbonsäure [enantiomerenreines Isomer] und 38.2 mg (0.263 mmol) (6,6-Dimethylmorpholin-3-yl)methanol [Racemat] in *N,N*-Dimethylformamid (1.01 mL) vorgelegt und mit 99.2 mg (134 μL, 0.786 mmol) *N,N*-Diisopropylethylamin versetzt. Anschließend wurden bei RT 100 mg (0.263 mmol) HATU zugegeben und es wurde über Nacht gerührt. Die Reaktionslösung wurde anschließend ohne weitere Aufarbeitung mittels präparativer RP-HPLC (Acetonitril/Wasser) gereinigt. Ausbeute: 55.1 mg (99% d. Th., Reinheit: 90%).
LC-MS (Methode 3A): $R_t$ = 2.02 min; MS (ESIpos): m/z = 492 $[M+H]^+$;
$^1$H-NMR (400 MHz, DMSO-$d_6$): δ [ppm] = 8.98 ($m_c$, 1H), 7.58 (s, 1H), 7.50-7.37 (m, 3H), 6.90 (br. s., 1H), 6.74 (br. s., 1H), 5.24 ($m_c$, 1H), 4.88 (br. s., 1H), 4.80-4.51 (m, 2H), 4.01-3.38 (m, 9H), 3.20-2.74 (m, 1H), 1.14 (br. s., 6H).

**Beispiel 5**

(2-{[1-(3-Chlorphenyl)-2-fluorethyl]amino}-7-methoxy-1,3-benzoxazol-5-yl)[5-(hydroxymethyl)-2,2-dimethylmorpholin-4-yl]methanon [enantiomerenreines Isomer 1]

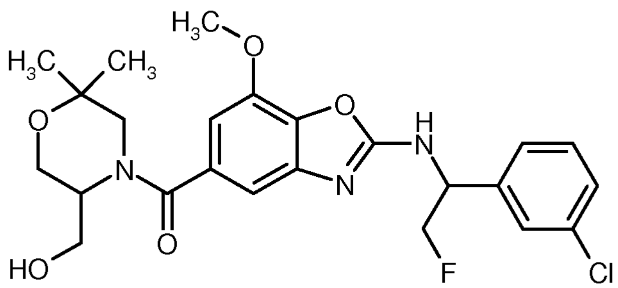

Diastereomerentrennung an chiraler Phase von 50.0 mg der Verbindung aus Beispiel 4 nach Methode 4D ergab nach nochmaliger Reinigung durch präparative RP-HPLC (Acetonitril/Wasser) 16.2 mg des Beispiels 5 (enantiomerenreines Isomer 1) und 22.1 mg des Beispiels 6 (enantiomerenreines Isomer 2).
HPLC (Methode 4E): $R_t$ = 5.05 min, >99.0% *de;*
LC-MS (Methode 1A): $R_t$ = 0.94 min; MS (ESIpos): m/z = 492 $[M+H]^+$.

**Beispiel 6**

(2-{[1-(3-Chlorphenyl)-2-fluorethyl]amino}-7-methoxy-1,3-benzoxazol-5-yl)[5-(hydroxymethyl)-2,2-dimethylmorpholin-4-yl]methanon [enantiomerenreines Isomer 2]

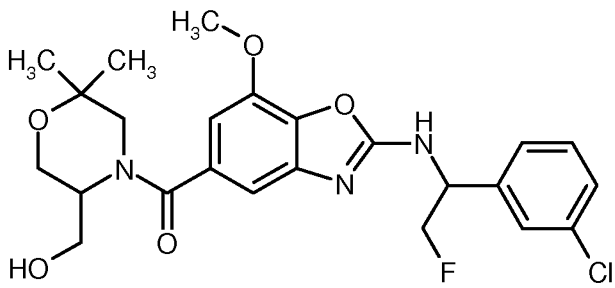

Diastereomerentrennung an chiraler Phase von 50.0 mg der Verbindung aus Beispiel 4 nach Methode 4D ergab nach nochmaliger Reinigung durch präparative RP-HPLC (Acetonitril/Wasser) 16.2 mg des Beispiels 5 (enantiomerenreines Isomer 1) und 22.1 mg des Beispiels 6 (enantiomerenreines Isomer 2).
HPLC (Methode 4E): $R_t$ = 6.50 min, >96.6% *de;*
LC-MS (Methode 1A): $R_t$ = 0.95 min; MS (ESIpos): m/z = 492 $[M+H]^+$.

Beispiel 7

(2-{[1-(3-Chlorphenyl)-2-fluorethyl]amino}-7-methoxy-1,3-benzoxazol-5-yl)[(5*R*)-2,5-dimethylmorpholin-4-yl]methanon [enantiomerenreines Isomer 2]

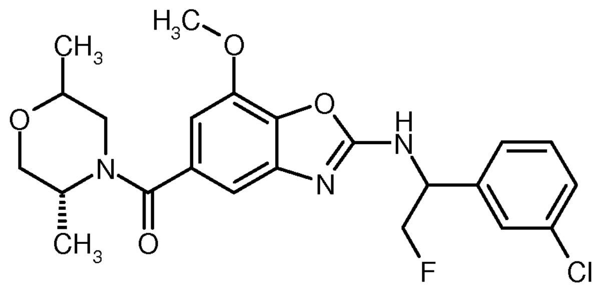

Es wurden 100 mg (0.130 mmol, Reinheit: 46%) 2-{[1-(3-Chlorphenyl)-2-fluorethyl]amino}-7-methoxy-1,3-benzoxazol-5-carbonsäure [enantiomerenreines Isomer] und 23.0 mg (0.150 mmol) (5*R*)-2,5-Dimethylmorpholin Hydrochlorid [enantiomerenreines Isomer 2] in *N,N*-Dimethylformamid (1.00 mL) vorgelegt und mit 65.3 mg (88.1 μL, 0.510 mmol) *N,N*-Diisopropylethylamin versetzt. Anschließend wurden bei RT 57.7 mg (0.150 mmol) HATU zugegeben und es wurde über Nacht gerührt. Die Reaktionslösung wurde anschließend ohne weitere Aufarbeitung mittels präparativer RP-HPLC (Acetonitril/Wasser) gereinigt. Ausbeute: 25.1 mg (40% d. Th.).
LC-MS (Methode 1A): $R_t$ = 1.01 min; MS (ESIpos): m/z = 462 $[M+H]^+$;
$^1$H-NMR (400 MHz, DMSO-$d_6$): δ [ppm] = 9.00 (d, 1H), 7.58 (s, 1H), 7.49-7.35 (m, 3H), 6.84 (br. s., 1H), 6.71 (br. s., 1H), 5.24 ($m_c$, 1H), 4.79-4.08 (m, 3H), 3.92 (s, 3H), 3.72-3.21 (m, 5H), 1.32-0.90 (m, 6H).

**Beispiel 8**

(2-{[(1-(3-Chlorphenyl)-2-fluorethyl]amino}-7-methoxy-1,3-benzoxazol-5-yl)[(5*R*)-2,5-dimethylmorpholin-4-yl]methanon [enantiomerenreines Isomer 1]

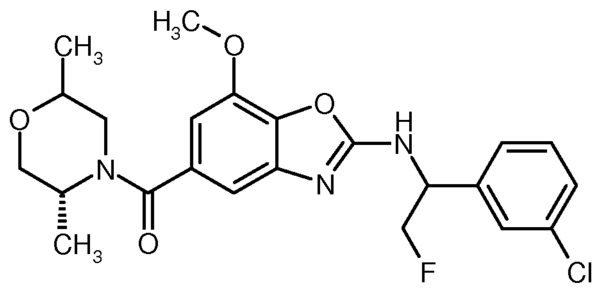

Es wurden 100 mg (0.130 mmol, Reinheit: 46%) 2-{[(1-(3-Chlorphenyl)-2-fluorethyl]amino}-7-methoxy-1,3-benzoxazol-5-carbonsäure [enantiomerenreines Isomer] und 23.0 mg (0.150 mmol) (5*R*)-2,5-Dimethylmorpholin Hydrochlorid [enantiomerenreines Isomer 1] in *N,N*-Dimethylformamid (1.00 mL) vorgelegt und mit 65.3 mg (88.1 μL, 0.510 mmol) *N,N*-Diisopropylethylamin versetzt. Anschließend wurden bei RT 57.7 mg (0.150 mmol) HATU zugegeben und über Nacht gerührt. Die Reaktionslösung wurde ohne weitere Aufarbeitung mittels präparativer RP-HPLC (Acetonitril/Wasser) gereinigt. Ausbeute: 27.4 mg (44% d. Th.).
LC-MS (Methode 1A): $R_t$ = 0.99 min; MS (ESIpos): m/z = 462 $[M+H]^+$;
$^1$H-NMR (400 MHz, DMSO-$d_6$): δ [ppm] = 9.00 (d, 1H), 7.58 (s, 1H), 7.50-7.34 (m, 3H), 6.83 (s, 1H), 6.69 (s, 1H), 5.24 ($m_c$, 1H), 4.81-4.52 (m, 2H), 4.04 (br. s., 1H), 3.97-3.88 (s, 4H), 3.84 (dd, 1H), 3.50 (d, 1H), 3.37 (dd, 1H), 3.27 (d, 1H), 1.23 (d, 3H), 1.15 (d, 3H).

**Beispiel 9**

(2-{[1-(3-Chlorphenyl)-2-fluorethyl]amino}-7-methoxy-1,3-benzoxazol-5-yl)[(5*R*)-2-(2-hydroxyethyl)-2,5-dimethylmorpholin-4-yl]methanon [1:1 Diastereomerengemisch, 2 Isomere]

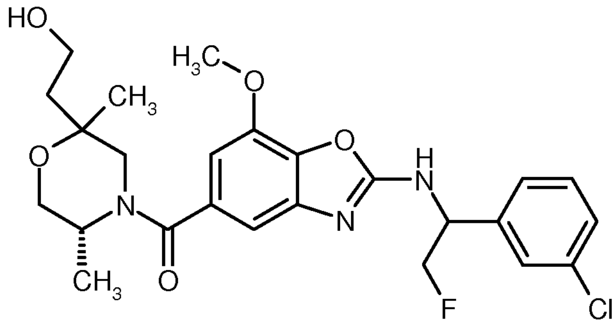

Es wurden 120 mg (0.250 mmol, Reinheit: 77%) 2-{[1-(3-Chlorphenyl)-2-fluorethyl]amino}-7-methoxy-1,3-benzoxazol-5-carbonsäure [Racemat] und 48.4 mg (0.300 mmol) 2-[(5R)-2,5-Dimethylmorpholin-2-yl]ethanol [Beispiel 31A, enantiomerenreines Isomer] in *N,N*-Dimethylformamid (1.17 mL) vorgelegt und mit 115 mg (154 μL, 0.890 mmol) *N,N*-Diisopropylethylamin versetzt. Anschließend wurden bei RT 116 mg (0.300 mmol) HATU zugegeben und es wurde 2 h gerührt. Die Reaktionslösung wurde ohne weitere Aufarbeitung mittels präparativer RP-HPLC (Acetonitril/Wasser) gereinigt. Ausbeute: 112 mg (84% d. Th.).
LC-MS (Methode 1A): $R_t$ = 0.93 min; MS (ESIpos): m/z = 506 $[M+H]^+$;
$^1$H-NMR (400 MHz, DMSO-$d_6$): δ [ppm] = 9.00 (dd, 1H), 7.58 (br. s., 1H), 7.50-7.32 (m, 3H), 6.82 (br. s., 1H), 6.67 (s, 1H), 5.24 ($m_c$, 1H), 4.80-4.52 (m, 2H), 4.31 (t, 1H), 3.92 (s, 3H), 3.73 (d, 1H), 2.96 (br. s., 0.5H), 2.02 ($m_c$, 1H), 1.43 (br. s., 0.5H), 1.29-1.02 (m, 6H), sechs Protonen verdeckt.

**Beispiel 10**

(2-{[1-(3-Chlorphenyl)-2-fluorethyl]amino}-7-methoxy-1,3-benzoxazol-5-yl)[(5*R*)-2-(2-hydroxyethyl)-2,5-dimethylmorpholin-4-yl]methanon [enantiomerenreines Isomer 1]

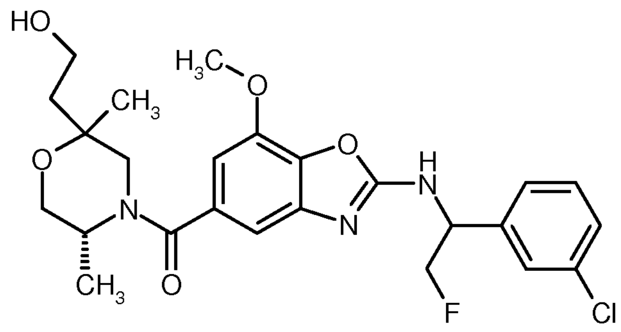

Diastereomerentrennung an chiraler Phase von 102 mg der Verbindung aus Beispiel 9 nach Methode 2D ergab nach erneuter Reinigung mittels präparativer RP-HPLC (Acetonitril/Wasser) 24.7 mg der Zielverbindung des Beispiels 10 (enantiomerenreines Isomer 1) und 24.0 mg der Zielverbindung des Beispiels 11 (enantiomerenreines Isomer 2).
HPLC (Methode 2E): $R_t$ = 13.6 min, >99.0% *de;*
LC-MS (Methode 1A): $R_t$ = 0.93 min; MS (ESIpos): m/z = 506 $[M+H]^+$;
$^1$H-NMR (400 MHz, DMSO-$d_6$): $\delta$ [ppm] = 9.00 (d, 1H), 7.59 (s, 1H), 7.51-7.36 (m, 3H), 6.83 (s, 1H), 6.67 (s, 1H), 5.24 ($m_c$, 1H), 4.80-4.52 (m, 2H), 4.32 (t, 1H), 3.92 (s, 3H), 3.73 (dd, 1H), 2.96 (br. s., 0.5H), 2.00 ($m_c$, 1H), 1.43 (br. s., 0.5H), 1.26-1.02 (m, 6H), sechs Protonen verdeckt.

**Beispiel 11**

(2-{[1-(3-Chlorphenyl)-2-fluorethyl]amino}-7-methoxy-1,3-benzoxazol-5-yl)[(5*R*)-2-(2-hydroxyethyl)-2,5-dimethylmorpholin-4-yl]methanon [enantiomerenreines Isomer 2]

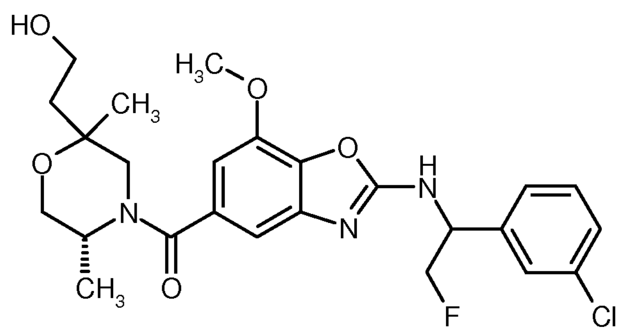

Diastereomerentrennung an chiraler Phase von 102 mg der Verbindung aus Beispiel 9 nach Methode 2D ergab nach erneuter Reinigung mittels präparativer RP-HPLC (Acetonitril/Wasser) 24.7 mg der Zielverbindung des Beispiels 10 (enantiomerenreines Isomer 1) und 24.0 mg der Zielverbindung des Beispiels 11 (enantiomerenreines Isomer 2).
HPLC (Methode 2E): $R_t$ = 15.6 min, 98.5% *de;*
LC-MS (Methode 1A): $R_t$ = 0.93 min; MS (ESIpos): m/z = 506 $[M+H]^+$;
$^1$H-NMR (400 MHz, DMSO-$d_6$): $\delta$ [ppm] = 9.01 (br. s., 1H), 7.58 (s, 1H), 7.51-7.29 (m, 3H), 6.81 (s, 1H), 6.67 (s, 1H), 5.24 (br. d., 1H), 4.86-4.48 (m, 2H), 4.32 (t, 1H), 3.92 (s, 3H), 3.73 (dd, 1H), 2.96 (br. s., 0.5H), 2.00 ($m_c$, 1H), 1.43 (br. s., 0.5H), 1.21 (d, 3H), 1.07 (br. s., 3H), sechs Protonen verdeckt.

**Beispiel 12**

(2-{[1-(3-Chlorphenyl)-2-fluorethyl]amino}-7-methoxy-1,3-benzoxazol-5-yl)[(5*R*)-2-(2-hydroxypropyl)-2,5-dimethylmorpholin-4-yl]methanon [1:1 Diastereomerengemisch, 2 Isomere]

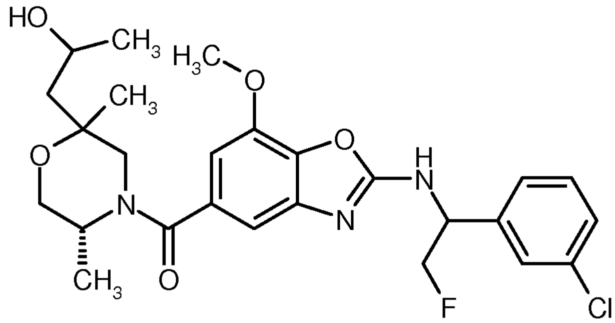

Es wurden 270 mg (0.170 mmol, Reinheit: 23%) 2-{[1-(3-Chlorphenyl)-2-fluorethyl]amino}-7-methoxy-1,3-benzoxazol-5-carbonsäure [Racemat] und 35.8 mg (0.210 mmol) 1-[(5*R*)-2,5-Dimethylmorpholin-2-yl]propan-2-ol [Beispiel 34A, enantiomerenreines Isomer] in *N,N*-Dimethylformamid (793 µL) vorgelegt und mit 78.0 mg (105 µL, 0.600 mmol) *N,N*-Diisopropylethylamin versetzt. Anschließend wurden bei RT 78.6 mg (0.210 mmol) HATU zugegeben und es wurde über Nacht gerührt. Die Reaktionslösung wurde ohne weitere Aufarbeitung mittels präparativer RP-HPLC (Acetonitril/Wasser) gereinigt. Das erhaltene Rohprodukt (20.0 mg) wurde nach Methode 1E nochmals gereinigt. Man erhielt 9.0 mg der Zielverbindung (10% d. Th.).
LC-MS (Methode 1A): $R_t$ = 0.98 min; MS (ESIpos): m/z = 520 $[M+H]^+$;
$^1$H-NMR (400 MHz, DMSO-$d_6$): δ [ppm] = 8.97 (dd, 1H), 7.58 (br. s., 1H), 7.50-7.33 (m, 3H), 6.81 (d, 1H), 6.67 (s, 1H), 5.24 ($m_c$, 1H), 4.81-4.52 (m, 2H), 4.22 (d, 1H), 3.92 (s, 3H), 3.70 (d, 2H), 2.97 (br. s., 1H), 1.96-1.85 (m, 1H), 1.35-1.01 (m, 10H), drei Protonen verdeckt.

## Beispiel 13

(2-{[1-(3-Chlorphenyl)-2-fluorethyl]amino}-7-methoxy-1,3-benzoxazol-5-yl)[5-(2-hydroxyethyl)-2-methylmorpholin-4-yl]methanon [1:1 Diastereomerengemisch, 2 Isomere]

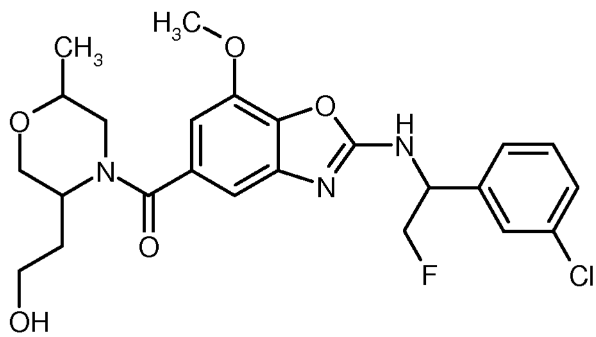

Es wurden 200 mg (0.25 mmol, Reinheit: 46%) 2-{[1-(3-Chlorphenyl)-2-fluorethyl]amino}-7-methoxy-1,3-benzoxazol-5-carbonsäure [enantiomerenreines Isomer] und 44.0 mg (0.30 mmol) 2-(6-Methylmorpholin-3-yl)ethanol [Racemat] in *N,N*-Dimethylformamid (2.00 mL) vorgelegt und mit 131 mg (176 µL, 1.01 mmol) *N,N*-Diisopropylethylamin versetzt. Anschließend wurde bei RT 115 mg (0.30 mmol) HATU zugegeben und es wurde 1 h gerührt. Es wurden weitere 22.0 mg (0.15 mmol) 2-(6-Methylmorpholin-3-yl)ethanol [Racemat], 65.5 mg (88 µL, 0.51 mmol) *N,N*-Diisopropylethylamin und 57.5 mg (0.15 mmol) HATU zugegeben und anschließend über Nacht bei RT gerührt. Die Reaktionslösung wurde ohne weitere Aufarbeitung mittels präparativer RP-HPLC (Acetonitril/Wasser) gereinigt. Ausbeute: 131 mg (97% d. Th.).
LC-MS (Methode 1A): $R_t$ = 0.90 min; MS (ESIpos): m/z = 492 $[M+H]^+$;
$^1$H-NMR (400 MHz, DMSO-$d_6$): δ [ppm] = 8.99 (br. d., 1H), 7.58 (s, 1H), 7.49-7.35 (m, 3H), 6.84 (br. s., 1H), 6.71 (br. s., 1H), 5.24 ($m_c$, 1H), 4.82-4.13 (m, 4H), 3.92 (s, 3H), 3.83-3.22 (m, 6H), 3.06-2.60 (m, 1H), 1.98-1.73 (m, 2H), 1.22-0.87 (m, 3H).

**Beispiel 14**

(2-{[1-(3-Chlorphenyl)-2-fluorethyl]amino}-7-methoxy-1,3-benzoxazol-5-yl)[(5*R*)-5-(2-hydroxyethyl)-2-methylmorpholin-4-yl]methanon [enantiomerenreines Isomer 1]

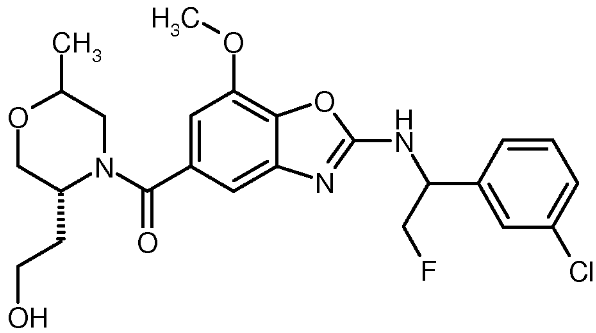

Diastereomerentrennung an chiraler Phase von 120 mg der Verbindung aus Beispiel 13 nach Methode 3D ergab 55.9 mg des Beispiels 14 (enantiomerenreines Isomer 1) und 56.2 mg des Beispiels 15 (enantiomerenreines Isomer 2).
HPLC (Methode 3E): $R_t$ = 8.39 min, 99.9% *de;*
LC-MS (Methode 1A): $R_t$ = 0.94 min; MS (ESIpos): m/z = 492 $[M+H]^+$;
$^1$H-NMR (400 MHz, DMSO-$d_6$): $\delta$ [ppm] = 8.99 (br. d., 1H), 7.58 (s, 1H), 7.50-7.30 (m, 3H), 6.84 (br. s., 1H), 6.71 (br. s., 1H), 5.23 ($m_c$, 1H), 4.80-4.14 (m, 4H), 3.92 (s, 3H), 3.83-3.22 (m, 6H), 3.03-2.60 (m, 1H), 2.01-1.75 (m, 2H), 1.19-0.91 (m, 3H).

**Beispiel 15**

(2-{[1-(3-Chlorphenyl)-2-fluorethyl]amino}-7-methoxy-1,3-benzoxazol-5-yl)[(5*S*)-5-(2-hydroxyethyl)-2-methylmorpholin-4-yl]methanon [enantiomerenreines Isomer 2]

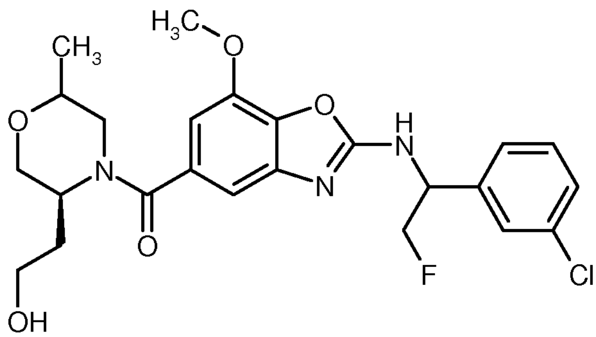

Methode 1:

Diastereomerentrennung an chiraler Phase von 120 mg der Verbindung aus Beispiel 13 nach Methode 3D ergab 55.9 mg des Beispiels 14 (enantiomerenreines Isomer 1) und 56.2 mg des Beispiels 15 (enantiomerenreines Isomer 2).
HPLC (Methode 3E): $R_t$ = 16.2 min, 99.9% *de;*
LC-MS (Methode 1A): $R_t$ = 0.93 min; MS (ESIpos): m/z = 492 $[M+H]^+$;
$^1$H-NMR (400 MHz, DMSO-$d_6$): $\delta$ [ppm] = 9.06-8.92 (m, 1H), 7.58 (s, 1H), 7.50-7.35 (m, 3H), 6.89-6.83 (m, 1H), 6.70 (br. s., 1H), 5.24 ($m_c$, 1H), 4.82-4.14 (m, 4H), 3.92 (s, 3H), 3.83-3.21 (m, 6H), 3.05-2.59 (m, 1H), 2.01-1.70 (m, 2H), 1.21-0.90 (m, 3H).

Methode 2:

Es wurden 500 mg (1.23 mmol, Reinheit: 89%) 2-{[1-(3-Chlorphenyl)-2-fluorethyl]amino}-7-methoxy-1,3-benzoxazol-5-carbonsäure [Beispiel 13A, enantiomerenreines Isomer] und 232 mg (1.60 mmol) 2-[(3*S*)-6-Methylmorpholin-3-yl]ethanol [Beispiel 42A, enantiomerenreines Isomer] in *N,N*-Dimethylformamid (20.0 mL) vorgelegt und mit 477 mg (643 μL, 3.69 mmol) *N,N*-Diisopropylethylamin versetzt. Es wurden bei RT 654 mg (1.72 mmol) HATU zugegeben, 14 h gerührt und anschließend ohne weitere Aufarbeitung mittels präparativer RP-HPLC (Acetonitril/Wasser) gereinigt. Das erhaltene Produkt wurde durch HPLC an chiraler Phase nach Methode 3D von Spuren des Minderisomers befreit. Ausbeute: 423 mg (70% d. Th.).

Drehwert: $[\alpha]_D^{20} = 63.79°$ (c = 0.625, Chloroform);

HPLC (Methode 3E): $R_t$ = 14.0 min, 99.9% *de;*

LC-MS (Methode 1A): $R_t$ = 0.93 min; MS (ESIpos): m/z = 492 $[M+H]^+$;

$^1$H-NMR (400 MHz, DMSO-$d_6$): δ [ppm] = 9.04-8.94 (m, 1H), 7.58 (s, 1H), 7.50-7.35 (m, 3H), 6.90-6.81 (m, 1H), 6.70 (br. s., 1H), 5.24 ($m_c$, 1H), 4.84-4.12 (m, 4H), 3.92 (s, 3H), 3.84-3.19 (m, 6H), 3.07-2.59 (m, 1H), 2.03-1.71 (m, 2H), 1.21-0.90 (m, 3H).

Nach Strukturbestimmung durch Komplexbildung von humanem α-Thrombin mit Beispiel 15 im Kristall handelt es sich bei dieser Verbindung um (2-{[(1*S*)-1-(3-Chlorphenyl)-2-fluorethyl]amino}-7-methoxy-1,3-benzoxazol-5-yl)[(2*S*,5*S*)-5-(2-hydroxyethyl)-2-methylmorpholin-4-yl]methanon mit der folgenden Formel

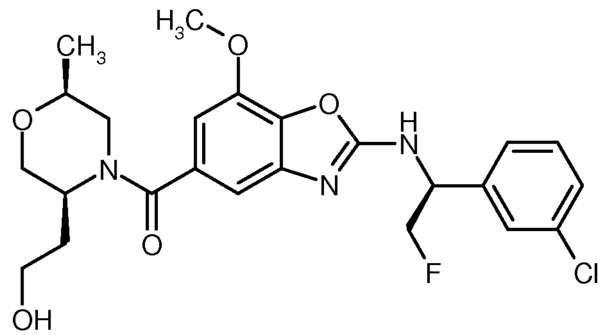

Beispiel 16

(2-{[1-(3-Chlorphenyl)-2-fluorethyl]amino}-7-methoxy-1,3-benzoxazol-5-yl)[5-(3-hydroxycyclobutyl)-2-methylmorpholin-4-yl]methanon [Diastereomerengemisch, 4 Isomere]

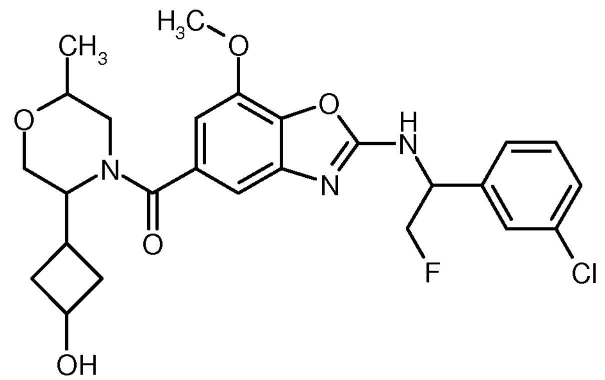

Es wurden 200 mg (0.33 mmol, Reinheit: 60%) 2-{[1-(3-Chlorphenyl)-2-fluorethyl]amino}-7-methoxy-1,3-benzoxazol-5-carbonsäure [enantiomerenreines Isomer] und 67.6 mg (0.40 mmol) 3-(6-Methylmorpholin-3-yl)cyclobutanol [Diastereomerengemisch, 4 Isomere] in *N,N*-Dimethylformamid (2.50 mL) vorgelegt und mit 170 mg (229 μL, 1.32 mmol) *N,N*-Diisopropylethylamin versetzt. Dann wurden bei RT 150 mg (0.40 mmol) HATU zugegeben und es wurde 2 h gerührt. Die Reaktionslösung wurde anschließend ohne weitere Aufarbeitung mittels präparativer RP-HPLC (Acetonitril/Wasser) gereinigt. Ausbeute: 83.9 mg (48% d. Th.).
LC-MS (Methode 1A): $R_t$ = 0.94, 0.95 min; MS (ESIpos): m/z = 518 $[M+H]^+$.

Beispiel 17

(2-{[1-(3-Chlorphenyl)-2-fluorethyl]amino}-7-methoxy-1,3-benzoxazol-5-yl)[5-(3-hydroxycyclobutyl)-2-methylmorpholin-4-yl]methanon [enantiomerenreines Isomer 1]

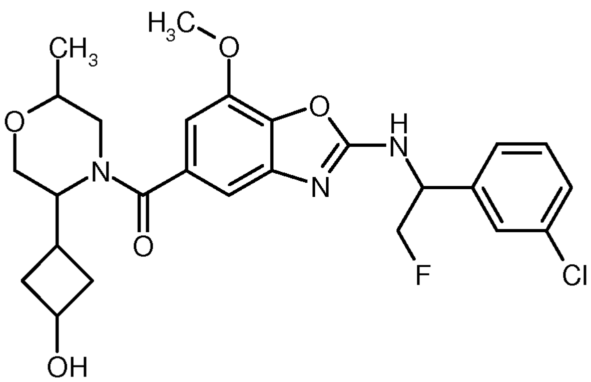

Diastereomerentrennung an chiraler Phase von 75.0 mg der Verbindung aus Beispiel 16 nach Methode 1D ergab 17.4 mg des Beispiels 17 (enantiomerenreines Isomer 1), 8.6 mg des Beispiels 18 (enantiomerenreines Isomer 2), 17.7 mg des Beispiels 19 (enantiomerenreines Isomer 3) und 9.5 mg des Beispiels 20 (enantiomerenreines Isomer 4).
HPLC (Methode 1E): $R_t$ = 11.1 min, >99% *de;*
LC-MS (Methode 1A): $R_t$ = 0.95 min; MS (ESIpos): m/z = 518 $[M+H]^+$.

**Beispiel 18**

(2-{[1-(3-Chlorphenyl)-2-fluorethyl]amino}-7-methoxy-1,3-benzoxazol-5-yl)[5-(3-hydroxycyclobutyl)-2-methylmorpholin-4-yl]methanon [enantiomerenreines Isomer 2]

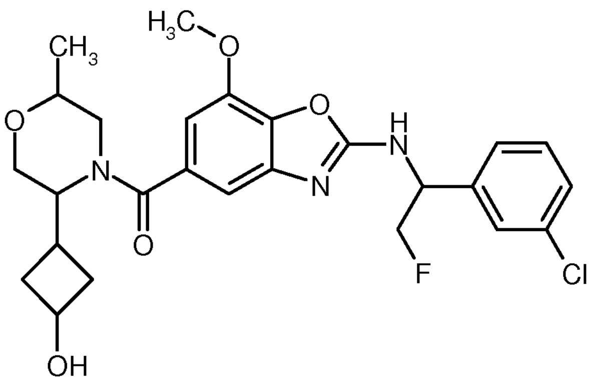

Diastereomerentrennung an chiraler Phase von 75.0 mg der Verbindung aus Beispiel 16 nach Methode 1D ergab 17.4 mg des Beispiels 17 (enantiomerenreines Isomer 1), 8.6 mg des Beispiels 18 (enantiomerenreines Isomer 2), 17.7 mg des Beispiels 19 (enantiomerenreines Isomer 3) und 9.5 mg des Beispiels 20 (enantiomerenreines Isomer 4).
HPLC (Methode 1E): $R_t$ = 12.6 min, 94.3:5.7 *dr*;
LC-MS (Methode 1A): $R_t$ = 0.95 min; MS (ESIpos): m/z = 518 $[M+H]^+$.

**Beispiel 19**

(2-{[1-(3-Chlorphenyl)-2-fluorethyl]amino}-7-methoxy-1,3-benzoxazol-5-yl)[5-(3-hydroxycyclobutyl)-2-methylmorpholin-4-yl]methanon [enantiomerenreines Isomer 3]

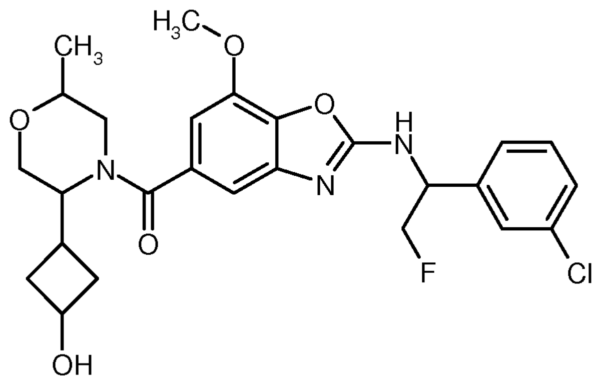

Diastereomerentrennung an chiraler Phase von 75.0 mg der Verbindung aus Beispiel 16 nach Methode 1D ergab 17.4 mg des Beispiels 17 (enantiomerenreines Isomer 1), 8.6 mg des Beispiels 18 (enantiomerenreines Isomer 2), 17.7 mg des Beispiels 19 (enantiomerenreines Isomer 3) und 9.5 mg des Beispiels 20 (enantiomerenreines Isomer 4).
HPLC (Methode 1E): $R_t$ = 14.5 min, >99% *de*;
LC-MS (Methode 1A): $R_t$ = 0.95 min; MS (ESIpos): m/z = 518 $[M+H]^+$.

**Beispiel 20**

(2-{[1-(3-Chlorphenyl)-2-fluorethyl]amino}-7-methoxy-1,3-benzoxazol-5-yl)[5-(3-hydroxycyclobutyl)-2-methylmorpholin-4-yl]methanon [enantiomerenreines Isomer 4]

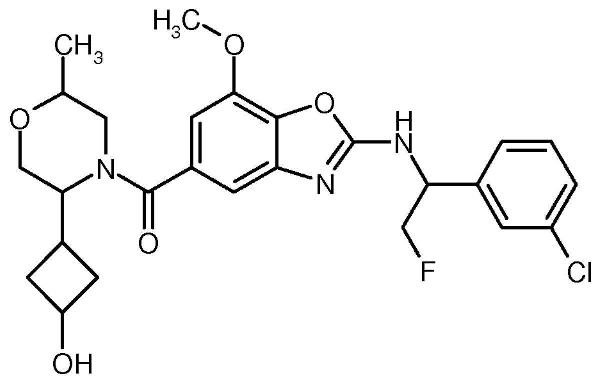

Diastereomerentrennung an chiraler Phase von 75.0 mg der Verbindung aus Beispiel 16 nach Methode 1D ergab 17.4 mg des Beispiels 17 (enantiomerenreines Isomer 1), 8.6 mg des Beispiels 18 (enantiomerenreines Isomer 2), 17.7 mg des Beispiels 19 (enantiomerenreines Isomer 3) und 9.5 mg des Beispiels 20 (enantiomerenreines Isomer 4).
HPLC (Methode 1E): $R_t$ = 17.2 min, 96.1:3.9 *dr*;
LC-MS (Methode 1A): $R_t$ = 0.94 min; MS (ESIpos): m/z = 518 $[M+H]^+$.

**Beispiel 21**

(2-{[1-(3-Chlorphenyl)-2-fluorethyl]amino}-7-methoxy-1,3-benzoxazol-5-yl)(*cis*-2-hydroxy-7-methyl-8-oxa-5-azaspiro[3.5]non-5-yl)methanon [enantiomerenreines Isomer 1]

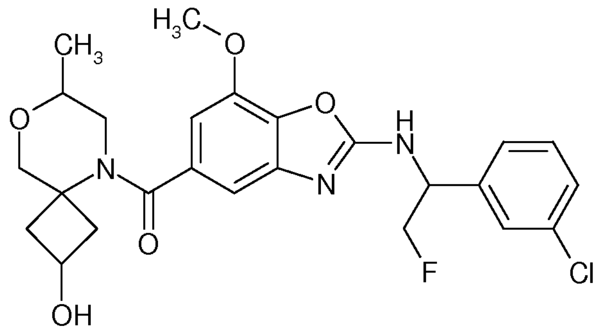

Es wurden 120 mg (0.152 mmol, Reinheit: 46%) 2-{[1-(3-Chlorphenyl)-2-fluorethyl]amino}-7-methoxy-1,3-benzoxazol-5-carbonsäure [enantiomerenreines Isomer] und 35.2 mg (0.182 mmol) *cis*-7-Methyl-8-oxa-5-azaspiro[3.5]nonan-2-ol Hydrochlorid [Beispiel 64A, enantiomerenreines Isomer 1] in *N,N*-Dimethylformamid (1.20 mL) vorgelegt und mit 78.4 mg (106 µL, 0.607 mmol) *N,N*-Diisopropylethylamin versetzt. Dann wurden 69.2 mg (0.182 mmol) HATU zugegeben und es wurde 1 h bei RT gerührt. Anschließend wurden 35.2 mg (0.182 mmol) *cis*-7-Methyl-8-oxa-5-azaspiro[3.5]nonan-2-ol Hydrochlorid [Beispiel 64A, enantiomerenreines Isomer 1], 78.4 mg (106 µL, 0.607 mmol) *N,N*-Diisopropylethylamin und 69.2 mg (0.182 mmol) HATU zugegeben und für weitere 2 h bei RT gerührt. Die Reaktionslösung wurde ohne weitere Aufarbeitung mittels präparativer RP-HPLC (Acetonitril/Wasser) gereinigt. Ausbeute: 67.3 mg (80% d. Th.).
LC-MS (Methode 1A): $R_t$ = 0.91 min; MS (ESIpos): m/z = 504 $[M+H]^+$;
$^1$H-NMR (400 MHz, DMSO-$d_6$): δ [ppm] = 9.00 (d, 1H), 7.58 (s, 1H), 7.51-7.34 (m, 3H), 6.93 (s, 1H), 6.78 (s, 1H), 5.24 ($m_c$, 1H), 5.08 (d, 1H), 4.80-4.51 (m, 2H), 3.93 (s, 3H), 3.89-3.80 (m, 1H), 3.62 (d, 1H), 3.52-3.43 (m, 2H), 2.90 (dd, 1H), 2.73-2.63 (m, 1H), 2.33 (br. s., 1H), 2.21-2.10 (m, 1H), 1.85 (br. t., 1H), 0.90 (d, 3H).

**Beispiel 22**

(2-{[1-(3-Chlorphenyl)-2-fluorethyl]amino}-7-methoxy-1,3-benzoxazol-5-yl)(*cis*-2-hydroxy-7-methyl-8-oxa-5-azaspiro[3.5]non-5-yl)methanon [enantiomerenreines Isomer 2]

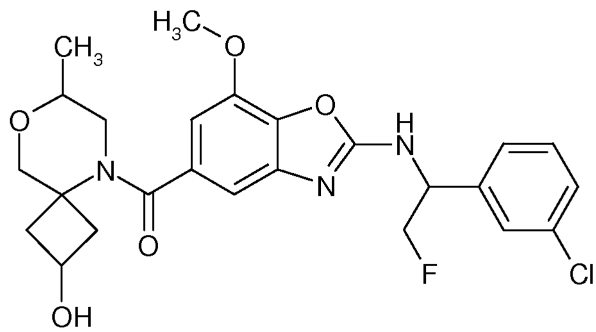

Es wurden 120 mg (0.152 mmol, Reinheit: 46%) 2-{[1-(3-Chlorphenyl)-2-fluorethyl]amino}-7-methoxy-1,3-benzoxazol-5-carbonsäure [enantiomerenreines Isomer] und 35.2 mg (0.182 mmol) *cis*-7-Methyl-8-oxa-5-azaspiro[3.5]nonan-2-ol Hydrochlorid [Beispiel 65A, enantiomerenreines Isomer 2] in *N,N*-Dimethylformamid (1.20 mL) vorgelegt und mit 78.4 mg (106 µL, 0.607 mmol) *N,N*-Diisopropylethylamin versetzt. Dann wurden 69.2 mg (0.182 mmol) HATU zugegeben und es wurde 1 h bei RT gerührt. Anschließend wurden 35.2 mg (0.182 mmol) *cis*-7-Methyl-8-oxa-5-azaspiro[3.5]nonan-2-ol Hydrochlorid [Beispiel 65A, enantiomerenreines Isomer 2], 78.4 mg (106 µL, 0.607 mmol) *N,N*-Diisopropylethylamin und 69.2 mg (0.182 mmol) HATU zugegeben und für weitere 2 h bei RT gerührt. Die Reaktionslösung wurde ohne weitere Aufarbeitung mittels präparativer RP-HPLC (Acetonitril/Wasser) gereinigt. Ausbeute: 44.2 mg (51% d. Th.).
LC-MS (Methode 1A): $R_t$ = 0.94 min; MS (ESIpos): m/z = 504 $[M+H]^+$;
$^1$H-NMR (400 MHz, DMSO-$d_6$): δ [ppm] = 9.01 (d, 1H), 7.58 (s, 1H), 7.49-7.36 (m, 3H), 6.92 (s, 1H), 6.80 (s, 1H), 5.24 ($m_c$, 1H), 5.09 (d, 1H), 4.80-4.52 (m, 2H), 3.93 (s, 3H), 3.90-3.80 (m, 1H), 3.62 (d, 1H), 3.51-3.42 (m, 2H), 2.90 (dd, 1H), 2.69 (dd, 1H), 2.38-2.29 (d, 1H), 2.22-2.13 (m, 1H), 1.84 (br. t., 1H), 0.89 (d, 3H).

**Beispiel 23**

4-[(2-{[1-(3-Chlorphenyl)-2-fluorethyl]amino-7-methoxy-1,3-benzoxazol-5-yl)carbonyl]-3-methyl-1,4-diazabicyclo[4.2.0]octan-2-on [enantiomerenreines Isomer]

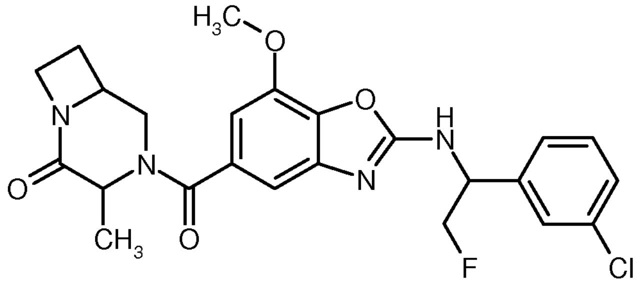

Es wurden 266 mg (0.337 mmol, Reinheit: 46%) 2-{[1-(3-Chlorphenyl)-2-fluorethyl]amino}-7-methoxy-1,3-benzoxazol-5-carbonsäure [enantiomerenreines Isomer] und 154 mg (1.10 mmol) 3-Methyl-1,4-diazabicyclo[4.2.0]octan-2-on [Beispiel 70A, enantiomerenreines Isomer 3] in *N*,*N*-Dimethylformamid (3.32 mL) vorgelegt und mit 378 mg (0.51 mL, 2.92 mmol) *N*,*N*-Diisopropylethylamin versetzt. Anschließend wurden bei RT 333 mg (0.877 mmol) HATU zugegeben und es wurde über Nacht gerührt. Die Reaktionslösung wurde ohne weitere Aufarbeitung mittels präparativer RP-HPLC (Acetonitril/Wasser) gereinigt. Ausbeute: 174 mg (quant.).
LC-MS (Methode 1A): $R_t$ = 0.94 min; MS (ESIpos): m/z = 487 $[M+H]^+$;
$^1$H-NMR (400 MHz, DMSO-$d_6$): δ [ppm] = 9.00 (d, 1H), 7.58 (s, 1H), 7.50-7.36 (m, 3H), 6.91 (s, 1H), 6.77 (s, 1H), 5.24 ($m_c$, 1H), 4.80-4.52 (m, 3H), 4.22 ($m_c$, 1H), 4.05 ($m_c$, 1H), 3.98-3.87 (m, 4H), 3.72 (m, 1H), 2.45-2.31 (m, 1H), 2.10-2.00 (m, 1H), 1.38 (d, 3H), ein Proton verdeckt.

**Beispiel 24**

{(3S)-4-[(2-{[1-(3-Chlorphenyl)-2-fluorethyl]amino}-7-methoxy-1,3-benzoxazol-5-yl)carbonyl]-6-methylmorpholin-3-yl}essigsäure [enantiomerenreines Isomer]

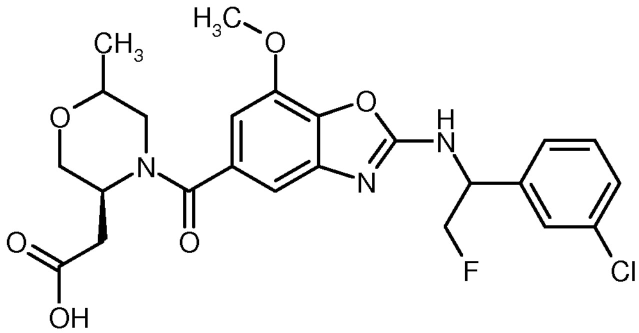

Es wurden 600 mg (1.22 mmol) (2-1[1-(3-Chlorphenyl)-2-fluorethyl]aminol-7-methoxy-1,3-benzoxazol-5-yl)[(5*S*)-5-(2-hydroxyethyl)-2-methylmorpholin-4-yl]methanon [Beispiel 15, enantiomerenreines Isomer 2] in Acetonitril (120 mL) vorgelegt, bei RT mit 611 mg (2.68 mmol) Periodsäure versetzt und 15 min gerührt. Anschließend wurde auf 0°C abgekühlt und mit 15.7 mg (0.07 mmol) Pyridiniumchlorochromat in Acetonitril (2 mL) versetzt. Es wurde 4 h bei 0°C gerührt (DC-Kontrolle: Dichlormethan/*iso*-Propanol 10:1) und anschließend auf ca. 20 mL im Vakuum eingeengt. Es wurde mit gesättigter wässriger Natriumhydrogensulfit-Lösung (150 mL) versetzt und mit Essigsäureethylester (3 x 100 mL) extrahiert. Die organische Phase wurde mit 1 N wässriger Hydrogenchlorid-Lösung (100 mL) und Wasser (100 mL) gewaschen, über Natriumsulfat getrocknet, filtriert und im Vakuum eingeengt. Der Rückstand wurde mittels präparativer RP-HPLC (Acetonitril/Wasser) gereinigt. Ausbeute: 312 mg (48% d. Th.).

Drehwert: $[\alpha]_D^{20}$ = 133.9° (c = 0.55, Chloroform);

LC-MS (Methode 7A): $R_t$ = 2.65 min; MS (ESIpos): m/z = 505 $[M+H]^+$;

$^1$H-NMR (400 MHz, DMSO-$d_6$): δ [ppm] = 12.33 (br. s., 1H), 8.99 (d, 1H), 7.58 (s, 1H), 7.50-7.33 (m, 3H), 6.95-6.80 (m, 1H), 6.72 (br. s., 1H), 5.24 ($m_c$, 1H), 4.85-4.15 (m, 3H), 3.91 (s, 3H), 3.83-3.23 (m, 4H), 3.05-2.56 (m, 3H), 1.22-0.91 (m, 3H).

**Beispiel 25**

(2-{[1-(3-Chlorphenyl)-2-fluorethyl]amino}-7-methoxy-1,3-benzoxazol-5-yl)|(5*R*)-5-(2-hydroxyethyl)-2,2-dimethylmorpholin-4-yl]methanon [Diastereomerengemisch, 2 Isomere]

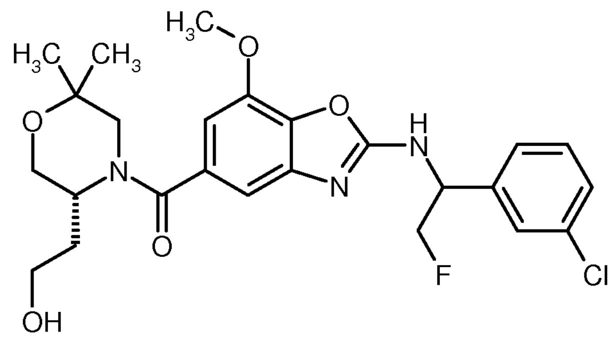

Es wurden 50.0 mg (0.08 mmol, Reinheit: 60%) 2-{[1-(3-Chlorphenyl)-2-fluorethyl]amino}-7-methoxy-1,3-benzoxazol-5-carbonsäure [enantiomerenreines Isomer] und 15.7 mg (0.10 mmol) 2-[(3R)-6,6-Dimethyl-morpholin-3-yl]ethanol [Beispiel 77A, Enantiomerengemisch, 2 Isomere] in *N,N*-Dimethylformamid (1.00 mL) vorgelegt und mit 42.5 mg (57.3 µl, 0.33 mmol) *N,N*-Diisopropylethylamin versetzt. Anschließend wurden bei RT 37.5 mg (0.10 mmol) HATU zugegeben und es wurde 2 h gerührt. Die Reaktionslösung wurde ohne weitere Aufarbeitung mittels präparativer RP-HPLC (Acetonitril/Wasser) gereinigt. Ausbeute: 30.7 mg (71% d. Th., Diastereomerenverhältnis: ca. 85:15).
HPLC (Methode 3E): $R_t$ = 6.07 min (Zielverbindung), 8.46 min (Nebenisomer): ca. 85:15 Diastereomerenverhältnis;
LC-MS (Methode 1A): $R_t$ = 0.95 min; MS (ESIpos): m/z = 506 $[M+H]^+$;
$^1$H-NMR (400 MHz, DMSO-$d_6$): δ [ppm] = 9.00 (d, 1H), 7.58 (s, 1H), 7.50-7.35 (m, 3H), 6.82 (br. s., 1H), 6.68 (s, 1H), 5.23 ($m_c$, 1H), 4.85-4.25 (m, 4H), 4.03-3.70 (m, 5H), 3.44 (br. s., 3H), 1.97-1.75 (m, 2H), 1.11 (br. s., 6H), ein Proton verdeckt.

**Beispiel 26**

(2-{[1-(3-Chlorphenyl)-2-fluorethyl]amino}-7-methoxy-1,3-benzoxazol-5-yl)[(4*R*)-4-ethoxy-2-(hydroxymethyl)pyrrolidin-1-yl]methanon [Diastereomerengemisch, 2 Isomere]

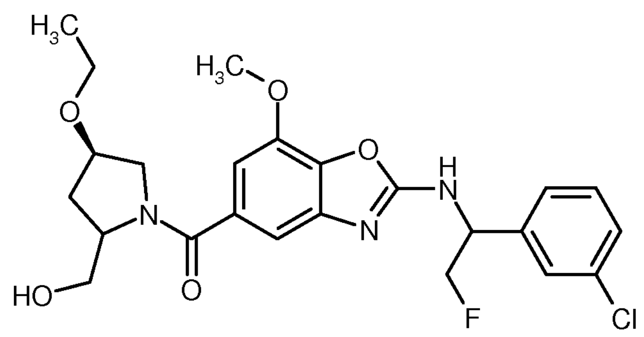

Es wurden 200 mg (0.49 mmol, Reinheit: 89%) 2-{[1-(3-Chlorphenyl)-2-fluorethyl]amino}-7-methoxy-1,3-benzoxazol-5-carbonsäure [Beispiel 13A, enantiomerenreines Isomer] und 106 mg (0.73 mmol) [(4*R*)-4-Ethoxypyrrolidin-2-yl]methanol [Diastereomerengemisch, 2 Isomere] in *N*,*N*-Dimethylformamid (3.25 mL) vorgelegt und mit 441 mg (595 µl, 3.42 mmol) *N*,*N*-Diisopropylethylamin versetzt. Anschließend wurden bei RT 223 mg (0.59 mmol) HATU zugegeben und über Nacht gerührt. Die Reaktionslösung wurde ohne weitere Aufarbeitung mittels präparativer RP-HPLC (Acetonitril/Wasser) gereinigt. Ausbeute: 192 mg (79% d. Th.).
LC-MS (Methode 1A): $R_t$ = 0.96 min; MS (ESIpos): m/z = 492 $[M+H]^+$;
$^1$H-NMR (400 MHz, DMSO-$d_6$): δ [ppm] = 9.03-8.96 (m, 1H), 7.59 (s, 1H), 7.50-7.36 (m, 3H), 7.02-6.94 (m, 1H), 6.87-6.80 (m, 1H), 5.25 ($m_c$, 1H), 4.86-4.51 (m, 3H), 4.24-4.10 (m, 1H), 3.92 (s, 4H), 3.72-3.37 (m, 3H), 3.32-3.11 (m, 2H), 2.23-1.89 (m, 2H), 1.17-0.95 (m, 3H), ein Proton verdeckt.

## Beispiel 27

(2-{[1-(3-Chlorphenyl)-2-fluorethyl]amino}-7-methoxy-1,3-benzoxazol-5-yl)[(4*R*)-4-ethoxy-2-(hydroxymethyl)pyrrolidin-1-yl]methanon [enantiomerenreines Isomer 1]

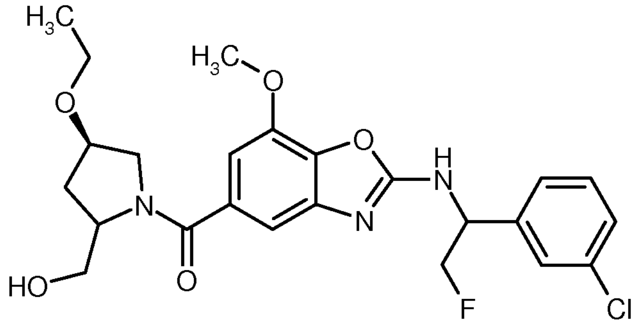

Diastereomerentrennung an chiraler Phase von 192 mg der Verbindung aus Beispiel 26 nach Methode 8D ergab nach nochmaliger Reinigung durch präparative RP-HPLC (Acetonitril/Wasser) 27.7 mg des Beispiels 27 (enantiomerenreines Isomer 1) und 6.7 mg des Beispiels 28 (enantiomerenreines Isomer 2).
HPLC (Methode 8E): $R_t$ = 8.59 min, >99.0% *de* (enantiomerenreines Isomer 1),
LC-MS (Methode 1A): $R_t$ = 0.93 min; MS (ESIpos): m/z = 492 $[M+H]^+$;
$^1$H-NMR (400 MHz, DMSO-$d_6$): δ [ppm] = 8.99 (br. s., 1H), 7.58 (s, 1H), 7.51-7.35 (m, 3H), 6.97 (s, 1H), 6.83 (s, 1H), 5.24 ($m_c$, 1H), 4.83-4.51 (m, 3H), 4.19 (br. s., 1H), 3.92 (s, 4H), 3.71-3.46 (m, 3H), 3.28-2.97 (m, 2H), 2.09-1.93 (m, 2H), 1.16-0.94 (m, 3H), ein Proton verdeckt.

## Beispiel 28

(2-{[1-(3-Chlorphenyl)-2-fluorethyl]amino}-7-methoxy-1,3-benzoxazol-5-yl)[(4*R*)-4-ethoxy-2-(hydroxymethyl)pyrrolidin-1-yl]methanon [enantiomerenreines Isomer 2]

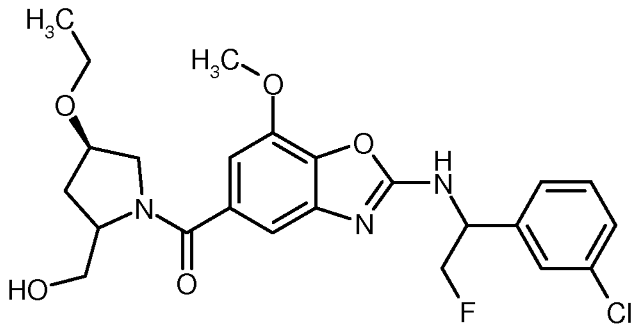

Diastereomerentrennung an chiraler Phase von 192 mg der Verbindung aus Beispiel 26 nach Methode 8D ergab nach nochmaliger Reinigung durch präparative RP-HPLC (Acetonitril/Wasser) 27.7 mg des Beispiels 27 (enantiomerenreines Isomer 1) und 6.7 mg des Beispiels 28 (enantiomerenreines Isomer 2).
HPLC (Methode 8E): $R_t$ = 15.9 min, >99.0% *de* (enantiomerenreines Isomer 2),
LC-MS (Methode 1A): $R_t$ = 0.96 min; MS (ESIpos): m/z = 492 $[M+H]^+$;
$^1$H-NMR (400 MHz, DMSO-$d_6$): $\delta$ [ppm] = 8.99 (br. s., 1H), 7.58 (s, 1H), 7.50-7.35 (m, 3H), 6.96 (s, 1H), 6.81 (s, 1H), 5.24 ($m_c$, 1H), 4.90-4.50 (m, 3H), 4.24-4.07 (m, 1H), 3.92 (s, 5H), 3.65-3.47 (br. s., 3H), 3.24-3.05 (m, 1H), 2.24-1.87 (m, 2H), 1.13-1.01 (m, 3H), ein Proton verdeckt.

**Beispiel 29**

(2-{[1-(3-Chlorphenyl)-2-fluorethyl]amino}-7-methoxy-1,3-benzoxazol-5-yl)[(4*R*)-4-ethoxy-2-(2-hydroxyethyl)pyrrolidin-1-yl]methanon [Diastereomerengemisch, 2 Isomere]

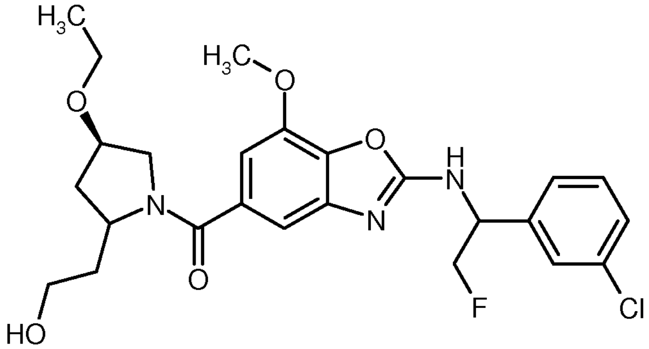

Es wurden 171 mg (0.42 mmol, Reinheit: 89%) 2-{[1-(3-Chlorphenyl)-2-fluorethyl]amino}-7-methoxy-1,3-benzoxazol-5-carbonsäure [Beispiel 13A, enantiomerenreines Isomer] und 100 mg (0.63 mmol) 2-[(4*R*)-4-Ethoxypyrrolidin-2-yl]ethanol [Diastereomerengemisch, 2 Isomere] in *N*,*N*-Dimethylformamid (2.79 mL) vorgelegt und mit 379 mg (511 µl, 2.93 mmol) *N*,*N*-Diisopropylethylamin versetzt. Dann wurden bei RT 191 mg (0.50 mmol) HATU zugegeben nachfolgend 2 h gerührt. Die Reaktionslösung wurde ohne weitere Aufarbeitung mittels präparativer RP-HPLC (Acetonitril/Wasser) gereinigt. Ausbeute: 145 mg (65% d. Th.).
LC-MS (Methode 1A): $R_t$ = 0.97 min; MS (ESIpos): m/z = 506 $[M+H]^+$;
$^1$H-NMR (400 MHz, DMSO-$d_6$): $\delta$ [ppm] = 9.08-8.94 (m, 1H), 7.59 (s, 1H), 7.52-7.32 (m, 3H), 6.95 (br. s., 1H), 6.80 (d, 1H), 5.24 ($m_c$, 1H), 4.81-4.52 (m, 2H), 4.46 (br. s., 1H), 4.20 (br. s., 1H), 3.97-3.85 (m, 4H), 3.68-3.37 (m, 3H), 3.30-3.10 (m, 2H), 2.23-1.97 (m, 2H), 1.85-1.48 (m, 2H), 1.15-0.90 (m, 3H), ein Proton verdeckt.

**Beispiel 30**

(2-{[1-(3-Chlorphenyl)-2-fluorethyl]amino}-7-methoxy-1,3-benzoxazol-5-yl)[(4*R*)-4-ethoxy-2-(2-hydroxyethyl)pyrrolidin-1-yl]methanon [enantiomerenreines Isomer 1]

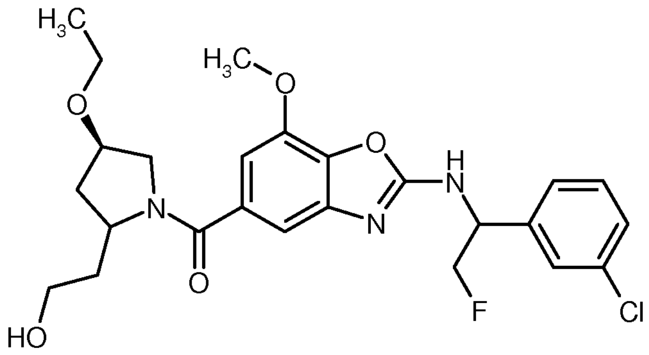

Diastereomerentrennung an chiraler Phase von 140 mg der Verbindung aus Beispiel 29 nach Methode 9D ergab 31.0 mg des Beispiels 30 (enantiomerenreines Isomer 1) und 73 mg des Beispiels 31 (enantiomerenreines Isomer 2).
HPLC (Methode 9E): $R_t$ = 8.08 min, >99.0% *de* (enantiomerenreines Isomer 1),
LC-MS (Methode 1A): $R_t$ = 0.98 min; MS (ESIpos): m/z = 506 $[M+H]^+$;
$^1$H-NMR (400 MHz, DMSO-$d_6$): δ [ppm] = 8.98 (d, 1H), 7.58 (s, 1H), 7.50-7.35 (m, 3H), 6.95 (s, 1H), 6.79 (s, 1H), 5.24 ($m_c$, 1H), 4.81-4.52 (m, 2H), 4.44 (br. s., 1H), 4.20 (br. s., 1H), 3.92 (s, 4H), 3.64-3.35 (m, 5H), 2.27-2.02 (m, 2H), 1.70 (br. s., 2H), 1.06 (t, 3H), ein Proton verdeckt.

**Beispiel 31**

(2-{[1-(3-Chlorphenyl)-2-fluorethyl]amino}-7-methoxy-1,3-benzoxazol-5-yl)[(4*R*)-4-ethoxy-2-(2-hydroxyethyl)pyrrolidin-1-yl]methanon [enantiomerenreines Isomer 2]

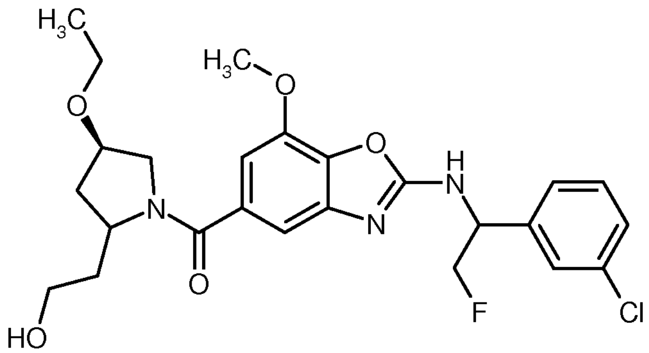

Diastereomerentrennung an chiraler Phase von 140 mg der Verbindung aus Beispiel 29 nach Methode 9D ergab 31.0 mg des Beispiels 30 (enantiomerenreines Isomer 1) und 73 mg des Beispiels 31 (enantiomerenreines Isomer 2).
HPLC (Methode 9E): Rt = 10.2 min, >99.0% *de* (enantiomerenreines Isomer 2),
LC-MS (Methode 1A): $R_t$ = 0.96 min; MS (ESIpos): m/z = 506 $[M+H]^+$;
$^1$H-NMR (400 MHz, DMSO-$d_6$): δ [ppm] = 8.99 (d, 1H), 7.59 (s, 1H), 7.50-7.29 (m, 3H), 6.95 (s, 1H), 6.81 (s, 1H), 5.24 ($m_c$, 1H), 4.85-4.51 (m, 2H), 4.46 (br. s., 1H), 4.20 (br. s., 1H), 3.98-3.84 (m, 4H), 3.59 (d, 3H), 3.29-3.08 (m, 2H), 2.21-1.97 (m, 2H), 1.82-1.72 (br. s., 1H), 1.66-1.47 (m, 1H), 0.99 (t, 3H), ein Proton verdeckt.

**Beispiel 32**

7-[(2- {[1-(3-Chlorphenyl)-2-fluorethyl]amino}-7-methoxy-1,3-benzoxazol-5-yl)carbonyl]-6-methyl-4,7-diazaspiro[2.5]octan-5-on [Diastereomerengemisch, 2 Isomere]

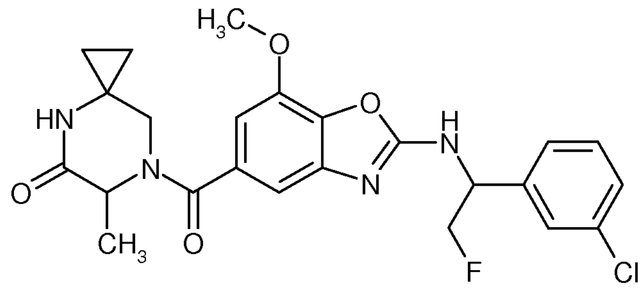

Es wurden 100 mg (0.24 mmol, Reinheit: 89%) 2-{[1-(3-Chlorphenyl)-2-fluorethyl]amino}-7-methoxy-1,3-benzoxazol-5-carbonsäure [Beispiel 13A, enantiomerenreines Isomer] und 100 mg (0.37 mmol) 6-Methyl-4,7-diazaspiro[2.5]octan-5-on Trifluoroacetat [Racemat] in *N*,*N*-Dimethylformamid (1.62 mL) vorgelegt und mit 221 mg (297 µl, 1.71 mmol) *N*,*N*-Diisopropylethylamin versetzt. Dann wurden bei RT 111 mg (0.29 mmol) HATU zugegeben nachfolgend 2 h gerührt. Die Reaktionslösung wurde ohne weitere Aufarbeitung mittels präparativer RP-HPLC (Acetonitril/Wasser) gereinigt. Ausbeute: 82.6 mg (68% d. Th.).
LC-MS (Methode 1A): $R_t$ = 0.89 min; MS (ESIpos): m/z = 487 $[M+H]^+$;
$^1$H-NMR (400 MHz, DMSO-$d_6$): δ [ppm] = 9.02 (dd, 1H), 8.15 (s, 1H), 7.58 (d, 1H), 7.51-7.29 (m, 3H), 6.84 (d, 1H), 6.71 (s, 1H), 5.25 ($m_c$, 1H), 4.96-4.46 (m, 3H), 3.92 (s, 3H), 3.69 (br.s., 1H), 3.21-2.99 (m, 1H), 1.42 (d, 3H), 0.90-0.30 (m, 4H).

## Beispiel 33

7-[(2-{[1-(3-Chlorphenyl)-2-fluorethyl]amino}-7-methoxy-1,3-benzoxazol-5-yl)carbonyl]-6-methyl-4,7-diazaspiro[2.5]octan-5-on [enantiomerenreines Isomer 1]

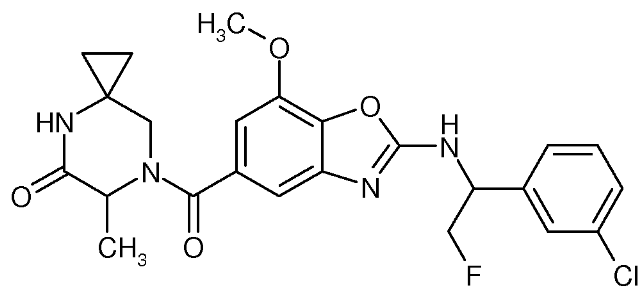

Diastereomerentrennung an chiraler Phase von 75.0 mg der Verbindung aus Beispiel 32 nach Methode 10D ergab nach nochmaliger Reinigung durch präparative RP-HPLC (Acetonitril/Wasser) 15.9 mg des Beispiels 33 (enantiomerenreines Isomer 1) und 23.5 mg des Beispiels 34 (enantiomerenreines Isomer 2).
HPLC (Methode 10E): $R_t$ = 4.68 min, >99.0% *de* (enantiomerenreines Isomer 1),
LC-MS (Methode 1A): $R_t$ = 0.91 min; MS (ESIpos): m/z = 487 $[M+H]^+$;
$^1$H-NMR (400 MHz, DMSO-$d_6$): δ [ppm] = 9.02 (d, 1H), 8.14 (s, 1H), 7.58 (s, 1H), 7.49-7.33 (m, 3H), 6.84 (s, 1H), 6.71 (s, 1H), 5.25 ($m_c$, 1H), 4.91-4.47 (m, 3H), 3.92 (s, 3H), 3.67 (br. s., 1H), 3.07 (br. s., 1H), 1.42 (d, 3H), 0.87-0.29 (m, 4H).

## Beispiel 34

7-[(2-{[1-(3-Chlorphenyl)-2-fluorethyl]amino}-7-methoxy-1,3-benzoxazol-5-yl)carbonyl]-6-methyl-4,7-diazaspiro[2.5]octan-5-on [enantiomerenreines Isomer 2]

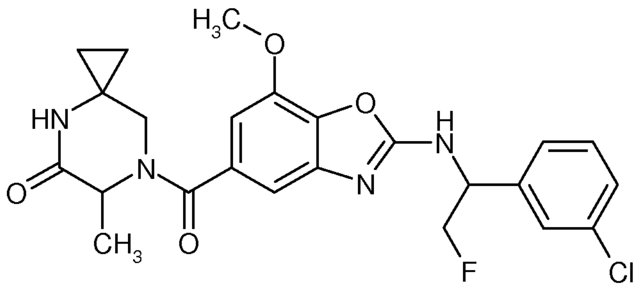

Diastereomerentrennung an chiraler Phase von 75.0 mg der Verbindung aus Beispiel 32 nach Methode 10D ergab nach nochmaliger Reinigung durch präparative RP-HPLC (Acetonitril/Wasser) 15.9 mg des Beispiels 33 (enantiomerenreines Isomer 1) und 23.5 mg des Beispiels 34 (enantiomerenreines Isomer 2).
HPLC (Methode 10E): $R_t$ = 6.24 min, >99.0% *de* (enantiomerenreines Isomer 2),
LC-MS (Methode 1A): $R_t$ = 0.91 min; MS (ESIpos): m/z = 487 $[M+H]^+$;
$^1$H-NMR (400 MHz, DMSO-$d_6$): δ [ppm] = 9.01 (d, 1H), 8.15 (s, 1H), 7.59 (s, 1H), 7.50-7.31 (m, 3H), 6.85 (s, 1H), 6.71 (s, 1H), 5.25 ($m_c$, 1H), 4.94-4.48 (m, 3H), 3.92 (s, 3H), 3.10 (br. s., 1H), 1.42 (d, 3H), 0.90-0.27 (m, 4H), ein Proton verdeckt.

## B) Bewertung der physiologischen Wirksamkeit

Die Eignung der erfindungsgemäßen Verbindungen zur Behandlung von thromboembolischen Erkrankungen kann in folgenden Assaysystemen gezeigt werden:

### a) Testbeschreibungen (*in vitro*)

### a.1) Messung der Thrombin-Hemmung in Puffer

Zur Bestimmung der Thrombin-Hemmung der oben aufgeführten Substanzen wird ein biochemisches Testsystem aufgebaut, in dem die Umsetzung eines Thrombin-Substrates zur Ermittlung der enzymatischen Aktivität von humanem Thrombin benutzt wird. Dabei spaltet Thrombin aus dem peptischen Substrat Aminomethylcoumarin ab, das fluoreszent gemessen wird. Die Bestimmungen werden in Mikrotiterplatten durchgeführt.

Zu testende Substanzen werden in unterschiedlichen Konzentrationen in Dimethylsulfoxid gelöst und 15 min mit humanem Thrombin (0.06 nmol/l gelöst in 50 mmol/l Tris-Puffer [C,C,C-Tris(hydroxymethyl)-aminomethan], 100 mmol/l Natriumchlorid, 0.1% BSA [bovines Serumalbumin], ph 7.4) bei 22°C inkubiert. Anschließend wird das Substrat (5 μmol/l Boc-Asp(OBzl)-Pro-Arg-AMC von der Firma Bachem) hinzugefügt. Nach einer Inkubation von 30 min wird die Probe bei einer Wellenlänge von 360 nm angeregt und die Emission bei 460 nm gemessen. Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen $IC_{50}$-Werte berechnet. Repräsentative Wirkdaten aus diesem Test sind in der folgenden Tabelle 1 aufgeführt (zum Teil als Mittelwerte aus Einzelbestimmungen):

**Tabelle 1**

| Beispiel-Nr. | $IC_{50}$ [nM] | | Beispiel-Nr. | $IC_{50}$ [nM] |
|---|---|---|---|---|
| 1 | 3.20 | | 2 | 31.00 |
| 3 | 2.40 | | 4 | 1.70 |
| 5 | 1.10 | | 6 | 9.70 |
| 7 | 5.30 | | 8 | 11.00 |
| 9 | 0.20 | | 10 | 130.00 |
| 11 | 0.01 | | 12 | 0.10 |
| 13 | 1.20 | | 14 | 2.00 |
| 15 | 0.72 | | 16 | 0.47 |
| 17 | 0.37 | | 18 | 0.30 |

(fortgesetzt)

| Beispiel-Nr. | $IC_{50}$ [nM] | | Beispiel-Nr. | $IC_{50}$ [nM] |
|---|---|---|---|---|
| 19 | 0.98 | | 20 | 1.40 |
| 21 | 1.40 | | 22 | 1.60 |
| 23 | 1.70 | | 24 | 4.40 |
| 25 | 4.30 | | 26 | 0.19 |
| 27 | 0.16 | | 28 | 2.30 |
| 29 | 1.10 | | 30 | 5.30 |
| 31 | 0.54 | | 32 | 0.70 |
| 33 | 0.38 | | 34 | 20.00 |

**a.2) Bestimmung der Selektivität**

Zum Nachweis der Selektivität der Substanzen bezüglich Thrombin-Hemmung werden die Prüfsubstanzen auf ihre Hemmung anderer humaner Serinproteasen wie Faktor Xa, Faktor XIIa, Faktor XIa, Trypsin und Plasmin hin untersucht. Zur Bestimmung der enzymatischen Aktivität von Faktor Xa (1.3 nmol/l von Kordia), Faktor XIIa (10 nmol/l von Kordia), Faktor XIa (0.4 nmol/l von Kordia), Trypsin (83 mU/ml von Sigma) und Plasmin (0.1 µg/ml von Kordia) werden diese Enzyme gelöst (50 mmol/l Tris-Puffer [C,C,C-Tris(hydroxymethyl)-aminomethan], 100 mmol/l Natriumchlorid, 0.1% BSA [bovines Serumalbumin], 5 mmol/l Calciumchlorid, pH 7.4) und für 15 min mit Prüfsubstanz in verschiedenen Konzentrationen in Dimethylsulfoxid sowie mit Dimethylsulfoxid ohne Prüfsubstanz inkubiert. Anschließend wird die enzymatische Reaktion durch Zugabe der entsprechenden Substrate gestartet (5 µmol/l Boc-Ile-Glu-Gly-Arg-AMC von Bachem für FXa, 5 µmol/l H-Pro-Phe-Arg-AMC von Bachem für Faktor XIIa, 5 nmol/l Boc-Ile-Glu-Gly-Arg-AMC von Bachem für Trypsin, 5 µmol/l Boc-Glu(OBzl)-Ala-Arg-AMC von Bachem für Faktor XIa, 50 µmol/l MeOSuc-Ala-Phe-Lys-AMC von Bachem für Plasmin). Nach einer Inkubationszeit von 30 min bei 22°C wird die Fluoreszenz gemessen (Anregung: 360 nm, Emission: 460 nm). Die gemessenen Emissionen der Testansätze mit Prüfsubstanz werden mit den Kontrollansätzen ohne Prüfsubstanz (ausschließlich Dimethylsulfoxid anstatt Prüfsubstanz in Dimethylsulfoxid) verglichen und aus den Konzentrations-Wirkungs-Beziehungen $IC_{50}$-Werte berechnet.

**a.3) Bestimmung der Thrombin-inhibitorischen Wirkung der potentiellen Inhibitoren in Plasmaproben**

Zur Bestimmung der Hemmung von Thrombin in Plasmaproben wird im Plasma vorhandene Prothrombinase durch Ecarin aktiviert. Anschließend wird die Thrombin-Aktivität bzw. deren Hemmung durch potentielle Inhibitoren mittels Zugabe eines Substrats fluoreszent gemessen.

Die zu testenden Substanzen werden in unterschiedlichen Konzentrationen in Dimethylsulfoxid gelöst und mit Wasser verdünnt. In weissen 96-Loch-Flachbodenplatten werden 20 µl Substanzverdünnung mit 20 µl Ecarin-Lösung (Ecarin Reagenz, Firma Sigma E-0504, Endkonzentration 20 mU pro Ansatz) in Ca-Puffer (200 mM Hepes + 560 mM Natriumchlorid + 10 mM Calciumchlorid + 0.4% PEG) bzw. mit 20 µl Ca-Puffer (als unstimulierte Kontrolle) gemischt. Desweiteren werden 20 µl fluorogenes Thrombinsubstrat (Firma Bachem I-1120, Endkonzentration 50 µmol/l) und 20 µl Citratplasma (Firma Octapharma) zugegeben und gut homogenisiert. Die Platte wird im SpectraFluorplus Reader mit einem Excitationsfilter 360 nm und Emissionsfilter 465 nm über 20 Minuten jede Minute gemessen. Die Ermittlung des $IC_{50}$-Wertes erfolgt, wenn ca. 70% des Maximalsignals erreicht ist (ca. 12 min). Repräsentative Wirkdaten aus diesem Test sind in der folgenden Tabelle 2 aufgeführt (zum Teil als Mittelwerte aus Einzelbestimmungen):

**Tabelle 2**

| Beispiel-Nr. | $IC_{50}$ [nM] | | Beispiel-Nr. | $IC_{50}$ [nM] |
|---|---|---|---|---|
| 1 | 21.0 | | 2 | 31.0 |
| 3 | 11.6 | | 4 | 9.8 |
| 5 | 4.8 | | 6 | 16.5 |
| 7 | 11.4 | | 8 | 25.3 |
| 9 | 14.3 | | 10 | 1400 |

(fortgesetzt)

| Beispiel-Nr. | $IC_{50}$ [nM] | | Beispiel-Nr. | $IC_{50}$ [nM] |
|---|---|---|---|---|
| 11 | 5.7 | | 12 | 14.7 |
| 13 | 7.8 | | 14 | 4.8 |
| 15 | 8.0 | | 16 | 5.6 |
| 17 | 13.2 | | 18 | 10.9 |
| 19 | 14.1 | | 20 | 8.4 |
| 21 | 6.2 | | 22 | 3.8 |
| 23 | 4.7 | | 24 | 4.5 |
| 25 | 4.7 | | 26 | 2 |
| 27 | 4.6 | | 28 | 56 |
| 29 | 15 | | 30 | 46 |
| 31 | 5 | | 32 | 7.3 |
| 33 | 8.4 | | 34 | 170 |

**a.4) Thrombin Generation Assay (Thrombogramm)**

Die Wirkung der Prüfsubstanzen auf das Thrombogramm (Thrombin Generation Assay nach Hemker) wird in vitro in Humanplasma (Octaplas® von der Firma Octapharma) bestimmt. Im Thrombin Generation Assay nach Hemker wird die Aktivität von Thrombin in gerinnendem Plasma durch die Messung der fluoreszenten Spaltprodukte des Substrats I-1140 (Z-Gly-Gly-Arg-AMC, Bachem) bestimmt. Um die Gerinnungsreaktion zu starten werden Reagenzien der Firma Thrombinoscope verwendet (PPP Reagenz: 30 pM recombinant tissue factor, 24 μM phospholipids in HEPES). Die Reaktion wird in Gegenwart variierender Konzentrationen an Prüfsubstanz oder dem entsprechenden Lösungsmittel durchgeführt. Außerdem wird ein Thrombin-Kalibrator der Firma Thrombinoscope verwendet, dessen amidolytische Aktivität zur Berechnung der Thrombinaktivität in einer Plasmaprobe benötigt wird.

Die Testdurchführung erfolgt nach Herstellerangaben (Thrombionsocpe BV): 4 μl der Prüfsubstanz oder des Lösungsmittels, 76 μl Plasma und 20 μl PPP-Reagenz oder Thrombin Calibrator werden 5 min bei 37°C inkubiert. Nach Zugabe von 20 μl 2.5 mM Thrombinsubstrat in 20 mM Hepes, 60 mg/ml BSA, 102 mM Calciumchlorid wird die Thrombin-Generierung über 120 min alle 20 s gemessen. Die Messung wird mit einem Fluorometer (Fluoroskan Ascent) der Firma Thermo Electron durchgeführt, der mit einem 390/460 nM Filterpaar und einem Dispenser ausgestattet ist. Durch die Verwendung der "thrombinoscope software" wird das Thrombogramm berechnet und graphisch dargestellt. Die folgenden Parameter werden berechnet: lag time, time to Peak, Peak, ETP (endogenous thrombin potential) und start tail.

**a.5) Bestimmung der antikoagulatorischen Wirkung**

Die antikoagulatorische Wirkung der Prüfsubstanzen wird in vitro in Human-, Kaninchen- und Rattenplasma bestimmt. Dazu wird Blut unter Verwendung einer 0.11 molaren Natriumcitrat-Lösung als Vorlage in einem Mischungsverhältnis Natriumcitrat/Blut 1/9 abgenommen. Das Blut wird unmittelbar nach der Abnahme gut gemischt und 15 Minuten bei ca. 4000 g zentrifugiert. Der Überstand wird abpipettiert.

Die Prothrombinzeit (PT, Synonyme: Thromboplastinzeit, Quick-Test) wird in Gegenwart variierender Konzentrationen an Prüfsubstanz oder dem entsprechenden Lösungsmittel mit einem handelsüblichen Testkit (Neoplastin® von der Firma Boehringer Mannheim oder Hemoliance® RecombiPlastin von der Firma Instrumentation Laboratory) bestimmt. Die Testverbindungen werden 3 Minuten bei 37°C mit dem Plasma inkubiert. Anschließend wird durch Zugabe von Thromboplastin die Gerinnung ausgelöst und der Zeitpunkt des Gerinnungseintritts bestimmt. Es wird die Konzentration an Prüfsubstanz ermittelt, die eine Verdoppelung der Prothrombinzeit bewirkt. Repräsentative Wirkdaten aus diesem Test sind in der folgenden Tabelle 3 aufgeführt (zum Teil als Mittelwerte aus Einzelbestimmungen):

**Tabelle 3**

| Beispiel-Nr. | $IC_{50}$ [μM] | | Beispiel-Nr. | $IC_{50}$ [μM] |
|---|---|---|---|---|
| 3 | 1.8 | | 7 | 1.9 |

(fortgesetzt)

| Beispiel-Nr. | $IC_{50}$ [μM] | | Beispiel-Nr. | $IC_{50}$ [μM] |
|---|---|---|---|---|
| 11 | 1 | | 12 | 1.5 |
| 13 | 1.4 | | 15 | 1.1 |
| 21 | 1.3 | | 25 | 1.1 |
| 27 | 1 | | 33 | 0.8 |

Die Thrombinzeit (TT) wird in Gegenwart variierender Konzentrationen an Prüfsubstanz oder dem entsprechenden Lösungsmittel mit einem handelsüblichen Testkit (Thrombin Reagent von der Firma Roche) bestimmt. Die Testverbindungen werden 3 Minuten bei 37°C mit dem Plasma inkubiert. Anschließend wird durch Zugabe des Thrombin Reagenz die Gerinnung ausgelöst und der Zeitpunkt des Gerinnungseintritts bestimmt. Es wird die Konzentration an Prüfsubstanz ermittelt, die eine Verdoppelung der Thrombinzeit bewirkt.

Die aktivierte partielle Thromboplastinzeit (APTT) wird in Gegenwart variierender Konzentrationen an Prüfsubstanz oder dem entsprechenden Lösungsmittel mit einem handelsüblichen Testkit (PTT Reagent von der Firma Roche) bestimmt. Die Testverbindungen werden 3 Minuten bei 37°C mit dem Plasma und dem PTT Reagenz (Cephalin, Kaolin) inkubiert. Anschließend wird durch Zugabe von 25 mM Calciumchlorid die Gerinnung ausgelöst und der Zeitpunkt des Gerinnungseintritts bestimmt. Es wird die Konzentration an Prüfsubstanz ermittelt, die eine Verdoppelung der APTT bewirkt.

**a.6) Thromboelastographie (Thromboelastogramm)**

Die Thromboelastographie wird mit Hilfe des Thromboelastographen ROTEM der Firma Pentapharm und dem dazu gehörenden Zubehör Cup and pin durchgeführt. Die Messung erfolgt in Vollblut, welches zuvor in Natrium-Citrat Monovetten der Firma Sarstedt entnommen wird. Das Blut wird in den Monovetten mit Hilfe eines Schüttlers in Bewegung gehalten und bei 37°C für 30 min. vorinkubiert.

Es wird eine 2 molare Stammlösung von Calciumchlorid in Wasser erstellt. Diese wird 1:10 mit einer wässrigen 0.9% Natriumchlorid-Lösung verdünnt. Zur Messung werden dieser 20 μl 200 mM Calciumchlorid-Lösung in die Cups vorgelegt (Endkonzentration Calciumchlorid 12.5 mM). Es werden 3.2 μl Substanz oder Lösemittel zugegeben. Die Messung wird durch Zugabe von 300 μl Vollblut gestartet. Nach der Zugabe wird kurz mit der Pipettenspitze auf und ab pipettiert ohne Luftblasen zu erzeugen. Die Messung erfolgt über 2.5 Stunden oder wird bei Beginn der Fibrinolyse gestoppt. Zur Auswertung werden folgende Parameter bestimmt: CT(clotting time/ [sec.]), CFT (clotting formation time/ [sec.]), MCF (maximum clot firmness / [mm]) und der alpha-Winkel [°]. Die Messpunkte werden alle 3 Sekunden erhoben und graphisch über die y-Achse als MCF [mm] und auf der x-Achse als Zeit [sec.] dargestellt.

**a.7) Inhibierung der an Thrombus gebundenen Gerinnungsfaktoren Thrombin**

Blutgerinnsel, die sich entweder vor Therapiebeginn mit Antikoagulantien, während Therapiepausen oder trotz Therapie bilden, enthalten große Mengen Gerinnungsfaktoren, die eine fortschreitende Thrombusbildung begünstigen können. Diese Gerinnungsfaktoren sind fest an den Thromus gebunden und können nicht ausgewaschen werden. In bestimmten klinischen Situationen kann hieraus ein Risiko für den Patienten entstehen. In den unten aufgeführten Versuchen lassen sich in humanen Thromben sowohl Thrombin als auch FXa mit biologischer (prokoagulatorischer) Aktivität nachweisen.

*In vitro* gebildete Thromben

Thromben werden *in vitro* aus humanem Plasma gebildet und auf Aktivität der gebundenen Gerinnungsfaktoren Thrombin und FXa untersucht. Hierzu werden 300μl Plasma mit 30 μl Lipidvesikeln und 30 μl einer wässrigen Calciumchlorid-Lösung in einer 48 MTP Platte gemischt, und 30 min inkubiert. Dieser und die folgenden Schritte werden bei 37°C und unter konstanter Bewegung durchgeführt (300 U/min). Die gebildeten Thromben werden in eine neue 48 MTP Platte transferiert und in 0.9%iger Natriumchlorid-Lösung zweimal 10 min gewaschen, wobei der Thrombus zwischen den Waschgängen auf Filterpapier abgetupft wird. Der Thrombus wird in Puffer B transferiert (Owens Veronal Puffer, 1% BSA) und 15 min inkubiert, auf Filterpapier abgetupft und 30 min in Testsubstanz in verschiedenen Konzentrationen in Puffer B inkubiert. Anschließend werden die Clots wie oben beschrieben zweimalig gewaschen. Die Thromben werden abgetupft und in Puffer D transferiert: (240 μl Owren's veronal Puffer, 1% BSA und 15.6 mM Calciumchlorid) und mit oder ohne 0.6 μM Prothrombin 45 min inkubiert. Die Reaktion wird durch 75 μl 1% EDTA-Lösung gestoppt. Thrombinaktivität wird separat im Thrombus in Puffer A (7.5 mM $Na_2EDTAx2H_2O$, 175 mM Natriumchlorid, 1% BSA, pH 8.4) oder im Überstand aus dem letzten Schritt gemessen. Hierzu wird das unter a.1) eingesetzte Thrombinsubstrat in der Endkonzentration 50 μM eingesetzt, und die resultierende Fluoreszenz im Fluoreszenz-Plattenausleser ausgemessen (360/465nm).

**a.8) Einfluss der Thrombininhibitoren auf die Thrombolyse in Plättchen-armem Plasma**

Die Wirkung der Prüfsubstanzen auf die in-vitro Thrombolyse in Plättchen-armem Plasma wird in Anwesenheit von Gewebs-Plasminogenaktivator (tPA) getesten. Hierzu wird unter Kontrolle mittels Trübungsmessung (UV-Absorption bei 405 nm) in einer Mikrotiterplatte in humanem Plasma unter Zugabe von Gewebefaktor (Tissue Factor) zunächst ein Gerinnsel gebildet, dessen Auflösung durch gleichzeitige Gabe von Gewebs-Plasminogenaktivator (tPA) in einem bestimmten Zeitfenster eingestellt wird. Zeitgleiche Zugabe verschiedener Mengen der Testsubstanz können zu einer Verkürzung der Thrombolyse-Zeit führen (Zeit zwischen der maximalen Trübung und des Wiedererreichens der Basislinie).

In einer 384-Loch-Mikrotiterplatte werden 63 μL humanes Plasma (Deutsches Rotes Kreuz, entspricht 90% Plasma im Test) mit 0.7 μL einer Ethanol/Wasser-Mischung (1:1), dieunterschiedliche Konzentrationen der Testsubstanzen enthält, 1.7 μL einer Lösung von humanem Thrombomodulin (finale Konzentration 10 nM) und 1.7 μL einer Lösung von humanem Gewebe-Plasminogenaktivator (Actilyse®, finale Konzentration 3 nM) versetzt. Die Koagulation wird durch den Zusatz von 3.5 μL einer Gewebefaktor-enthaltenden Lösung (Recombiplastin 2G in einer 1:100-Verdünnung in 0.2 M Calciumchlorid-Lösung) bei 37°C gestartet. Danach wird sofort mit der Trübungsmessung (UV-Absortionsmessung bei 405 nm) in Minutenabständen begonnen. Die Thrombolyse-Zeit wird als Zeit zwischen der maximalen Absorption und dem Wiedererreichen der Basislinie berechnet.

**b) Bestimmung der antithrombotischen Wirkung (*in vivo*)**

**b.1) Arteriovenöses Shunt- und Blutungs-Modell (Kombi-Modell Ratte)**

Nüchterne männliche Ratten (Stamm: HSD CPB:WU) mit einem Gewicht von 300-350 g werden mit Inactin (150 - 180 mg/kg) narkotisiert. Die Thrombusbildung wird in einem arteriovenösen Shunt in Anlehnung an die von Christopher N. Berry et al., Br. J. Pharmacol. (1994), 113, 1209-1214 beschriebene Methode ausgelöst. Dazu werden die linke Vena jugularis und die rechte Arteria carotis freipräpariert. Ein extracorporaler Shunt wird mittels eines 10 cm langen Polyethylenschlauchs (PE 60) zwischen den beiden Gefäßen gelegt. Dieser Polyethylenschlauch ist in der Mitte in einen weiteren 3 cm langen Polyethylenschlauch (PE 160), der zur Erzeugung einer thrombogenen Oberfläche einen aufgerauhten und zu einer Schlinge gelegten Nylonfaden enthält, eingebunden. Der extrakorporale Kreislauf wird 15 Minuten lang aufrechterhalten. Dann wird der Shunt entfernt und der Nylonfaden mit dem Thrombus sofort gewogen. Das Leergewicht des Nylonfadens ist vor Versuchsbeginn ermittelt worden.

Zur Bestimmung der Blutungszeit wird unmittelbar nach Öffnung des Shunt-Kreislaufs die Schwanzspitze der Ratten mit einer Rasierklinge um 3 mm kupiert. Der Schwanz wird in 37°C temperierte physiologische Kochsalzlösung gelegt und die Blutung aus der Schnittwunde über 15 Minuten beobachtet. Es werden die Zeit bis zum Sistieren der Blutung für mind. 30 Sekunden (initiale Blutungszeit), die Gesamtblutungszeit innerhalb von 15 Minuten (kumulative Blutungszeit) sowie die Menge des Blutverlusts über die photometrische Bestimmung des aufgefangenen Hämoglobins ermittelt.

Die Prüfsubstanzen werden vor Anlegung des extrakorporalen Kreislaufs und des Schwanzspitzenschnitts entweder intravenös über die kontralaterale Jugularvene als Einzelbolus bzw. als Bolus mit anschließender Dauerinfusion oder oral mittels Schlundsonde wachen Tieren verabreicht.

**b.2) Eisen(II)chlorid-Schädigungs- und Blutungs-Modell (Kombi-Modell II, Ratte)**

Männliche Ratten (Stamm: HsdRCCHan:Wist) mit einem Gewicht von 300g-325g werden mit Inactin (180 mg/kg) intraperitoneal narkotisiert. Die Thrombusbildung wird mit Eisen(II)chlorid in der Arteria carotis ausgelöst. Dazu wird die rechte Arteria carotis freipräpariert. Dann wird ein Flussmesskopf angeschlossen und 10 Minuten der Blutfluss aufgezeichnet. Danach werden Arterie und Umgebung trocken gelegt. Parafilm (10 x 8 mm) und Filterpapier (10 x 6 mm geknickt) werden unter die A. carotis gelegt und mit 20μl Eisen(II)chlorid-Lösung (Eisen(II)chlorid Tetrahydrat Reagent Plus 99%, Sigma, 5%ige Lösung in Wasser wird hergestellt) benetzt. Ein kleines Stück Filterpapier wird von oben auf die A. carotis aufgelegt und ebenfalls mit Eisen(II)chlorid-Lösung benetzt. Die so belegte A. carotis wird mit einem feuchten Tupfer bedeckt und 5 Minuten belassen. Danach werden Parafilm und Filterpapier entfernt und die Arterie mit physiologischer Natriumchlorid-Lösung gespült. Der Flussmesskopf wieder angebracht und der Blutfluss 30 Minuten aufgezeichnet. Danach wird die Messung gestoppt und das freipräparierte Stück A.carotis mit Gefäßklemmen abgeklemmt und herausgeschnitten. Der sich im Gefäß befindliche Thrombus wird mithilfe einer Pinzette aus dem Gefäß entfernt und sofort gewogen.

Zur Bestimmung der Blutungszeit wird nach Schädigung und Wiederanbringen des Flussmesskopfes die Schwanzspitze der Ratte mit einer Rasierklinge um 3 mm kupiert. Der Schwanz wird in 37°C temperiertes Wasser gelegt und die Blutung aus der Schnittwunde über 15 Minuten beobachtet. Es werden die Zeit bis zum Sistieren der Blutung für mind. 30 Sekunden (initiale Blutungszeit), die Gesamtblutungszeit innerhalb von 15 Minuten (kumulative Blutungszeit) sowie die Menge des Blutverlusts über die photometrische Bestimmung des aufgefangenen Hämoglobins ermittelt.

Die Prüfsubstanzen werden entweder intravenös über die Jugularvene als Einzelbolus direkt vor Beginn des Versuchs bzw. als Bolus (vor Beginn) mit anschließender Dauerinfusion gegeben.

**b.3) Venöse Reperfusion und Blutungs-Modell am Kaninchen (Kombi-Modell-Kaninchen)**

Männliche Neuseeländer Kaninchen mit einem Gewicht von 2.8 - 3.4 kg werden mit einer intramuskulären Ketamin/Rompun Bolusinjektion narkotisiert. Anschließend wird das Tier an den für die Operation notwendigen Stellen rasiert. Über eine Verweilkanüle wird über die linke Ohrvene eine kontinuierliche Narkoseinfusion (Ketamin/Rompun) verabreicht. Die rechte und linke Vena femoralis, sowie die rechte Arteria femoralis werden mit einem Polyethylenschlauch (PE50) katheterisiert. Anschließend wird die Vena jugularis vorsichtig freipräpariert, so dass das Gefäß möglichst wenig strapaziert und geschädigt wird und sich kein Fett mehr an dem Gefäß befindet. Mittels einer geeigneten Flussmessanlage (Powerlab, Transonic TS420 inkl. Flussmesskopf) wird der Fluss in der Vena jugularis aufgenommen (Lab Chart Software). Dem Kaninchen wir vor Beginn des Versuches aus der Arteria femoralis zweimal 1.4 ml Citrat-Blut entnommen und die basale Blutungszeit wird am Ohrrand bestimmt. Sobald der Fluss in der Vena jugularis für 10 min konstant gelaufen ist (vollständige Regeneration des Gefäßes nach Präparation), wird die Vene mit kleinen Gefäßklemmen 2 cm lang abgeklemmt. In einer Petrischale wird das vorher entnommene Citrat-Blut (300 μl) mit Calciumchlorid (0.25 M, 90 μl) und Thrombin (25 U/ml, 60 μl) gemischt. 180 μl der Blut/Calciumchlorid/Thrombin-Mischung werden schnell mit einer 1 ml Spritze aufgezogen und durch eine 27G Kanüle in das abgeklemmte Gefäßsegment injiziert. Die Einstichstelle wird eine Minute lang mit einer Pinzette abgeklemmt, damit kein Blut entweichen kann. Zwei Minuten nach Injektion des Thrombus wird die Prüfsubstanz als Bolus und Infusion durch den linken venösen Femoralkatheter appliziert und angeschlossen. 14 Minuten nach Thrombusinjektion wird Gewebe-Plasminogenaktivator als Bolus und Infusion (Actilyse®, 20μg/kg Bolus & 150 μg/kg/h Infusion) an der rechten Vena femoralis angeschlossen. 15 Minuten nach Thrombusinjektion wird die Stase geöffnet und der Flussmesskopf angeschlossen. Der Blutfluss im Gefäß wird über 120 Minuten aufgenommen, wobei das Gefäß mit warmer 0.9%iger wässriger Natriumchlorid-Lösung feuchtgehalten wird. Nach 105 Minuten Reperfusion wird erneut die Ohrblutungszeit bestimmt. Am Ende des Versuches, nach 120 Minuten Reperfusion, wird 1.4 ml Citrat-Blut abgenommen, das Tier schmerzfrei durch 1.5 ml T61 Bolusinjektion abgetötet und das Thrombusgewicht in der Vena jugularis bestimmt. Das vor und nach dem Versuch abgenommene Blut dient zur Plasmagewinnung und ex-vivo Gerinnungszeitbestimmung.

Die Fläche unter der Blutfluß-Zeit-Kurve wird berechnet (AUC) und zur maximal erzielbaren Fläche, die aus dem Blutfluß vor dem Versuch und der Zeit (120 min) errechnet wird in Beziehung gesetzt. Die ausschließlich mit Gewebe-Plasminogenaktivator erzielte Fläche wird von der mit der jeweiligen Substanz beziehungsweise Dosierung erzielten Fläche subtrahiert. Die dadurch entstehende Fläche gilt als Mass für die Verbesserung der Reperfusion durch die zu testende Substanz (Tabelle 4).

**Tabelle 4: Synergistische antithrombotische Wirkung der Kombination von Beispiel 15 mit Rivaroxaban**

| Erhöhung der Reperfusion (Blutfluß-Zeit-Fläche) nach Behandlung mit | | |
|---|---|---|
| Verbindung aus Beispiel 15 [bolus 0.33 mg/kg; Dauerinfusion 0.43 mg/kg/h] | Rivaroxaban [bolus 0.08 mg/kg; Dauerinfusion 0.09 mg/kg/h] | Kombination der Verbindung aus Beispiel 15 [bolus 0.33 mg/kg; Dauerinfusion 0.43 mg/kg/h] mit Rivaroxaban [bolus 0.08 mg/kg; Dauerinfusion 0.09 mg/kg/h] |
| 12.2 % | 13.0 % | 49.5 % |
| Nicht signifikanter Effekt ($p > 0.05$) | Nicht signifikanter Effekt ($p > 0.05$) | Signifikanter Effekt ($p < 0.05$) |

**c) Bestimmung der Pharmakokinetik**

**c.1) Pharmakokinetik nach intravenöser Gabe der Testsubstanz**

Männliche Wistar Ratten werden anästesiert und ihnen wird ein Katheter in die vena jugularis gesetzt. Am nächsten Tag wird eine definierte Dosis der Testsubstanz als Lösung durch Injektion in die Schwanzvene verabreicht. Blutproben werden mittels des Katheters über einen Zeitraum von 7 Stunden gesammelt (9 Zeitpunkte).

Weiblichen Beagle Hunden wird eine definierte Dosis der Testsubstanz als Lösung über die vena cephalica als 15 min Infusion verabreicht. Blutproben werden mittels eines Katheters in der vena cephalica über einen Zeitraum von 7 Stunden gesammelt (12 Zeitpunkte).

Das Blut wird in Heparingefäßen zentrifugiert. Die Plasmaprobe wird zur Proteinfällung mit Acetonitril versetzt und zentrifugiert. Die Testsubstanz im Überstand wird mittels LC/MS-MS quantifiziert. Die ermittelten Plasmakonzentrationen der Testsubstanz werden zur Berechnung der pharmakokinetischen Parameter wie AUC (Fläche unter der Plasmakonzentrations-Zeit-Kurve), $V_{ss}$ (Verteilungsvolumen), $C_{max}$ (höchste Konzentration der Testsubstanz im Plasma nach Verabreichung), $t_{1/2}$ (Halbwertszeit) und CL (Totale Clearance der Testsubstanz vom Plasma) verwendet. Für die Berechnung der Blut-Clearance wird die Blut-Plasma-Verteilung mittels Inkubation der Testsubstanz in Blut bestimmt. Nach Abtrennung des Plasmas durch Zentrifugation wird die Konzentration der Testsubstanz im Plasma mittels LC/MS-MS bestimmt.

**c.2) Pharmakokinetik nach peroraler Gabe der Testsubstanz**

Männliche Wistar Ratten werden anästesiert und ihnen wird ein Katheter in die vena jugularis gesetzt. Am nächsten Tag wird eine definierte Dosis der Testsubstanz peroral verabreicht. Blutproben werden mittels des Katheters über einen Zeitraum von 24 Stunden gesammelt (9 Zeitpunkte).

Weiblichen Beagle Hunden wird eine definierte Dosis der Testsubstanz peroral verabreicht. Blutproben werden mittels eines Katheters in der vena cephalica über einen Zeitraum von 24 Stunden gesammelt (9 Zeitpunkte).

Das Blut wird in Heparingefäßen zentrifugiert. Die Plasmaprobe wird zur Proteinfällung mit Acetonitril versetzt und zentrifugiert. Die Testsubstanz im Überstand wird mittels LC/MS-MS quantifiziert. Die ermittelten Plasmakonzentrationen der Testsubstanz werden zur Berechnung der pharmakokinetischen Parameter wie AUC (Fläche unter der Plasmakonzentrations-Zeit-Kurve), $C_{max}$ (höchste Konzentration der Testsubstanz im Plasma nach Verabreichung), $t_{1/2}$ (Halbwertszeit) und F (Bioverfügbarkeit) verwendet.

**c.3) Caco-2 Permeabilitätsassay**

Die *in vitro* Permeabilität der Testsubstanz durch einen Caco-2 Zellmonolayer wird mittels eines etablierten *in vitro* Systems zur Vorhersage der Permeabilität durch den Gastrointestinaltrakt bestimmt [1]. Caco-2 Zellen (ACC Nr. 169, DSMZ, Deutsche Sammlung von Mikroorganismen und Zellkulturen, Braunschweig) werden auf 24 Well-Platten ausgesät und für 14 bis 16 Tage kultiviert. Die Testsubstanz wird in DMSO gelöst und auf eine Konzentration von 2 μM in Transportpuffer (HBSS, Hanks Buffered Salt Solution, Gibco/Invitrogen, supplementiert mit Glukose (Endkonzentration 19.9 mM) und HEPES (Endkonzentration 9.8 mM)) verdünnt. Zur Bestimmung der apikalen nach basolateralen Permeabilität ($P_{app}$ A-B) wird die Testsubstanz der apikalen Seite und Transportpuffer der basolateralen Seite des Zellmonolayers zugegeben. Zur Bestimmung der basolateralen nach apikalen Permeabilität ($P_{app}$ B-A) wird die Testsubstanz der basolateralen Seite und Transportpuffer der apikalen Seites des Zellmonolayers zugegeben. Proben aus dem Donor-Kompartment werden zu Beginn des Experimentes zur Bestimmung der Massenbilanz genommen. Nach einer Inkubationszeit von 2 Stunden bei 37°C wurden Proben aus beiden Kompartimenten genommen. Proben werden mittels LC/MS-MS quantifiziert und die Permeationskoeffizienten berechnet. Die Permeabilität von Lucifer Yellow wird für jeden Zellmonolayer bestimmt, um die Unversehrtheit des Zellmonolayers zu gewährleisten. Die Permeabilität von Atenolol (Marker für niedrige Permeabilität) und von Sulfasalazin (Marker für aktive Exkretion) wird in jedem Zellbatch zur Qualitätskontrolle der Zellen mitbestimmt.

Literatur: Artursson, P. and Karlsson, J. (1991). Correlation between oral drug absorption in humans and apparent drug permeability coefficients in human intestinal epithelial (Caco-2) cells. Biochem. Biophys.175 (3), 880-885.

**c.4) *In vitro* Clearance Bestimmungen mit Hepatozyten**

Inkubationen mit frischen Primär-Hepatozyten werden bei 37°C in einem Gesamtvolumen von 1.5 mL mit einem modifizierten Janus ® Roboter (Perkin Elmer) unter Schütteln durchgeführt. Die Inkubationen enthalten typischerweise 1 Mio lebende Leberzellen / mL, ~ 1 μM Substrat und 0.05 M Kalium-Phosphatpuffer (pH = 7.4). Die finale ACN Konzentration in der Inkubation ist ≤ 1%.

Aliquots von 125 μl werden den Inkubationen nach 2, 10, 20, 30, 50, 70 und 90 min entnommen und in 96 well Filterplatten überführt (0.45μm Low-Binding Hydrophilic PTFE; Millipore: MultiScreen Solvinert). Diese enthalten jeweils 250 μl ACN um die Reaktion abzustoppen. Nach der Zentrifugation werden die Filtrate mit MS/MS analysiert (üblicherweise API 3000).

Die in vitro Clearances werden aus den Halbwertszeiten des Substanzabbaus berechnet wobei folgende Gleichungen benutzt werden:

- $CL'_{intrinsic}$ [mL/(min.kg)] = (0.693 / in vitro t1/2 [min]) · (Lebergewicht [g Leber /kg Körpergewicht]) (Zellzahl [1.1·10^8] / Lebergewicht [g]) / (Zellzahl [1·10^6] / Inkubationsvolumen [mL])

$CL_{blood}$ wird ohne Berücksichtigung der freien Fraktion ("nonrestricted well stirred model") nach folgender Gleichung berechnet.

- $CL_{blood}$ well-stirred [L/(h·kg)] = ($Q_H$ [L/(h·kg)]·$CL'_{intrinsic}$ [L/(h·kg)]) / ($Q_H$ [L/(h·kg)] + $CL'_{intrinsic}$ [L/(h·kg)])

Die spezies-spezifischen Hochrechnungsfaktoren mit denen gerechnet wird sind in folgender Tabelle 5 zusammengefaßt:

**Tabelle 5**

| male / female | Mouse m | Mouse f | Rat m/f | Dog m/f | Cyno f | Man m/f |
|---|---|---|---|---|---|---|
| Cell number / g Liver [Mio cells] | 110 | 110 | 110 | 110 | 110 | 110 |
| Liver [g] / kg Body Weight | 50 | 43 | 32 | 39 | 30 | 21 |
| Liver Blood Flow [L/(h·kg)] | 5.4 | 5.4 | 4.2 | 2.1 | 2.5 | 1.3 |

$F_{max}$ Werte, die die maximal mögliche Bioverfügbarkeit - bezogen auf die hepatische Extraktion-angeben werden wie folgt berechnet:

$$F_{max} \text{ well-stirred } [\%] = (1-(CL_{blood} \text{ well-stirred } [L/(h\cdot kg)] / Q_H [L/(h\cdot kg)])) \cdot 100$$

**c.5) CYP-Inhibitionstest**

Inhibitorische Eigenschaften eines Wirkstoffes auf die Cytochrome P450 (CYP) des menschlichen Organismus können massive klinische Auswirkungen (Arzneimittel-Wechselwirkungen) nach sich ziehen, da ein Hauptteil der verschriebenen Medikamente durch diese Enzyme abgebaut (verstoffwechselt) werden. Daran beteiligt sind dabei vor allem die CYP-Isoenzyme der 1A- und 2C-Familie, CYP2D6 sowie mit einem Anteil von fast 50 % CYP3A4. Um diese möglichen Arzneimittel-Wechselwirkungen (Drug-Drug Interactions, DDI) auszuschließen bzw. zu minimieren, wird die Fähigkeit von Substanzen, CYP1A2, CYP2C8, CYP2C9, CYP2D6 und CYP3A4 im Menschen inhibieren zu können, mit Humanlebermikrosomen (Pool aus verschiedenen Individuen) untersucht. Dies geschieht durch Messung von CYP-isoformspezifischen Metaboliten, die sich aus Standardsubstraten, wie zum Beispiel Phenacetin, Amodiaquin, Diclofenac, Dextromethorphan, Midazolam und Testosteron, bilden. Die Inhibitionseffekte werden bei sechs verschiedenen Konzentrationen der Testverbindungen untersucht (1.5, 3.1, 6.3, 12.5, 25 sowie 50 μM als Höchstkonzentration oder 0.6, 1.3, 2.5, 5, 10 sowie 20 μM als Höchstkonzentration), mit dem Ausmaß der CYP-isoformspezifischen Metabolitenbildung der Standardsubstrate in Abwesenheit der Testverbindungen verglichen und die entsprechenden $IC_{50}$-Werte berechnet. CYP-isoformspezifische Standard-Inhibitoren wie zum Beispiel Furafyllin, Montelukast, Sulfaphenazol, Fluoxetin und Ketoconazol dienen als Kontrolle der erhaltenen Ergebnisse. Um Hinweise auf mögliche mechanism-based Inhibitioren (MBI) auf CYP3A4 zu erhalten, werden vor der Zugabe von Midazolam oder Testosteron als Standardsubstrate für CYP3A4 die Humanlebermikrosomen 30 Minuten in Gegenwart des zu untersuchenden Inhibitors inkubiert. Eine Verringerung des erhaltenen $IC_{50}$-Wertes im Vergleich zum Ansatz ohne Vorinkubation dient als Hinweis für eine mechanism-based Inhibition. Als Positivkontrolle dient Mibefradil.

Durchführung: Die Inkubationen der Standardsubstrate mit Humanlebermikrosomen (14-100 μg/mL) in Gegenwart der Testverbindung (als potentiellem Inhibitor) werden bei 37 °C in 96-Lochplatten auf einer Workstation (Tecan, Genesis; Hamiltion, MICROLAB STARLET) durchgeführt. Die Inkubationszeiten betragen 10-15 Minuten. Die Testverbindungen werden bevorzugt in Acetonitril gelöst (1.0, 2.0 bzw. 2.5, 5.0 mM Stammlösung). Die 96-Lochplatten werden hergestellt durch sequentielle Zugabe einer Stammlösung von NADP+, EDTA, Glukose-6-phosphat und Glukose-6-phosphat-Dehydrogenase in Phosphat-Puffer (pH 7.4), der Testverbindung, sowie einer Lösung von Standardsubstrat und Humanlebermikrosomen in Phosphat-Puffer (pH 7.4). Das Gesamtvolumen beträgt 200 μl. Auf der 96-Lochplatte befinden sich außerdem die entsprechenden Kontrollinkubationen mit und ohne Standardinhibitor. Die Inkubationen

werden nach der jeweiligen Inkubationszeit durch Zugabe von 100 µl Acetonitril abgestoppt, worin sich ein geeigneter interner Standard befindet. Gefällte Proteine werden durch Zentrifugation abgetrennt (3000 rpm, 10 Minuten, 10 °C). Die erhaltenen Überstände der jeweiligen Platten werden auf einer Platte vereinigt und mittels LC-MS/MS analysiert. Aus den erhaltenen Messdaten werden die $IC_{50}$- Werte generiert und zur Bewertung des inhibitorischen Potenzials der Testverbindung herangezogen.

**c.6) Zellulärer *in vitro*-Test zur Bestimmung der Induktion arzneimittelabbauender cytochromaler Enzyme (CYPs) in primären Hepatozyten des Menschen**

Enzyminduktion ist eine unerwünschte Eigenschaft eines Arzneimittels, die eine breite und sichere Anwendbarkeit des Wirkstoffs in Frage stellt. Folge der Enzyminduktion ist ein beschleunigter Abbau (Metabolisierung) von Arzneistoffen in der Leber. Die kombinierte Einnahme von einem Enzyminduktor und anderen Medikamenten wie beispielsweise Immunsuppressiva, Gerinnungsmittel oder auch Verhütungsmittel kann zu einer völligen Unwirksamkeit der Arzneimittel führen.

Ziel der Untersuchung ist es Substanzen zu finden die diese unerwünschte Arzneimittelwechselwirkung nicht aufweisen. Die Identifizierung von Enzyminduktoren erfolgt mit Hilfe von primären Hepatozyten des Menschen in Langzeitkultur. Zur Kultivierung der Zellen werden Hepatozyten auf einer Collagen I-Schicht ausplattiert (Dichte von 100000 Zellen/$cm^2$) und die angewachsenen Zellen dann mit einer zweiten Collagenschicht (Sandwich-Technik) überschichtet. (Kern A, Bader A, Pichlmayr R, and Sewing KF, Biochem Pharmacol., 54, 761-772 (1997). Um den Einfluss der Testsubstanzen auf die Regulation der Leberenzyme zu erhalten werden die Hepatozyten in Langzeit-Kultur mehrere Tage mit den Wirkstoffen inkubiert.

Testablauf: Die Zellen werden nach einer zweitägigen Regenerationsphase in Williams Medium E, 10% FCS, Prednisolon, Insulin, Glucagon und L-Glutamin, Penicillin and Streptomycin mit den Testsubstanzen behandelt. Dazu werden Stammlösungen der Wirkstoffe mit einer Konzentration von 1 mg/mL in Acetonitril oder Methanol hergestellt und in 8 Verdünnungsschritten (1:3) in Zellkulturmedium zu den Zellkulturen pipettiert und in einem Zellinkubator (96% Luftfeuchtigkeit, 5% v/v Kohlendioxid, 37°C) ca. 5 Tage inkubiert. Das Zellkulturmedium wird täglich gewechselt. Nach dieser Inkubationszeit werden die Zellkulturen mit Cytochrom P450(CYP)-spezifischen Substraten inkubiert um die Aktivität der Leberenzyme CYP1A2, CYP3A4, CYP2B6 und CYP2C19 zu bestimmen. Die abgestoppten Proben werden entweder direkt analysiert oder bis zur Analyse bei -20°C gelagert.

Dazu werden die Medien der Zellkulturen mit geeigneten C18-reversed-phase Säulen und variablen Gemischen Acetonitril und 10 mM Ammoniumformiat chromatographiert (HPLC-MS/MS).

Die massenspektrometrischen Daten dienen zur Quantifizierung der Substratumsätze und daraus abgeleitet der Berechnung der Leberenzymaktivitäten. Wirkstoffe mit ungünstigen Eigenschaften auf die Regulation der Leberenzyme werden nicht weiter verfolgt.

**d) Reinigung, Kristallalisation, und Einkristallstrukturbestimmung von humanem α-Thrombin (=FIIa) im Komplex mit Beispiel 15**

**d.1)** Kristallisation von humanen **α-Thrombin**

Das Protein α-Thrombin wurde von der Firma Haemochrom gekauft (Uniprot P00734, Aminosäure 328 bis 622) . Ein vial mit 5000U (~1.7 mg) des Proteins wird mit einem 20 mM Phosphatpuffer bei pH 7.5, 350 mM Natriumchlorid-Kristallisationspuffer und 2 mM Benzamidin auf eine Konzentration von ca. 10 mg/ml gebracht. Die Konzentration des α-Thrombins wird mit dem NanoDrop® ND-1000 Spectrophotometer überprüft. Zu der α-Thrombin Lösung wird ein Hirudin Fragment (gekauft bei der Firma Bachem) im molaren Verhältnis 1:4 hinzugefügt. Die Mischung wird mindestes 2 Stunden bei 4°C inkubiert. Messbare Einkristalle können mit Hilfe der hangingdrop Methode bei 10°C erhalten werden. Hierzu werden gleiche Volumina der Proteinlösung und der Reservoirlösung (0.02M Phosphat Puffer bei pH 7.5, 27% PEG 8000, 100 mM Natriumchlorid-Lösung) zusammen pipettiert und mit Kristallkeimen von α-Thrombin versetzt. Kristalle von α-Thrombin entstehen meist über Nacht.

**d.2) Komplexbildung von humanen α-Thrombin mit Beispiel 15 im Kristall**

Eine 50 mM DMSO-Lösung von Beispiel 15 wurde mit dem Reservoirbuffer zu einer finalen Konzentration von 5 mM verdünnt. α-Thrombin Kristalle wurden in 2 µl dieser Lösung überführt und über Nacht in dieser Lösung belassen (=soaking).

**d.3) Datensammlung und Prozessierung**

Der gesoakte Kristall wurde für sehr kurze Zeit in eine Lösung mit 0.02 M Phosphat Puffer bei pH 7.5, 27% PEG 8000, 100 mM Natriumchlorid-Lösung und 15% Glycerol gegeben und anschliessend in flüssigen Stickstoff schockgefroren. Der Kristall wurde auf einem Bruker Proteum System bei 100K und einer Wellenlänge von 1.5418Å vermessen. Als Detektionsgerät diente ein CCD Zähler. Die Daten wurden mit dem Programm SAINT integriert und mit dem Programm SADABS skaliert (beide enthalten im Bruker Proterum Programm Paket). Der Kristall streute bis zu einer Auflösung von 1.6Å und kristallierte in der monoklinen Raumgruppe C2 mit Zellkanten von a = 69.166Å, b = 70.343Å und c = 71.372Å mit einem Molekül in der asymetrischen Einheit.

**d.4) Struktur Bestimmung und Verfeinerung**

Die Struktur von α-Thrombin konnte mit der Methode des molekeularen Ersatzes (*Molecular Replacement*) mit einer weiteren internen Struktur als Suchmodell und dem Programm PHASER (CCP4 Programmpaket) gelöst werden. Beispiel 15 wurde mit Hife des Programms Discovery Studio als 3D Modell erzeugt und mit dem Programm PRODRG ein Parameterfile erzeugt. Beispiel 15 wurde manuell in die Elektronendichte plaziert und im Programm COOT in der Elektronendichte minimiert. Die weitere Verfeinerung erfolgte iterativ mit den Programmen REFMAC5.5 und COOT (beide CCP4 Programmpaket) zu einem finalen R1-Wert von 20.52% und Rfree Wert von 24.73%. Daten und Verfeinerungsstatistiken sind in Tabelle 6 zusammengefasst.

**Tabelle 6:** Datensammlung und Verfeinerungsstatistiken für humanes α-Thrombin im Komplex mit Beispiel 15.

| | |
|---|---|
| Wellenlänge | 1,5418 Å |
| Auflösung (äußerste Schale) | 71,27-1,59 (1,69-1,59) Å |
| Reflexe (beobachtet/gemittelt) | 130734/40347 |
| Vollständigkeit[a] | 89,2% (90,2%) |
| I/s[a] | 12,95 (2.85) |
| Rmerge[ab] | 0,064 (0,27) |
| Raumgruppe | C2 |
| Zellparameter | |
| *a* | 69,166Å |
| *b* | 70,343Å |
| *c* | 71.372Å |
| *b* | 100,28° |
| Rcryst[c] | 0.2052 |
| Rfree[d] | 0,2473 |
| Wilson Temperaturfaktor | 14,3 $Å^2$ |
| RMSD Bindungslängen[e] | 0,023 Å |
| RMSD Bindungswinkel | 2,212° |

[a] Die Werte in Klammern sind für die äußerste Auflösungsschale
[b] $R_{merge} = \sum hkl |I_{hkl} - <I_{hkl}>| / \sum hkl <I_{hkl}>$; $I_{hkl}$ ist die Intensität der Reflexe hkl und $<I_{hkl}>$ ist der Mittelwert von mehrfach gemessenen Intensitäten
[c] $R_{cryst} = \sum |F_{obs} - F_{calc}| / \sum F_{obs}$; $F_{obs}$ and $F_{calc}$ sind die beobachteten und berechneten Structurfaktoren
[d] 5% Testset
[e] RMSD, *root man square deviation* vom Paramterset der idealen Bindungsgeometrie

**d.5) Absolutstrukturbestimmung von Beispiel 15 in humanen α-Thrombin**

Der Komplex von α-Thrombin mit Beispiel 15 kristallisiert mit einem Molekül in der asymetrischen Einheit. Die Stereochemie von Beispiel 15 wird eindeutig durch die Kenntnis der Stereochemie des Proteins α-Thrombin bestimmt. In Beispiel 15 liegt an allen Stereozentren (C13, C28 und C31) eindeutig die S-Konfiguration vor.

Struktur aus Beispiel 15: (2-{[(1*S*)-1-(3-Chlorphenyl)-2-fluorethyl]amino}-7-methoxy-1,3-benzoxazol-5-yl)[(2*S*,5*S*)-5-(2-hydroxyethyl)-2-methylmorpholin-4-yl]methanon mit der folgenden Formel

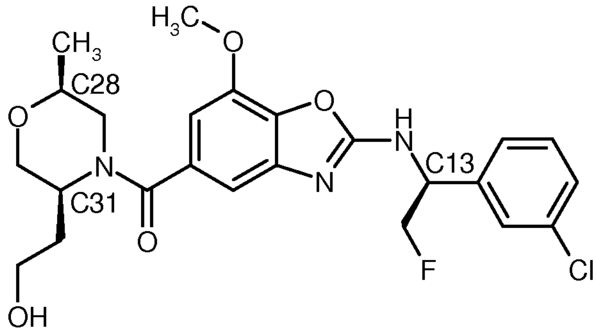

C) **Ausführungsbeispiele für pharmazeutische Zusammensetzungen**

Die erfindungsgemäßen Substanzen können folgendermaßen in pharmazeutische Zubereitungen überführt werden:

**Tablette:**

Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

Orale Suspension:

Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6 h gerührt.

**Intravenös applizierbare Lösung:**

Zusammensetzung:

1 mg der Verbindung von Beispiel 1, 15 g Polyethylenglykol 400 und 250 g Wasser für Injektionszwecke.

Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel

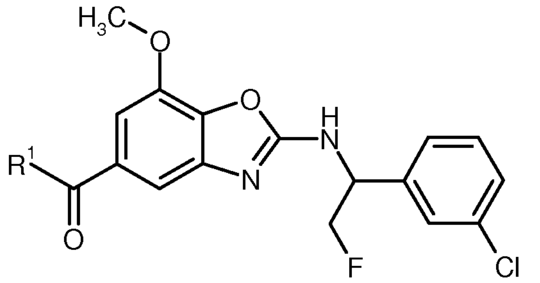

(I),

in welcher

$R^1$ für eine Gruppe der Formel

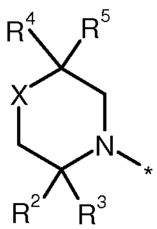

oder

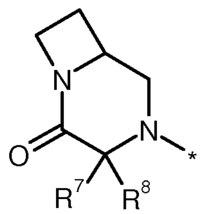

oder

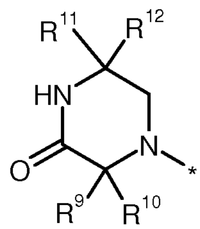

oder

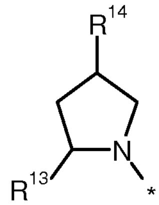

steht, wobei * die Anknüpfstelle an die Carbonylgruppe ist,
X für ein Sauerstoffatom, ein Schwefelatom oder CH-$R^6$ steht,
wobei
$R^6$ für Hydroxy steht,
$R^2$ für Wasserstoff, $C_1$-$C_6$-Alkyl, $C_3$-$C_6$-Cycloalkyl oder Phenyl steht,
worin Alkyl und Cycloalkyl substituiert sein können mit einem Substituenten ausgewählt aus der Gruppe bestehend aus Hydroxy, Methoxy, Cyano, Hydroxycarbonyl, Aminocarbonyl, Methylsulfonyl, Difluormethoxy und Trifluormethoxy,
oder
worin Alkyl und Cycloalkyl substituiert sein können mit 1 bis 3 Substituenten Fluor,
$R^3$ für Wasserstoff oder $C_1$-$C_4$-Alkyl steht,
oder $R^2$ und $R^3$ zusammen mit dem Kohlenstoffatom an das sie gebunden sind einen Cyclopropyl-Ring, Cyclobutyl-Ring oder Cyclopentyl-Ring bilden,
worin der Cyclobutyl-Ring und Cyclopentyl-Ring substituiert sein können mit einem Substituenten Hydroxy,
$R^4$ für Wasserstoff oder $C_1$-$C_6$-Alkyl steht,
worin Alkyl substituiert sein kann mit einem Substituenten Hydroxy,
$R^5$ für $C_1$-$C_4$-Alkyl steht,
oder $R^4$ und $R^5$ zusammen mit dem Kohlenstoffatom an das sie gebunden sind einen Cyclopropyl-Ring, Cyclobutyl-Ring oder Cyclopentyl-Ring bilden,
worin der Cyclobutyl-Ring und Cyclopentyl-Ring substituiert sein können mit einem Substituenten Hydroxy,
$R^7$ für Wasserstoff oder $C_1$-$C_6$-Alkyl steht,
worin Alkyl substituiert sein kann mit einem Substituenten Hydroxy oder Cyano,
oder
worin Alkyl substituiert sein kann mit 1 bis 3 Substituenten Fluor,
$R^8$ für Wasserstoff steht,
$R^9$ für Wasserstoff oder $C_1$-$C_6$-Alkyl steht,
worin Alkyl substituiert sein kann mit einem Substituenten Hydroxy oder Cyano,
oder
worin Alkyl substituiert sein kann mit 1 bis 3 Substituenten Fluor,
$R^{10}$ für Wasserstoff steht,
$R^{11}$ für $C_1$-$C_4$-Alkyl steht,

worin Alkyl substituiert sein kann mit einem Substituenten Hydroxy,
$R^{12}$ für Wasserstoff oder $C_1$-$C_4$-Alkyl steht,
oder $R^{11}$ und $R^{12}$ zusammen mit dem Kohlenstoffatom an das sie gebunden sind einen Cyclopropyl-Ring, Cyclobutyl-Ring oder Cyclopentyl-Ring bilden,
worin der Cyclobutyl-Ring und Cyclopentyl-Ring substituiert sein können mit einem Substituenten Hydroxy,
$R^{13}$ für Hydroxymethyl oder Hydroxyethyl steht,
$R^{14}$ für Methoxy oder Ethoxy steht,

oder eines ihrer Salze, ihrer Solvate oder der Solvate ihrer Salze.

**2.** Verbindung nach Anspruch 1, **dadurch gekennzeichnet, dass** $R^1$ für eine Gruppe der Formel

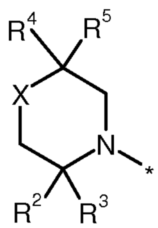

oder

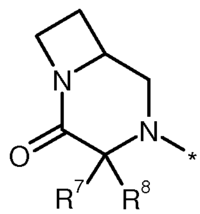

oder

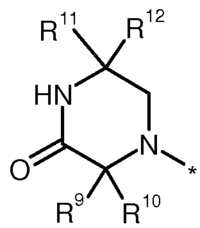

oder

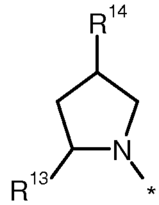

steht, wobei * die Anknüpfstelle an die Carbonylgruppe ist,
X für ein Sauerstoffatom oder CH-$R^6$ steht,
wobei
$R^6$ für Hydroxy steht,
$R^2$ für Wasserstoff, $C_1$-$C_4$-Alkyl oder $C_3$-$C_6$-Cycloalkyl steht,
worin Alkyl substituiert sein kann mit einem Substituenten Hydroxy oder Hydroxycarbonyl,
und
worin Cycloalkyl substituiert sein kann mit einem Substituenten Hydroxy,
$R^3$ für Wasserstoff oder $C_1$-$C_4$-Alkyl steht,
oder $R^2$ und $R^3$ zusammen mit dem Kohlenstoffatom an das sie gebunden sind einen Cyclobutyl-Ring bilden,
worin der Cyclobutyl-Ring substituiert sein kann mit einem Substituenten Hydroxy,
$R^4$ für Wasserstoff oder $C_1$-$C_4$-Alkyl steht,
worin Alkyl substituiert sein kann mit einem Substituenten Hydroxy,
$R^5$ für $C_1$-$C_4$-Alkyl steht,
$R^7$ für Wasserstoff oder $C_1$-$C_4$-Alkyl steht,
$R^8$ für Wasserstoff steht,
$R^9$ für Wasserstoff oder $C_1$-$C_4$-Alkyl steht,
worin Alkyl substituiert sein kann mit einem Substituenten Hydroxy,
$R^{10}$ für Wasserstoff steht,
$R^{11}$ und $R^{12}$ zusammen mit dem Kohlenstoffatom an das sie gebunden sind einen Cyclopropyl-Ring bilden,
$R^{13}$ für Hydroxymethyl oder Hydroxyethyl steht,
$R^{14}$ für Methoxy oder Ethoxy steht,

oder eines ihrer Salze, ihrer Solvate oder der Solvate ihrer Salze.

**3.** Verbindung nach einem der Ansprüche 1 oder 2, **dadurch gekennzeichnet, dass**

$R^1$ für eine Gruppe der Formel

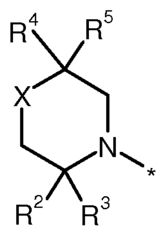

steht, wobei * die Anknüpfstelle an die Carbonylgruppe ist,
X für ein Sauerstoffatom steht,
$R^2$ für Methyl, Ethyl oder Cyclobutyl steht,
worin Methyl und Ethyl substituiert sind mit einem Substituenten Hydroxy,
und
worin Cyclobutyl substituiert ist mit einem Substituenten Hydroxy,
$R^3$ für Wasserstoff steht,
$R^4$ für Wasserstoff oder Methyl steht,
und $R^5$ für Methyl steht,
oder $R^2$ für Methyl steht,
$R^3$ für Wasserstoff oder Methyl steht,
$R^4$ für Methyl, Ethyl oder Propyl steht, worin Methyl, Ethyl und Propyl substituiert sind mit einem Substituenten Hydroxy,
und $R^5$ für Methyl steht,
oder $R^2$ und $R^3$ zusammen mit dem Kohlenstoffatom an das sie gebunden sind einen Cyclobutyl-Ring bilden, worin der Cyclobutyl-Ring substituiert ist mit einem Substituenten Hydroxy,
$R^4$ für Wasserstoff oder Methyl steht,
und $R^5$ für Methyl steht,

oder eines ihrer Salze, ihrer Solvate oder der Solvate ihrer Salze.

4. Verbindung nach einem der Ansprüche 1 oder 2, **dadurch gekennzeichnet, dass**

$R^1$ für eine Gruppe der Formel

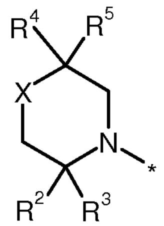

oder

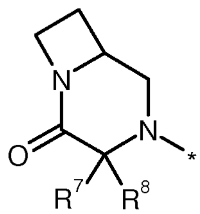

steht, wobei * die Anknüpfstelle an die Carbonylgruppe ist,
X für ein Sauerstoffatom steht,
$R^2$ für Methyl oder Ethyl steht,
worin Methyl und Ethyl substituiert sind mit einem Substituenten Hydroxy,
$R^3$ für Wasserstoff steht,
oder $R^2$ und $R^3$ zusammen mit dem Kohlenstoffatom an das sie gebunden sind einen Cyclobutyl-Ring bilden, worin der Cyclobutyl-Ring substituiert ist mit einem Substituenten Hydroxy,
$R^4$ für Wasserstoff oder Methyl steht,
$R^5$ für Methyl steht,
$R^7$ für Wasserstoff oder Methyl steht,
$R^8$ für Wasserstoff steht,

oder eines ihrer Salze, ihrer Solvate oder der Solvate ihrer Salze.

5. Verbindung nach einem der Ansprüche 1 bis 4, **dadurch gekennzeichnet, dass**

$R^1$ für eine Gruppe der Formel

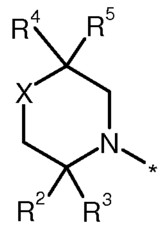

steht, wobei * die Anknüpfstelle an die Carbonylgruppe ist,
X für ein Sauerstoffatom steht,
$R^2$ für Methyl oder Ethyl steht, worin Methyl und Ethyl substituiert sind mit einem Substituenten Hydroxy,
$R^3$ für Wasserstoff steht,
oder $R^2$ und $R^3$ zusammen mit dem Kohlenstoffatom an das sie gebunden sind einen Cyclobutyl-Ring bilden, worin der Cyclobutyl-Ring substituiert ist mit einem Substituenten Hydroxy,
$R^4$ für Wasserstoff oder Methyl steht,
$R^5$ für Methyl steht,

oder eines ihrer Salze, ihrer Solvate oder der Solvate ihrer Salze.

6. (2-{[(1S)-1-(3-Chlorphenyl)-2-fluorethyl]amino}-7-methoxy-1,3-benzoxazol-5-yl)[(2*S*,5*S*)-5-(2-hydroxyethyl)-2-methyl-morpholin-4-yl]methanon nach Anspruch 1 mit der folgenden Formel

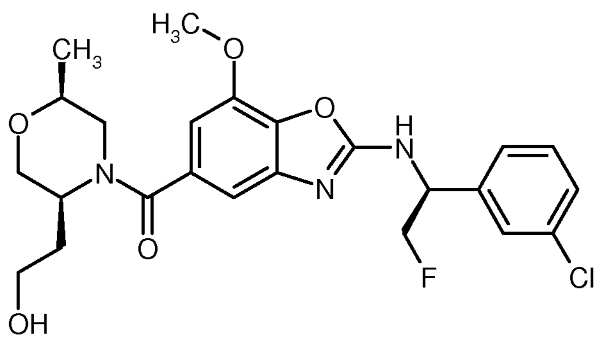

oder eines ihrer Salze, ihrer Solvate oder der Solvate ihrer Salze.

7. (2-{[(1*S*)-1-(3-Chlorphenyl)-2-fluorethyl]amino}-7-methoxy-1,3-benzoxazol-5-yl)[(2*S*,5*S*)-5-(2-hydroxyethyl)-2-methyl-morpholin-4-yl]methanon nach Anspruch 1 mit der folgenden Formel

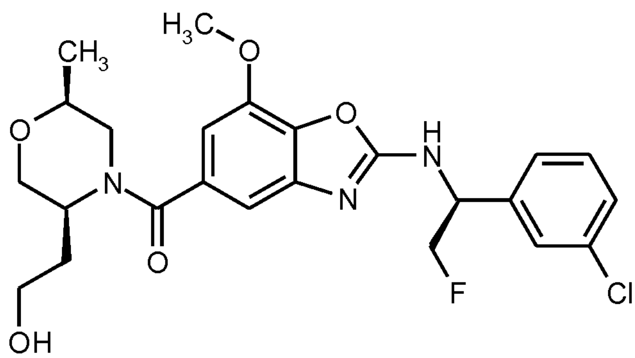

8. Verfahren zur Herstellung einer Verbindung der Formel (I) oder eines ihrer Salze, ihrer Solvate oder der Solvate ihrer Salze nach Anspruch 1, **dadurch gekennzeichnet, dass** eine Verbindung der Formel

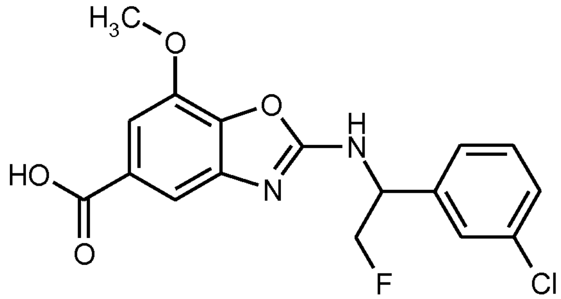

(II),

mit einer Verbindung der Formel

$R^1$-H (III),

in welcher

$R^1$ die in Anspruch 1 angegebene Bedeutung hat,
mit Dehydratisierungsreagenzien umgesetzt wird.

**9.** Verbindung nach einem der Ansprüche 1 bis 5 zur Behandlung und/oder Prophylaxe von Krankheiten.

**10.** Verbindung nach einem der Ansprüche 1 bis 5 zur Verwendung in einem Verfahrenzur Behandlung und/oder Prophylaxe von Krankheiten, unter Verwendung einer therapeutisch wirksamen Menge einer erfindungsgemäßen Verbindung.

**11.** Verbindung nach einem der Ansprüche 1 bis 5 zur Verwendung in einem Verfahrenzur Behandlung und/oder Prophylaxe von thromboembolischen Erkrankungen, unter Verwendung einer therapeutisch wirksamen Menge einer erfindungsgemäßen Verbindung.

**12.** Verbindung nach einem der Ansprüche 1 bis 5 zur Verwendung in einem Verfahrenzur Behandlung und/oder Prophylaxe von akutem Koronarsyndrom (ACS), venösen Thromboembolien, venösen Thrombosen, insbesondere in tiefen Beinvenen und Nierenvenen, Lungenembolien, Schlaganfall und/oder Thromboseprophylaxe im Rahmen von chirurgischen Eingriffen, insbesondere im Rahmen von chirurgischen Eingriffen bei Patienten, die eine Krebserkrankung haben, unter Verwendung einer therapeutisch wirksamen Menge einer erfindungsgemäßen Verbindung.

**13.** Arzneimittel enthaltend eine Verbindung nach einem der Ansprüche 1 bis 5 in Kombination mit einem inerten, nichttoxischen, pharmazeutisch geeigneten Hilfsstoff.

**14.** Arzneimittel nach Anspruch 13 zur Behandlung und/oder Prophylaxe von thromboembolischen Erkrankungen.

**15.** Kombination enthaltend

(A) (2-{[(1*S*)-1-(3-Chlorphenyl)-2-fluorethyl]amino}-7-methoxy-1,3-benzoxazol-5-yl)[(2*S*,5*S*)-5-(2-hydroxyethyl)-2-methylmorpholin-4-yl]methanon mit der folgenden Formel

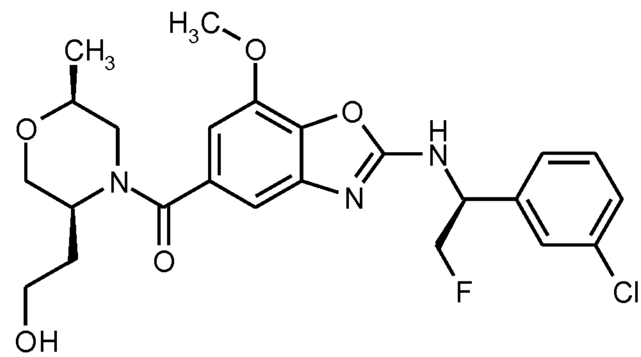

oder eines ihrer Salze, ihrer Solvate oder der Solvate ihrer Salze
und
(B) 5-Chlor-*N*-({(5*S*)-2-oxo-3-[4-(3-oxo-4-morpholinyl)-phenyl]-1,3-oxazolidin-5-yl}-methyl)-2-thiophen-carboxamid (Rivaroxaban) mit der Strukturformel

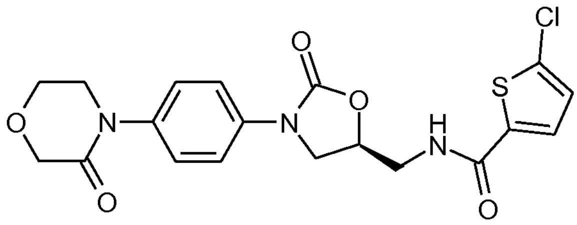

## Claims

**1.** Compound of the formula

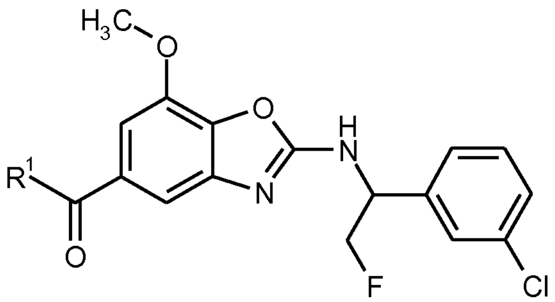

(I),

in which

$R^1$ represents a group of the formula

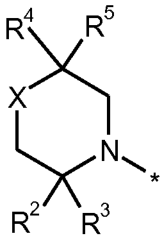
or
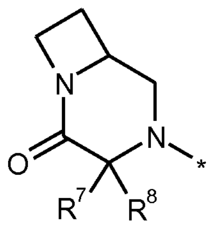
or
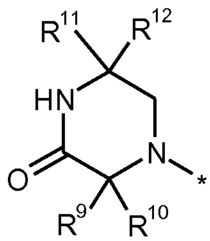
or
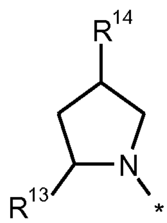

where * is the point of attachment to the carbonyl group,

X represents an oxygen atom, a sulphur atom or CH-$R^6$,
where
$R^6$ represents hydroxyl,
$R^2$ represents hydrogen, $C_1$-$C_6$-alkyl, $C_3$-$C_6$-cycloalkyl or phenyl,
where alkyl and cycloalkyl may be substituted by a substituent selected from the group consisting of hydroxy, methoxy, cyano, hydroxycarbonyl, aminocarbonyl, methylsulphonyl, difluoromethoxy and trifluoromethoxy, or where alkyl and cycloalkyl may be substituted by 1 to 3 fluorine substituents,
$R^3$ represents hydrogen or $C_1$-$C_4$-alkyl,
or $R^2$ and $R^3$ together with the carbon atom to which they are attached form a cyclopropyl ring, cyclobutyl ring or cyclopentyl ring,
where the cyclobutyl ring and the cyclopentyl ring may be substituted by a hydroxyl substituent,

$R^4$ represents hydrogen or $C_1$-$C_6$-alkyl,
where alkyl may be substituted by a hydroxyl substituent,
$R^5$ represents $C_1$-$C_4$-alkyl,
or $R^4$ and $R^5$ together with the carbon atom to which they are attached form a cyclopropyl ring, cyclobutyl ring or cyclopentyl ring,
where the cyclobutyl ring and the cyclopentyl ring may be substituted by a hydroxyl substituent,
$R^7$ represents hydrogen or $C_1$-$C_6$-alkyl,
where alkyl may be substituted by a hydroxyl or cyano substituent,
or
where alkyl may be substituted by 1 to 3 fluorine substituents,
$R^8$ represents hydrogen,
$R^9$ represents hydrogen or $C_1$-$C_6$-alkyl,
where alkyl may be substituted by a hydroxyl or cyano substituent,
or
where alkyl may be substituted by 1 to 3 fluorine substituents,
$R^{10}$ represents hydrogen,
$R^{11}$ represents $C_1$-$C_4$-alkyl,
where alkyl may be substituted by a hydroxyl substituent,
$R^{12}$ represents hydrogen or $C_1$-$C_4$-alkyl,
or $R^{11}$ and $R^{12}$ together with the carbon atom to which they are attached form a cyclopropyl ring, cyclobutyl ring or cyclopentyl ring,
where the cyclobutyl ring and the cyclopentyl ring may be substituted by a hydroxyl substituent,
$R^{13}$ represents hydroxymethyl or hydroxyethyl,
$R^{14}$ represents methoxy or ethoxy,

or one of the salts thereof, solvates thereof or solvates of the salts thereof.

**2.** Compound according to Claim 1, **characterized in that**

$R^1$ represents a group of the formula

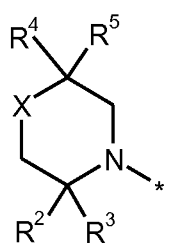 or 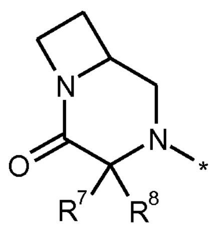 or 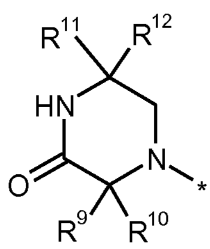 or

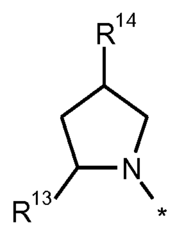

where * is the point of attachment to the carbonyl group,

X represents an oxygen atom or CH-$R^6$,
where
$R^6$ represents hydroxyl,
$R^2$ represents hydrogen, $C_1$-$C_4$-alkyl or $C_3$-$C_6$-cycloalkyl,
where alkyl may be substituted by a hydroxyl or hydroxycarbonyl substituent,
and

where cycloalkyl may be substituted by a hydroxyl substituent,
$R^3$ represents hydrogen or $C_1$-$C_4$-alkyl, or
$R^2$ and $R^3$ together with the carbon atom to which they are attached form a cyclobutyl ring,
where the cyclobutyl ring may be substituted by a hydroxyl substituent,
$R^4$ represents hydrogen or $C_1$-$C_4$-alkyl,
where alkyl may be substituted by a hydroxyl substituent,
$R^5$ represents $C_1$-$C_4$-alkyl,
$R^7$ represents hydrogen or $C_1$-$C_4$-alkyl,
$R^8$ represents hydrogen,
$R^9$ represents hydrogen or $C_1$-$C_4$-alkyl,
where alkyl may be substituted by a hydroxyl substituent,
$R^{10}$ represents hydrogen,
$R^{11}$ and $R^{12}$ together with the carbon atom to which they are attached form a cyclobutyl ring,
$R^{13}$ represents hydroxymethyl or hydroxyethyl,
$R^{14}$ represents methoxy or ethoxy,

or one of the salts thereof, solvates thereof or solvates of the salts thereof.

**3.** Compound according to either of Claims 1 and 2, **characterized in that**

$R^1$ represents a group of the formula

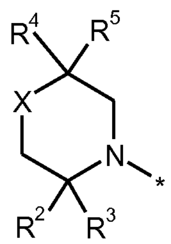

where * is the point of attachment to the carbonyl group,
X represents an oxygen atom,
$R^2$ represents methyl, ethyl or cyclobutyl,
where methyl and ethyl are substituted by a hydroxyl substituent,
and
where cyclobutyl is substituted by a hydroxyl substituent,
$R^3$ represents hydrogen,
$R^4$ represents hydrogen or methyl,
and $R^5$ represents methyl,
or $R^2$ represents methyl,
$R^3$ represents hydrogen or methyl,
$R^4$ represents methyl, ethyl or propyl, where methyl, ethyl and propyl are substituted by a hydroxyl substituent,
and $R^5$ represents methyl,
or $R^2$ and $R^3$ together with the carbon atom to which they are attached form a cyclobutyl ring,
where the cyclobutyl ring is substituted by a hydroxyl substituent,
$R^4$ represents hydrogen or methyl, and
$R^5$ represents methyl,

or one of the salts thereof, solvates thereof or solvates of the salts thereof.

**4.** Compound according to either of Claims 1 and 2, **characterized in that**

$R^1$ represents a group of the formula

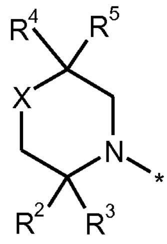

or

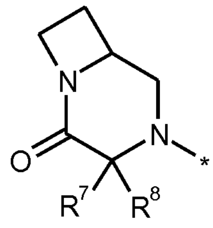

where * is the point of attachment to the carbonyl group,

X represents an oxygen atom,
$R^2$ represents methyl or ethyl, where methyl and ethyl are substituted by a hydroxyl substituent,
$R^3$ represents hydrogen,
or $R^2$ and $R^3$ together with the carbon atom to which they are attached form a cyclobutyl ring, where the cyclobutyl ring is substituted by a hydroxyl substituent,
$R^4$ represents hydrogen or methyl,
$R^5$ represents methyl,
$R^7$ represents hydrogen or methyl,
$R^8$ represents hydrogen,

or one of the salts thereof, solvates thereof or solvates of the salts thereof.

**5.** Compound according to any of Claims 1 to 4, **characterized in that**

$R^1$ represents a group of the formula

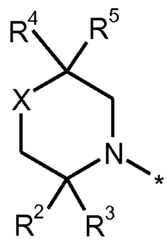

where * is the point of attachment to the carbonyl group,

X represents an oxygen atom,
$R^2$ represents methyl or ethyl, where methyl and ethyl are substituted by a hydroxyl substituent,
$R^3$ represents hydrogen,
or $R^2$ and $R^3$ together with the carbon atom to which they are attached form a cyclobutyl ring, where the cyclobutyl ring is substituted by a hydroxyl substituent,
$R^4$ represents hydrogen or methyl,
$R^5$ represents methyl,

or one of the salts thereof, solvates thereof or solvates of the salts thereof.

**6.** (2-{[(1*S*)-1-(3-Chlorophenyl)-2-fluoroethyl]amino}-7-methoxy-1,3-benzoxazol-5-yl)[(2*S*,5*S*)-5-(2-hydroxyethyl)-2-methylmorpholin-4-yl]methanone according to Claim 1 of the formula below

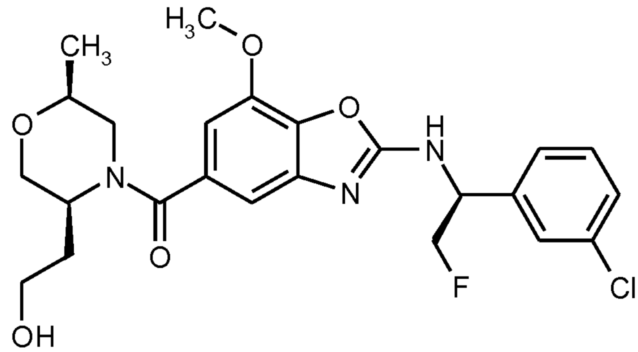

or one of the salts thereof, solvates thereof or solvates of the salts thereof.

**7.** (2-{[(1*S*)-1-(3-Chlorophenyl)-2-fluoroethyl]amino}-7-methoxy-1,3-benzoxazol-5-yl)[(2*S*,5*S*)-5-(2-hydroxyethyl)-2-methylmorpholin-4-yl]methanone according to Claim 1 of the formula below

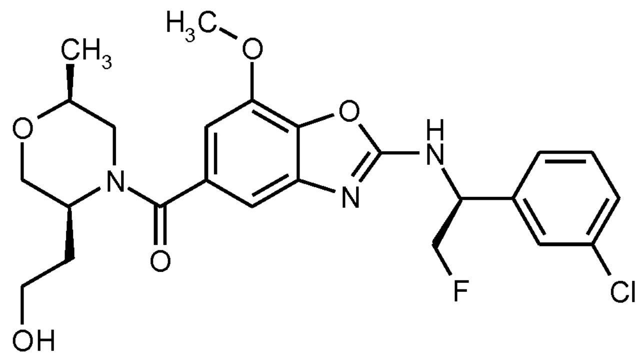

.

**8.** Process for preparing a compound of the formula (I) or one of the salts thereof, solvates thereof or solvates of the salts thereof according to Claim 1, **characterized in that** a compound of the formula

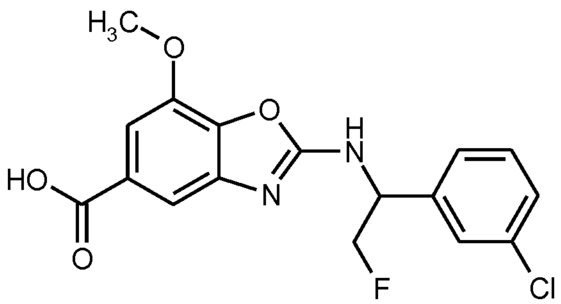

(II)

is reacted with a compound of the formula

$R^1$-H (III)

in which
$R^1$ has the meaning given in Claim 1
with dehydrating agents.

**9.** Compound according to any of Claims 1 to 5 for the treatment and/or prophylaxis of diseases.

**10.** Compound according to any of Claims 1 to 5 for use in a method for the treatment and/or prophylaxis of diseases, using a therapeutically effective amount of a compound according to the invention.

**11.** Compound according to any of Claims 1 to 5 for use in a method for the treatment and/or prophylaxis of thromboembolic disorders, using a therapeutically effective amount of a compound according to the invention.

**12.** Compound according to any of Claims 1 to 5 for use in a method for the treatment and/or prophylaxis of acute coronary syndrome (ACS), venous thromboembolisms, venous thromboses, in particular in deep leg veins and kidney veins, pulmonary embolisms, stroke and/or thrombosis prophylaxis in the context of surgical interventions, in particular in the context of surgical interventions in patients suffering from cancer, using a therapeutically effective amount of a compound according to the invention.

**13.** Medicament comprising a compound according to any of Claims 1 to 5 in combination with an inert, nontoxic, pharmaceutically suitable excipient.

**14.** Medicament according to Claim 13 for the treatment and/or prophylaxis of thromboembolic disorders.

**15.** Combination comprising

(A) (2-{[(1*S*)-1-(3-chlorophenyl)-2-fluoroethyl]amino}-7-methoxy-1,3-benzoxazol-5-yl)[(2*S*,5*S*)-5-(2-hydroxyethyl)-2-methylmorpholin-4-yl]methanone of the formula below

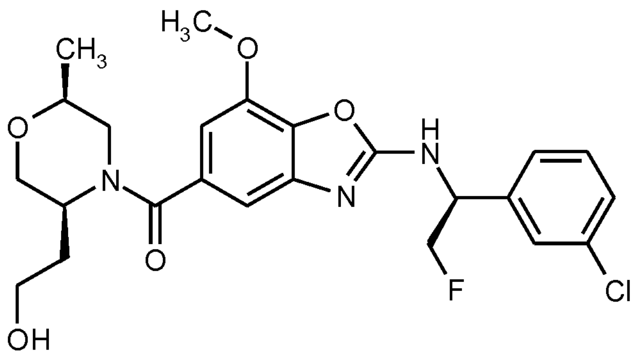

or one of the salts thereof, solvates thereof or solvates of the salts thereof
and
(B) 5-chloro-*N*-({(5*S*)-2-oxo-3-[4-(3-oxo-4-morpholinyl)phenyl]-1,3-oxazolidin-5-yl}methyl)-2-thiophenecarboxamide (rivaroxaban) having the structural formula

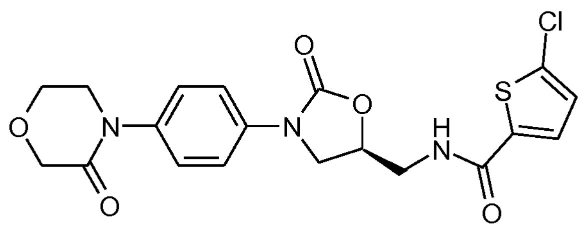

.

**Revendications**

**1.** Composé de formule

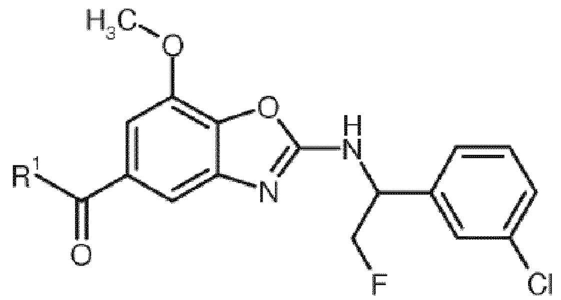

(I),

dans laquelle

$R^1$ représente un groupe de formule

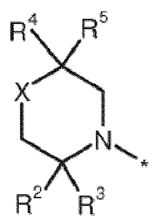

ou

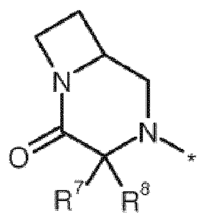

ou

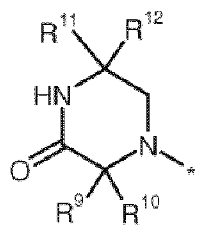

ou

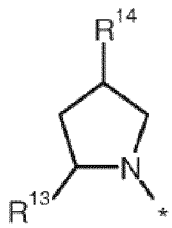

* étant l'emplacement de liaison au groupe carbonyle,

X représentant un atome d'oxygène, un atome de soufre ou CH-$R^6$, $R^6$ représentant hydroxy,

$R^2$ représentant hydrogène, alkyle en $C_1$-$C_6$, cycloalkyle en $C_3$-$C_6$ ou phényle,

l'alkyle et le cycloalkyle pouvant être substitués avec un substituant choisi dans le groupe constitué par hydroxy, méthoxy, cyano, hydroxycarbonyle, aminocarbonyle, méthylsulfonyle, difluorométhoxy et trifluorométhoxy,

ou

l'alkyle et le cycloalkyle pouvant être substitués avec 1 à 3 substituants fluor,

$R^3$ représentant hydrogène ou alkyle en $C_1$-$C_4$,

ou

$R^2$ et $R^3$ formant ensemble avec l'atome de carbone auquel ils sont reliés un cycle cyclopropyle, un cycle cyclobutyle ou un cycle cyclopentyle,

le cycle cyclobutyle et le cycle cyclopentyle pouvant être substitués avec un substituant hydroxy,

$R^4$ représentant hydrogène ou alkyle en $C_1$-$C_6$,

l'alkyle pouvant être substitué avec un substituant hydroxy,

$R^5$ représentant alkyle en $C_1$-$C_4$,

ou

$R^4$ et $R^5$ formant ensemble avec l'atome de carbone auquel ils sont reliés un cycle cyclopropyle, un cycle cyclobutyle ou un cycle cyclopentyle,

le cycle cyclobutyle et le cycle cyclopentyle pouvant être substitués avec un substituant hydroxy,

$R^7$ représentant hydrogène ou alkyle en $C_1$-$C_6$,

l'alkyle pouvant être substitué avec un substituant hydroxy ou cyano,

ou

l'alkyle pouvant être substitué avec 1 à 3 substituants fluor,

$R^8$ représentant hydrogène,
$R^9$ représentant hydrogène ou alkyle en $C_1$-$C_6$,

l'alkyle pouvant être substitué avec un substituant hydroxy ou cyano,
ou
l'alkyle pouvant être substitué avec 1 à 3 substituants fluor,

$R^{10}$ représentant hydrogène,
$R^{11}$ représentant alkyle en $C_1$-$C_4$,
l'alkyle pouvant être substitué avec un substituant hydroxy,
$R^{12}$ représentant hydrogène ou alkyle en $C_1$-$C_4$,
ou
$R^{11}$ et $R^{12}$ formant ensemble avec l'atome de carbone auquel ils sont reliés un cycle cyclopropyle, un cycle cyclobutyle ou un cycle cyclopentyle,
le cycle cyclobutyle et le cycle cyclopentyle pouvant être substitués avec un substituant hydroxy,
$R^{13}$ représentant hydroxyméthyle ou hydroxyéthyle,
$R^{14}$ représentant méthoxy ou éthoxy,
ou un de ses sels, ses solvates ou des solvates de ses sels.

**2.** Composé selon la revendication 1, **caractérisé en ce que**
$R^1$ représente un groupe de formule

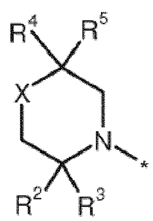 ou 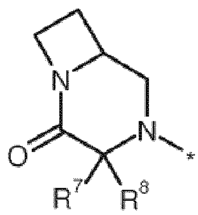 ou 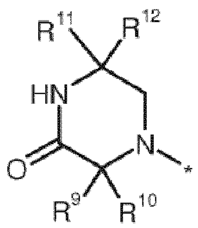 ou 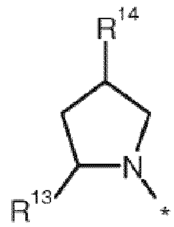

* étant l'emplacement de liaison au groupe carbonyle,

X représentant un atome d'oxygène ou CH-$R^6$, $R^6$ représentant hydroxy,
$R^2$ représentant hydrogène, alkyle en $C_1$-$C_4$ ou cycloalkyle en $C_3$-$C_6$,
l'alkyle pouvant être substitué avec un substituant hydroxy ou hydroxycarbonyle,
et
le cycloalkyle pouvant être substitué avec un substituant hydroxy,
$R^3$ représentant hydrogène ou alkyle en $C_1$-$C_4$,
ou
$R^2$ et $R^3$ formant ensemble avec l'atome de carbone auquel ils sont reliés un cycle cyclobutyle,
le cycle cyclobutyle pouvant être substitué avec un substituant hydroxy,
$R^4$ représentant hydrogène ou alkyle en $C_1$-$C_4$,
l'alkyle pouvant être substitué avec un substituant hydroxy,
$R^5$ représentant alkyle en $C_1$-$C_4$,
$R^7$ représentant hydrogène ou alkyle en $C_1$-$C_4$,
$R^8$ représentant hydrogène,
$R^9$ représentant hydrogène ou alkyle en $C_1$-$C_4$,
l'alkyle pouvant être substitué avec un substituant hydroxy,
$R^{10}$ représentant hydrogène,
$R^{11}$ et $R^{12}$ formant ensemble avec l'atome de carbone auquel ils sont reliés un cycle cyclopropyle,
$R^{13}$ représentant hydroxyméthyle ou hydroxyéthyle,
$R^{14}$ représentant méthoxy ou éthoxy,
ou un de ses sels, ses solvates ou des solvates de ses sels.

**3.** Composé selon l'une quelconque des revendications 1 ou 2, **caractérisé en ce que**
$R^1$ représente un groupe de formule

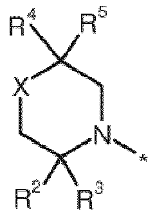

* étant l'emplacement de liaison au groupe carbonyle,
X représentant un atome d'oxygène,
$R^2$ représentant méthyle, éthyle ou cyclobutyle,
le méthyle et l'éthyle pouvant être substitués avec un substituant hydroxy,
et
le cyclobutyle pouvant être substitué avec un substituant hydroxy,
$R^3$ représentant hydrogène,
$R^4$ représentant hydrogène ou méthyle,
et
$R^5$ représentant méthyle,
ou
$R^2$ représentant méthyle,
$R^3$ représentant hydrogène ou méthyle,
$R^4$ représentant méthyle, éthyle ou propyle,
le méthyle, l'éthyle et le propyle étant substitués avec un substituant hydroxy,
et
$R^5$ représentant méthyle,
ou
$R^2$ et $R^3$ formant ensemble avec l'atome de carbone auquel ils sont reliés un cycle cyclobutyle,
le cycle cyclobutyle étant substitué avec un substituant hydroxy,
$R^4$ représentant hydrogène ou méthyle,
et
$R^5$ représentant méthyle,
ou un de ses sels, ses solvates ou des solvates de ses sels.

4. Composé selon l'une quelconque des revendications 1 ou 2, **caractérisé en ce que** $R^1$ représente un groupe de formule

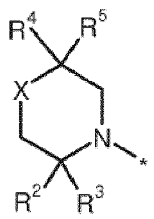

ou

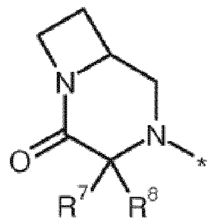

* étant l'emplacement de liaison au groupe carbonyle,
X représentant un atome d'oxygène,
$R^2$ représentant méthyle ou éthyle,
le méthyle et l'éthyle étant substitués avec un substituant hydroxy,
$R^3$ représentant hydrogène,
ou
$R^2$ et $R^3$ formant ensemble avec l'atome de carbone auquel ils sont reliés un cycle cyclobutyle,
le cycle cyclobutyle étant substitué avec un substituant hydroxy,
$R^4$ représentant hydrogène ou méthyle,
$R^5$ représentant méthyle,
$R^7$ représentant hydrogène ou méthyle,
$R^8$ représentant hydrogène,
ou un de ses sels, ses solvates ou des solvates de ses sels.

**5.** Composé selon l'une quelconque des revendications 1 à 4, **caractérisé en ce que** $R^1$ représente un groupe de formule

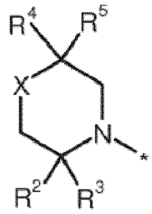

* étant l'emplacement de liaison au groupe carbonyle,
X représentant un atome d'oxygène,
$R^2$ représentant méthyle ou éthyle,
le méthyle et l'éthyle étant substitués avec un substituant hydroxy,
$R^3$ représentant hydrogène,
ou
$R^2$ et $R^3$ formant ensemble avec l'atome de carbone auquel ils sont reliés un cycle cyclobutyle,
le cycle cyclobutyle étant substitué avec un substituant hydroxy,
$R^4$ représentant hydrogène ou méthyle,
$R^5$ représentant méthyle,
ou un de ses sels, ses solvates ou des solvates de ses sels.

**6.** (2-{[(1*S*)-1-(3-chlorophényl)-2-fluoroéthyl]amino}-7-méthoxy-1,3-benzoxazol-5-yl)[(2*S*,5*S*)-5-(2-hydroxyéthyl)-2-méthyl-morpholin-4-yl]méthanone selon la revendication 1, ayant la formule suivante

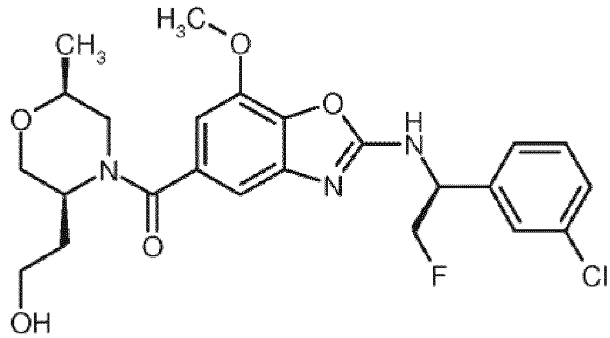

ou un de ses sels, de ses solvates ou des solvates de ses sels.

**7.** (2-{[(1S)-1-(3-chlorophényl)-2-fluoroéthyl]amino}-7-méthoxy-1,3-benzoxazol-5-yl)[(2*S*,5*S*)-5-(2-hydroxyéthyl)-2-méthyl-morpholin-4-yl]méthanone selon la revendication 1, ayant la formule suivante

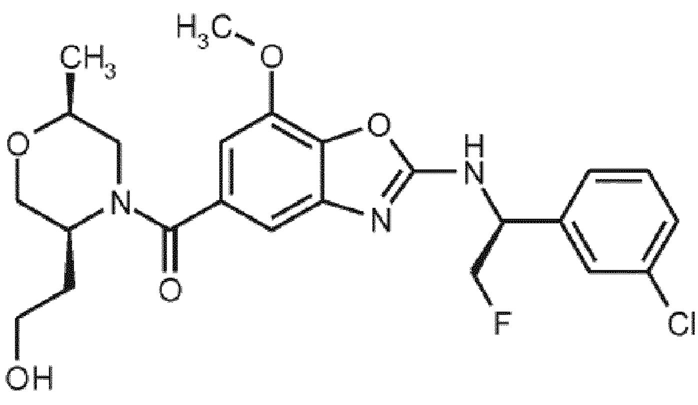

**8.** Procédé de fabrication d'un composé de formule (I) ou un de ses sels, de ses solvates ou des solvates de ses sels

selon la revendication 1, **caractérisé en ce qu'**un composé de formule

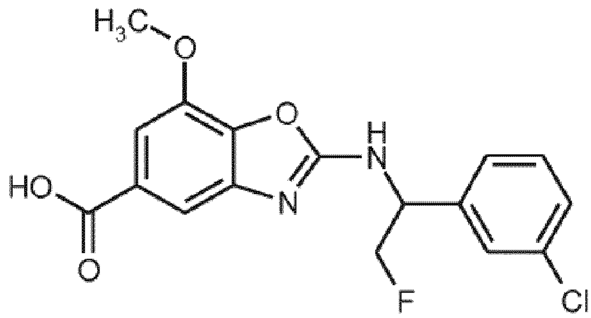

(II),

est mis en réaction avec un composé de formule

$R^1$-H (III)

dans laquelle
$R^1$ a la signification indiquée dans la revendication 1,
avec des réactifs de déshydratation.

**9.** Composé selon l'une quelconque des revendications 1 à 5, pour le traitement et/ou la prophylaxie de maladies.

**10.** Composé selon l'une quelconque des revendications 1 à 5, destiné à une utilisation dans un procédé pour le traitement et/ou la prophylaxie de maladies, en utilisant une quantité thérapeutiquement efficace d'un composé selon l'invention.

**11.** Composé selon l'une quelconque des revendications 1 à 5, destiné à une utilisation dans un procédé pour le traitement et/ou la prophylaxie de maladies thromboemboliques, en utilisant une quantité thérapeutiquement efficace d'un composé selon l'invention.

**12.** Composé selon l'une quelconque des revendications 1 à 5, destiné à une utilisation dans un procédé pour le traitement et/ou la prophylaxie du syndrome coronarien aigu (SCA), des thromboembolies veineuses, des thromboses veineuses, notamment dans les veines profondes des jambes et dans les veines rénales, des embolies pulmonaires, de l'accident vasculaire cérébral et/ou pour le prophylaxie de la thrombose dans le cadre d'interventions chirurgicales, notamment dans le cadre d'interventions chirurgicales chez des patients atteints d'une maladie cancéreuse, en utilisant une quantité thérapeutiquement efficace d'un composé selon l'invention.

**13.** Médicament contenant un composé selon l'une quelconque des revendications 1 à 5 en combinaison avec un adjuvant inerte, non toxique, pharmaceutiquement approprié.

**14.** Médicament selon la revendication 13, pour le traitement et/ou la prophylaxie de maladies thromboemboliques.

**15.** Combinaison contenant

(A) de la (2-{[(1*S*)-1-(3-chlorophényl)-2-fluoroéthyl]aminol-7-méthoxy-1,3-benzoxazol-5-yl)[(2S,5S)-5-(2-hydroxyéthyl)-2-méthyl-morpholin-4-yl]méthanone ayant la formule suivante

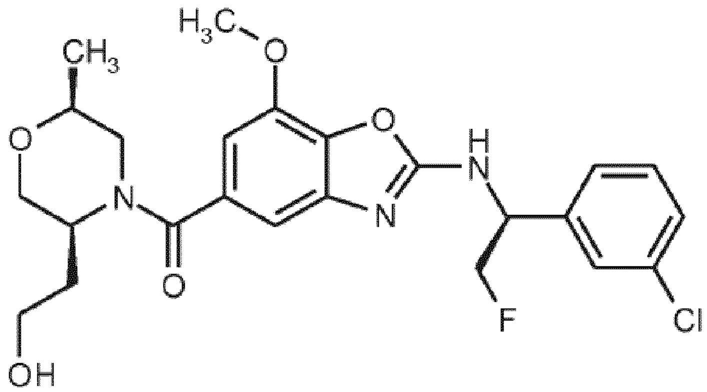

ou un de ses sels, de ses solvates ou des solvates de ses sels,
et
(B) du 5-chloro-N-({(5S)-2-oxo-3-[4-(3-oxo-4-morpholinyl)-phényl]-1,3-oxazolidin-5-yl}-méthyl)-2-thiophène-carboxamide (Rivaroxaban) de formule structurale

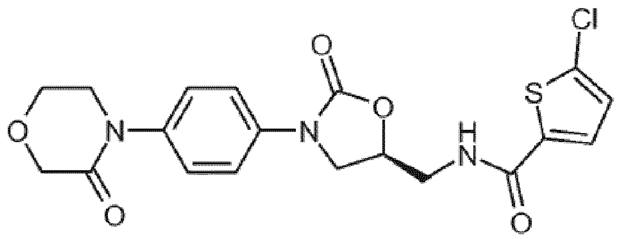

.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9837075 A
- WO 2007140982 A
- EP 0535521 A
- WO 0147919 A
- WO 2004094380 A1, M.-J. Blanco-Pilado
- US 2006292073 A1


### In der Beschreibung aufgeführte Nicht-Patentliteratur


- **EUGENE BRAUNWALD.** Heart Disease: A Textbook of Cardiovascular Medicine. W.B. Saunders Company, 1997
- Pschyrembel, Klinisches Wörterbuch. Walter de Gruyter Verlag, 1994, 610
- Römpp Lexikon Chemie. Georg Thieme Verlag, 1998
- **J. HIRSH ; J. DALEN ; D.R. ANDERSON et al.** Oral anticoagulants: Mechanism of action, clinical effectiveness, and optimal therapeutic range. *Chest,* 2001, vol. 119, 8S-21S
- **J. ANSELL ; J. HIRSH ; J. DALEN et al.** Managing oral anticoagulant therapy. *Chest,* 2001, vol. 119, 22S-38S
- **P.S. WELLS ; A.M. HOLBROOK ; N.R. CROWTHER et al.** Interactions of warfarin with drugs and food. *Ann. Intern. Med.,* 1994, vol. 121, 676-683
- **A. CASIMIRO-GARCIA ; D. A. DUDLEY ; R. J. HEEMSTRA ; K. J. FILIPSKI ; C. F. BIGGE ; J. J. EDMUNDS.** *Expert Opin. Ther. Patents,* 2006, vol. 16 (2), 119-145
- **R. LOK et al.** *J. Org. Chem.,* 1996, vol. 61, 3289-3297
- **X. ZOU et al.** *J. Fluorane Chem.,* 2010, vol. 131, 340-344
- **B. H. HOFF et al.** *Tetrahedron,* 2009, vol. 65, 9550-9556
- **W. LACÔTE et al.** *Org. Lett.,* 2011, vol. 13, 5990-5993
- **T. J. TEWSON et al.** *Synthesis,* 2002, vol. 6, 766-770
- **B. L. FERINGA ; B. DE LANGE.** *Heterocycles,* 1988, vol. 27, 1197-1205
- **F. HORIUCHI ; M. MATSUI.** *Agr. Biol.Chem.,* 1973, vol. 37, 1713-1716
- **K. OGURA ; G. TSUCHIHASHI et al.** *Bull. Chem. Soc. Jpn.,* 1984, vol. 57, 1637-1642
- **T. M. SHOUP ; M. M. GOODMAN.** *J. Labelled. Cpd. Radiopharm.,* 1999, vol. 42, 215-225
- **B. L. FERINGA.** *Tetrahedron,* 1989, vol. 45, 6799-6818
- **N. BERRY et al.** *Br. J. Pharmacol.,* 1994, vol. 113, 1209-1214
- **ARTURSSON, P. ; KARLSSON, J.** Correlation between oral drug absorption in humans and apparent drug permeability coefficients in human intestinal epithelial (Caco-2) cells. *Biochem. Biophys.,* 1991, vol. 175 (3), 880-885
- **KERN A ; BADER A ; PICHLMAYR R ; SEWING KF.** *Biochem Pharmacol.,* 1997, vol. 54, 761-772